# EUROPEAN PATENT SPECIFICATION

(11) **EP 2 044 109 B1**
(45) Date of publication and mention of the grant of the patent: **21.05.2014**
(21) Application number: 07756833.5
(22) Date of filing: 09.02.2007
(51) Int. Cl.: C07K 14/43

(54) **IDENTIFICATION AND USE OF TARGET GENES FOR CONTROL OF PLANT PARASITIC NEMATODES**
IDENTIFIZIERUNG UND VERWENDUNG VON ZIELGENEN ZUR STEUERUNG PFLANZENPARASITÄRER NEMATODEN
IDENTIFICATION ET UTILISATION DE GENES CIBLES EN VUE DE LUTTER CONTRE DES NEMATODES PARASITES DE PLANTES

(30) Priority: 10.02.2006 US 772265 P
(43) Date of publication of application: 08.04.2009
(73) Proprietor: Monsanto Technology, LLC, St. Louis, MO 63167 (US)
(72) Inventor: BOUKHAROV, Andrey A., St Louis, MO 63167 (US); DU, Zijin, St. Louis, MO 63167 (US); GUO, Liang, St. Louis, MO 63167 (US); HRESKO, Michelle C., St. Louis, MO 63167 (US); KOVALIC, David K., St Louis, MO 63167 (US); LI, Zhaolong, St. Louis, MO 63167 (US); LU, Maolong, St. Louis, MO 63167 (US); MCCARTER, James P., St. Louis, MO 63167 (US); MILLER, Nancy M., St. Louis, MO 63167 (US); VAUDIN, Mark, St. Louis, MO 63167 (US); WILLIAMS, Deryck J., St. Louis, MO 63167 (US); WU, Wei, St. Louis, MO 63167 (US)
(74) Representative: Von Kreisler Selting Werner - Partnerschaft von Patentanwälten und Rechtsanwälten mbB
(86) International application number: PCT/US2007/061932
(87) International publication number: WO 2007/095469

(56) References cited:
- WO-A-01/37654
- US-A1- 2004 098 761
- US-A1- 2005 188 438
- LEE MYON HEE ET AL: "Regulation of gene expression, cellular localization, and in vivo function of Caenorhabditis elegans DNA topoisomerase I" GENES TO CELLS, vol. 6, no. 4, April 2001 (2001-04), pages 303-312, XP002517459 ISSN: 1356-9597
- DATABASE EMBL Heterodera glycines cDNA 19 March 2003 (2003-03-19), XP002517460 retrieved from EBI Database accession no. CB374546
- URWIN P E ET AL: "INGESTION OF DOUBLE-STRANDED RNA BY PREPARASITIC JUVENILE CYST NEMATODES LEADS TO RNA INTERFERENCE" MOLECULAR PLANT-MICROBE INTERACTIONS, APS PRESS, ST. PAUL, MN, US, vol. 15, no. 8, August 2002 (2002-08), pages 747-752, XP001146898 ISSN: 0894-0282 cited in the application

### SEQUENCE LISTING

<110> MONSANTO TECHNOLOGY, LLC
<120> IDENTIFICATION AND USE OF TARGET GENES FOR CONTROL OF PLANT PARASITIC NEMATODES
<130> MNDI:002WO
<140> EP 07756833.5
   <141> 2007-02-09
<150> US 60/772,265
   <151> 2006-02-10
<160> 1934
<170> PatentIn version 3.3
<210> 1
<400> 1
   000
<210> 2
<400> 2
   000
<210> 3
<400> 3
   000
<210> 4
<400> 4
   000
<210> 5
<400> 5
   000
<210> 6
<400> 6
   000
<210> 7
<400> 7
   000
<210> 8
<400> 8
   000
<210> 9
<400> 9
   000
<210> 10
<400> 10
   000
<210> 11
<400> 11
   000
<210> 12
<400> 12
   000
<210> 13
<400> 13
   000
<210> 14
<400> 14
   000
<210> 15
<400> 15
   000
<210> 16
<400> 16
   000
<210> 17
<400> 17
   000
<210> 18
<400> 18
   000
<210> 19
<400> 19
   000
<210> 20
<400> 20
   000
<210> 21
<400> 21
   000
<210> 22
<400> 22
   000
<210> 23
<400> 23
   000
<210> 24
<400> 24
   000
<210> 25
<400> 25
   000
<210> 26
<400> 26
   000
<210> 27
<400> 27
   000
<210> 28
<400> 28
   000
<210> 29
<400> 29
   000
<210> 30
<400> 30
   000
<210> 31
<400> 31
   000
<210> 32
<400> 32
   000
<210> 33
<400> 33
   000
<210> 34
<400> 34
   000
<210> 35
<400> 35
   000
<210> 36
<400> 36
   000
<210> 37
<400> 37
   000
<210> 38
<400> 38
   000
210> 39
<400> 39
   000
<210> 40
<400> 40
   000
<210> 41
<400> 41
   000
<210> 42
<400> 42
   000
<210> 43
<400> 43
   000
<210> 44
<400> 44
   000
<210> 45
<400> 45
   000
<210> 46
<400> 46
   000
<210> 47
<400> 47
   000
<210> 48
<400> 48
   000
<210> 49
<400> 49
   000
<210> 50
<400> 50
   000
<210> 51
<400> 51
   000
<210> 52
<400> 52
   000
<210> 53
<400> 53
   000
<210> 54
<400> 54
   000
<210> 55
<400> 55
   000
<210> 56
<400> 56
   000
<210> 57
<400> 57
   000
<210> 58
<400> 58
   000
<210> 59
<400> 59
   000
<210> 60
<400> 60
   000
<210> 61
<400> 61
   000
<210> 62
<400> 62
   000
<210> 63
<400> 63
   000
<210> 64
<400> 64
   000
<210> 65
<400> 65
   000
<210> 66
<400> 66
   000
<210> 67
<400> 67
   000
<210> 68
<400> 68
   000
<210> 69
<400> 69
   000
<210> 70
<400> 70
   000
<210> 71
<400> 71
   000
<210> 72
<400> 72
   000
<210> 73
<400> 73
   000
<210> 74
<400> 74
   000
<210> 75
<400> 75
   000
<210> 76
<400> 76
   000
<210> 77
<400> 77
   000
<210> 78
<400> 78
   000
<210> 79
<400> 79
   000
<210> 80
<400> 80
   000
<210> 81
<400> 81
   000
<210> 82
<400> 82
   000
<210> 83
<400> 83
   000
<210> 84
<400> 84
   000
<210> 85
<400> 85
   000
<210> 86
<400> 86
   000
<210> 87
<400> 87
   000
<210> 88
<400> 88
   000
<210> 89
<400> 89
   000
<210> 90
<400> 90
   000
<210> 91
<400> 91
   000
<210> 92
<400> 92
   000
<210> 93
<400> 93
   000
<210> 94
<400> 94
   000
<210> 95
<400> 95
   000
<210> 96
<400> 96
   000
<210> 97
<400> 97
   000
<210> 98
<400> 98
   000
<210> 99
<400> 99
   000
<210> 100
<400> 100
   000
<210> 101
<400> 101
   000
<210> 102
<400> 102
   000
<210> 103
<400> 103
   000
<210> 104
<400> 104
   000
<210> 105
<400> 105
   000
<210> 106
<400> 106
   000
<210> 107
<400> 107
   000
<210> 108
<400> 108
   000
<210> 109
<400> 109
   000
<210> 110
<400> 110
   000
<210> 111
<400> 111
   000
<210> 112
<400> 112
   000
<210> 113
<400> 113
   000
<210> 114
<400> 114
   000
<210> 115
<400> 115
   000
<210> 116
<400> 116
   000
<210> 117
<400> 117
   000
<210> 118
<400> 118
   000
<210> 119
<400> 119
   000
<210> 120
<400> 120
   000
<210> 121
<400> 121
   000
<210> 122
<400> 122
   000
<210> 123
<400> 123
   000
<210> 124
<400> 124
   000
<210> 125
<400> 125
   000
<210> 126
<400> 126
   000
<210> 127
<400> 127
   000
<210> 128
<400> 128
   000
<210> 129
<400> 129
   000
<210> 130
<400> 130
   000
<210> 131
<400> 131
   000
<210> 132
<400> 132
   000
<210> 133
<400> 133
   000
<210> 134
<400> 134
   000
<210> 135
<400> 135
   000
<210> 136
<400> 136
   000
<210> 137
<400> 137
   000
<210> 138
<400> 138
   000
<210> 139
<400> 139
   000
<210> 140
<400> 140
   000
<210> 141
<400> 141
   000
<210> 142
<400> 142
   000
<210> 143
<400> 143
   000
<210> 144
<400> 144
   000
<210> 145
<400> 145
   000
<210> 146
<400> 146
   000
<210> 147
<400> 147
   000
<210> 148
<400> 148
   000
<210> 149
<400> 149
   000
<210> 150
<400> 150
   000
<210> 151
<400> 151
   000
<210> 152
<400> 152
   000
<210> 153
<400> 153
   000
<210> 154
<400> 154
   000
<210> 155
<400> 155
   000
<210> 156
<400> 156
   000
<210> 157
<400> 157
   000
<210> 158
<400> 158
   000
<210> 159
<400> 159
   000
<210> 160
<400> 160
   000
<210> 161
<400> 161
   000
<210> 162
<400> 162
   000
<210> 163
<400> 163
   000
<210> 164
<400> 164
   000
<210> 165
<400> 165
   000
<210> 166
<400> 166
   000
<210> 167
<400> 167
   000
<210> 168
<400> 168
   000
<210> 169
<400> 169
   000
<210> 170
<400> 170
   000
<210> 171
<400> 171
   000
<210> 172
<400> 172
   000
<210> 173
<400> 173
   000
<210> 174
<400> 174
   000
<210> 175
<400> 175
   000
<210> 176
<400> 176
   000
<210> 177
<400> 177
   000
<210> 178
<400> 178
   000
<210> 179
<400> 179
   000
<210> 180
<400> 180
   000
<210> 181
<400> 181
   000
<210> 182
<400> 182
   000
<210> 183
<400> 183
   000
<210> 184

<400> 184
   000
<210> 185
<400> 185
   000
<210> 186
<400> 186
   000
<210> 187
<400> 187
   000
<210> 188
<400> 188
   000
<210> 189
<400> 189
   000
<210> 190
<400> 190
   000
<210> 191
<400> 191
   000
<210> 192
<400> 192
   000
<210> 193
<400> 193
   000
<210> 194
<400> 194
   000
<210> 195
<400> 195
   000
<210> 196
<400> 196
   000
<210> 197
<400> 197
   000
<210> 198
<400> 198
   000
<210> 199
<400> 199
   000
<210> 200
<400> 200
   000
<210> 201
<400> 201
   000
<210> 202
<400> 202
   000
<210> 203
<400> 203
   000
<210> 204
<400> 204
   000
<210> 205
<400> 205
   000
<210> 206
<400> 206
   000
<210> 207
<400> 207
   000
<210> 208
<400> 208
   000
<210> 209
<400> 209
   000
<210> 210
<400> 210
   000
<210> 211
<400> 211
   000
<210> 212
<400> 212
   000
<210> 213
<400> 213
   000
<210> 214
<400> 214
   000
<210> 215
<400> 215
   000
<210> 216
<400> 216
   000
<210> 217
<400> 217
   000
<210> 218
<400> 218
   000
<210> 219
<400> 219
   000
<210> 220
<400> 220
   000
<210> 221
<400> 221
   000
<210> 222
<400> 222
   000
<210> 223
<400> 223
   000
<210> 224
<400> 224
   000
<210> 225
<400> 225
   000
<210> 226
<400> 226
   000
<210> 227
<400> 227
   000
<210> 228
<400> 228
   000
<210> 229
<400> 229
   000
<210> 230
<400> 230
   000
<210> 231
<400> 231
   000
<210> 232
<400> 232
   000
<210> 233
<400> 233
   000
<210> 234
<400> 234
   000
<210> 235
<400> 235
   000
<210> 236
<400> 236
   000
<210> 237
<400> 237
   000
<210> 238
<400> 238
   000
<210> 239
<400> 239
   000
<210> 240
<400> 240
   000
<210> 241
<400> 241
   000
<210> 242
<400> 242
   000
<210> 243
<400> 243
   000
<210> 244
<400> 244
   000
<210> 245
<400> 245
   000
<210> 246
<400> 246
   000
<210> 247
<400> 247
   000
<210> 248
<400> 248
   000
<210> 249
<400> 249
   000
<210> 250
<400> 250
   000
<210> 251
<400> 251
   000
<210> 252
<400> 252
   000
<210> 253
<400> 253
   000
<210> 254
<400> 254
   000
<210> 255
<400> 255
   000
<210> 256
<400> 256
   000
<210> 257
<400> 257
   000
<210> 258
<400> 258
   000
<210> 259
<400> 259
   000
<210> 260
<400> 260
   000
<210> 261
<400> 261
   000
<210> 262
<400> 262
   000
<210> 263
<400> 263
   000
<210> 264
<400> 264
   000
<210> 265
<400> 265
   000
<210> 266
<400> 266
   000
<210> 267
<400> 267
   000
<210> 268
<400> 268
   000
<210> 269
<400> 269
   000
<210> 270
<400> 270
   000
<210> 271
<400> 271
   000
<210> 272
<400> 272
   000
<210> 273
<400> 273
   000
<210> 274
<400> 274
   000
<210> 275
<400> 275
   000
<210> 276
<400> 276
   000
<210> 277
<100> 277
   000
<210> 278
<400> 278
   000
<210> 279
<400> 279
   000
<210> 280
<400> 280
   000
<210> 281
<400> 281
   000
<210> 282
<400> 282
   000
<210> 283
<400> 283
   000
<210> 284
<400> 284
   000
<210> 285
<400> 285
   000
<210> 286
<400> 286
   000
<210> 287
<400> 287
   000
<210> 288
<400> 288
   000
<210> 289
<400> 289
   000
<210> 290
<400> 290
   000
<210> 291
<400> 291
   000
<210> 292
<400> 292
   000
<210> 293
<400> 293
   000
<210> 294
<400> 294
   000
<210> 295
<400> 295
   000
<210> 296
<400> 296
   000
<210> 297
<400> 297
   000
<210> 298
<400> 298
   000
<210> 299
<400> 299
   000
<210> 300
<400> 300
   000
<210> 301
<400> 301
   000
<210> 302
<400> 302
   000
<210> 303
<400> 303
   000
<210> 304
<400> 304
   000
<210> 305
<400> 305
   000
<210> 306
<400> 306
   000
<210> 307
<400> 307
   000
<210> 308
<400> 308
   000
<210> 309
<400> 309
   000
<210> 310
<400> 310
   000
<210> 311
<400> 311
   000
<210> 312
<400> 312
   000
<210> 313
<400> 313
   000
<210> 314
<400> 314
   000
<210> 315
<400> 315
   000
<210> 316
<400> 316
   000
<210> 317
<400> 317
   000
<210> 318
<400> 318
   000
<210> 319
<400> 319
   000
<210> 320
<400> 320
   000
<210> 321
<400> 321
   000
<210> 322
<400> 322
   000
<210> 323
<400> 323
   000
<210> 324
<400> 324
   000
<210> 325
<400> 325
   000
<210> 326
<400> 326
   000
<210> 327
<400> 327
   000
<210> 328
<400> 328
   000
<210> 329
<400> 329
   000
<210> 330
<400> 330
   000
<210> 331
<400> 331
   000
<210> 332
<400> 332
   000
<210> 333
<400> 333
   000
<210> 334
<400> 334
   000
<210> 335
<400> 335
   000
<210> 336
<400> 336
   000
<210> 337
<400> 337
   000
<210> 338
<400> 338
   000
<210> 339
<400> 339
   000
<210> 340
<400> 340
   000
<210> 341
<400> 341
   000
<210> 342
<400> 342
   000
<210> 343
<400> 343
   000
<210> 344
<400> 344
   000
<210> 345
<400> 345
   000
<210> 346
<400> 346
   000
<210> 347
<400> 347
   000
<210> 348
<400> 348
   000
<210> 349
<400> 349
   000
<210> 350
<400> 350
   000
<210> 351
<400> 351
   000
<210> 352
<400> 352
   000
<210> 353
<400> 353
   000
<210> 354
<400> 354
   000
<210> 355
<400> 355
   000
<210> 356
<400> 356
   000
<210> 357
<400> 357
   000
<210> 358
<400> 358
   000
<210> 359
<400> 359
   000
<210> 360
<400> 360
   000
<210> 361
<400> 361
   000
<210> 362
<400> 362
   000
<210> 363
<400> 363
   000
<210> 364
<400> 364
   000
<210> 365
<400> 365
   000
<210> 366
<400> 366
   000
<210> 367
<400> 367
   000
<210> 368
<400> 368
   000
<210> 369
<400> 369
   000
<210> 370

<400> 370
   000
<210> 371
<400> 371
   000
<210> 372
<400> 372
   000
<210> 373
<400> 373
   000
<210> 374
<400> 374
   000
<210> 375
<400> 375
   000
<210> 376
<400> 376
   000
<210> 377
<400> 377
   000
<210> 378
<400> 378
   000
<210> 379
<400> 379
   000
<210> 380
<400> 380
   000
<210> 381
<400> 381
   000
<210> 382
<400> 382
   000
<210> 383
<400> 383
   000
<210> 384
<400> 384
   000
<210> 385
<400> 385
   000
<210> 386
<400> 386
   000
<210> 387
<400> 387
   000
<210> 388
<400> 388
   000
<210> 389
<400> 389
   000
<210> 390
<400> 390
   000
<210> 391
<400> 391
   000
<210> 392
<400> 392
   000
<210> 393
<400> 393
   000
<210> 394
<400> 394
   000
<210> 395
<400> 395
   000
<210> 396
<400> 396
   000
<210> 397
<400> 397
   000
<210> 398
<400> 398
   000
<210> 399
<400> 399
   000
<210> 400
<400> 400
   000
<210> 401
<400> 401
   000
<210> 402
<400> 402
   000
<210> 403
<400> 403
   000
<210> 404
<400> 404
   000
<210> 405
<400> 405
   000
<210> 406
<400> 406
   000
<210> 407
<400> 407
   000
<210> 408
<400> 408
   000
<210> 409
<400> 409
   000
<210> 410
<400> 410
   000
<210> 411
<400> 411
   000
<210> 412
<400> 412
   000
<210> 413
<400> 413
   000
<210> 414
<400> 414
   000
<210> 415
<400> 415
   000
<210> 416
<400> 416
   000
<210> 417
<400> 417
   000
<210> 418
<400> 418
   000
<210> 419
<400> 419
   000
<210> 420
<400> 420
   000
<210> 421
<400> 421
   000
<210> 422
<400> 422
   000
<210> 423
<400> 423
   000
<210> 424
<400> 424
   000
<210> 425
<400> 425
   000
<210> 426
<400> 426
   000
<210> 427
<400> 427
   000
<210> 428
<400> 428
   000
<210> 429
<400> 429
   000
<210> 430
<400> 430
   000
<210> 431
<400> 431
   000
<210> 432
<400> 432
   000
<210> 433
<400> 433
   000
<210> 434
<400> 434
   000
<210> 435
<400> 435
   000
<210> 436
<400> 436
   000
<210> 437
<400> 437
   000
<210> 438
<400> 438
   000
<210> 439
<400> 439
   000
<210> 440
<400> 440
   000
<210> 441
<400> 441
   000
<210> 442
<400> 442
   000
<210> 443
<400> 443
   000
<210> 444
<400> 444
   000
<210> 445
<400> 445
   000
<210> 446
<400> 446
   000
<210> 447
<400> 447
   000
<210> 448
<400> 448
   000
<210> 449
<400> 449
   000
<210> 450
<400> 450
   000
<210> 451
<400> 451
   000
<210> 452
<400> 452
   000
<210> 453
<400> 453
   000
<210> 454
<400> 454
   000
<210> 455
<400> 455
   000
<210> 456
<400> 456
   000
<210> 457
<400> 457
   000
<210> 458
<400> 458
   000
<210> 459
<400> 459
   000
<210> 460
<400> 460
   000
<210> 461
<400> 461
   000
<210> 462
<400> 462
   000
<210> 463
<400> 463
   000
<210> 464
<400> 464
   000
<210> 465
<400> 465
   000
<210> 466
<400> 466
   000
<210> 467
<400> 467
   000
<210> 468
<400> 468
   000
<210> 469
<400> 469
   000
<210> 470
<400> 470
   000
<210> 471
<400> 471
   000
<210> 472
<400> 472
   000
<210> 473
<400> 473
   000
<210> 474
<400> 474
   000
<210> 475
<400> 475
   000
<210> 476
<400> 476
   000
<210> 477
<400> 477
   000
<210> 478
<400> 478
   000
<210> 479
<400> 479
   000
<210> 480
<400> 480
   000
<210> 481
<400> 481
   000
<210> 482
<400> 482
   000
<210> 483
<400> 483
   000
<210> 484
<400> 484
   000
<210> 485
<400> 485
   000
<210> 486
<400> 486
   000
<210> 487
<400> 487
   000
<210> 488
<400> 488
   000
<210> 489
<400> 489
   000
<210> 490
<400> 490
   000
<210> 491
<400> 491
   000
<210> 492
<400> 492
   000
<210> 493
<400> 493
   000
<210> 494
<400> 494
   000
<210> 495
<400> 495
   000
<210> 496
<400> 496
   000
<210> 497
<400> 497
   000
<210> 498
<400> 498
   000
<210> 499
<400> 499
   000
<210> 500
<400> 500
   000
<210> 501
<400> 501
   000
<210> 502
<400> 502
   000
<210> 503
<400> 503
   000
<210> 504
<400> 504
   000
<210> 505
   <211> 4554
   <212> DNA
   <213> Heterodera glycines
<220>
   <223> genomic virtual cDNA Sequence=SeqID_544; coordinates=414..516 ,571..770,826..999,1053..1206,1261..1487,1533..1646,1694..1879 ,1940..2062,2107..2220,2273..2438,2500..2708,2757..2861,2912..3058 ,3115..3189,3234..3438,...
<220>
   <223> genomic virtual cDNA coordinates=3490..3677,3725..3931,3990..4076 ,4126..4226,4274..4409
<220>
   <223> coding sequence=SeqID_786; coordinates=414..516,571..770,826..999 ,1053..1206,1261..1487,1533..1646,1694..1879,1940..2062,2107..2220 ,2273..2438,2500..2708,2757..2861,2912..3058,3115..3189,3234..3438 ,3490..3677,3725..3931,...
<220>
   <223> coding coordinates=3990..4076,4126..4226,4274..4409
<220>
   <223> peptide sequence=SeqID_1031
<400> 505
   ttcgaccaat tcgagattcc gaaaccaaat tcgaacaaaa agtcgaaaat tcaaaatttt 60
   cgaatttcgg gtttttggcc gaacaaggtc taaatacatt aaaatgaact ttaatgtcat 120
   cagtgcttca ataaaagatt gtttaaacca tttttagcct aaaaatcaaa attcgaaatt 180
   tttgaatttt cgactttttg ttcgaatttg gtttcggaat ctcgaattgg tcgaaatgga 240
   tgtcgtattc gtgatcagcg accccgaaaa cccaataata cttgttttca ggagttttta 300
   aaccaaaaaa aaaattgaaa atttctggcc aaaaaagggg acccaaaccc aatgagtacc 360
   gccgcgacaa atgcctttga tataattgtt tattgtggtt tttcctgttt agttatgcgc 420
   ttggacattc cgccatggaa cacttgcgca aggcgtcggt tctcgtcagt ggcctcggct 480
   ccgtgggcgt ggaggtggcg aagaatctga tccttggtgc cgccattctt tgattttact 540
   ttgcatgact aaacaatcgc cgcttttcag gcggtgttcg ctcggtgact cttcacgaca 600
   caaaagcgtt ggcgttcaga gatttgtcgg cgcatttcta cgccaaggaa agcgacgtgg 660
   gaacgaaccg tgccaaagtt tgcttcgaca aattggcgga gctcaacgac actgtgaact 720
   gtgcattgtc tgtggaaaat ctgtcggagg atttcgtcaa acaattcgac gtcagcaaac 780
   gcttccttcc ttttcccgtc tcgcctaatt ccgtcgttct ttcagttggt cgttctcacc 840
   gacgcgtcct tttccactca gacggaagtg aacggttgga tgcgcaacca tcgcaaaatg 900
   ttcatctcgg cagacgcgcg cggacttttc tcttatgcat ttgtggactt gggcgacgaa 960
   ttcgttgtga acgattccga cggggagacg tgcaaagagg tggaaattgg aataattaat 1020
   ttaattgcga attgaatggt tccgattttc aggtgctcgt tgagtacatc gactgcgaaa 1080
   gcggcgacat gacgacactc gaaggcgcat ttcaccagtt ggaggacggg gaccacgttg 1140
   tgttcaccga ctttgagcac gccgaactga acgaacttcc accagtgcca gtcactttga 1200
   agggaagtga gtgccaccac tgatcatttg ttggttcatc tacagtcctc ccatttttag 1260
   gcaaagtctt caacattgga aatgctctcg cggccttttc tggcgcagtt caaggccccg 1320
   tttcgcgtgg acgtgttcag caagtcaaag tgcccaagaa aatgtccttc aagccattgt 1380
   ctcgcgcact cgccgagccc gactttatga tttgggactt tgccaagttc gacgaacctg 1440
   cacaacttca cgccctttgg caggctttgt acagttttga ggagatggtt cgtcatcttg 1500
   cgactttcca tttggttggg ttttcctttc agaaaaacta cagccctttg ccccgttccg 1560
   atgccgacgc aaaattgtta aaggaaatgc tgccaaaaga tgcgcccgaa atgccggact 1620
   cactgctctt caacttctct taccaggtcc cttttgtgcc tttttcaact tcgaattcct 1680
   cccccgtctt aaggcgtgcg gaaatctcca acccatcgct tcgctcatcg gcggtttggt 1740
   ggcccaagag gcgatgaagg cagtgacgca ccacacgacg cccctcaagc agtttctgta 1800
   caccgattct cttgaggcgt tgccgggcgc cgattcgacc tttgacgccg ccaagttgga 1860
   gaagaacgat tgtgcaccgg tgagtttttg gacggacaac taattgctgt tggagtgttc 1920
   aaaaactgtc catttgcagc gcaacaatcg ctacgacgga caggcggccg ttttcgggtg 1980
   ggcctttcag gaggcgctgc agcaacagaa atggttcatt gttggcgccg gagcgattgg 2040
   ctgtgagctg ctgaagaaca tggtcagtca tttgctgcca attgtaataa aagtacgttt 2100
   tgccaggcga tgatgggctt gggctgtggc aacagtggct cgctgaccat caccgacccc 2160
   gacaccatcg aagtgtccaa tttgaaccgc cagtttctgt ttcgtcgtcc tgatgttggg 2220
   gtcagttgcg agtcttaaat gttaccaaat tcttgtcatc gcctttctgt agaaaaagaa 2280
   gtccgaagtc gccgcccgtt ccgtcaaagc gtttaactcg accattcaca tcaatccgat 2340
   gtctgagatc gtcgccgagt cgaccgagca catcttcggc gacgacttct tcgcccgtct 2400
   gaacggcgtc tgtaatgcgc tggacaatgt cgaagcacgt aagaaattag gagaaggggg 2460
   ggggcagact caaattggca cttttgccca ttttgccagg ccgttacgtt gaccgccgct 2520
   gcgtgtacta tcagcttccg ctgctcgagt ccggcacaat gggcgcaaag ggcaacacgc 2580
   aagtggtctt cccgcacttg acggagtctt acggctccac gtccgacccg cccgagaagg 2640
   agacgcccat ctgcacgctg cgcaatttcc cctaccaaat ccatcacacc attcaatggg 2700
   cgcgagctgt gaaatttatt gacctaattt cagttgtagg aaaagtcccg tttaagaagt 2760
   tcaccgacta tttcacttcg gttgcggaga cggtcaacca atacttggac aatgtgaacg 2820
   actttttgca ccgagtgaac caaatgactt tttctcagcg ggtcctttct ttttccatct 2880
   tttatctttt tttatccgag ttcttttgca gatcgaagtg ctcagtacgc ttgagcgtgc 2940
   gttggtcgtc gaatacccga aaaatgcgga agaatgtgtg agttgggccc gtgaactgtt 3000
   cgacacgctt taccacaacg acattgccca aatgctgcac aatttccctt ccagcatggt 3060
   cgttttcctt tccatccatc catttttttc ttttttatat aaatttgctt ttagcttact 3120
   gcccaaggcg acaaattttg gagtggaacc aaacgctgtc cgcacacttt gaacttcgac 3180
   gttaacaacg tgactgccca atttaagcca atgaacccgt tgtccatttt tagcctgaac 3240
   acttcgagtt cgtcttctcc gcttccattt tgcgtgccca atcttacagt ttggctccga 3300
   tcactgagcg caaaaaagtg gcacaattgg ccatggcgta ctgctccaaa ccgttcagac 3360
   cacaagaggg agtgcgcatc gctgtgaccg acgcggaggc gactgccaat gatggcaaca 3420
   cagcaaatgg cgatggaggt gggcattttt gattcattta tcatcatatt ttcgattaca 3480
   attttccaga cgaaactgta cttcggctga acgatctgaa cgtgaaattg gccaggctga 3540
   acttggagaa cattcgccgc atgacgccca ttgactttga gaaggacgac gactccaacc 3600
   accacgtcga cttcgtgacg agtgcctcca atttgcgagc cgataattac gacatcgaaa 3660
   aggctgatcg gatgaaagtg ggttgatttc tcgacggatt tatcccgaat gatttcgcat 3720
   tcagacaaaa caaattgcgg gcaaaatcat cccggcgttg gccaccacaa ctgcgctcgt 3780
   ctcagggctc gtctgcatcg aactctacaa aaccattgag gcggatggaa ggcgctcgac 3840
   ggctcagatc gaacacatca aaaatgcatt tatcaacttg gctacgcctt tcattgcgtt 3900
   cagcgagccg ggcaaagcgc agaagaagaa ggtgagtttt gattctgttt gggctatttc 3960
   cgtcattcgt tattttctcc gcttttcagt acctcgacat tgacttcact ctttgggacc 4020
   gtttcgaagt tgacggaccg atgacattgg ggcaactgat tgattgggtt gaggtggtca 4080
   ttgcttccac cgtttaactt tttgcatttt tttctgttcg tttagagtaa gagtggcctc 4140
   accgtttcaa tgatttcttc cggcgtctcc cttttgtatg cgttctttca gcccgcagaa 4200
   aaagttgccg agcgcaagaa catggagtga gttttaattg gctttttgtt tctgtttaat 4260
   tgtcaaactt cagtcttatt gcggttttcg aggaagtgtc tcgtggcaaa gtgccggacc 4320
   atcgccgagc gatcgttctt gaggcgttga cccagaatga ggaaaacgaa gacgttgata 4380
   tcccattcat taaatataat ttccgttaaa tcattgttct ttgagtacgc tgtatattgg 4440
   atattgttgt gtctagagat cggaaattat acaatgctgt ttaggctatc aggacttgaa 4500
   aaaattgggc gcaattgtct gagccgcaaa cacactgatc attatttctt caat 4554
<210> 506
   <211> 2252
   <212> DNA
   <213> Heterodera glycines
<220>
   <223> genomic virtual cDNA Sequence=SeqID_545; coordinates=1699..1622 ,1310..1023,967..884,837..651,604..518,466..350,298..206,150..71
<220>
   <223> coding sequence=SeqID_787; coordinates=1699..1622,1310..1023,967..884, 837..651,604..518,466..350,298..206,150..71
<220>
   <223> peptide sequence=SeqID_1032
<400> 506
   cggtaatgag gggtgggggg ggagagtgat cggcgatcaa ttctttggcc gccaaagatc 60
   tgccggctcg tcaagtcatc agcttcacgt acgaattgta attggacacg tcgcgcttga 120
   gaatctcacg caccggaccc atcacgcgct ctgaaggcaa ttaattggcc aatgtgaaat 180
   ggtggaaaat gccaaacaaa ctgaccaaat cgtgcccggt ccaatttcag acatttcagc 240
   agagttttgg ccacgacggt ggcggcacga gggcgatcga gaaggagggc gatttcccct 300
   ttaatgagcc ggtaattaat gcagcggagg agtgagtgcc aaaaccgacc gaaatagtca 360
   gagggtccca attttccgac cacttcgaag ggcgcgtcgt cgttcggtct ctgcagcacc 420
   tcagcttcgc cctccacaat gatgaagaat tcgtccccag gatgtcctta aatggtcgct 480
   aattgccctc cgtctgtctt ccccttcgct cttttaccct gctccaccac gtgggtgccc 540
   gaggggaatt ggcactgttc caacgcatcc gccacattgg ccctttccca tttgtctaag 600
   tcggctgtaa aagagagaag aatgaatggg gcagaatggc attatattac caagaatctg 660
   aactttgctc aggaattcgt cgtacatttt ccgctttcgc atcgtcgacc ccataagaat 720
   ggcgcggtaa gtgatgcggt cgatggccca cagtttgacc gtgttcgtct tcgcgactac 780
   ggtggctgcg cgcggagtgc cataaatcag tgccaactcg ccaaatgaac ctccctcctt 840
   caaaaaacag tttaaagcat ttgcggctgc ctttccgtca caccttaatg ctcaacacga 900
   actcgccatt gacgaacaca tcgacctcgc cactgtcaat cacataaaaa ttgtcgccct 960
   cttcaccctg aattgtcaat tgtccgtctt ttgtcgtcca atttgcttcg cttttcctcc 1020
   acctgctcaa tgattgtctc cccgaggtgt ttctccaccg gaaacatggc gtcgaaaatg 1080
   gccttttcct cgtccaagtc caaatgcgca aacagcacat ttttgcacat cgccgctcgc 1140
   aaagcctttt gcacgtcgtc gtctttcggg atgaccactc gctggtaatt ggccgcctca 1200
   ttttcgtccg gcacctccgc cgacacgccc aaccgacggc gattgccctg gggcagtcgg 1260
   ggcggctcct ccaaagtgtc gtcgtcatcc ggcacggaat ggacagtttc ctatgaattt 1320
   atgggagggt gaatttggca taaaagggag tccaagaaaa aaatgctatt ccatataaac 1380
   caactaatga aacgattgag tgcccgcaaa aagagagtta accttttgag aagcgaaccg 1440
   ttgcattttg aaataattag ggacaaatga gggattgggc aaaggcaaag ttgggatggg 1500
   agaaagcgag gtttgcacag ccgaagagaa gcggtcgcgc aaatcgaccc cgaaatgtta 1560
   gccttagcgc cccctcagca gatccgcgga atttttcatt caattcgagg aaaatgcaaa 1620
   catcggacgg acggaggaaa cggacgcgtt tctcaaactc accacggaga cggacaatca 1680
   acattctcat gcactccatc aagtcacgcg aataaaccat caaaattcaa atgctctctt 1740
   ctcttcctcc cccctccatt ggccatctct ccgatgtttc ccccattttc cttcctcgtc 1800
   catttgtccc cttctcccct ccttttgctc cattatcctc gtctcattat tttcttcctc 1860
   ctctttattt atctgcttct gatcaccttg tttgtcttct cggccaaaac ctttcttggt 1920
   gacccctctc cgccgccttc cctttgctgc gcctgcgaat tctcttcccc ttttccctcc 1980
   gcctctccgg cgctaagatt tgcgcgtcgc cggcgcaaat tggtcaaatg ttgacccatc 2040
   tgaccgaaag cacttcagag agagccaaaa tcaacacgca atttgcattg accagccgaa 2100
   attgccaaga ataagcggag atacgacgaa ggattagcca atttggtgaa ggaaataagg 2160
   catgaaagaa ggggagagat gaaggcgaag aacgaatgaa aaacggcgga tgacgacgaa 2220
   tgaacgcttc tcgggggacg agggggaaga tg 2252
<210> 507
<400> 507
   000
<210> 508
   <211> 7265
   <212> DNA
   <213> Heterodera glycines
<220>
   <223> genomic virtual cDNA Sequence=SeqID_547; coordinates=6173..6048 ,5864..5722,5653..5485,5422..5376,5037..4901,4511..4426,4078..3892 ,3420..3089,2742..2623,2119..2008,1908..1860,1404..1174,452..296 ,117..1
<220>
   <223> coding sequence=SeqID_811; coordinates=6173..6048,5864..5722 ,5653..5485,5422..5376,5037..4901,451.1..4926,4078..3892,3420..3089 ,2742..2623,2119..2008,1908..1860,1404..1174,452..296,117..1
<220>
   <223> peptide sequence=SeqID_1053
<400> 508
   cgtttgctgc tcatgttgtt gctgttgtgc caacagttgt tggccaccaa tgttgcgccg      60
   ctttcgtcgt ccatgggaca tgaacagttc gttggtgccc gagtccaagc agttgctcta     120
   cagtagagtg agatgaatat tttgccaaat tttgtgccaa agtccaggct tttttttaaa     180
   tgtcgagctc taatctcatc aaattccaga atattctcta aatggcttgg aattcagtac     240
   ggtgtcatgg tactgtaatg aatgaacaca ttttttgtgc cgaaaaatca ccgaccggcg      300
   gacatccgtt gggtccggca cagaacatga cggtgcacac aatttgtatg tcgtaggagc     360
   cgtccaaacg gaacgccttc atctcggcct tgagtcgatt cttgtcaaag tcttgctcca     420
   ccgggccaac cagggcaggg tagcggggac agctttaaaa agtgccgtaa ataattccgg     480
   ccgttaaatg tggcactttc ccactcccat ttggaaataa gtggcacgga ctctaaaaag     540
   gcgacacctt tcataaatgc caaggcaaac gacgatagag atgaaaaaag atgaaaaaca     600
   attttttttt tttgaatgga gcataccaat tgaactgttt caaaaacacg ccatggaccc     660
   cccaagcccg tagccaggaa aaattttcgg ggggggggtt tttttttttt tttttttttt      720
   gcaaatttat ccaataattt ttttttcctg ctgggtaaca actggctttt ttgcactaaa      780
   aatgaccaaa ttttggccgt tttaaggcaa aaaacttgta aaaatagtgg aaaatgtagg     840
   tccggcggtg ccgataggcg ccgccggaaa caaatttttg gaaatttgag tgctatttgt     900
   tacgaaaatt gtctataaaa tttttaaatt ttcgtttggg gggggcaatt cgaaaaaatt     960
   ttccattatt tgctggaaaa tttttaaaaa ttttcatttc ggggggggga attccccccc     1020
   cttccccccc cctggctacg ggcctgggac ccccccccgt aaaaagatgg caaatgcttt     1080
   acaaaaattg ccccctccca aaaaagcaaa taccatacaa aaattggcaa aaaaacggca     1140
   aatgccatac aaagagcatt ggctcctcct cacccgttgt cgatgatggt gaatggcact     1200
   ggctcgcctt cgccgcccac ccgagtggcc acacattcgg tgaccatcat attgtggggc     1260
   ccggcctgct cgctgttcac caccaagtgc agctgatcac cgatggatgc cttctccagc     1320
   ggttggttgt ccttgtcgag gaacgacagt tgcgcagtga tcggcggcgg tgtgccggcg     1380
   ctcagcactt ggggctcggc ctccctttga tgggtggaca aacaattaat aggattgtta     1440
   attaattagt agtcggatac aaaaaaataa aaataaacac aagaattaat taatgagttt     1500
   gggttggaat tggacattga atggaaattt gccaatgtag cgctcaatta attaattaaa     1560
   ctaattaata agttggtggg tgtgaaaatt gtcgtatgag ttaattagtt caggccattt     1620
   ttcagcaagc tggattatac aaattaatta aattaattat taacgaatga ttttgggatg     1680
   aaatttgcca atgaagccag tgcttattaa ttagtcaact aattaatgag tggggttaaa     1740
   aattgttatg ttaattaatt aagtattaaa ttagttaatt cataataact gggctggttc 1800
   cccgtggccc aattcaaaat attctaaacg cactatcgtt tcccgactga cagacaaact 1860
   ttgtctcgaa tatggtggtt acgatcactg tgccctcaaa gccgggttct tggccggcgg 1920
   catcgccacc tccggcacca ccgccgccgc ctccggcact gactacggca ccgccagcgc 1980
   tgttgctggt ccgcaccaag cggtcaatgt ccatgttcac gtcgcatgac acgcggtaga 2040
   agcggtccca ctccgtggtg acctggtgcg gcaacactcc gtccaacgag acgaccacca 2100
   acgcctccaa tttgccgtcc tgtggacaga aagatttttg ataaagcaat tcgttttgat 2160
   aaaaaaacag aggaggtctg atcagctttt accggcaatg attggacaaa tttatatctg 2220
   attttgatca agcacccatt tcctattggt atatttattt gatcaaaaaa agacaatggg 2280
   tctgattggc ttttgtccgc aattatagga caaatgtata attttatgat ctggttatga 2340
   tttatttggt caataatacc ctcccccaat cagtgaccaa atgatttatt ggtatttaaa 2400
   ttcaaattta tcaacaagcc acaagtctcc agagcgcatt gccagaaaaa ggaaaaagct 2460
   cattgttgct actaaatagg ctcttcttgc aaaaagctga ggctatttaa atcagaaact 2520
   gattgattgt atggcttatt cagtctgatc aaattcaaag taatgctttg aagagaaact 2580
   gatcaggaga agagcaataa actaatttcc ttgcttttgg accttaattg tagtgccaca 2640
   tgccggcgac acatttggcg gcgggaatgt gatggaaatt tgctttgcat tctcgaccac 2700
   ttgggagcac gtgctgaact tctccgccgc ataaatggca cccttaaatg gagcgggggg 2760
   ggggggggtc taattcagaa aaaacgcata aaaagaaaaa aacgaagtta agttaaatta 2820
   aatggttgta aattctggac aatttctctc cccccccccc cccgtccgat aaattaaaaa 2880
   aaattttata agaaatttca ttgaatatgc ttttaaatct ctgaaaattt tcaacaattt 2940
   ttattttgaa tttttttaat tttccatgag ggcggaaacg gactgctacc aaatatgact 3000
   attattggtt aacgagccgg accattttct tgagaaattt caagaaacat ttttttgtaa 3060
   cgccggccca accgtcgaac agacggaccg tgtacggctc tttggtgttc acaatgaagc 3120
   ggatgccgtc attggtgcac agtgccatgc ccccaaactg ggactcgcag ttgaactcgt 3180
   agaagtcgta ggacggattc tttttgatgt tcgccgtcgc ttggcccacc gcataaatgt 3240
   ggcacgtgct ctgtgtgcgg tcgtaattga tcgatttgca gttcaaattt tggaagcaca 3300
   gcgaaatgca gtcagacacg gtgacgccct gggcgctggc caccggttgg ccctcaatgg 3360
   acacgccgat gaagcggttg aatgccgcct gttgacggca cgtggacgag ctcagatcgg 3420
   ctgtgaattt tttttttttt tggttagtga tggattgatg aaaaaatgtg gcacaatgat 3480
   tgattgggcc gaccgatcgg tggaaatttg gccgggataa agctatgccc aaaaaaatgt 3540
   gaaactttaa gcccataatt tttggcttct ctctaattga tcagcgccaa cggtgattag 3600
   aatcttaaga atgaacccgg gacttattct gaactttatt aagcgttgat caatctgaat 3660
   cagactcgat aaagtgatca aaatccaatt gaattcggct aaggatcagc ttaaccgttt 3720
   tttgggcttc atcactcaaa gaagagtgat cagagaatct cacaggatgc gcaccgatac 3780
   ccattctgat cactttttga cttgttggtc agacttcgga gtgaccaacc atgaacaaaa 3840
   attagtgatc agagaatttc gggtaaaaat attgtttttt ttgcctctca cccgccacac 3900
   aaatgggctc gtaatagtct ttgctgtgca ccgccacttg ttgatcagcg cgcgagaatg 3960
   tgtcctcgtc gctgacaatg cagtttttct cgttgggcga gaattccacc gagcgacaga 4020
   aggtgttcag cgagtccagg cacttctgca gacagttgga ctcttgtggc tcaacagact 4080
   gtaaacaaaa tgacattggt tggatactgt aaactgtaaa atgggtgtgg aatactgtaa 4140
   aaacggagag tgagggtact gtagctgtac ataaaaatgg gtgagaatag caaacttaaa 4200
   aacggagggt gaatttggtt tagcaaacta taaacatgaa ggcggccggg taagaaaagg 4260
   aaaaaggcct aaaaacgggc atggaataca gaaaactgag gatatggtgg acctggtaca 4320
   gaataatgta aaaatgtgct gttacagtaa aacaaaataa aaattcttcc aatcattttt 4380
   catactgttt ttttcgtgat aaatttgctc aatttcccgg ttcacctgag tgtggtcata 4440
   gaacttgatc tcgaactggg tggtcttgac gaacacaagg ttgccgccct gctgaccgca 4500
   gcgattttgt cctggaaaga aaatcaatca attcaaagcc atggattttc taaaaaaaaa 4560
   aatcgttatt tattttgttt tcaatcccca tcaaaaggag ggctttatct cagggagggg 4620
   ctcaatttta agaaatacgg tagtatttta acaacattta acccccgaac cgtcgggact 4680
   gatgactcga gataatgtaa tttcattttt tcaaggtcag gattcgaatg agtcaggatt 4740
   tcgggactag cccggcttgg ttgataagct ccgaatgatc cgggattttc atttttagaa 4800
   actcgaaagc ctaatctaaa aaccgatttt tgtggactcg tctcccgacc ggaaaatgac 4860
   cccgagaacc cgttgccacc actcggacaa caacacgcac tgttcaagca gttgttgtca 4920
   tagtaatcca ccggctggtt ggtcatccgc aaatgggtgg gcttcgagcg gcggtcctcc 4980
   gtgctcatct cacacgcgta tgagtcataa ttgtagttga cggaacggca cacgaatctg 5040
   gcacaacaca aaagaaaaat tgctgcaatg aatggatgtg tgggtgagag tctcggtttt 5100
   tatgagatca gaaaatttct cgggattttt tgccattttc gggcaaaatt tgcgactcaa 5160
   aaattttttc ccgatttttt gggtacttca tatatataca tatatatcca aaaaaccttt 5220
   tatttctgtg ttttttttac taaaaatata tgacttactc atctaaattc attccctgcc 5280
   atttttgagg caaaaatttc tactcaaaat aaaatttttc caggttttct gggtctctct 5340
   attcacggga taaattttcg gttgaaaaaa ctcactcttt ttccagaagg catgcgctca 5400
   agcactgctc ccgggaaggc acctttaaaa cggggcggta aattttaaaa tttcacaata 5460
   tttttcgaat tccgaaccat ttacctgaat acttttccgc acaaagccgt ccagcgacgt 5520
   tttgcgcatg cgttcaaatg aaaaggagcg gtaggtgcac gcctcgtgcg cgttcacgcc 5580
   cggcaagcag gtcttctcgt agtagaaatt gtttgcggat gggtttgggt tcaggtcggc 5640
   agtgccggcc gggctgaaca tggaatgcca aggaaaaaat caaaaattcc tccgcgccat 5700
   gccgcaccaa acattcccca ccttgcggca tcgtcaaaaa agtagcacgt ctcgcgtccg 5760
   tcaaagttaa aagatgcgca cagcctctga gcaccggtcg tgggttcgat gttgttccgg 5820
   cagtattgcg agcaggtcag gtcatggcca gccgtggaaa caacctggaa attagtaaaa 5880
   acaattatgt aaaatcattt taattgacca attctgtccc agcctaatat gttctgctga 5940
   tgtagtttat ggattatttt atgcgacccg ttcactcgga gcagatttga ttgaaaataa 6000
   ctgctaaatt ctggatccga caataaaaaa gtgcccttcc ggctcaccaa tggcgtgccg 6060
   tccagacggg cacgtgaaat gcgaacaaat gttgctgttt catgattggc acattcaaac 6120
   gcgccaaatg tgccgggtgt ggcgagtgcc aaaagcactg caacaaatgg caccagcgca 6180
   atcggtgccg gaggggacaa atgccgagga cgacggactg gtgccgatga tgtgggtggt 6240
   agtggtgccg aacacgacaa tgttctgttg ttggcacgac gcattttccc ggattgagag 6300
   aaattattcg gcgcgacctt tcgagggcat aaaattgtcg atgaatttaa ttagacaaga 6360
   atttcgggga taaaatgggt acaagaatac catgggattt gaaaacggaa aattcgctgg 6420
   ccgcggcacg tcaaatgaga gaaatagtag gcaaaaaaaa aggagggatt gatctgactt 6480
   gcactagggg ttgtcccaaa cgaagcatca ttatcactaa taatggatgc gcgaatttat 6540
   gtgtcaaggg aaaaccctcc tcccggaaaa tggggtcgag taaaactcta tactgtaaca 6600
   tctgtacagt attattgacc agttctggtt ggtctacact tcaatgaagg gtaaacccct 6660
   gcctgaatcg ggaacatccc gatttattga gtcccaatcc cgattctcga cataaatccc 6720
   gaatccatgt catagtgccg gaggcttaaa actcgcctcg cacattcaca aacgaatgtt 6780
   gtgcaaacgg ttttccgtaa tgggataaga cggtggaagg ggaaggaaga ggaggaggag 6840
   gcgaaggaat gggaacacag cacaaccaga gcaaaggaag acggaggatg atgatggtat 6900
   ggctgtgacc gcaacggaat ggaagggggg ggggaagaat ttgggtcata aacccccgag 6960
   gggttctcat gcccaacggg gcgggagggg gtgggggagg aggtgtttcg cagcagtaag 7020
   tggataatac cgccttcgga agacactttg tgtgttatag agagatgctc atccgactat 7080
   ctcgaattta caccgctatt ttggcgggat ttacgcgaaa cttcgtaaaa aactggcaca 7140
   tttgccctaa ggccacatga ttttcgagtg tcgagaatgg gtgggtcaag aggcgggaga 7200
   caatgaatta gctagcatta tattatgatc attattgcct ccttcatgat ttacgctctt 7260
   gaagg 7265

<210> 509
   <211> 4406
   <212> DNA
   <213> Heterodera glycines
<220>
   <223> genomic virtual cDNA Sequence=SeqID_548; coordinates=1563..1778 ,2003..2279,2504..2699,3122..3454,3556..3696,3885..3998,4064..4269
<220>
   <223> coding sequence=SeqID_813; coordinates=1563..1778,2003..2279 ,2504..2699,3122..3454,3556..3696,3885..3998,4064..4269
<220>
   <223> peptide sequence=SeqID_1055
<400> 509
   aaaatttttc cctacgttca aattttcttg aaattttgca taggcccata ttttgacccc     60
   ctctatcgga tccctgttta ttttatgaaa gttatgtttt taactcgaga tcggctcttc    120
   catttttaca agggaaaatc ccaagaaaat ttaaagtttt gcaaagtttt ggcgttctga    180
   ttaatagata aataataaac ttatttgcac tgggagccca aaattgaatc gccatgttta    240
   aaatttaaac atcactccag cgcactgatt tacccgggca atgcgggtac atgaatcatg    300
   ccgaagagca acggatgttg gcgcatttga agagcacgtg aaaaggagga gagtattgga    360
   atggaggaca ttttgggaaa ctttaattaa agaatgaaaa aggtagcagc agcagcacac    420
   agtcacacac agcagaacct attcccttcc tacacataca tatcctttca ttttcttttt    480
   cttatcagca aaacaacaaa aggtgataat ttgaatcggc gtctgtcgcc gggtactgct    540
   cgagaatcct gtgctaggcc tagagagtcc cacgactcgg gaaatatgta gtcggaggac    600
   tgactataca tatgcaactt tgcagattcc aatccaatcg aaactaagac ttttgccgga    660
   ttatttcctt ttcataaggc atttatactt taggcactca gagttttgca ttgtgaaatt    720
   tcaaatttca caacatatga taattggcat tatgtttttt tgaaaaagtg ttttttaaat    780
   tctatcaaat ttaaaattgt ttcctttgcc ttttctacca ctctttaatt gcctgcgatt    840
   gatttttgat tgtcgtttat ggaagagttc acattttttt ccatccattc ctttacaaaa    900
   attggaggga attctcttcc tatttatgtc atgtcgcaaa agaatatgca tggaatggta    960
   ctgctgttgt atgggcccat attttttgag attgatggga tggaattgga gaataaaaat   1020
   gagcacaaga aaaggaagga aaggtgaagg ccttccatcg tgtgatcttt ggtcttttcc   1080
   atcagctcac aaatattcga gtggaaagga gggggggggg aaggggtaac aacgattcat   1140
   ttttggtgtg caatcgcatt gaaaggagag aaagtggaaa aatgaaaatg gcaccgcccc   1200
   gtcccctttc acatatttga tgggtgtgta tattcttaat ttgttatttg catggtttta   1260
   tttatggaat ttcatttttc tggttctttt ttatttcatt tcattttaaa gaacttggta   1320
   aattaccttt tgtttgttcg aatttcttga atttgatgtt tcgctttatt ctgactgaca   1380
   gtgtccgact agtcagctca atttgcgcta aaaatcggat ttttctgtga gaatttaagc   1440
   ctcatttgca ctttcacatt tttctctccc atacataaat aattatgccc agtctaacct   1500
   ccacaatttt tagtccgacc aaattttcca ctcattttgc gtccaaaaca ttccatccaa   1560
   aaatgaccat ttctgcgacc gctgacgttg acaagctcca ggatgcggcc aaccgaatgc   1620
   gcatttcgtc cattgagcag acgtgcgccg ccaactcggg ccacccgacc agcagttgca   1680
   gcgcagcgga aattgtggcc actctgttct ttgccgaaat gcgttatgac gtgaaggacc 1740
   cgcgcagcgc atccgccgac cgtttcattc tgtccaaggt ttgcaaaaag tcgcgggaac 1800
   tcgctgatcg cgcgaaattc ggttgtgtcc ggcacgggtc gtacgactat ttttattgga 1860
   attgggtatt ccagttagtg gatgccatta tcggggagtg atttaatatt ccaattcgtt 1920
   gcttctacta tatttccaca attcggactt tcctccaata ttcccatccc acatcaattg 1980
   gccatacaat ttcaatttcc agggtcacgc ttgcccaatt ctgtacgccg cttgggaaga 2040
   ggcgggcctg ctcagccgtg accaagtgct ctccctgcgc aagattgact ccgacattga 2100
   aggacatccg accccgcggc tcaacttcat cgacgtggcc accggctcgc tgggccaggg 2160
   tctgtcatgc gctgccggca tggcgtacac tggcaaatat gtggaccatt cgtcctaccg 2220
   cgtgtactgt ttgatgggcg acggagagag tgcggaaggc tccgtgtggg aggcagcagg 2280
   tgaactcaaa atccacggat agaggagacg tagtcaaagc ctgggaacct acgggaaaat 2340
   attttttcat gaccgttagt ttctgttgaa actacattta aaaattaatc ataatttgac 2400
   ataagccgga atcgggattg catggtttag gagaaataaa aaaaattttg ttgttttttt 2460
   ttctaatttt atgttggggc aacgaatatt tgtccctttt cagcatttgc aagtttctac 2520
   aagttggaca atctggtggc cattgtggac gttaaccgac ttggacaaag tcaggagacc 2580
   caactgcagc acgacattga agcgtatgtg caacggtttg ccgcgttcgg ttggaatgcc 2640
   atcggattgg aggacggaca cgacatctcg gcacttttga atgcttacgc caatgccaag 2700
   taaactcagg gacagtattt ttccttttcc tgaattactg attgattgtg agccgaaatt 2760
   cctccttttc cttcctgctg ctggggagca ggacaaaacg tcgtcggggc ataatgtcga 2820
   cgcaaaaatt tttaattaga aaaaaaatta aaatttttta tttacctagt ctaattcgaa 2880
   atttttttct aaaataaaat cttataaaga aaacaaggga aaaaatgtcc ccgttttttt 2940
   tcttaaaact tgagcagatt ttattttcat taacacttag attttcaaaa aaagacaaat 3000
   atttttttaa aatttttctt ggacttttta aacaacattt tttcatcgac tttttggggt 3060
   gtgcccttcc ccctggactg gtattttaac acaatcccat tgaatttata ttggcgttaa 3120
   ggaacgtgac tggcaaaccc acggcaatca tcgccaaaac gctcaagggc aagggcattg 3180
   acggcgttgc cgacctgaac aaccagcacg ggaagccggt gtcgaaggac aaggcggagc 3240
   ccatccgggc attgctcaaa agtgacaagc cggcaaattg gaccatcccg gcaccgattc 3300
   gggacgcacc ggcggtcgac ttaggcatcg gtgccatcgc aatgagtgca ccgccgcagt 3360
   acaaaatggg cgaaaaggtg gccactcgac aggcgtacgg aaacgccttg ctgaagctgg 3420
   cggaggccaa caaacgcgtc attgccttag acgggtggga cagataatag gcacctaccg 3480
   aatttaaatg gatttaaata aaaaaaaatc ctttttgatg tgtcaattca atttttaatt 3540
   gttttttccg tttagtgaca tctccaactc caccttctcc aacctggtgc aaaagaagct 3600
   acccgagcag tttgtccagt gttacattgc tgagcagaac atggtcggtg ttggagttgg 3660
   catggcgtgc cgtgggcgtg tgcttccgca tgtcaggtgg gcagaaagaa gccttttgga 3720
   ttgccaaatg gccaagaaga caaactgctg ttatattaag gggattttaa aaacaattaa 3780
   aaattttttt gttacactga atttacaaca gaaaaaaaac caccgggttt ttgccaaaat 3840
   ttttattaaa tttgccccct attttatgca ttttactttt ttagcacttt tgccgccttc 3900
   ttcacccgtg ccggtgacca aattcgcatg ggcgccgtgt ccttcacaaa tgtcaaattc 3960
   accggctctc actgcggcgt ttccatcggc gaggacgggt aaactgctgc tggcagagca 4020
   cttggaaaaa aagggaattg cttcaatttc tgttttgttt cagtcccagt caaatggccc 4080
   tcgaggactt ggcgctgttc agcgctttgc ccgccagtgt cgtcctctac ccatccgacg 4140
   cggtgtcggc cgaacgtgcg actgagttgg ccgccaatta cacgggcatc acttacacgc 4200
   gcgtctctcg acccgccacc ccagtgctct acacaagtga cgagaagttc gagctgggca 4260
   agtgcaaggt gggcagcgat gggaggctag aaaattttca aaaatgtttt tgaattttaa 4320
   atagggtttc ataaataatt ttgtagtcgt cactaatttc gttagaattt caatttaatg 4380
   gggtggtcgg tgtcggttta tgtaaa 4406
<210> 510
   <211> 2359
   <212> DNA
   <213> Heterodera glycines
<220>
   <223> genomic virtual cDNA Sequence=SeqID_549; coordinates=2122..2072 ,1912..1809,1752..1659,1599..1492,1401..1296,1237..1116,925..735
<220>
   <223> coding sequence=SeqID_814; coordinates=2122..2072,1912..1809 ,1752..1659,1599..1492,1401..1296,1237..1116,925..735
<220>
   <223> peptide sequence=SeqID_1056
<400> 510
   ttttaagaaa aaatcataga atttcttata agaaaattta aaaaaaaaat tagaaaaaat 60
   tttaaggatt ttttttgaaa tttatgataa attagtaagt tttttcgaaa aattttccaa 120
   aaaatcacta aaaaattttt tcaaaaattt ttcattggaa tttccatata aaaaaattct 180
   aagaattttt ttcttgaaaa tttccaaatt ttttaaaaaa aatttaaaaa ttttccaaaa 240
   aatcactaaa aaattttttc aaaaattttt cattggaatt tccatataaa aaaattctaa 300
   gaattttttc ttgaaaattt ccaaattttt taaaaaaaat ttttggcgaa attatgtccc 360
   ggcgaaattt cggcggcgaa attatgtccc ggcgaaattt cggccggcga aattccgtcc 420
   ggcgaaattt cggcggcgta atttcggcgg cgaaatttcg gcggcgaaat tttggggtgc 480
   acccacaaca aagcaatacc aataaatcaa acccacacat ttcgaaatgc acttaattta 540
   cactcacatt caccaaatat tcatgcatat catccttttc attttgtttt ttcttccatt 600
   ttgttccctt ttttgtttcc catttccaaa ttacggaatt ttaatattta attaatttcc 660
   cttaaatttg tttgtacgta tttttgtatt tcatagtaac aatttagtga ataaaaatgt 720
   aattattatc agttttgaaa tggaattccc caccttttcc gtgctttccc aacactccga 780
   aagagaagga ctcgtcgatg aaaatgcgca ctttgtgtcg ccatttcagc tcgatcagtt 840
   tgggcagtgg gcaaatgtcg cccgaatttg tgtaaattcc ttcgacaacg atcaaacggc 900
   gaatggacgc tttccttttc ggattctgcg aaaatggctt tttgctgaat tctacagaac 960
   aaattcttac aagtataaaa aatttaaatg ggtttgctaa acaatctaat ccgatataat 1020
   aatccgaatt ttggtgcggc tcaattgggg agggccgtcg cgcggaatgc attccctcgg 1080
   caattctttt gtttcccctt tcccgttctc cccaccattt tgtctttctc ctccaactcg 1140
   accattttgc gctccaagtc cgccatgtca ttgtgttcaa accataaaat gtcacttctc 1200
   gacgccatca gtccggattg tatggcgaaa ttaacaccta aaaacatggc ttttattttc 1260
   catggccaac aaatgattgg gcgactcccg gggacctttg tccgcaaaaa tgacatcgcc 1320
   cctccttgaa taagctggga tggcacttgc gatggtgggg aatccgtagc tgtacagaat 1380
   ggcctcttca cagcccatga actgtcaatt tgaatgacaa ttacggtatg acaatacagt 1440
   gtgaaagtgc cgccaaaacg aactatctgc aaatttttgc tttgtcccta cctcagccaa 1500
   ttccctttcc aagtccaaat gtacgtccac cgttccgtag aaattcctgg gcccgcatga 1560
   gcccacgccg tatttgaaaa tggcattttt ggctacttcc tatgaaatgg aaagcaaaac 1620
   aaaaatattt ttaaataagc catgtatgaa caacaaactt caattttctg gtttccgata 1680
   aagccgaaaa aatcagtcga cgccaaattg acgcatcgtt tgtcatcgat gacaacaaat 1740
   ttgcccattc gcctgtattt ggtttgtatc cgtgataccg gtccaaacaa ttcaaatgaa 1800
   aatcgaaccc ttcaattgaa cggggccgat aattcgggtc gttttgttcc ctttccgagt 1860
   aaagcggcac caacggactc ggctcccaat tcgcaataac cgcttccttt tcctgcacac 1920
   aaaaaccaat ctttgccagc agagaatggc cgtttttaat cgaattcaaa tggcacttgg 1980
   gtaatcgctt ttttttttgt tttgttgaat ttatgggttt tttcgacgcc aaaatttctg 2040
   tcgccgtccc atcgttcttt ccctcacctc ttcgctcagt tccgcttttt tccccttttt 2100
   ctttcgcttc cctcccatca ttttaaagcg ccaacacatg caaatggtca aaacagttgc 2160
   gaccagtaaa ggccagctgc gaattgtcaa ggcgaaaacg cagagcaaaa cggaggatgc 2220
   catgccaaag gaacgcaccg tttgggtgag agggggcgcc atcggagagg cgaaatcgga 2280
   cagaatgcaa accaaaaata tattaacatt atcaaacatt gcttattgtt agtgtacaaa 2340
   ataacaaata caattcgca 2359
<210> 511
   <211> 5091
   <212> DNA
   <213> Heterodera glycines
<220>
   <223> genomic virtual cDNA Sequence=SeqID_550; coordinates=4426..4285 ,4185..4085,3917..3429,3274..3130,3087..2964,2907..2848,2792..2547 ,2493..2376,2320..2235,2181..2083,2037..1863,1681..1549,1161..1097 ,1046..965,914..840,697..495,453..304,204..1
<220>
   <223> coding sequence=SeqID_790; coordinates=4426..4285,4185..4085 ,3917..3429,3274..3130,3087..2964,2907..2848,2792..2547,2493..2376 ,2320..2235,2181..2083,2037..1863,1681..1549,1161..1097,1046..965 ,914..840,697..495,453..304,204..1
<220>
   <223> peptide sequence=SeqID_1233
<400> 511
   aaaagccgtc ccaacagacc ctccctggac gagttgaagc ttttccatgg ccaacttagg 60
   cgccgatgcg ccctttggcg gcacaattcc cggctccgcg taacaatcct tcaccaaaaa 120
   gtccaattcg tcgtccatgg ccatcaattc ccagcgaagt tccaaaatat cgcccaactt 180
   tgcctgtggc acatttcaat tagcctccct tcttcccgtt ctctcctcgc cttttctcac 240
   ctgcaaaagc ggctcgcgca gttcagttgt gctaggtttg ccggcggcgt cttccgagcg 300
   aacttctctc atcggcatca tttgcattag caccgggttg cccagttcga tctttccgcg 360
   cggttggatc agcgacggct gcggcccctg cagcgtcaaa ttggctgcag cggtcacttt 420
   gcctcccaac atgctcgaat aattgcagga aatctgaatg aagggcaaag tcagtgactg 480
   atcggtcggc gaacttcgta aagttggtcc atcgaagtga cgaggccggg gatgcccaaa 540
   ttgttggtct gaacgaccac caccgttttg tacgtgccgt tgtccatgtc cacgaggcca 600
   caatccctct gcgattggcg acgttgtctg aacattatag caaaacttgc gaaagccttt 660
   tttgaacact gtttttcgcg gtttccaaaa gaaccttctg tttttgtgct tcttccgatg 720
   cttctcttcc acttccattg gaatgctttc caattgttcc gttccctcaa gcgaagtgcc 780
   attggttttg tcaacggaat gtgccgtctg cgagtgcgcc ttggggcgac gatgagaaat 840
   gccttccggg gaggtcaaag tgatcggttt catgccaaag tttgcaggaa gtccgagcac 900
   taaagtgacg ctgtccttgg cctccacctg cgaatgttcg gctcaaatgt ccgtccgttg 960
   gcactttctg tccccacctc aactcggcac gtgtcgtatt tgttcttgac gaacatcact 1020
   cccgagaagg gttcgctgtg ttttatctga caaacaaaca gagcatttgg ccaaagatgg 1080
   tcaagggacg gcccacctga acacttatgc cctcgtaatt gcaaatcgtg tgcacttcgc 1140
   tgccggggat ttggatgggc gacggcaact gattcgggtc ggggccgttg ttggccactg 1200
   agaagccatt gcccccactt gactccggca ccgatttggt ctcagatgcc tgctgaaagt 1260
   gtttttgata ggtgtttttt tgtcatatcc gcttggattc cccccccccc cccgggattt 1320
   ttctacgtca ttgcctgggg ttggtctttg tcctttggcg aggatggcac tccgaatgcc 1380
   ttcgactctc tgtccgtttc tgtcccttcg ttttctgttt gaattggccc tttcgttggc 1440
   ccttttctca gtccgtccgt ttttatcgtc ggctaaattc aaagttttct tttttctttt 1500
   ctctttcctt tgcctttttg ttaagtgcgc cgttcgttcg ttcgttcagt ccaacttttt 1560
   tgtcctcttt tgcgcctttt ccttcagaag tgccgctcac caatgccttt ttttcctttt 1620
   tttacattaa tttcccaatt tttgatttga tccctttcat ttctcctcat cccatatccc 1680
   tcttcccctt attctctcac ctctccctcc tttctttctc tctctctctc tcctctttct 1740
   ctctctcctc tccctcatta tctctctttc tctctctctc tctcctcttt ctctctctcc 1800
   tctccctcat tatctctctt tctctctctt ctatctcagt ttgtctttct tttctttctc 1860
   actttgcttt ttggcacatt cgctgcgctt gattcctccg attggacttc tttcagtgca 1920
   caaacattcc gataaaagtc cacttgttca ctcttctgca tcgcccccga cactgtgccc 1980
   aattgtccga tcactgcgca tgtgccattt cgccgattgt aattaagtcc ttcacaactg 2040
   aaatgtattt cataaatttt aatgttttta atcaaaaacc gttgtccggc accggcacac 2100
   aaattcaaac atttggagag aggcacagaa tgcaattctt tgctcagttc gcccccgacc 2160
   cattgggaat acaaatggcg actttgtggg taaaattggt atagtttgtc atttccgaaa 2220
   ttggaaattg tcacctgaac gcggccccct cggggtcgac gcatcccatt gattggccct 2280
   cgggcgatgt ctccaaacag tggaggtcat aaagatcgac cttcagcaga acaaagcatg 2340
   atcaatctcg accagtcagc acttggccag cccacctgtc ccttcggctc ggggcgcagc 2400
   gaaccgcggc cgtccaaata aagcaaattg caaatgccgg acgatttgtc gaagttgaag 2460
   gaacggcatt tgtcgggaga gtgagcggca catctaagaa aggcaaatca ttcgttggcc 2520
   ggagaaagtg ccggtcattc actcactttt gggcacattc gcgcacattc gcctgcaccg 2580
   tttccactac gtgaatggca ccgtcccctt cgggcagtgc tgcatttaat gtccgcacga 2640
   ttttcggact tcgcgccccc tctccggcgc tgcactgttc cgactccaag cgacagttta 2700
   tgtcgaaata gtcgaccaca aagtcgtcct cctcaggaat gaacaggtcc ggcttagaat 2760
   ggcgcgactc cgcgttcaaa atgcattctt gcctaggggg acaaattgga agaggaaagg 2820
   aggggggggg gcgggggacg gactcactcg ttgtaataat acatcaccga tttacacaca 2880
   aagttttgtc ccgtctcagg aggagggctg aacaatgaac agcttcgact cgtcaattaa 2940
   ttaatgaata ttcattggct gactttgtgc attggtccaa acacattgtc actgacggca 3000
   cattttccat cacaaaagag gcaaagccca ccaaaagcat ttgcggcact cgcgtaaacg 3060
   acggaacgcc gcggcacgta cgcggggcta agaaaggagc gcatttgagg ctgggaatag 3120
   cgaactcaca ggcaaagcat ttcttctcga aataagtcca tcccggcact tcggccaaat 3180
   tggcacggcc caaagggcgc gagcgttcgt ccgacaaaac gcaactgcgc gtcaagttac 3240
   tgtacatgaa cgatttgcag ttgaagtcgt ttgccttttg agggaagggg tcatccaaca 3300
   cttcctcatt tgtccatttt cattgattcg tttgtccgtt tttcgttgct ttttctctct 3360
   tttcgcccaa ttgtccgttt tttgttcctt tttctctttt ttcgtccatt tttcggtttt   3420
   tttgttaccg taacacttaa ccgttgcgtg cacaactctt cacattcgct cagctccaac   3480
   ccttcaaaac tgtggtccaa ttcgtccgac aattggcgtc cttccaccgc cacgtattgc   3540
   gttacgtacg gtgcgtaaca ttgtgctgca attgcgaatt gttggtcgtt caaaatgccg   3600
   cttgttttga aattgtcaaa atgccaatgg cactcacatc cggcgcaatt gttgtccaag   3660
   tagatgaccg tgtcctcgtg ctcgtccacg aaaaggtcag gccgatccaa tcggtcctcc   3720
   gtgttcaaaa tgcattccgc gtccacaggg taatacatga ccgactcgca ttcgaagccg   3780
   aacgtatcga acgcattcag acaagcggac agacagcggt ggactgagtc ggcggagacc   3840
   acctcacgcg cgaaacccac caaaatcttt tgttcatgaa cgtcgaaatg gaaaggcgag   3900
   gaacagatgc gctcactctg cagaaaattt gggggaggaa tcagtggcat aatgaaaacg   3960
   gattgagatg tgaccgaggg gactctgcca aaaccaaaac aaacgcccca gggatggaat   4020
   ggcatggccc ttcccattaa taccatttgg caatttccct tggtattttg gcactttcac   4080
   ccaccttcac acaattcttc gtcaccgtcc gtgtcgccat ttcttcacca aattctgcca   4140
   cggcaccttc agatgccgac ttcggaccga ccaattggca ttgtcctccc ccgccggcgg   4200
   ccaattgggt gtaacggacg ccaacgcaat cctaaagagg gacaaacaaa acaatgaact   4260
   tacgccagtg gattaaacat acatttgcga cggaacaaag ttcggtacat tcgagaaacc   4320
   ctccggggac gggcgccgat cggagaggga cagtgccgga ggcgacggaa gtgttgtggt   4380
   ggaggaggaa aacaggcgaa tcgcccgcag ggcattccga caaaagtgct aaacgcacaa   4440
   aatggccatt caacaagaga ccctcataat caatcaattg gattattttg gcatttcatt   4500
   tttacctttt gtttttgctc cattaattaa gttaattacc cctcccccca attataatgc   4560
   tttgttttca gtgttttccg ttcgacccat agaatttgct ttgtgaaaag caattcaggt   4620
   gaaggtcacc cgcaagagtg tctcctcttc ttcgtcaaga taatgatgat taagatgacg   4680
   gtccgccaaa ccgccgtcac tggcgatgga aaaggcggcg gagaacacaa atcaaaaagc   4740
   aaacccccca gggctattca aagaggaaag acattggcac atcaaaaaaa acttacgaat   4800
   ggactgatcg gcggagatgg caatgacaaa tgagagaaaa aagagaggga aaagcgatcg   4860
   gacggaagga aggccaaggc gaagacaatg cctttctctt ctgtggggga aaggccaact   4920
   ggacaaagag aggcgcatgc ttaaggaaaa atgggggaaa agccaataaa agggaaaagc   4980
   gagggaaagg aaagaaaaag ggaaaagcga atattttacg aggaatttga tcggaggctt   5040
   ttgctttgat ttgaccatat acaaacggcg aaagtggctt tctcttctcc g            5091
<210> 512
<400> 512
   000
<210> 513
   <211> 3796
   <212> DNA
   <213> Heterodera glycines
<220>
   <223> genomic virtual cDNA Sequence=SeqID_552; coordinates=3606..3564 ,3501..3338,2995..2869,2319..2122,1375..1154,455..258
<220>
   <223> coding sequence=SeqID_804; coordinates=3602..3564,3501..3338 ,2995..2869,2319..2122,1375..1154,455..258
<220>
   <223> peptide sequence=SeqID_1046
<400> 513
   aaaaactccg aaaacccaaa ttcctaggcc cgtcactgat taaactttcc cttcctggat 60
   aagtccgtgt cgaccaacac acacattttt ttttaatttt ttttttattc aacctaaata 120
   ctcaatcaaa taacaaacaa cacaatagca gcagcaacac catttccatt tcattgtaca 180
   caaaatgtgt gtgcatattt gtggtggtga tcaaagcatc cctcctcatc cgtcgctgat 240
   caaaatgaat ggaccgatca gacgaccggc ttctgcggcg gctcgatcac cttcagctcc 300
   ggctcgtcct tgggcggctt tggcgccgac gactcgggcg tccacccgcg gccgggcagt 360
   cgccggaaga tcaccttctc cgtcttcatc agtgcccaca cattgcgctt ggcgtacagt 420
   agcacaaacg tgagcagtgg caacagcgtg atcaactgag tgtgtgtgta tatgtgaaaa 480
   tttttgaaaa aatgaaattt tcccggctgg taaggcacca gcgaagccat caaaaggatc 540
   tggtgccgat gaaaaaaaat tttttttttt tttttggatt tgtagggtta taaatttgcc 600
   gattttcgag gcaataaaaa agaaaaatgg gggataacac tttttgaaat ctatcaattt 660
   attggcaatt ttgcaaaaaa aaaaatcctc cggcagtgct aaccggcacc gttgaacggg 720
   tgcactcggt gtacccctga aacggctccg ctgaaatgtg gcgggacaaa acttcgcgaa 780
   aaacattttt ttttattgcc aagcctaatt cggaaagaaa atttcaaaga aataaaaaac 840
   aagtttgttc catgaatttt tgttttcaaa aaatttcaaa acaaattttt gagtgccggc 900
   gacgctacgg cggcaacgtt ttgttcagtt tgaaacgttt ctcgagtctc gggaatttag 960
   gtggatgtag agcagtccaa taaacagagc atcccatctt tgccctcccc ccggggtacg 1020
   aattccggac ttgttgttcc cccggaattg cagattccgt tttttttttt atttgggggg 1080
   cctagaggtc tacgaaacta taactctggg gtaagtgcca accaccgcaa atgacaacgg 1140
   caaattggct gaccttccat gcccagcgtt tgcgcgtgtc gtgccattgt tcgccggccc 1200
   agcgcaagaa ggtgcaaatg tccttggcct gttgcgacat tgttgccggt gtgccatcct 1260
   catagtcaat gccttcgtca aacagttgtt gcggcatggc caacacacag ccgttgggga 1320
   agtacgggtt gaacgccttg ccctcctcca cgtcaatgcc cgccggtgca tcaaagccat 1380
   agctgaacca aacaaaatgc aacaatggga aattttgggg aattgaaaaa acgaggaatt 1440
   aaatttttat tggaaatcaa agtccaactt ccccatttcg tccgtatccg acaaacaccg 1500
   caaatgctat ttaaatgcga gaattttaca ggattttgaa aagaaacaat tcaacaaatg 1560
   aggggggggg gggggtagat tctctctgac aatgaaatca tcaagagtct gatcacaatc 1620
   tgagtattgt aagcatcttg atttcttgat tcactgacag gtgtacaaaa aacaaaaatt 1680
   taaaaaaatg gtcgaatttt tggaaaattt acctattcaa taaacagtaa ttcttcataa 1740
   aactaaaaaa ttcgtttgaa attttttcaa aaatctcgcg tatttttcac tagagtaggt 1800
   cgggaccggg aattttgggc aaaaccggga ccgggacttt tcgggttgaa aacccgggaa 1860
   tttttggctg ggaaaaccgg gaaaaaaccg ggactagtcc cggtcccggt caaaaccggg 1920
   acttcggtac tcagttgtcc gtgttttggt cccggtcccg ctctactcta tttttcaccg 1980
   tacgactaac tttttcgctg ctcttccagt cctgggttgg aaattttttt ttaatttttt 2040
   tgatttttgg tgggtctaaa gaatcttggg acctccgtca ctgtacacta cgctttttta 2100
   aaaatgctga caacaacaaa caaacccggt caacagatgg aacacgtagt cctcggcccc 2160
   ttcccgggcc cacataatca gtgacaaatc gggtggcaat gcgccactgt tcgccgccgc 2220
   ggcagctttg ttgtttttgt aggggcttgg caacgtgtcg ttgagcagcc cgggacgaag 2280
   aatttccaca ccattatcgt ccaagtccgg gatcagtgcc tgaagggaat gagttttttt 2340
   ttttgaaatt ttcgccattt ttaggggtta aaggccccga cccagtgcgg ggggttcgaa 2400
   ttgtaagaca tatttgtttt agaaaaattt tgtttttatt gtttttcgac tttttatgaa 2460
   atttactgct tgatgaggat gttgcaaacc gaaattttta aatttatcca gcgatttccg 2520
   acaaattttg tcgggaaata ttcctccccg atacccgact tttcatatca agcagagtgt 2580
   caaggcttgg ctctacattg aacatgattt atccaaaact tttggctccg tgttatcatt 2640
   tagtatttga taattcaggg agattttgtt gtatttagct tgttaaattt ttgaaaacta 2700
   aaaatcgtta ccaaaaaggt tgaaaaccct gattttttaa aattaaataa acccataaag 2760
   aaccattgag acattaaaca ttttacggtt cgtgcaacat gttaaaattt aaaaaaagac 2820
   tcaaaaataa tgctcaaatt ttgcaaattt tacggcaaaa aaaaaaacct cggcagcctc 2880
   cgtcttcgcc tcatcccgtg tcatgaaatt gtcgacaaaa tggttgtaac ggatgaattt 2940
   caaggaatgg catgcggcac acacttgctt gtacacttgg taaccgcgac gaaccctgaa 3000
   aaaataaaat aattcataaa aaacatggag gggttccaat tttgggtatt atttgaggga 3060
   ccaaaaaact catccataat ggttttgctt gcatatatcc accaaactaa atagaaaagt 3120
   gcccttttta ttgcgaggta aatagaaagg attgatttca acggtttctt ggacgacgag 3180
   ttttttttaa tttcaggcgt tgattaaaaa aaaaaaaatt tttttttttt aaatcgggcc 3240
   aaatccaaat tttgtttagg caattggaga cattacggtt gaaaacttgg cacattaaaa 3300
   taaagatgtt aaatgtggaa caagaaaaaa aacggacgat cgcatgtcaa ttgcgtccat 3360
   cagtccgccg tgggaccatg gcaaatggct cgggtgcaca acaaagtcgt aggcacgcgc    3420
   accgctattc tccaacgagt acagcaacac actgccaccg gtggccaatg cgccgaacaa    3480
   cacaaatttg gatgctttac gctgaaattt ggcgaaaatc aggcggagaa attcaaaaaa    3540
   aaatttttgt ttgtttggct taccaagtta agccctaatg ccgctgctga ccgaaatgac    3600
   ataatttaga aagaaaatat tggggtattt tgttcggtct aaacatggaa aaagaggtaa    3660
   taatggcatt aataaaattt ttggcgctat atggaaagtg accagttttg aaaatttttt    3720
   caaaaggggg gacccttttg ttttgcaaaa tttttttaag atagtcataa aaatgtattc    3780
   tctggggttt ttgggg                                                    3796
<210> 514
   <211> 3395
   <212> DNA
   <213> Heterodera glycines
<220>
   <223> genomic virtual cDNA Sequence=SeqID_553; coordinates=669..708 ,755..883,927..1076,1125..1327,1393..1576,1979..2218,2281..2518 ,2569..2706
<220>
   <223> coding sequence=SeqID_806; coordinates=669..708,755..883,927..1076 ,1125..1327,1393..1576,1979..2218,2281..2518,2569..2706
<220>
   <223> peptide sequence=SeqID_1048
<400> 514
   atggactgcg actcatcgat ggcattttgc cagtggcatt tcgatgacca cccaatggcc 60
   cgggggaagt ctctcttcgc ggataggtgg cacattgtgc cgagcttttg tgtgccattt 120
   cgttttgttt tatttgcttt tttttagtat ttatcctcaa tgtttcaaaa atttaagctg 180
   atgcaaattc gtcgttcccg agtttgtttt tgtcatttgt tctaatgcca accgaaggag 240
   aaggaaaaag tcagaaaaaa gcggccaaaa atttgtcaaa cgaagtgagt ttttcagaga 300
   aattgaagcg gaggatgaat aaataatttt ttgtttaagt aaaaagtgaa ttttgtccaa 360
   tttggcaata tttaatataa cattactgta agagggaatt tatatatttt tttttcgaaa 420
   cgaaaccttt cataaaaata attttttatt tgattttcgg tctcaatttg tactttttat 480
   ttgattctct ctcccttttt tgttttataa gttttatatc aaattttcat ttgaagctca 540
   taaattttcg tttttagcgt caaaaagtta aacttttcct caacccgcga caaaaaatgc 600
   caccgacgac aagagaacac attaaggtat ttttttccaa actagtattt ccaatctttt 660
   tctgccagga atcccaatat ggctttgttt acggcgtttc gggaccgggt tggttttatt 720
   tcgcctcatt ttttgataat tttttacttt taagtggtaa ctgctgagaa aatgtccggt 780
   gctgcaatgt acgaactcgt cagagtcggc cattctgaat tggtgggcga aatcatccga 840
   ttggaaggcg actacgccac gattcaagtc tacgaggaga cttgtaagct cgtcaagttc 900
   gataagttca ctcgtcctcc gttaagcgtc cgtcacaatc ggtgaccccg tgctgcggac 960
   ggggcagcct ttgtctgttg agcttggccc gggcattatg ggctccattt ttgacggaat 1020
   acaacggcca ttgcacgaca tttcagaaat gaccaaatcc atttacattc cgaagggtaa 1080
   aatttgtgat tttattccct tcgaatttta atttgacatt ttaggggtca atgtgaaggc 1140
   attaaaccgt gcaaaacgat gggaatttat gccgaggaaa gatttgcgag ttggggacca 1200
   cgtgacgggc ggagatatca tcggaatggt gcacgaaagt gcgttggtca agcacaaaat 1260
   tatggtggag ccgaaggcgt gcggaacggt caaattcatc gccccgcaag gccaatacac 1320
   cgtcgaagtt ggcatttgga cgaaatgcca aacttattga ctctttcccc cccccccccc 1380
   ccctccacaa aggacaccat tttggaagtc gaattcgctg gacacaccca aaagcttacg 1440
   atgatgcaca tttggccagt cagaaacccc cgcccgtccg ccgaaaaatt agcggccaat 1500
   ttccctctcc tctgtgggca gcgcgtgttg gacgcgctct tcccgtgtgt ccagggcggc 1560
   acaacggcaa ttcctggtaa acagttattt ttgctctcat tggcacttcg gcgtttttct 1620
   attttatatt tttttaaatt ttcaatctat tttgttggtc taataaaact ttgtgaaacg 1680
   tgctaataga gaccgaaatt ttgaaatgac cagtacttgg aaaagggacc gggacgggat 1740
   ccccccgatc ccaatttttg gagcggagcc cgacgagact tttcaaaaac gatgaaaaaa 1800
   tgtaataact atttttttaa cttttctccg acgatgatta gggagagtaa ttccatattt 1860
   gcagtgtaat tggtcgcttt tcaaattttt gctccgttag ctctgttttt acccaatttc 1920
   cttataattt ttcttcccca gtcttttaat ctgttgtcat ttgtccgtcc tttttcaggt 1980
   gcctttggct gtggtaaaac tgtcatttcg cagtcgttgt ccaaatattc caactccgat 2040
   gccattattt acgtgggctg tggcgagcgt ggcaacgaaa tgtcggaagt gttgcgcgac 2100
   tttcccgaac tgacgatgga agtggatgga gtgaccacgt ccatcatgga gaggacggcg 2160
   ctggtggcca acacttcgaa tatgcccgtg gcggcgcgtg aagcgtccat ttacacgggt 2220
   gggcactttg ccaagactgt atttttattt ttcgttaaat ctcccctccc ccttccgcag 2280
   gaattactct ggccgaatac ttccgcgaca tgggtctgaa cgtggccatg atggccgact 2340
   cgacctcccg atgggccgag gcgctccgtg agatttcggg ccgtctcggg gaaatgccgg 2400
   ctgactcggg ttaccccgcc tatttggccg cacggctcgc ttccttctac gaaagggcgg 2460
   gaaaagtgaa atgttttggg tcgcctgaac gcgaagggtc cgtcactatt gtcggcgcgt 2520
   aagtgcatgt cgaccgatgg cactttccct tcccttccct ttgctcagag tgtcgccgcc 2580
   cggcggagac ttcgccgacc ccgtcacatc ggcaaccctc ggcattgtcc aagtgttttg 2640
   gggactcgac aaaaaattgg cccaacgaaa gcacttccct tcaatcaatt ggctcatttc 2700
   ttacaggtgg gccaaattgg gaaaaagtga agaaaaagga aagggtggac tttgtttatt 2760
   acaaaaaaaa gtcaaaaaca atggttgtgc gatatcatcg gccaacactt tacggacatt 2820
   agctaatctt tcgtcaataa ttgcgtcctt tttcgtccgt tctgtccctc cctttgtctt 2880
   tttctgtctc aaaaattgct gaactgttcc accggttgtt cctcgctgtg cgcgagttgc   2940
   gcgtgtcgcg ttgcctccgg caatgcgtaa ttttcccccg gcgcgtgctg ctgtctgcac   3000
   ctctgtcttt ctctgttttg tgctgctgcc cttcccttta tgcagccgac cattgacgac   3060
   agacaaacgc actaaacagc accttctttc tctgctttgt ctttgtattt tagggtccat   3120
   tgcttttatt ggacctttga ccctccgctt ttattctcag ttaattctca ttacggctgt   3180
   ttgattcgac tcactgattt gtcccctccg ctcatataat cgatgtattg gtaccaaatc   3240
   accatgaaac caatcgccag aacattgaac agaaaacaga ggacaaagcc tccctcaatt   3300
   gcgcttattg tcaccaaaac tttccagtgg aattcggtct gaaaattgca tttcatcgcc   3360
   gtcataaatt cacaaaatga tttcacctga tagaa                               3395
<210> 515
<400> 515
   000
<210> 516
<400> 516
   000
<210> 517
<400> 517
   000
<210> 518
<400> 518
   000
<210> 519
   <211> 3041
   <212> DNA
   <213> Heterodera glycines
<220>
   <223> genomic virtual cDNA Sequence=SeqID_559; coordinates=2870..2753 ,2689..2444,2348..2235,2089..1982,1939..1776,1623..1374,1318..1137 ,1082..981,928..843,783..536
<220>
   <223> coding sequence=SeqID_789; coordinates=2866..2753,2689..2444 ,2348..2235,2089..1982,1939..1776,1623..1374,1318..1137,1082..981 ,928..843,783..668
<220>
   <223> peptide sequence=SeqID_1033
<400> 519
   ctgttggtgc tgctgttgtt gaacctgaga caccggaatt tggcccgtca aaaaattttg     60
   aagcattttt aaagatagaa aaataaaaat atttttgccc gtctaagtca caaagtttag    120
   agagtgtaag taagtgtaag gcaattggat gaattaaaaa ggaacgggga aaatggaaaa    180
   taaaaaatat atattaaaaa gagggattga ttgaaaagcg aatacgtaaa aagtggggat    240
   gaatgaggaa aagaagggcc aaagaatagt gagtacaaag aagaaaggat aaacactcgg    300
   atggtcaatg cgataatcgg gcgtggccaa atgagcagtc atcaagaaat aaaaatctat    360
   gaagcaagtg gaacatatgt tgtaaaagtg aaagagaaaa agcaactcaa gaaaaagatg    420
   gaaagggtag agatttattt tattcaataa aacgaaatga aaaaaacaat gaaattattg    480
   ttgatgtagg aagggaggtg atgagtcgct aaattaggat aaaaaatttt aagggaaaag    540
   tacagaaaat cacaacacga caaattaatt aacgaaccgg cgaatgctta aatcgtaaat    600
   gatgccaaaa tttacaatgt ctgactacag ctgtgtaaaa agtacagtga aattggttcc    660
   actattgtta ggttcgcgtt ttcacttcct ttgcttcttc ccccgcagcg ccttttgccc    720
   cttcgagtcc ttcaatttgc ttgccaagat attcccaatt gtgcaccatt aacttttgga    780
   ttgctgaaaa agatgtttaa ttatctggcc acacgcatag ttctcttatt atccctactc    840
   acctaacaaa gcgtggtacc gaacgtttgg gtcctctgca ctcaacaact tcatcactgc    900
   ttgtttgcct tggtattgtt cgacaattct aaataaaatg aaattgtttt gttcttctga    960
   aattttgcgg taggataaac gtttttccgc gatggtaatg ccggacgtat tcgccaatgt   1020
   catgagcagc aacactgagc ggcaatgggt cgctgctggt ctcgagaatt ttgatgagta   1080
   ttctgagaat ttgtttttgt tatttataag ctttataaaa aaaagtaata atccacttta   1140
   tcaattcaaa gttcttttca ttgaagcgtt gtgcattttc gcgccagaat ttctccaatt   1200
   tatgcaccgg actccactgg agtctgcccg atcggacctc ggcacagtat tcgtcaaacg   1260
   aactcaaatc ctgcaccgag gcgtgaagac gttcagacag aaattcaatg tcgtccacct   1320
   gaatttgatt taatttccca tccaaaattg ccaaaagtta tgcagtctct caccaagtcc   1380
   gggtcgtcaa actttttggc gtccatcaat tcaagggttt tcagcgtttt gcattggacc   1440
   atttgcagtg ccgattcgcg caccatctct ctattgtcaa ttttctccaa caaatttcgg   1500
   aaggtcgcga gaatgatacg aatcactttt tcctttgagc attcggagag aatatcgccc   1560
   aacggttgga taatgcccaa tccctggaca aattgagcga tgttactgtc gaaggtaagg   1620
   cacctgaaca gcactttgaa tattgattga cataaatatg tcgggcattt tttaggtaaa   1680
   ttttttttta tcgtcttact aaaatgggga aattaaatta acaaactttt ttcaggatta   1740
   tttttgggtt aaaagtgcga aaaaacccaa ccgaccaaag gcaaaaaatg agttggtatt   1800
   gaagttgaaa attggcacga ccattcaatg ccaaaagaat gcttttaacg ccgtcatact   1860
   caacgaattt tcgtcgatac ttttcctttc gtaacatcat ttgtagacat cgaactgatg   1920
   tatttatgta ttcgttgctc tattgaaaaa tatttttaat agcattttag gaagggcaac 1980
   caaccgagtt ttttatttcc tgttgaagga aattgaaata agcttgcagt tcttgtccat 2040
   ccataagcac agagccatag caagccaatt ttgagaatat tgcagacatc ttcagaatgg 2100
   aaaaaggtta atgataacag attataagca cgacactatt aaggaaattt tcatgattaa 2160
   acattgagac aaaaaatgaa atgaacgagt cccttttaaa atttctaaaa ccaatttagg 2220
   aaaactaaac tcacttggtt gacaatgaaa ctgtcatttc gcgttaaaat cccttggaac 2280
   cacgaccaaa tagttcgttt ttgccgtctt gcataatcat ggaaaatctc tacccgggat 2340
   ttgtcctcct gaaaacaacc aagaattaaa ttaaatatat ttaaaaataa attaaattca 2400
   ataaaaaacg attttaatta aagtttaaat ggaaatgaaa gacctgcaaa aggtcgtcga 2460
   aaagggtgag aacgtaacgg atcagctgat ccttcgcaac ttcggtgatc aaattgataa 2520
   tactccgtgc cgaattcgtc ttgtccttgg ctaacgcttc gtccctctcc tgcttgtttt 2580
   tggccttttc caattcaata atgaaattgt actcattttg aggcaccaat tgagagcgca 2640
   aatacgatga ccaattcggt ttattcgccg gatttcgcac ttcggcagcc gaaaattaag 2700
   ttttcattga ataaaattta agtaaaaatg taattggaat caatgaacac acctcaagct 2760
   gaagtttgct ggtagctgtt gtcatgtcca caggcgtaac gtgatgccca gccgcaattt 2820
   cctcaacgtc aacagcaaaa gtagcgccat tctgttccgt aaccatttct aaaattgaag 2880
   tgaaagaaaa acaatatttt tgaaatatga attgcataat aataaaaaaa atgaattttg 2940
   ccgatcaaca ttaagaattt tgctaaccga aaaatggata atcagagacc aaaagttact 3000
   tttaaaaagg cattcgtttc ggaaatttaa taattttaat t 3041
<210> 520
<400> 520
   000
<210> 521
<400> 521
   000
<210> 522
<400> 522
   000
<210> 523
   <211> 2986
   <212> DNA
   <213> Heterodera glycines
<220>
   <223> genomic virtual cDNA Sequence=SeqID_564; coordinates=2..38,93..310 ,542..703,1004..1150,1207..1242,1289..1609,1859..1945,2013..2083 ,2142..2179,2263..2366,2475..2604
<220>
   <223> coding sequence=SeqID_805; coordinates=2..38,93..310,542..703 ,1004..1150,1207..1242,1289..1609,1859..1945,2013..2083,2142..2179 ,2263..2366,2475..2528
<220>
   <223> peptide sequence=SeqID_1047
<400> 523
   ccccaatgag acggatgaca aaaccaaaga aataaaatgt gttttttgtg cttaacccaa     60
   gtaaaaccca tcatatttgg tgtttctttc tgtttcagcg cttgacgagg atgaaattgc    120
   cgcacttagt aattacaaca tgggaccgta cgcggatcag ttgaagcagg cggagaagga    180
   cattgatgaa attcagaagc gcataaacac tctttgcgga gtgaaagaga gcgacacggg    240
   gctggcgccg cccattcttt gggacattgc ggccgacaaa atggccatgt cccatgagca    300
   gccgctgcag gtgaatttta ttttgatttt ggtcaataag acttgtttaa aatcttaaag    360
   atgagtgaaa atgaaaatag tttaaggaaa aactgaaaat gctaaatttt aatgttaggg    420
   attaggaaat ggtcaaattc ttggtgggtc aaaaatatcg aaaatttaca ataataattc    480
   gcgacgctcc accttaggca aatgttgcat tcaatacaaa ttaaaatatc tttttaaaaa    540
   ggtggctcgc tgcacaaaaa tcatcaaaga agagggcaaa gaaacgcgtt acatgatcaa    600
   tgtgaagcag ttcgccaagt tcgtcgtgga cctgcacgaa aatgtggcgc ccactgacat    660
   tgaggaggga atgcgagtgg gggtggaccg caacaaatac caggcaatgt gcacattaat    720
   ttatttcaga atttgcgttt cacagttact taaacttttc tgtgggttgg accattttta    780
   agggatttga caaaatacgc catgaattga ttttaaaaat taaaaaagtg ggcatttgac    840
   taattgtaag taaaggcaca acaaaatctt cctttaaata agagagcagc atcttgatct    900
   cccctccccc gaaaattttc cacaaaattt tctgatgtat tctaagtttt tcaaaaaacg    960
   atttctcacc atccatcggc ttacaaaaca tttcattttt aagattcatt tgcctttgcc   1020
   ggcaaagatt gacgcgtccg ttacgatgat gcaagtggag gacaagccgg acgttaccta   1080
   cgcggacatt ggcgggtgcg aagaacagat caaaaagttg cgtgaagtgg tcgagtttcc   1140
   gttgcttcag gtcggcaatt caatctctgt ttttgttttt gttccatttt gcctctttat   1200
   ttttagcctg agcgtttcac gagtttgggc attgagcctc cggtgcgttt tgccacacct   1260
   tgggattgtc caacgttttc catttcagaa gggcgttttg ttttttggtc cgccgggcac   1320
   cggcaaaact ttgtgtgccc gcgcggtcgc caatcggacg gacgcgtgtt tcatccgcgt   1380
   catcggttcc gaattagtca aaaaatacgt tggcgaaggc gcgcgcatgg tgcgcgagct   1440
   gttttcgctg gctaaaacga aaaaggcgtg cattctcttc ttcgacgaag tcgacgccat   1500
   cggcggagcg cgatttgacg acggaaaagg gggcgacaac gaagtgcaac ggacgatgct 1560
   cgagttggtc aaccaactgg acggattcga ctcacgcggg gccatcaagg tgggtggggg 1620
   gatgtgtctt ccattttggc ttgaagggga aggcaaaggg ggaggtcaac cgaaattttt 1680
   gcgaacgggt ccggtctttt aaatcgacga cttttagctt tttttgcggc gccctttttg 1740
   tcaaaaaaac ttagggaacg ggcaggggac ccggcggtac caataggcgc agccgtggat 1800
   taattattga atttttgatg aattgttaag aaaatttgtt caggatttct aatttcaggt 1860
   tttgatggcc accaacagac cggacacact cgacccggcg ctcattcgtc ccggtcgcat 1920
   tgaccgacgc attgaatttt ccttggtaac caatgaaaat aaatgccgat ctgacaatga 1980
   gtacacacac tgctcattat gaatcttttc agcctgacct caaggcacga ggaaacattc 2040
   tccaaattca caccaaacgg atgagcgtcg accggaacat tcggtcggtc attgtttttg 2100
   acatacattt aaaatggagt acaacatgtt cggtgtttca ggtacgaatt gattgctcga 2160
   ctctgtccaa acacgacggg tgaacgaacg cccattaaaa tgaaccctct tacaaatagc 2220
   gattgcttaa ttaaccagaa aaagtacgtt ttgctttttg aggtgccgac ttgcgcagcg 2280
   tttgcactga ggcgggaatg ttcgctttgc gtgcacgtcg aaaggtcata acggagcaag 2340
   actttctcaa ggctgttcag aaagtggtac gttttccttt tgattgtgcc aagtggattc 2400
   ttttatttgg cttatccaaa gtccttcgtt tgttcgttct ctttcgccct ttgtatttta 2460
   cgttttaatt taaggtgaaa agctacgcca agttcagttc aacgccggcg tatatgacgc 2520
   acaactgaca atacagttct tacaaaacgg acttttttat atttgtgcac ttttgtttca 2580
   ttacaatata aatgaggaaa ccaattccgc agaatgtctt gttaacaagg actactttta 2640
   cggcttttgg aaattcaagc gtaaaaattg agtggcggtt tcgtaaatgc cgtgttaata 2700
   atgcagtccc ccccccatgt gtatgcggaa aaaagtggtt tgtgcccagg gatggcacgg 2760
   aattccgtct tcctaaattt tattaagtag taaagctaaa gcaccaccca aaacaattga 2820
   ccgataagtg tttaaaattt accaataaat aaataatatt caaagggaag taactgtcgt 2880
   ctttggtgcc gtttcttcat ctttcgctcc gtcttcgaac atttgaccca ttgcgttcag 2940
   ccgagcgtag cattccgcaa cgccgaagaa ttcaatcagt tctttg 2986
<210> 524
   <211> 3451
   <212> DNA
   <213> Heterodera glycines
<220>
   <223> genomic virtual cDNA Sequence=SeqID_565; coordinates=3450..3418 ,3349..3248,3029..2900,2457..2222,1770..1558,1374..1249,1145..1021 ,957..835,583..492,371..165
<220>
   <223> coding sequence=SeqID_816; coordinates=3450..3418,3349..3248 ,3029..2900,2457..2222,1770..1558,1374..1249,1145..1021,957..835 ,583..492,371..165
<220>
   <223> peptide sequence=SeqID_1058
<400> 524
   tgcgttcaca gttctgtaaa attttaaact tgcttggatt tttctgtaaa atctaaaatc     60
   ttttggagtt agacaattac gggaaacttt agcactttta attgggtagt agtcaatcaa    120
   gactgatgaa ttgtagtgct cgttggcatt ggcatttccg tcccatgccc acccactcac    180
   ttgccagcca aacaccaatt tggacagctc acggcccgac atgccggcag tcagcgaggc    240
   gatttgcttc attttggaca gccagtcaaa gtcgcccagc ttcaggcgtt gggtccgtgc    300
   cccggaggtg gccgggtcgg caatgtactg cttgaagtac tggagcagta gacgttcacg    360
   ctccagctca cctttggaaa aaaatagaaa attttaggca tcacaaaaat ttcgtgaaaa    420
   tttttcattc gttgattcga agtgtgccaa tgccattcgt aaagctattt attcctggga    480
   tttccactca ccgggcagtc caaattcgac aagttcgtcc agacgatcgt tgacagccca    540
   gtcgaactgc tccggctggt tgctggccac taccaacatg aacctaatgg aaaagccatc    600
   tttaataaag atattcaagt aaacaaatcc ttaaaaagat ttaaaaaaag catccccagt    660
   tttcaaacta agaaactttg caagaaacct taaaaagttt tacaaatttt tttttcattt    720
   ttcatagaaa tcgtgttgtt tgaattttct ttttaatttt agggtgatgg aaacgagctg    780
   cccctaaaaa ttgtggcagc caaataattc gtaaatgaag caccactgac tgacttcctc    840
   gactgttccc cggttctgaa gaggaacgcg ttgagcatgg cgcgcatgtc ctcgctgatc    900
   tgttccgtgc ttcgcttgcg cagaaaagcg tccgcttcgt ccacaaagag caccaaactg    960
   acaaaaaatg caggaaaatt attcatttga aaaacggcta attaatttca aatggcccac   1020
   cctttgcgcg acgtttgggc ccagtcaaaa attttatgaa ttgcggacac tccgtcacgg   1080
   cccatcggtg ccacgtcacc gccggtcatt atggcgtagt cgagacccga atggcgagcc   1140
   aaactctgat aaacaattta aaatattaaa ataggggaat gaatttgcat gaccccatag   1200
   aaaggaggta aaatttccca agaaaatgca acaaaatggt ggaccaacct tggcgaacat   1260
   tgtcttcccc gtgcctggcg gtccgtagaa caacacattg cggaacagtc caaagttacg   1320
   ctttgtgttc ctcgtggtga tcgcaatgtc acgcagacgc gactcgaggc ttggctaaaa   1380
   caattctgta gcaaaatttt tatttattac aacaaatgac catttttaga ggacattgat   1440
   tcaattaaga tttaaaaata gaaaaatgtg ggtcggaaag catcgaaagg cactgcccgt   1500
   gggggggggg gggggtaaat ctctccataa agcacggtaa atgatttgcg cactcacatt   1560
   gagcacaatg cccttcagcg ggtcctcgcg ggcacggaac agtgtccgca cactcgcaat   1620
   tggatgtttg aacagctcga acagtgtgag gcgggacgtt tcacgcacca gggacggctt   1680
   accaagccgt gcttccacct gtttggccac aacgccagtg ccacgcttgg cactgtacca   1740
   accgaccgcc agcacagtca gaccaccgac ctttttggga ggaaggaggg gtcatttaaa   1800
   aagtggaagt atttataaat tgcagttata aatatagttg ttactgtatg taaaattcag 1860
   atatgggtat tgtaaaatag taaaaataat tttataaaag aaatggaatt aaaattgaaa 1920
   actcatagtc aaaaaagtca ttctaaatca cattttatct ccaaaatcaa aattctaaaa 1980
   gccattacat atacaaaagc tcatttttca acaaaatcgg atacaatttc atcaagtcga 2040
   caataaaatt gaaaattagc cctttcaaaa ttgaaaaaac tgaaaatgag taattgtttc 2100
   acattctttt tgctagcatt tgttaatttt ttttctattt agataaaacc ataaaaaacg 2160
   gaaaacattt ttagataaaa tacaaatata attaacaaat tgggcataaa aaagcactca 2220
   ctgccgccat aattttggag cgatcggaga gaaactcgga cagtccagcg cccacaacgc 2280
   cggcggttgt ccgaatctgt tccgtgaccg tcttgcgtcg ctcctcctca ctcgcacgca 2340
   tcatctccag gttcacgtca cggttgtcgc gtgcggcacg cgccttggcg cgcacgtccg 2400
   cgtcaatgcg ttccaagtcg tatttgtgcc gtagcgacag ctcatgctca atggtcgctg 2460
   caaaaatttg tggtttttta caaattttta cgaaacttat aagcaacatt tttcagggga 2520
   aaatctaatt aaaaaatttc tttgacagca ccgtagtgaa acttttttat aggttaggtt 2580
   ttacgcaatt tgttaaaatt tttgagggag aaacattgga aatagcattt tttggttcaa 2640
   tttcttacca aatttcgtta aattttgcaa aattgccacc gacaacgcct atcgttacca 2700
   cggacgacaa aattcccgca aaattttcag acccaaaaat gcataaaatt tatttttcag 2760
   tccccaaaaa atgcacaaaa tttgtctttc tgtcaaaaaa tgctacaaaa tttggcttat 2820
   agcgtatata attttaaaat tcaaaaaaat ttctggttac gggcctgccc tttacacaat 2880
   gcttttagac aaaacccacc ctttctgagt gcctcctgct tcttcaccga ctcctcctgc 2940
   ctgcgcaatg tttcttcctg cattcgtgcc ttcatgctca tctcctcctc ttgtcgcttc 3000
   cgtgccaatt ggtcctggta gtcggctctc tgcaattttt ttaaaaatta aattgccaaa 3060
   ttttttatta ttttggtata aaaccatgga attgctgaat ttttatgaaa aaaattaaaa 3120
   ttaaaccgaa aacgggaggc taaaattaaa tttttttttt ttttcatcca taggataccc 3180
   ctccaaccgg ataaaatatc gaaaaaaacc caaaaatttg actgattgag tgttgtgctg 3240
   cacttactgc gcgcgcatgt tttgtctcct caattaacgt tttgcgcctt tcttcctccg 3300
   caacgcgcac attctccgat tgcatggccg caattcgagc ctccaattgc tggaaaaaaa 3360
   taaatggaaa ttctgaattt ttggacgggt gcatttttca gtgatattgc gactgacctt 3420
   ctgctgcaaa tcgtactcct tctgcttggt g 3451

<210> 525
   <211> 3201
   <212> DNA
   <213> Heterodera glycines
<220>
   <223> genomic virtual cDNA Sequence=SeqID_569; coordinates=725..844 ,889..1015,1574..1695,1752..1793,2207..2386,2438..2566,2616..2807
<220>
   <223> coding sequence=SeqID_797; coordinates=749..844,889..1015,1574..1695 ,1752..1793,2207..2386,2438..2566,2616..2675
<220>
   <223> peptide sequence=SeqID_1040
<400> 525
   tggcatttcc cgccaccgga gcagtcgccg aattgttttg ttgctgagca taattgatga     60
   aactttgctg ctgaccgttt gctgaagcgt aagcgccgta attgaattga gtctgctgat    120
   atccgaacat tctctgctag tgtcaatttg gcgagcacaa taaaaaagga aggctaaaaa    180
   taaaaaaaga ataaaataca ttaaagctta ttatatgtaa tccaacaaat ttgattaagc    240
   taaaaatgag atttttttta caaaatatgt aaaaagcagc aggagaactc ctcttgcgac    300
   agtcctctat tgttgtaatc gataaaaaat aatcgctcta atgacaattt tcgttaattt    360
   ttttcaaaat aatcatgaaa tttatgaaaa attttggcac ccccacaaag tgtccaagcc    420
   acgaaatgtc caaaagagta aaaacccacg aaatgtccaa aatgtttcaa aatttataaa    480
   aacaaaatga gtctaattta ggatttttag ggttttcggg gtcgctgaat acgaatatac    540
   cctttatatt accccttgcc taaccttagg cccatctgaa taataaaaaa ctcaaattcg    600
   agacttggac atttcgtggg ctggacattt tgtgggtcag cgaaaatttt atttgttaca    660
   acgtttctac ttttacatct gaatctataa gtactacttc cctattttaa aataataatt    720
   attaagtgaa ttttcgactt atctataaat gtttctgacc cgcagcgaat acgacagggg    780
   agtgaacact ttttccccgg agggccgtct gttccaagtg gaatatgcca tcgaaactgt    840
   caaggtgagt tggaagaatt caattcagat tcaccataat tttcatagct tggttccaca    900
   agcatcggaa ttcacaccaa agaaggcgtt cttttggctg cggaaaggcg ttcaatgagc    960
   aaattggtgg tggacgactc aatgagcaaa atttcggaag ttgagaagca cattggtaag   1020
   gcccattttc cacaatatat gtatttagtc aaatgggtac taatgtcgtt tacagaccat   1080
   aaaaaatagg gtaatactgc cgatggcccc cccccttttt ttggagaaaa attcttcctg   1140
   ttctaaaaag tcgtgaaaac acgttatggg ctttttaaag tcgctgatca cgaactgaca   1200
   ttcattttga ccaatttcca atccaggacc cgaattcgaa cataagtcga gaattaattt   1260
   taccaaaatt cacttttgtt agaatctgaa cggaattcag gtccggggtt cggatgcaat   1320
   ggtttatagt taattttttg cttttttaat ctattttaga acataaaagt gaatttgaaa   1380
   ttttttgagt tttcgacttt ttgttcgagt tcgggtccgg ggttcgaaat aggtcgctaa   1440
   tggatgccgg attcatgagc agcgaccctg atttatttta atggtctgta aacgaaataa   1500
   ataccgtcaa ttgttacaat aaatttgttg ttaaattatt tgatcccctc ttttttaatc   1560
   aatttgtgtt tagccgtcgc ctcggccggt ctcatcgcag attcacgcac ttgggtcgaa   1620
   cacgcgcggg tggaggctca acacttttgg tttacttacg gtggcaaaat tcgggtggaa   1680
   gacattactc aaaaggtgga taaggaaaat taaaagtggt cgttttcatt ttcgttttat   1740
   ttttctttca ggtctcaaga ttggcactgc attttgggga cgacgactca actgtatctt   1800
   ttttcaaatt tgattttcac ttaaaaatca tgtttcaaac atgtttcatt ttaactattt   1860
   tgattatgtg ggtcaggctg ccccaagggg gtggggagag atacgtaccc cccccccctc   1920
   cctccccaag accacttatc cctcctaaaa ttctgaaggg attttaaaaa attttccttg   1980
   atagatacca atcgaaagcg ctggtccaaa tccctcaacc tttttcaaaa atacaaaact   2040
   catctttttt tacaaaatct gataataaag ttttactgcg ctattatgga accatcggat   2100
   cacttttccc atttagaaag tctttttgtt ttaaattttt gcaaacactt ttttgcttaa   2160
   tcatttctct tccattggca tttttccaat tcattcttgc tttcagatca gtctcggccg   2220
   tccgtttgga gtttctatgc tttttgccgg cattgatcac acgggtgcgc atctcttcca   2280
   tttggacccg tccgggacgt acattaaatg tttggccaag gccatcggtg ccggctccga   2340
   tgcagcggaa caaacgctgc aagagcactg taaaaactgc gacaaagtga ggaaaatgca   2400
   ctcattgctt ttcacatttt atcattatca ttcgcagaaa atggaaatgg ccgaggcaaa   2460
   acaagtcgca ctgaacacac tcaaacagct gatggaagag aaaatcaatt ccaaaaatgt   2520
   ggaaattgtt atgattaagc cgcagacgga caaggaaggc aaaacggtag caattggaag   2580
   aaaatgcata ttttgttatt tattcccatt ttcagttggg caaaattgtg tggttagagg   2640
   aatcggagtt gcaagaaatc atttcgcgat tgtagtcgaa ggggacggat gagagaagga   2700
   aaatgggctt tgcactgccc cttttatgat tggatgacct tttgttattc tctgcctttt   2760
   tgtgactttt tcagtgtata aggcaaatga aagcaattaa ttgagaattt ggctaatatc   2820
   caataaatgt ttcaattctc tgctgaggca cagtgtccgc cactgactga cactaatgac   2880
   aagagtttaa cgatgcgtag cactaataaa ccgtatttct cacacaccac acaaaatttt   2940
   catatgagga ggctctgatc gctcccaaaa aaagacatta aaaaacaatg tcagatgata   3000
   aggcggagtc aatcagtcca actcttatca gaatttattg aacgcttttt tgttggaata   3060
   aaacgaaata tcatggaact aattgacata tttttgggcc cggtaaatgc caaaattggt   3120
   cgaatctttc agcagacaag aacgggacgg cacaacagac agtgccgtca atgtcttctg   3180
   gcacagtgtc tccccttcgt g                                              3201
<210> 526
<400> 526
   000
<210> 527
<400> 527
   000
<210> 528
<400> 528
   000
<210> 529
<400> 529
   000
<210> 530
<400> 530
   000
<210> 531
<400> 531
   000
<210> 532
<400> 532
   000
<210> 533
<400> 533
   000
<210> 534
<400> 534
   000
<210> 535
   <211> 5201
   <212> DNA
   <213> Heterodera glycines
<220>
   <223> genomic virtual cDNA Sequence=SeqID_580; coordinates=3891..3838 ,3752..3586,3506..3342,3201..3025,2979..2832,2739..2593,2526..2445 ,2394..2273,2217..2024,1930..1837,1785..1487,1351..1234,1183..928 ,562..411
<220>
   <223> coding sequence=SeqID_792; coordinates=3891..3838,3752..3586 ,3506..3342,3201..3025,2979..2832,2739..2593,2526..2445,2394..2273 ,2217..2024,1930..1837,1785..1487,1351..1234,1183..928,562..411
<220>
   <223> peptide sequence=SeqID_1035
<400> 535
   ctttgctttc gcgtggattt gcccgagaga ttataaaccg agtccaaaag ctacggaaac 60
   aggtgcttac agtgggacaa ttacagtttt ctaagattat ttttgtgatg gcagggcaaa 120
   ttggtcgaga ctgacaaggc cgttgcatat tgtactgtaa tgccagagga ttcaccgctt 180
   ggcttggcac tcaaagaaca tcgcgagaac atccaacagg caactggcac tccgttgttc 240
   atgaacgaaa cccccaatgg ggacactttt ctggcggaaa gctgtgacaa aatcaaaaat 300
   ttcgatttcc aggtagcatt gacattacgc cattaggaat tttaaatgta tgtttatgta 360
   gctgaggttg ctaagactga acgattgaag aaatgcactg aagaatttca ttatacatta 420
   aatacatagg tatatgtatg tatgtgtaag aatttttggc tttcgttgtt acaaatgcac 480
   tcaattgctt tatattctat tgtttttatt tttgttgaga ttgtttgatt agtgtatacc 540
   agggttgttg tgaatcgaag aataaaatgc gaaatacgaa acgggtactt ttcaattttt 600
   ggcctacatc tctatattta agccagatta aattatttag gtttttcctg ggatgaaaaa 660
   tgtgctgatt actaacctga tctcgttttg taaaaaaaac acatcgattt ttcttaatac 720
   aagttaagat ttgtgatcag cacattttta gaggatggtc aatgactgaa tacatacact 780
   gtactagagc caataccgat caaaagtggc aacattgaga cgaagttctt gttcgaatcc 840
   aatcacaaaa aaacaattaa caaggggaat catttgacca tcaaacattt tgtcagcttt 900
   tatcttctcc ccccccccca ttttttcatt ggttttccct ttcgtttttc tgttgtgtct 960
   ctctcgtctt tcttttctgc tcttccttcg cctgtctctt catttccttt tccttctgca 1020
   gttgtatctt ccgttgctct ttttctctct gcgctgcctc tttctgttct ctctctttaa 1080
   ttagtgtctc tctgtccacc aatttaacac aagtgtcttt gctgtggtcc tccaaccgaa 1140
   cgcccaattc cgggaggatt tcgtcgcgca gtctgtcaca ctcctggaaa atggagcaaa 1200
   cttagtttga aatattaact ggtttaaatg tactttcaaa attccaatgt ttttctgtgc 1260
   ccgagcctcg cttctgacga cctctcgaaa ctgtgcaagt atttccaagt acggcatgat 1320
   aatcgcttct ctgtcgtcag aagttgcgca gctaaaatga gaattttttt gattccatgg 1380
   cttaataatc attgcgccat tcttaaattt ttaaaagtaa aaacaaaaag ttattaattt 1440
   ggtatggtcg ttgcagaata tcgagcttac tcaagtaata ttttaccagt ctttagtaaa 1500
   gaattcgtat ttgcccacgt ccggctgtac gccaaatatg caaagcaatt ctctgacgaa 1560
   attggcacat tttgtcagca gttggcaatt aagtacggct tcctcctgtt tccggaccat 1620
   aatgtaagtg ttcagaaggg aaatgatcgt cctgattttg tcaagaactg cgcgtgtgtc 1680
   gatggagtcg cacatggcca aatgcacgga cactttgatc gcgttgaatt tgttcagcac 1740
   ttccatctcc tcttcaccgt attttttgaa gcacttgaaa tttttaaatt tttcgttatc 1800
   atttaaaatt atttttacct gggacaattc atccacttct ttaatttgct ttcttataat 1860
   atccttcaca ttgaggaaga attcgcagaa acatttttcg aaatgaatgg catgttccat 1920
   cgtcgattcg ctgaacattt tgacaataat tattttttag gattgcaatt tcatttttgc 1980
   cataattgtc taattccgta tttcctccat ttccttggtc cacctgtagt ccaaattgtc 2040
   cgtccaattg tgcatcagga aaaggaggcg cagttgtcga gcagtgtatt tgttcaacgc 2100
   ctcccttatc gtcacaaagt tcttcaaact cttcgacatt tttagccctt cgattcgtaa 2160
   tgtgccgcaa tggaggaaaa agttaaccca atgatcgttg tcattgtacg cctcacactt 2220
   aaaggaaaaa atattcaaaa cggaattttc catcaacagt tagagtactc acttgagcaa 2280
   tttcgttgtc atggtgggga aatttgagat caaatccgcc ggcgtgaatg tccaatttgt 2340
   ccccgcaaat ggccccactc attgccgaac attcgatgtg ccaaccgggc cgacctgaac 2400
   agaaaacaaa ttggaaaaca ggtgatgtgg aataaatgac acacctttgc cccagggact 2460
   ttcccagaag ggctctccgt ctttggaggc tttccaaagc gcaaaatccg acggcgagcg 2520
   cttttcctac aaaaactgca ataaattcga caattaatta attggggaaa aaggggtgca 2580
   gttgttgcgc actttcaaat tgtccactgc cacgctgagt tcgccttccc cctctcgcaa 2640
   atgtttttcc gcactttctg catcagcata cgcctcgggg aggagttttg cataggaatg 2700
   gttgggtttt gatgcgaatt gggcagtgtt aaaatacacc taaaaggaat aaaattgggt 2760
   aaaaagaaag gctaaagaaa catttataaa aggatcactg tagagatcaa atgcattcaa 2820
   aaaaaactta ctgatccatc agacacataa gcgtatccgt tgcggatgat ttgctgcaca 2880
   taatcaatga tttcgggaat gtactcgctg accctggtca acacgtccgg ctcaagaaca 2940
   tttaacgcat tcatatcgca caaaaattca ttttcatacc ttaaaaaaca tgactcaaat 3000
   aaaggcatat tcggatttcc ttactttctg gccaatttcg cgaaaatgtc gtgtttagtg 3060
   accgtatttc cccgggtaac gtccaaccaa tcggagagca cttctttcgc actttccacc 3120
   aatttgcgca ccaaaacagt gcgaacatca gctgattgcc cattgtctgg ctgggaaata 3180
   cgcgttttga attgaacgag atcagtctcg aatttggcaa tctgaaaatt tacggattag 3240
   aaattggaac aatcaaataa aggcaaggca cagactcgaa taatccggca cttaattttt 3300
   gaagtgttca aacgaaataa tctttaaaat tcggcttcca ctcactgacc gtatcgacaa 3360
   aaagttttct tttgtcggtg tcgggttcaa tggatgcctt tttgagataa tgctgaagtg 3420
   ctgcttgaag atgttgttcc aattcctcaa atccaaccga atcaatgcca ttcaaatatt 3480
   gctcaaaaag aaagttttgg cgtgccctta aaaaggcaaa acaaataatt tttaaatata 3540
   tcattaaagt tcgtttatcc aaaattaggg gaatcggaaa agaacctttt gatgattttg 3600
   tcgtcaatgt ccgttacatt catcacatag agcacgtcgt aattaaaata atcggtcagc 3660
   actcgcctta caatatcaaa ggacagataa gctctcgcgt gtcccatatg ggcggaatcg 3720
   taaacagtcg gaccacaaat ataacatttc acctgaacag ataaaaagaa atgattaatg 3780
   aactttgctc cccttctaaa atacccaaaa atgccttttc aaaccctcag aattcacctg 3840
   attccctgaa atgggttcaa atgtttcttt gtcgcgcgta aaactattca tcagccgtaa 3900
   acggggcttt tcctcattgt tttgcttttt gtaggccgtc catttgcaag gcattgcact 3960
   aacggagcaa atttgagtaa aattaaaaag aacagtcgct gaaaaacgga atgtgtatgg 4020
   aggaatagaa cgaataattt gcataaacta aaaaaaggag aaaaggtatg tatttcggtg 4080
   gattaaatgg ttgaggaaaa ttaaaaagaa ttaaaaatac aacgcatttg cctatagaaa 4140
   agtaaactat aaataagtta caaagaaaaa gtgcagaaaa tagaaacaaa atccatcttt 4200
   ttaaatggcc aaataaatga aagtgaaatc ggtaacggac aggcgaaagc atccgatcga 4260
   ctcgcaattg gcgcccgttt gttttgggcg caatttccaa aatgtgtgga tgatgatgca 4320
   atccgagtga gtcgctcgcc cacccatcca tcacccgccc cacttatatg taagccatcc 4380
   ttatttgata tgagaagcaa ttccccacct gacgaatgag ttgaggaatt ccaattacga 4440
   atgttagata aaattcgatt tgcaaaatct atgtacataa atgttttgtc catggacaag 4500
   gaaaggtttg agatttttag atttggaaaa tttacactgc gttgtcaaag tgaaatgttt 4560
   gcttacaata tgtttcctac cattgttgca tttaattatt aattaggaat atcattagtt 4620
   cgcctaattg gcttttgcgc cccaccaact tccctccctc cctcgcttcg cttcttcatt 4680
   catctatcaa cgtgcattcc catttattgt taaatgacat atgtaggtat tatttattgt 4740
   tgattcattt gttcgttccc tttttctgcc aatttccaaa acgactcgcg atgtgtgcgt 4800
   ttttcgtttt tctctattcg ttccaattgt tggattgaat tttagggagt gagcgaaaga 4860
   gggacgataa gaaagggtag tcaatgaacg aataataaat acaacaccaa aactatcgtt 4920
   gacgttcctt cccttttctt catctcgaag aagtgttttc tcgaggcgtt taaactgtgt 4980
   tttacttcaa tccttcaaaa ctcaaaagtt cgattaaatt caattttgaa agaagcaaaa 5040
   gaggaaagag acggagagaa aagcggcggg cgaatgcacc ggcggaatga gtgaatgcat 5100
   ctgccgtccg tctgccgtcc gtctgccgat cagacgatgg gcactcactc actttccctc 5160
   ccagcttttc ctctttcaac tcgccgttca tcaattcact a 5201
<210> 536
<400> 536
   000
<210> 537
<400> 537
   000
<210> 538
   <211> 2477
   <212> DNA
   <213> Heterodera glycines
<220>
   <223> genomic virtual cDNA Sequence=SeqID_583; coordinates=182..230 ,285..535,578..742,784..869,918..1077,1515..1618,1661..1937
<220>
   <223> coding sequence=SeqID_808; coordinates=182..230,285..535,578..742 ,784..869,918..1077,1515..1618,1661..1844
<220>
   <223> peptide sequence=SeqID_1050
<400> 538
   aaggtggcta attaatggga cctagtaaaa aaagaatttt taaaaaatag agaatttttc 60
   atttttctag gactaaattt attttaaatc tttaaaaaaa cagttatttt aataaaagtt 120
   gggtacaagt tgccccgata atcctttgat ttcttaagaa tttgagcgca tttttctttc 180
   catgatcaaa agattgacga atggaactga gctcaaactt agtaaaaacg gtcagtcctt 240
   tgatttttgt cttaatagtt ttggctatgc catccatttg acaggtacgg aactcagttt 300
   tcggcctggt ctaattcatg gaggagaatt ggagttcgac tgtggggttc aacgttgtat 360
   cagttacttt ttggagcctc tcattctcat ggcgcctttt tgcaaaaaac ctttgaatgt 420
   gaatctgacc ggtgttacaa attccgtcga tgaactcagc atcgacgcca ttcgggctac 480
   ttggctcccg gtcttttccc gctttgttct tgccgaccaa tgcacagaaa taaaagtcat 540
   aaaactttat ttattgagtt tttttttata ttttcagatc aaagccagag gtttcaagcc 600
   gagtggcggt ggttcagtga ctttctccag tcccatcaaa aggaatttgc gtgctgtgca 660
   atgcattaag ccaggaaaag tttgcaagat cagaggtctc gcttacgtat gcaaagtgac 720
   gccatcaatc gcatctcgaa tggtaattac gccgtcgttt cgtgtccttt tacacttttt 780
   cagattgacg gagctaaaaa gatgcttcac ggaaacattt ctgacattta cattacgatt 840
   gaccagagga agggccccca gggcggactg tccgttttta tttattttaa ttttacaaat 900
   atatatttgt tttaaagatc tcccggcttt ggaatcttct tgacggctga aaccaccgaa 960
   ggcgtatttt tccatggaga agcgatgtcg cgccctaagg gccaaaacga agaccaaaca 1020
   gtgccggaag aaattgggga acaggcggca agacgtttgc tggatgaaat tcatatggta 1080
   agtgctttct cttatcatgg gttcgaactg ttcattttta cacatatatc ttgttttttt 1140
   ctaatcattc caaagaggtc aacaaaaacc tacaccgcta ccttttccag ggatttttta 1200
   aatattttta aaattatttt tctctcgatt ttctgatttt aaatatttat ttactaatca 1260
   ggacgggacc gctcctttct tctctaccgt tttccgcgcc cctggaaagt tattttttaa 1320
   atttttatga aaatttttga gtcaaaaaag tcgactaggt ttttttgttt tcatctggtc 1380
   agaatccgaa tttcaaaaat tatgttatat tcgtgaaagg agaggccctg acaagctact 1440
   gacatatgga atctagatta tgcccataaa cggaaaaata ttgtattata gtgtgataag   1500
   aaagaaagct ttagggaggc tgtgcagatt cgtcctgtca atctttggcc gccactttca   1560
   tgactctctg cgacagagat gtttcgaaat ttctccttgg tccactcaca attaacaggt   1620
   gcatttttca attctttgtc tttttattac tcgtttttag cgtacacacc cttcggaatt   1680
   tgcaaatgtt tttcgagcaa atgtttaaat tggaggaatg gtggaaggtt atgaaagaaa   1740
   tcgtcggaaa aggcggaaat gcgatgaaaa acggaagtga ggacaaatgt ttgatgacct   1800
   gtgttggggt tggtttcttc aacatcaaca aagaccaact ttaattgttc ttttttgtta    1860
   attttatatt tgtttttatt tattgtaata agactgaccg attgaattcg gcaaaaaata   1920
   catttcttat tttaaaatga attaatggtt aatttggaaa acaaacggaa agtgatgaaa   1980
   tgactgaaga aggtgcaaat acgacattta cggctgcttt gattagcgat ggacgtgctg   2040
   aaaatcagtt ggagcaaaag aaacccagcc aattgcattc cctcccattt tccatggatt   2100
   ttaatgggcc ggcattggtc tccaaattct tccagactga ggatggcgct aatgggctca   2160
   aagcagcact gttccgagga catttgcttt tgggcaaaac ggtcagagcg ccggagggtt   2220
   ttgcgtttta tgcagtcaaa gagagtgaac attcacaatc agaagaggga ggtgaggagc   2280
   aacagttgaa aaggacgggg ttttttctta gaattggaaa gtggcagcaa aggaaaacat   2340
   tctcattaat atttccatag agatgagcac gtacaatgtt gtggactctg ttccgtcgat   2400
   ggatgtttgg gagtacgacg aaccaccaga aagtcgtaag aaatgcctgc agaatgcatt   2460
   ggttgctctg caagttg                                                  2477
<210> 539
   <211> 5202
   <212> DNA
   <213> Heterodera glycines
<220>
   <223> genomic virtual cDNA Sequence=SeqID_584; coordinates=399..494 ,600..667,712..910,956..1089,1136..1330,1390..1498,1560..1700 ,1742..1891,1953..2108,2153..2314,2359..2617,2669..3028,3086..3371 ,3418..3538,3589..3740,...
<220>
   <223> genomic virtual cDNA coordinates=3788..4064,4114..4224,4279..4434 ,4497..4615,4660..4834,4895..5063
<220>
   <223> coding sequence=SeqID_809; coordinates=399..494,600..667,712..910 ,956..1089,1136..1330,1390..1498,1560..1700,1742..1891,1953..2108 ,2153..2314,2359..2617,2669..3028,3086..3371,3418..3538,3589..3740 ,3788..4064,4114..4224,...
<220>
   <223> coding coordinates=4279..4434,4497..4615,4660..4834,4895..5005
<220>
   <223> peptide sequence=SeqID_1051
<400> 539
   attgaaaata aaacattaaa tcaatttgcc ttatttttgc acaagtcttt aatttaatct 60
<210> 540
<400> 540
   000
<210> 541
<400> 541
   000
<210> 542
   <211> 4451
   <212> DNA
   <213> Heterodera glycines
<220>
   <223> genomic virtual cDNA Sequence=SeqID_558; coordinates=2428..4112
<220>
   <223> coding sequence=SeqID_788; coordinates=2428..4112
<220>
   <223> peptide sequence=SeqID_1028
<400> 542
   atgaagtcgt gagtcatgtc gaagaatgcg aaaattttct tgcaacttcg aatgaatact     60
   tcaaaaataa ttatgtagca acagatgatc aaaatgtgtc aatcgataga attttgattg    120
   aaattctgga aaaaaggcaa ttccagattc ggaaaaacaa agtctgttgg aattggtttc    180
   gcaattcaag agtattttcg ctgtttccga ttcacagcta acccaaacaa cccttattca    240
   gcatgaaatt gagacaggaa ttgcagcacc agtgagatcg aaaatgagac cagtgcctta    300
   cgcatataga gaaaaagtgg ccgaaatgtt gaaagattat ttagaaaggg atttaattcg    360
   tccttcatat tccccgtggg ccagcccagt tatcgtgcca aagcgcgatg ggtcgctccg    420
   tttttgtgtt gactatcgtg ccgtgaattc gataacacga aaggatagtt ttccattgcc    480
   aaacatagac aatactcttg ttactgggag gtaaaaaatt ttttacaacg cttgatttca    540
   tgtcaggcta ctggcaaatt cggatgaaac ccgaatcggt cgaaaaaacg gcgttcataa    600
   ctgagtttgg gctccacgag tttgtggtaa tgccgtgggt tatcgaatgc agtggccact    660
   tttcagcgtt ttatgaatcg actttttgaa ggaatgatca ataaattttt atttgtttat    720
   atcgatgatg tgttaattgc ttccgaatca cttgaaagcc atctcgaaca ctagaaatgc    780
   gtttttgagc gtattgaaaa atcaggctta aaattgaact ctcgaaatgc caatttagtg    840
   ccgaagaatt gccgttttta ggtcacactt tgactagaaa tggaatcaaa atgaacgaag    900
   aaaaaataca accagtcaaa aatttaccgg ttcctaaaac aaaaaaagaa ttacagtcat    960
   tattaggctt cttcacttat tataggaagt ttatttactc ggttggaacc attgcagcac   1020
   cactttttcg tttgttaaag aaggagacaa aatttgaaat tggcgaaatg gaaaagaatg   1080
   caattgataa attgaaagag aaaattttaa gtgatgttgt actttatttc ccaaattttg   1140
   aggcagcgaa aaatgaccca ttgaggaatt tcattataat gacggatgcc agtaaaattg   1200
   gcatttcggc aattctcagt cagcagaatg aaaaaggcag aattcgtcca atttattttg   1260
   cctcaaggca atgtaacaaa tttgagtcgt tatgcgccga ccgagctgga agcattggca   1320
   attcgatttg gagtcatcag ttcataacaa tgattcctac tagagtaata acggaccata   1380
   aatcgctgat cggaatgttt aaaagtaaaa aggaaaccgg aaatgcacga gttgacaaat   1440
   ggatccttga attaaactca aaattcactc tgaaagttga ataccaaccg ggggaaaaga   1500
   atatgattgc ggatgttttg tcaagaacat tcgagacaaa agagtgtgaa acgttagaaa   1560
   ttgctgcgcc ggaagatatt gcattgattg ggatgttaag aattgaattg gggcaagaaa   1620
   agaatttgac tgttcatgag aaaatggaat gtgttcgtga aacaaaagaa agcgaaatga   1680
   aatttgttta tgattttttg agtcagagat ttattcctga agatcctagg caacaaaaaa   1740
   gtctgatgga catttgccac aaatatattt tgctagatga attgttgtat ttttgtaaat 1800
   cggacggaaa attgaaactt ttcgttccag aaaaattcag aaatgagttg ttgagacagc 1860
   ggcatgaaag taaatgtgca gagcatatga gtggcaaaaa attattcatt caactttcgg 1920
   aattttatta ttggccaaat atgctgggag attgtgtccg tgagcatttg aaatgtcgaa 1980
   tctgtgcgca tacgcgaatt gcgcgttcaa accaaccacc aatgcgagtt gttaactcgt 2040
   cagagccttt ggaattagtg tgtttagaca ttctcgatgt tggaccaagc aattcgaacc 2100
   aaaaatattg taagccggga ctcgggtctc ggctaccgat tgggattgaa ggggagaggc 2160
   tcctccccat tggaacgccc accgggattc cccttttaag ccatggctct cccagccgtc 2220
   gaggccagtc agtcagtcaa ttccccagtc atcactccca ctactggccc tccccctaag 2280
   ccgaccgatg ggacggagcc tttccgcggg ggctacggcc cagtccaaca atccgtcggt 2340
   ggacggagga gatcgatctc agtggtcgga actaagcagg ccgaatcccg aaacccggca 2400
   cggcttaact ggtagcaccg gatggtttat tgtgagctgt tgaaaatgat ggagtcggag 2460
   taccaacagt tacgcgaact gtctgccggg cgcatggtcg atttggactc gctgatcgcc 2520
   ttcatccgtg ctgctcagct tgaattgctt tggatccatc agcgtgagga attggaagtg 2580
   acacgcaact ggagcgcctg gacattccga tgctgcaaaa ctacttcaag cagctgctcc 2640
   atgagattga gctgcacgag aagcagttca acgacgtgca caaccagggc gccgcgctga 2700
   tcaaccaacg ccacccggcg ggcgaagtga tcgaggtgta cctgcgcacc atgcagaacc 2760
   aatgggattg gatgctgaac ctgagcaagt gcctggaggg ccacctgcgc gacgcgtaca 2820
   atgccaaggc atttgtggag gagtcggagc aggtggagca gcagatgcag caacagttgg 2880
   accatcttga tcgtgaatat tctcgtactg atttttcgct ggacgatggt gaacagatgt 2940
   tgcgacagct tgactctgtc cgtgaataca tccaacaatt gcatgcgcga ctcatttcac 3000
   tgaccgacag gtgtgcccaa atttcaccgc tttggcaacg tggcgaacgc atttcaaaac 3060
   cgctgccggt gatggcactt tgcgagtaca agagcaagga caaaattcac atcagacaag 3120
   gggatgagtg cactctgttg gacaaccatg accaaattca gtggcgagtg cgcggtatgg 3180
   acggcgttga ggcttccgtc ccatcggtgg tgttccgcat tcctccgcct gacccgcgat 3240
   tggcctcgtt cctcagccga ctgcacaacc aattcgagaa gttgcgcaaa ctgtgggatc 3300
   gcacccatca tttggtccgt tacaacatgg tgctgaacac aatgcgcaca gtgcgtgcat 3360
   gggacttgga cactttcctg tcgattccat ccgatcaacg ggacgagatc atcaaagctt 3420
   tgaatgagga cgtgaacaag ctgctgtccg agatggaccc caacgatccc ttggccaagc 3480
   ggcttaagga cgagctgcgg atgacgaacg aacatttcta cgatttgttg gcacgcgctc 3540
   agaaagagcc ggagcccgac tactccaacc agttcgatgc tgcatgtgcc gagttggtgc 3600
   gcaagttcga ggagggctgg aagcagctga accaccgggt gcaggaacga gtggccacca 3660
   acttggagga gttggaacgc caaatcaaag agcacaaggc attcgaagat gcactgcaga 3720
   cgcttgacgt ggacgtgtcg aatgtgaagg aactgtaccg ccagctgccc cacccaacgc 3780
   ccacccagcg tgcagcgcac gacaacatgg tgcagcgctg gcaagacatt tgggagctgt 3840
   cgcgcatgta cgtggagcgg ctgaaggggc ttgaatcggt gttgaacggg ttggacgaag 3900
   tgacggacat tgtgcgacgc catgagatca cgctggcctc ctttgacgac atgccggccg 3960
   cgctggagcg cctgcgcggc gtgcacgccc agctgctgga gctgaatatg gtactgcaac 4020
   agcaggaaaa catcgtcaag aagctgaacc gggacactgc aacgctgcgc caacatgttg 4080
   cgcgcacgcg tttcggcgct gcaaaccatc cggtgctacc agttaagccg tgccgggttt 4140
   cgggattcgg cctgcttagt tccgacccct gagatcgatc tcctccgtcc accgacggat 4200
   tgttggactg ggccgtagcc cccgcggaaa ggctccgtcc catcggtcgg ctttggggga 4260
   gggccagtag tgggagtgat gactggggaa ttgactgact gactggcctc gacggctggg 4320
   agagccatcg cttaaaaggg gaatcccggt gggcgttcca atggggaggt gcctctcccc 4380
   ttcaatccca atcggtagcc gagacccgag tcccgactta cacctccacc ccagggcagg 4440
   aaccgtccct g 4451
<210> 543
<400> 543
   000
<210> 544
<400> 544
   000
<210> 545
<400> 545
   000
<210> 546
<400> 546
   000
<210> 547
<400> 547
   000
<210> 548
<400> 548
   000
<210> 549
<400> 549
   000
<210> 550
<400> 550
   000
<210> 551
<400> 551
   000
<210> 552
<400> 552
   000
<210> 553
<400> 553
   000
<210> 554
<400> 554
   000
<210> 555
<400> 555
   000
<210> 556
<400> 556
   000
<210> 557
<400> 557
   000
<210> 558
<400> 558
   000
<210> 559
<400> 559
   000
<210> 560
<400> 560
   000
<210> 561
<400> 561
   000
<210> 562
<400> 562
   000
<210> 563
<400> 563
   000
<210> 564
<400> 564
   000
<210> 565
<400> 565
   000
<210> 566
<400> 566
   000
<210> 567
<400> 567
   000
<210> 568
<400> 568
   000
<210> 569
   <211> 912
   <212> DNA
   <213> Heterodera glycines
<220>
   <223> genomic DNA sequence=SeqID_525; coding sequence=SeqID_797
<400> 569
   agtgaatttt cgacttatct ataaatgttt ctgacccgca gcgaatacga caggggagtg      60
   aacacttttt ccccggaggg ccgtctgttc caagtggaat atgccatcga aactgtcaag     120
   cttggttcca caagcatcgg aattcacacc aaagaaggcg ttcttttggc tgcggaaagg     180
   cgttcaatga gcaaattggt ggtggacgac tcaatgagca aaatttcgga agttgagaag     240
   cacattgccg tcgcctcggc cggtctcatc gcagattcac gcacttgggt cgaacacgcg     300
   cgggtggagg ctcaacactt ttggtttact tacggtggca aaattcgggt ggaagacatt     360
   actcaaaagg tctcaagatt ggcactgcat tttggggacg acgactcaac tatcagtctc     420
   ggccgtccgt ttggagtttc tatgcttttt gccggcattg atcacacggg tgcgcatctc     480
   ttccatttgg acccgtccgg gacgtacatt aaatgtttgg ccaaggccat cggtgccggc     540
   tccgatgcag cggaacaaac gctgcaagag cactgtaaaa actgcgacaa aaaaatggaa     600
   atggccgagg caaaacaagt cgcactgaac acactcaaac agctgatgga agagaaaatc     660
   aattccaaaa atgtggaaat tgttatgatt aagccgcaga cggacaagga aggcaaaacg     720
   ttgggcaaaa ttgtgtggtt agaggaatcg gagttgcaag aaatcatttc gcgattgtag     780
   tcgaagggga cggatgagag aaggaaaatg ggctttgcac tgcccctttt atgattggat     840
   gaccttttgt tattctctgc ctttttgtga ctttttcagt gtataaggca aatgaaagca     900
   attaattgag aa                                                         912
<210> 570
<400> 570
   000
<210> 571
<400> 571
   000
<210> 572
<400> 572
   000
<210> 573
<400> 573
   000
<210> 574
<400> 574
   000
<210> 575
<400> 575
   000
<210> 576
<400> 576
   000
<210> 577
<400> 577
   000
<210> 578
<400> 578
   000
<210> 579
<400> 579
   000
<210> 580
<400> 580
   000
<210> 581
<400> 581
   000
<210> 582
<400> 582
   000
<210> 583
<400> 583
   000
<210> 584
<400> 584
   000
<210> 585
<400> 585
   000
<210> 586
<400> 586
   000
<210> 587
<400> 587
   000
<210> 588
<400> 588
   000
<210> 589
<400> 589
   000
<210> 590

<400> 590
   000
<210> 591
<400> 591
   000
<210> 592
<400> 592
   000
<210> 593
<400> 593
   000
<210> 594
<400> 594
   000
<210> 595
<400> 595
   000
<210> 596
<400> 596
   000
<210> 597
<400> 597
   000
<210> 598
<400> 598
   000
<210> 599
<400> 599
   000
<210> 600
<400> 600
   000
<210> 601
<400> 601
   000
<210> 602
<400> 602
   000
<210> 603
<400> 603
   000
<210> 604
<400> 604
   000
<210> 605
<400> 605
   000
<210> 606
<400> 606
   000
<210> 607
<400> 607
   000
<210> 608
<400> 608
   000
<210> 609
<400> 609
   000
<210> 610
<400> 610
   000
<210> 611
<400> 611
   000
<210> 612
<400> 612
   000
<210> 613
<400> 613
   000
<210> 614
<400> 614
   000
<210> 615
<400> 615
   000
<210> 616
<400> 616
   000
<210> 617
<400> 617
   000
<210> 618
<400> 618
   000
<210> 619
<400> 619
   000
<210> 620
<400> 620
   000
<210> 621
<400> 621
   000
<210> 622
<400> 622
   000
<210> 623
<400> 623
   000
<210> 624
<400> 624
   000
<210> 625
<400> 625
   000
<210> 626
<400> 626
   000
<210> 627
<400> 627
   000
<210> 628
<400> 628
   000
<210> 629
<400> 629
   000
<210> 630
<400> 630
   000
<210> 631
<400> 631
   000
<210> 632
<400> 632
   000
<210> 633
<400> 633
   000
<210> 634
<400> 634
   000
<210> 635
<400> 635
   000
<210> 636
<400> 636
   000
<210> 637
<400> 637
   000
<210> 638
<400> 638
   000
<210> 639
<400> 639
   000
<210> 640
<400> 640
   000
<210> 641
<400> 641
   000
<210> 642
<400> 642
   000
<210> 643
<400> 643
   000
<210> 644
<400> 644
   000
<210> 645
<400> 645
   000
<210> 646
<400> 646
   000
<210> 647
<400> 647
   000
<210> 648
<400> 648
   000
<210> 649
<400> 649
   000
<210> 650
<400> 650
   000
<210> 651
<400> 651
   000
<210> 652
<400> 652
   000
<210> 653
<400> 653
   000
<210> 654
<400> 654
   000
<210> 655
<400> 655
   000
<210> 656
<400> 656
   000
<210> 657
<400> 657
   000
<210> 658
<400> 658
   000
<210> 659
<400> 659
   000
<210> 660
<400> 660
   000
<210> 661
<400> 661
   000
<210> 662
<400> 662
   000
<210> 663
<400> 663
   000
<210> 664
<400> 664
   000
<210> 665
<400> 665
   000
<210> 666
<400> 666
   000
<210> 667
<400> 667
   000
<210> 668
<400> 668
   000
<210> 669
<400> 669
   000
<210> 670
<400> 670
   000
<210> 671
<400> 671
   000
<210> 672
<400> 672
   000
<210> 673
<400> 673
   000
<210> 674
<400> 674
   000
<210> 675
<400> 675
   000
<210> 676
<400> 676
   000
<210> 677
<400> 677
   000
<210> 678
<400> 678
   000
<210> 679
<400> 679
   000
<210> 680
<400> 680
   000
<210> 681
<400> 681
   000
<210> 682
<400> 682
   000
<210> 683
<400> 683
   000
<210> 684
<400> 684
   000
<210> 685
<400> 685
   000
<210> 686
<400> 686
   000
<210> 687
<400> 687
   000
<210> 688
<400> 688
   000
<210> 689
<400> 689
   000
<210> 690
<400> 690
   000
<210> 691
<400> 691
   000
<210> 692
<400> 692
   000
<210> 693
<400> 693
   000
<210> 694
<400> 694
   000
<210> 695
<400> 695
   000
<210> 696
<400> 696
   000
<210> 697
<400> 697
   000
<210> 698
<400> 698
   000
<210> 699
<400> 699
   000
<210> 700
<400> 700
   000
<210> 701
<400> 701
   000
<210> 702
<400> 702
   000
<210> 703
<400> 703
   000
<210> 704
<400> 704
   000
<210> 705
<400> 705
   000
<210> 706
<400> 706
   000
<210> 707
<400> 707
   000
<210> 708
<400> 708
   000
<210> 709
<400> 709
   000
<210> 710
<400> 710
   000
<210> 711
<400> 711
   000
<210> 712
<400> 712
   000
<210> 713
<400> 713
   000
<210> 714
<400> 714
   000
<210> 715
<400> 715
   000
<210> 716
<400> 716
   000
<210> 717
<400> 717
   000
<210> 718
<400> 718
   000
<210> 719
<400> 719
   000
<210> 720
<400> 720
   000
<210> 721
<400> 721
   000
<210> 722
<400> 722
   000
<210> 723
<400> 723
   000
<210> 724
<400> 724
   000
<210> 725
<400> 725
   000
<210> 726
<400> 726
   000
<210> 727
<400> 727
   000
<210> 728
<400> 728
   000
<210> 729
<400> 729
   000
<210> 730
<400> 730
   000
<210> 731
<400> 731
   000
<210> 732
<400> 732
   000
<210> 733
<400> 733
   000
<210> 734
<400> 734
   000
<210> 735
<400> 735
   000
<210> 736
<400> 736
   000
<210> 737
<400> 737
   000
<210> 738
<400> 738
   000
<210> 739
<400> 739
   000
<210> 740
<400> 740
   000
<210> 741
<400> 741
   000
<210> 742
<400> 742
   000
<210> 743
<400> 743
   000
<210> 744
<400> 744
   000
<210> 745
<400> 745
   000
<210> 746
<400> 746
   000
<210> 747
<400> 747
   000
<210> 748
<400> 748
   000
<210> 749
<400> 749
   000
<210> 750
<400> 750
   000
<210> 751
<400> 751
   000
<210> 752
<400> 752
   000
<210> 753
<400> 753
   000
<210> 754
<400> 754
   000
<210> 755
<400> 755
   000
<210> 756
<400> 756
   000
<210> 757
<400> 757
   000
<210> 758
<400> 758
   000
<210> 759
<400> 759
   000
<210> 760
<400> 760
   000
<210> 761
<400> 761
   000
<210> 762
<400> 762
   000
<210> 763
<400> 763
   000
<210> 764
<400> 764
   000
<210> 765
<400> 765
   000
<210> 766
<400> 766
   000
<210> 767
<400> 767
   000
<210> 768
<400> 768
   000
<210> 769
<400> 769
   000
<210> 770
<400> 770
   000
<210> 771
<400> 771
   000
<210> 772
<400> 772
   000
<210> 773
<400> 773
   000
<210> 774
<400> 774
   000
<210> 775
<400> 775
   000

<210> 776
<400> 776
   000
<210> 777
<400> 777
   000
<210> 778
<400> 778
   000
<210> 779
<400> 779
   000
<210> 780
<400> 780
   000
<210> 781
<400> 781
   000
<210> 782
<400> 782
   000
<210> 783
<400> 783
   000
<210> 784
<400> 784
   000
<210> 785
<400> 785
   000
<210> 786
   <211> 3021
   <212> DNA
   <213> Heterodera glycines
<220>
   <223> genomic DNA sequence=SeqID_505; cDNA sequence=SeqID_544
<220>
   <223> peptide sequence=SeqID_1031
<400> 786
   tatgcgcttg gacattccgc catggaacac ttgcgcaagg cgtcggttct cgtcagtggc 60
   ctcggctccg tgggcgtgga ggtggcgaag aatctgatcc ttggcggtgt tcgctcggtg 120
   actcttcacg acacaaaagc gttggcgttc agagatttgt cggcgcattt ctacgccaag 180
   gaaagcgacg tgggaacgaa ccgtgccaaa gtttgcttcg acaaattggc ggagctcaac 240
   gacactgtga actgtgcatt gtctgtggaa aatctgtcgg aggatttcgt caaacaattc 300
   gacttggtcg ttctcaccga cgcgtccttt tccactcaga cggaagtgaa cggttggatg 360
   cgcaaccatc gcaaaatgtt catctcggca gacgcgcgcg gacttttctc ttatgcattt 420
   gtggacttgg gcgacgaatt cgttgtgaac gattccgacg gggagacgtg caaagaggtg 480
   ctcgttgagt acatcgactg cgaaagcggc gacatgacga cactcgaagg cgcatttcac 540
   cagttggagg acggggacca cgttgtgttc accgactttg agcacgccga actgaacgaa 600
   cttccaccag tgccagtcac tttgaaggga agcaaagtct tcaacattgg aaatgctctc 660
   gcggcctttt ctggcgcagt tcaaggcccc gtttcgcgtg gacgtgttca gcaagtcaaa 720
   gtgcccaaga aaatgtcctt caagccattg tctcgcgcac tcgccgagcc cgactttatg 780
   atttgggact ttgccaagtt cgacgaacct gcacaacttc acgccctttg gcaggctttg 840
   tacagttttg aggagatgaa aaactacagc cctttgcccc gttccgatgc cgacgcaaaa 900
   ttgttaaagg aaatgctgcc aaaagatgcg cccgaaatgc cggactcact gctcttcaac 960
   ttctcttacc aggcgtgcgg aaatctccaa cccatcgctt cgctcatcgg cggtttggtg 1020
   gcccaagagg cgatgaaggc agtgacgcac cacacgacgc ccctcaagca gtttctgtac 1080
   accgattctc ttgaggcgtt gccgggcgcc gattcgacct ttgacgccgc caagttggag 1140
   aagaacgatt gtgcaccgcg caacaatcgc tacgacggac aggcggccgt tttcgggtgg 1200
   gcctttcagg aggcgctgca gcaacagaaa tggttcattg ttggcgccgg agcgattggc 1260
   tgtgagctgc tgaagaacat ggcgatgatg ggcttgggct gtggcaacag tggctcgctg 1320
   accatcaccg accccgacac catcgaagtg tccaatttga accgccagtt tctgtttcgt   1380
   cgtcctgatg ttgggaaaaa gaagtccgaa gtcgccgccc gttccgtcaa agcgtttaac   1440
   tcgaccattc acatcaatcc gatgtctgag atcgtcgccg agtcgaccga gcacatcttc   1500
   ggcgacgact tcttcgcccg tctgaacggc gtctgtaatg cgctggacaa tgtcgaagca   1560
   cgccgttacg ttgaccgccg ctgcgtgtac tatcagcttc cgctgctcga gtccggcaca   1620
   atgggcgcaa agggcaacac gcaagtggtc ttcccgcact tgacggagtc ttacggctcc   1680
   acgtccgacc cgcccgagaa ggagacgccc atctgcacgc tgcgcaattt cccctaccaa   1740
   atccatcaca ccattcaatg ggcgcgagct aagttcaccg actatttcac ttcggttgcg   1800
   gagacggtca accaatactt ggacaatgtg aacgactttt tgcaccgagt gaaccaaatg   1860
   actttttctc agcggatcga agtgctcagt acgcttgagc gtgcgttggt cgtcgaatac   1920
   ccgaaaaatg cggaagaatg tgtgagttgg gcccgtgaac tgttcgacac gctttaccac   1980
   aacgacattg cccaaatgct gcacaatttc ccttccagca tgcttactgc ccaaggcgac   2040
   aaattttgga gtggaaccaa acgctgtccg cacactttga acttcgacgt taacaaccct   2100
   gaacacttcg agttcgtctt ctccgcttcc attttgcgtg cccaatctta cagtttggct   2160
   ccgatcactg agcgcaaaaa agtggcacaa ttggccatgg cgtactgctc caaaccgttc   2220
   agaccacaag agggagtgcg catcgctgtg accgacgcgg aggcgactgc caatgatggc   2280
   aacacagcaa atggcgatgg agacgaaact gtacttcggc tgaacgatct gaacgtgaaa   2340
   ttggccaggc tgaacttgga gaacattcgc cgcatgacgc ccattgactt tgagaaggac   2400
   gacgactcca accaccacgt cgacttcgtg acgagtgcct ccaatttgcg agccgataat   2460
   tacgacatcg aaaaggctga tcggatgaaa acaaaaacaa ttgcgggcaa aatcatcccg   2520
   gcgttggcca ccacaactgc gctcgtctca gggctcgtct gcatcgaact ctacaaaacc   2580
   attgaggcgg atggaaggcg ctcgacggct cagatcgaac acatcaaaaa tgcatttatc   2640
   aacttggcta cgcctttcat tgcgttcagc gagccgggca aagcgcagaa gaagaagtac   2700
   ctcgacattg acttcactct ttgggaccgt ttcgaagttg acggaccgat gacattgggg   2760
   caactgattg attgggttga ggtgagtaag agtggcctca ccgtttcaat gatttcttcc   2820
   ggcgtctccc ttttgtatgc gttctttcag cccgcagaaa aagttgccga gcgcaagaac   2880
   atggatctta ttgcggtttt cgaggaagtg tctcgtggca aagtgccgga ccatcgccga   2940
   gcgatcgttc ttgaggcgtt gacccagaat gaggaaaacg aagacgttga tatcccattc   3000
   attaaatata atttccgtta a                                             3021
<210> 787
   <211> 1014
   <212> DNA
   <213> Heterodera glycines
<220>
   <223> genomic DNA sequence=SeqID_506; cDNA sequence=SeqID_545
<220>
   <223> peptide sequence=SeqID_1032
<400> 787
   atggagtgca tgagaatgtt gattgtccgt ctccgtggtg agtttgagaa acgcgtccgt      60
   ttcctccgtc cgtccgatga aactgtccat tccgtgccgg atgacgacga cactttggag     120
   gagccgcccc gactgcccca gggcaatcgc cgtcggttgg gcgtgtcggc ggaggtgccg     180
   gacgaaaatg aggcggccaa ttaccagcga gtggtcatcc cgaaagacga cgacgtgcaa     240
   aaggctttgc gagcggcgat gtgcaaaaat gtgctgtttg cgcatttgga cttggacgag     300
   gaaaaggcca ttttcgacgc catgtttccg gtggagaaac acctcgggga gacaatcatt     360
   gagcagggtg aagagggcga caatttttat gtgattgaca gtggcgaggt cgatgtgttc     420
   gtcaatggcg agttcgtgtt gagcattaag gagggaggtt catttggcga gttggcactg     480
   atttatggca ctccgcgcgc agccaccgta gtcgcgaaga cgaacacggt caaactgtgg     540
   gccatcgacc gcatcactta ccgcgccatt cttatggggt cgacgatgcg aaagcggaaa     600
   atgtacgacg aattcctgag caaagttcag attcttgccg acttagacaa atgggaaagg     660
   gccaatgtgg cggatgcgtt ggaacagtgc caattcccct cgggcaccca cgtggtggag     720
   cagggacatc ctggggacga attcttcatc attgtggagg gcgaagctga ggtgctgcag     780
   agaccgaacg acgacgcgcc cttcgaagtg gtcggaaaat tgggaccctc tgactatttc     840
   ggggaaatcg ccctccttct cgatcgccct cgtgccgcca ccgtcgtggc caaaactctg     900
   ctgaaatgtc tgaaattgga ccgggcacga tttgagcgcg tgatgggtcc ggtgcgtgag     960
   attctcaagc gcgacgtgtc caattacaat tcgtacgtga agctgatgac ttga          1014
<210> 788
   <211> 1685
   <212> DNA
   <213> Heterodera glycines
<220>
   <223> genomic DNA sequence=SeqID_542; cDNA sequence=SeqID_558
<220>
   <223> peptide sequence=SeqID_1028
<400> 788
   tattgtgagc tgttgaaaat gatggagtcg gagtaccaac agttacgcga actgtctgcc 60
   gggcgcatgg tcgatttgga ctcgctgatc gccttcatcc gtgctgctca gcttgaattg 120
   ctttggatcc atcagcgtga ggaattggaa gtgacacgca actggagcgc ctggacattc 180
   cgatgctgca aaactacttc aagcagctgc tccatgagat tgagctgcac gagaagcagt 240
   tcaacgacgt gcacaaccag ggcgccgcgc tgatcaacca acgccacccg gcgggcgaag 300
   tgatcgaggt gtacctgcgc accatgcaga accaatggga ttggatgctg aacctgagca 360
   agtgcctgga gggccacctg cgcgacgcgt acaatgccaa ggcatttgtg gaggagtcgg 420
   agcaggtgga gcagcagatg cagcaacagt tggaccatct tgatcgtgaa tattctcgta 480
   ctgatttttc gctggacgat ggtgaacaga tgttgcgaca gcttgactct gtccgtgaat 540
   acatccaaca attgcatgcg cgactcattt cactgaccga caggtgtgcc caaatttcac 600
   cgctttggca acgtggcgaa cgcatttcaa aaccgctgcc ggtgatggca ctttgcgagt 660
   acaagagcaa ggacaaaatt cacatcagac aaggggatga gtgcactctg ttggacaacc 720
   atgaccaaat tcagtggcga gtgcgcggta tggacggcgt tgaggcttcc gtcccatcgg 780
   tggtgttccg cattcctccg cctgacccgc gattggcctc gttcctcagc cgactgcaca 840
   accaattcga gaagttgcgc aaactgtggg atcgcaccca tcatttggtc cgttacaaca 900
   tggtgctgaa cacaatgcgc acagtgcgtg catgggactt ggacactttc ctgtcgattc 960
   catccgatca acgggacgag atcatcaaag ctttgaatga ggacgtgaac aagctgctgt 1020
   ccgagatgga ccccaacgat cccttggcca agcggcttaa ggacgagctg cggatgacga 1080
   acgaacattt ctacgatttg ttggcacgcg ctcagaaaga gccggagccc gactactcca 1140
   accagttcga tgctgcatgt gccgagttgg tgcgcaagtt cgaggagggc tggaagcagc 1200
   tgaaccaccg ggtgcaggaa cgagtggcca ccaacttgga ggagttggaa cgccaaatca 1260
   aagagcacaa ggcattcgaa gatgcactgc agacgcttga cgtggacgtg tcgaatgtga 1320
   aggaactgta ccgccagctg ccccacccaa cgcccaccca gcgtgcagcg cacgacaaca 1380
   tggtgcagcg ctggcaagac atttgggagc tgtcgcgcat gtacgtggag cggctgaagg 1440
   ggcttgaatc ggtgttgaac gggttggacg aagtgacgga cattgtgcga cgccatgaga 1500
   tcacgctggc ctcctttgac gacatgccgg ccgcgctgga gcgcctgcgc ggcgtgcacg 1560
   cccagctgct ggagctgaat atggtactgc aacagcagga aaacatcgtc aagaagctga 1620
   accgggacac tgcaacgctg cgccaacatg ttgcgcgcac gcgtttcggc gctgcaaacc 1680
   atccg 1685
<210> 789
   <211> 1482
   <212> DNA
   <213> Heterodera glycines
<220>
   <223> genomic DNA sequence=SeqID_519; cDNA sequence=SeqID_559
<220>
   <223> peptide sequence=SeqID_1033
<400> 789
   atggttacgg aacagaatgg cgctactttt gctgttgacg ttgaggaaat tgcggctggg     60
   catcacgtta cgcctgtgga catgacaaca gctaccagca aacttcagct tgaggctgcc    120
   gaagtgcgaa atccggcgaa taaaccgaat tggtcatcgt atttgcgctc tcaattggtg    180
   cctcaaaatg agtacaattt cattattgaa ttggaaaagg ccaaaaacaa gcaggagagg    240
   gacgaagcgt tagccaagga caagacgaat tcggcacgga gtattatcaa tttgatcacc    300
   gaagttgcga aggatcagct gatccgttac gttctcaccc ttttcgacga ccttttgcag    360
   gaggacaaat cccgggtaga gattttccat gattatgcaa gacggcaaaa acgaactatt    420
   tggtcgtggt tccaagggat tttaacgcga aatgacagtt tcattgtcaa ccaaatgtct    480
   gcaatattct caaaattggc ttgctatggc tctgtgctta tggatggaca agaactgcaa    540
   gcttatttca atttccttca acaggaaata aaaaactcgg ttagcaacga atacataaat    600
   acatcagttc gatgtctaca aatgatgtta cgaaaggaaa agtatcgacg aaaattcgtt    660
   gagtatgacg gcgttaaaag cattcttttg gcattgaatg gtcgtgccaa ttttcaactt    720
   caataccaac tcattttttg cctttggtgc cttaccttcg acagtaacat cgctcaattt    780
   gtccagggat tgggcattat ccaaccgttg ggcgatattc tctccgaatg ctcaaaggaa    840
   aaagtgattc gtatcattct cgcgaccttc cgaaatttgt tggagaaaat tgacaataga    900
   gagatggtgc gcgaatcggc actgcaaatg gtccaatgca aaacgctgaa aacccttgaa    960
   ttgatggacg ccaaaaagtt tgacgacccg gacttggtgg acgacattga atttctgtct   1020
   gaacgtcttc acgcctcggt gcaggatttg agttcgtttg acgaatactg tgccgaggtc   1080
   cgatcgggca gactccagtg gagtccggtg cataaattgg agaaattctg gcgcgaaaat   1140
   gcacaacgct tcaatgaaaa gaactttgaa ttgataaaaa tactcatcaa aattctcgag   1200
   accagcagcg acccattgcc gctcagtgtt gctgctcatg acattggcga atacgtccgg   1260
   cattaccatc gcggaaaaac aattgtcgaa caataccaag gcaaacaagc agtgatgaag   1320
   ttgttgagtg cagaggaccc aaacgttcgg taccacgctt tgttagcaat ccaaaagtta   1380
   atggtgcaca attgggaata tcttggcaag caaattgaag gactcgaagg ggcaaaaggc   1440
   gctgcggggg aagaagcaaa ggaagtgaaa acgcgaacct aa                       1482
<210> 790
   <211> 2697
   <212> DNA
   <213> Heterodera glycines
<220>
   <223> genomic DNA sequence=SeqID_511; cDNA sequence=SeqID_550
<220>
   <223> peptide sequence=SeqID_1233
<400> 790
   cttttgtcgg aatgccctgc gggcgattcg cctgttttcc tcctccacca caacacttcc     60
   gtcgcctccg gcactgtccc tctccgatcg gcgcccgtcc ccggagggtt tctcgaatgt    120
   accgaacttt gttccgtcgc aagacaatgc caattggtcg gtccgaagtc ggcatctgaa    180
   ggtgccgtgg cagaatttgg tgaagaaatg gcgacacgga cggtgacgaa gaattgtgtg    240
   aagagtgagc gcatctgttc ctcgcctttc catttcgacg ttcatgaaca aaagattttg    300
   gtgggtttcg cgcgtgaggt ggtctccgcc gactcagtcc accgctgtct gtccgcttgt    360
   ctgaatgcgt tcgatacgtt cggcttcgaa tgcgagtcgg tcatgtatta ccctgtggac    420
   gcggaatgca ttttgaacac ggaggaccga ttggatcggc ctgacctttt cgtggacgag    480
   cacgaggaca cggtcatcta cttggacaac aattgcgccg gatgtgagtg ccattggcat    540
   tttgacaatt tcaaaacaag cggcattttg aacgaccaac aattcgcaat tgcagcacaa    600
   tgttacgcac cgtacgtaac gcaatacgtg gcggtggaag gacgccaatt gtcggacgaa    660
   ttggaccaca gttttgaagg gttggagctg agcgaatgtg aagagttgtg cacgcaacgg    720
   ttaagtgtta cggcaaacga cttcaactgc aaatcgttca tgtacagtaa cttgacgcgc    780
   agttgcgttt tgtcggacga acgctcgcgc cctttgggcc gtgccaattt ggccgaagtg    840
   ccgggatgga cttatttcga gaagaaatgc tttgcctccc cgcgtacgtg ccgcggcgtt    900
   ccgtcgttta cgcgagtgcc gcaaatgctt ttggtgggct ttgcctcttt tgtgatggaa    960
   aatgtgccgt cagtgacaat gtgtttggac caatgcacaa accctcctcc tgagacggga   1020
   caaaactttg tgtgtaaatc ggtgatgtat tattacaacg agcaagaatg cattttgaac   1080
   gcggagtcgc gccattctaa gccggacctg ttcattcctg aggaggacga ctttgtggtc   1140
   gactatttcg acataaactg tcgcttggag tcggaacagt gcagcgccgg agagggggcg    1200
   cgaagtccga aaatcgtgcg gacattaaat gcagcactgc ccgaagggga cggtgccatt   1260
   cacgtagtgg aaacggtgca ggcgaatgtg cgcgaatgtg cccaaaaatg tgccgctcac   1320
   tctcccgaca aatgccgttc cttcaacttc gacaaatcgt ccggcatttg caatttgctt   1380
   tatttggacg gccgcggttc gctgcgcccc gagccgaagg gacaggtcga tctttatgac   1440
   ctccactgtt tggagacatc gcccgagggc caatcaatgg gatgcgtcga ccccgagggg   1500
   gccgcgttca gtcgccattt gtattcccaa tgggtcgggg gcgaactgag caaagaattg   1560
   cattctgtgc ctctctccaa atgtttgaat ttgtgtgccg gtgccggaca ttgtgaagga   1620
   cttaattaca atcggcgaaa tggcacatgc gcagtgatcg gacaattggg cacagtgtcg   1680
   ggggcgatgc agaagagtga acaagtggac ttttatcgga atgtttgtgc actgaaagaa   1740
   gtccaatcgg aggaatcaag cgcagcgaat gtgccaaaaa gcaaaaggga tatgggatga   1800
   ggagaaatga aagggatcaa atcaaaaatt gggaaattaa tgtaaaaaaa ggaaaaaaag   1860
   gcattggtga gcggcacttc tgaaggaaaa ggcgcaaaag aggacaaaaa agttggaccg   1920
   cccatccaaa tccccggcag cgaagtgcac acgatttgca attacgaggg cataagtgtt   1980
   cagataaaac acagcgaacc cttctcggga gtgatgttcg tcaagaacaa atacgacacg   2040
   tgccgagttg aggtggggac agaaaacagc gtcactttag tgctcggact tcctgcaaac   2100
   tttggcatga aaccgatcac tttgacctcc ccggaaggca aaggttcttt tggaaaccgc   2160
   gaaaaacagt gttcaaaaaa ggctttcgca agttttgcta taatgttcag acaacgtcgc   2220
   caatcgcaga gggattgtgg cctcgtggac atggacaacg gcacgtacaa aacggtggtg 2280
   gtcgttcaga ccaacaattt gggcatcccc ggcctcgtca cttcgatgga ccaactttac 2340
   gaaatttcct gcaattattc gagcatgttg ggaggcaaag tgaccgctgc agccaatttg 2400
   acgctgcagg ggccgcagcc gtcgctgatc caaccgcgcg gaaagatcga actgggcaac 2460
   ccggtgctaa tgcaaatgat gccgatgaga gaagctaatt gaaatgtgcc acaggcaaag 2520
   ttgggcgata ttttggaact tcgctgggaa ttgatggcca tggacgacga attggacttt 2580
   ttggtgaagg attgttacgc ggagccggga attgtgccgc caaagggcgc atcggcgcct 2640
   aagttggcca tggaaaagct tcaactcgtc cagggagggt ctgttgggac ggctttt 2697
<210> 791
<400> 791
   000
<210> 792
   <211> 2175
   <212> DNA
   <213> Heterodera glycines
<220>
   <223> genomic DNA sequence=SeqID_535; cDNA sequence=SeqID_580
<220>
   <223> peptide sequence=SeqID_1035
<400> 792
   atgaatagtt ttacgcgcga caaagaaaca tttgaaccca tttcagggaa tcaggtgaaa    60
   tgttatattt gtggtccgac tgtttacgat tccgcccata tgggacacgc gagagcttat   120
   ctgtcctttg atattgtaag gcgagtgctg accgattatt ttaattacga cgtgctctat   180
   gtgatgaatg taacggacat tgacgacaaa atcatcaaaa gggcacgcca aaactttctt   240
   tttgagcaat atttgaatgg cattgattcg gttggatttg aggaattgga acaacatctt   300
   caagcagcac ttcagcatta tctcaaaaag gcatccattg aacccgacac cgacaaaaga   360
   aaactttttg tcgatacggt cagtgatctc gttcaattca aaacgcgtat ttcccagcca   420
   gacaatgggc aatcagctga tgttcgcact gttttggtgc gcaaattggt ggaaagtgcg   480
   aaagaagtgc tctccgattg gttggacgtt acccggggaa atacggtcac taaacacgac   540
   attttcgcga aattggccag aaagtatgaa aatgaatttt tgtgcgatat gaatgcgtta   600
   aatgttcttg agccggacgt gttgaccagg gtcagcgagt acattcccga aatcattgat   660
   tatgtgcagc aaatcatccg caacggatac gcttatgtgt ctgatggatc agtgtatttt   720
   aacactgccc aattcgcatc aaaacccaac cattcctatg caaaactcct ccccgaggcg   780
   tatgctgatg cagaaagtgc ggaaaaacat ttgcgagagg gggaaggcga actcagcgtg   840
   gcagtggaca atttgaaaga aaagcgctcg ccgtcggatt ttgcgctttg gaaagcctcc 900
   aaagacggag agcccttctg ggaaagtccc tggggcaaag gtcggcccgg ttggcacatc 960
   gaatgttcgg caatgagtgg ggccatttgc ggggacaaat tggacattca cgccggcgga 1020
   tttgatctca aatttcccca ccatgacaac gaaattgctc aatgtgaggc gtacaatgac 1080
   aacgatcatt gggttaactt tttcctccat tgcggcacat tacgaatcga agggctaaaa 1140
   atgtcgaaga gtttgaagaa ctttgtgacg ataagggagg cgttgaacaa atacactgct 1200
   cgacaactgc gcctcctttt cctgatgcac aattggacgg acaatttgga ctacagcgaa 1260
   tcgacgatgg aacatgccat tcatttcgaa aaatgtttct gcgaattctt cctcaatgtg 1320
   aaggatatta taagaaagca aattaaagaa aaaaatttca agtgcttcaa aaaatacggt 1380
   gaagaggaga tggaagtgct gaacaaattc aacgcgatca aagtgtccgt gcatttggcc 1440
   atgtgcgact ccatcgacac acgcgcagtt cttgacaaaa tcaggacgat catttccctt 1500
   ctgaacactt acattatggt ccggaaacag gaggaagccg tacttaattg ccaactgctg 1560
   acaaaatgtg ccaatttcgt cagagaattg ctttgcatat ttggcgtaca gccggacgtg 1620
   ggcaaatacg aattctttac taaagactgc tgcgcaactt ctgacgacag agaagcgatt 1680
   atcatgccgt acttggaaat acttgcacag tttcgagagg tcgtcagaag cgaggctcgg 1740
   gcacagaaaa acattggaat tttgaaagag tgtgacagac tgcgcgacga aatcctcccg 1800
   gaattgggcg ttcggttgga ggaccacagc aaagacactt gtgttaaatt ggtggacaga 1860
   gagacactaa ttaaagagag agaacagaaa gaggcagcgc agagagaaaa agagcaacgg 1920
   aagatacaac tgcagaagga aaaggaaatg aagagacagg cgaaggaaga gcagaaaaga 1980
   aagacgagag agacacaaca gaaaaacgaa agggaaaacc aatttcttcg attcacaaca 2040
   accctggtat acactaatca aacaatctca acaaaaataa aaacaataga atataaagca 2100
   attgagtgca tttgtaacaa cgaaagccaa aaattcttac acatacatac atatacctat 2160
   gtatttaatg tataa 2175
<210> 793
   <211> 1506
   <212> DNA
   <213> Heterodera glycines
<220>
   <223> genomic DNA sequence=SeqID_532; cDNA sequence=SeqID_576
<220>
   <223> peptide sequence=SeqID_1036
<400> 793
   atggcgaaat ctgcaattga tgttaatcgc ggcatcacag aagaccaaac agctaccata 60
   agaaattatt tcacacaacc gcacttaatt tacaaaactg tcactggagt gcgcgggcct 120
   ttggtcatat tgggcgatgt taaattccca caatacgctg aaattgtgaa acttacactg 180
   cctaatggag aaaaacgcat gggacaagta ctcgaagtaa cgaaggacaa agctgtcgtt 240
   caggtgtttg aaggtacgtc aggtatcgat gcaaaaaaca caatttgcga gttcactggc 300
   gatattatga gaacacccgt ttccgaggat atgcttggaa gaatattcaa tggatccggc 360
   acacccatcg acaaagggcc caatgttttg gcggaggatt acttggacat cgatggacag 420
   cctatcaacc catggtccag aatttatccc gaagaaatga ttcagaccgg catttccgca 480
   attgatgtta tgaatagtat cgccagggga cagaaaatcc caattttttc tgctgcaggt 540
   cttccgcata atgagatcgc tgctcaaatt gtacgccaag ggggcctcgt caaacagccg 600
   aacagaaaga aggaaaatga ggtggagggt gacgacgact ttgccattgt gtttgctgca 660
   atgggtgtca acatggagac agcgcgcttt ttcaaacaag actttgaaga aaacggttcc 720
   atggaaaatg tgtgcctttt ccttaacttg gctaatgacc caaccatcga acggatcatc 780
   acaccacgac tcgcgctgac ttgtgcagaa tttctggctt accaatgcca aaaacacgtc 840
   ttggttgtgc tcactgacat gtcttattac gctgaagcac ttcgcgaagt gtccgccgcc 900
   cgagaggaag tgcccggtcg tcgtggtttt cccggttaca tgtacactga cttggccaga 960
   atatacgaac gtgccgggcg tgttgaaggc cgaaaggggt caatcacaca aattccaatt 1020
   ttgtctatgc ctaatgacga catcacccac ccaattcccg atttgaccgg ttacataacg 1080
   gaaggccaaa tttatgtgga tcgccaactt cacaatcgtc agatttaccc tccaataaat 1140
   gtacttccat cgctgtcgcg tctgatgaaa tcggccattg gcgagggaat gacccgtgcc 1200
   gatcattcgg atgtgtccaa ccaactttac gcatgttatg ccattggtaa agacgtgcaa 1260
   gcaatgaaag cggtggttgg agaggaggca ctgtcgtcgg atgatttgct ctacttggag 1320
   tttctcacga aatttgaaaa gaatttcatt tcacaaggga attatgaaaa ccggacggtg 1380
   ttcgactcac tcgacattgg ttggcagctg ttgcgcattt ttccaagaga aatgttgaag 1440
   cgaattccag cgaaaacatt ggataaatat tacccaaggg gaggcactgg ggcgaggaac 1500
   gaatga 1506
<210> 794
<400> 794
   000
<210> 795
<400> 795
   000
<210> 796
<400> 796
   000
<210> 797
   <211> 756
   <212> DNA
   <213> Heterodera glycines
<220>
   <223> genomic DNA sequence=SeqID_525; cDNA sequence=SeqID_569
<220>
   <223> peptide sequence=SeqID_1040
<400> 797
   atgtttctga cccgcagcga atacgacagg ggagtgaaca ctttttcccc ggagggccgt     60
   ctgttccaag tggaatatgc catcgaaact gtcaagcttg gttccacaag catcggaatt    120
   cacaccaaag aaggcgttct tttggctgcg gaaaggcgtt caatgagcaa attggtggtg    180
   gacgactcaa tgagcaaaat ttcggaagtt gagaagcaca ttgccgtcgc ctcggccggt    240
   ctcatcgcag attcacgcac ttgggtcgaa cacgcgcggg tggaggctca acacttttgg    300
   tttacttacg gtggcaaaat tcgggtggaa gacattactc aaaaggtctc aagattggca    360
   ctgcattttg gggacgacga ctcaactatc agtctcggcc gtccgtttgg agtttctatg    420
   ctttttgccg gcattgatca cacgggtgcg catctcttcc atttggaccc gtccgggacg    480
   tacattaaat gtttggccaa ggccatcggt gccggctccg atgcagcgga acaaacgctg    540
   caagagcact gtaaaaactg cgacaaaaaa atggaaatgg ccgaggcaaa acaagtcgca    600
   ctgaacacac tcaaacagct gatggaagag aaaatcaatt ccaaaaatgt ggaaattgtt    660
   atgattaagc cgcagacgga caaggaaggc aaaacgttgg gcaaaattgt gtggttagag    720
   gaatcggagt tgcaagaaat catttcgcga ttgtag                              756
<210> 798
<400> 798
   000
<210> 799
<400> 799
   000
<210> 800
<400> 800
   000
<210> 801
<400> 801
   000
<210> 802
<400> 802
   000
<210> 803
<400> 803
   000
<210> 804
   <211> 948
   <212> DNA
   <213> Heterodera glycines
<220>
   <223> genomic DNA sequence=SeqID_513; cDNA sequence=SeqID_552
<220>
   <223> peptide sequence=SeqID_1046
<400> 804
   atgtcatttc ggtcagcagc ggcattaggg cttaacttgc gtaaagcatc caaatttgtg 60
   ttgttcggcg cattggccac cggtggcagt gtgttgctgt actcgttgga gaatagcggt 120
   gcgcgtgcct acgactttgt tgtgcacccg agccatttgc catggtccca cggcggactg 180
   atggacgcaa ttgacatgcg atcggttcgt cgcggttacc aagtgtacaa gcaagtgtgt 240
   gccgcatgcc attccttgaa attcatccgt tacaaccatt ttgtcgacaa tttcatgaca 300
   cgggatgagg cgaagacgga ggctgccgag gcactgatcc cggacttgga cgataatggt 360
   gtggaaattc ttcgtcccgg gctgctcaac gacacgttgc caagccccta caaaaacaac 420
   aaagctgccg cggcggcgaa cagtggcgca ttgccacccg atttgtcact gattatgtgg 480
   gcccgggaag gggccgagga ctacgtgttc catctgttga ccgggttttt tgatgcaccg 540
   gcgggcattg acgtggagga gggcaaggcg ttcaacccgt acttccccaa cggctgtgtg 600
   ttggccatgc cgcaacaact gtttgacgaa ggcattgact atgaggatgg cacaccggca 660
   acaatgtcgc aacaggccaa ggacatttgc accttcttgc gctgggccgg cgaacaatgg 720
   cacgacacgc gcaaacgctg ggcatggaag ttgatcacgc tgttgccact gctcacgttt 780
   gtgctactgt acgccaagcg caatgtgtgg gcactgatga agacggagaa ggtgatcttc 840
   cggcgactgc ccggccgcgg gtggacgccc gagtcgtcgg cgccaaagcc gcccaaggac 900
   gagccggagc tgaaggtgat cgagccgccg cagaagccgg tcgtctga 948
<210> 805
   <211> 1275
   <212> DNA
   <213> Heterodera glycines
<220>
   <223> genomic DNA sequence=SeqID_523; cDNA sequence=SeqID_564
<220>
   <223> peptide sequence=SeqID_1047
<400> 805
   cccaatgaga cggatgacaa aaccaaagaa ataaaatttt cagcgcttga cgaggatgaa      60
   attgccgcac ttagtaatta caacatggga ccgtacgcgg atcagttgaa gcaggcggag     120
   aaggacattg atgaaattca gaagcgcata aacactcttt gcggagtgaa agagagcgac     180
   acggggctgg cgccgcccat tctttgggac attgcggccg acaaaatggc catgtcccat     240
   gagcagccgc tgcaggtggc tcgctgcaca aaaatcatca aagaagaggg caaagaaacg     300
   cgttacatga tcaatgtgaa gcagttcgcc aagttcgtcg tggacctgca cgaaaatgtg     360
   gcgcccactg acattgagga gggaatgcga gtgggggtgg accgcaacaa ataccagatt     420
   catttgcctt tgccggcaaa gattgacgcg tccgttacga tgatgcaagt ggaggacaag     480
   ccggacgtta cctacgcgga cattggcggg tgcgaagaac agatcaaaaa gttgcgtgaa     540
   gtggtcgagt ttccgttgct tcagcctgag cgtttcacga gtttgggcat tgagcctccg     600
   aagggcgttt tgttttttgg tccgccgggc accggcaaaa ctttgtgtgc ccgcgcggtc     660
   gccaatcgga cggacgcgtg tttcatccgc gtcatcggtt ccgaattagt caaaaaatac     720
   gttggcgaag gcgcgcgcat ggtgcgcgag ctgttttcgc tggctaaaac gaaaaaggcg     780
   tgcattctct tcttcgacga agtcgacgcc atcggcggag cgcgatttga cgacggaaaa     840
   gggggcgaca acgaagtgca acggacgatg ctcgagttgg tcaaccaact ggacggattc     900
   gactcacgcg gggccatcaa ggttttgatg gccaccaaca gaccggacac actcgacccg     960
   gcgctcattc gtcccggtcg cattgaccga cgcattgaat tttccttgcc tgacctcaag    1020
   gcacgaggaa acattctcca aattcacacc aaacggatga gcgtcgaccg gaacattcgg    1080
   tacgaattga ttgctcgact ctgtccaaac acgacgggtg ccgacttgcg cagcgtttgc    1140
   actgaggcgg gaatgttcgc tttgcgtgca cgtcgaaagg tcataacgga gcaagacttt    1200
   ctcaaggctg ttcagaaagt ggtgaaaagc tacgccaagt tcagttcaac gccggcgtat    1260
   atgacgcaca actga                                                     1275
<210> 806
   <211> 1322
   <212> DNA
   <213> Heterodera glycines
<220>
   <223> genomic DNA sequence=SeqID_514; cDNA sequence=SeqID_553
<220>
   <223> peptide sequence=SeqID_1048
<400> 806
   gaatcccaat atggctttgt ttacggcgtt tcgggaccgg tggtaactgc tgagaaaatg      60
   tccggtgctg caatgtacga actcgtcaga gtcggccatt ctgaattggt gggcgaaatc     120
   atccgattgg aaggcgacta cgccacgatt caagtctacg aggagacttc gtccgtcaca     180
   atcggtgacc ccgtgctgcg gacggggcag cctttgtctg ttgagcttgg cccgggcatt     240
   atgggctcca tttttgacgg aatacaacgg ccattgcacg acatttcaga aatgaccaaa     300
   tccatttaca ttccgaaggg ggtcaatgtg aaggcattaa accgtgcaaa acgatgggaa     360
   tttatgccga ggaaagattt gcgagttggg gaccacgtga cgggcggaga tatcatcgga     420
   atggtgcacg aaagtgcgtt ggtcaagcac aaaattatgg tggagccgaa ggcgtgcgga     480
   acggtcaaat tcatcgcccc gcaaggccaa tacaccgtcg aagacaccat tttggaagtc     540
   gaattcgctg gacacaccca aaagcttacg atgatgcaca tttggccagt cagaaacccc     600
   cgcccgtccg ccgaaaaatt agcggccaat ttccctctcc tctgtgggca gcgcgtgttg     660
   gacgcgctct tcccgtgtgt ccagggcggc acaacggcaa ttcctggtgc ctttggctgt     720
   ggtaaaactg tcatttcgca gtcgttgtcc aaatattcca actccgatgc cattatttac     780
   gtgggctgtg gcgagcgtgg caacgaaatg tcggaagtgt tgcgcgactt tcccgaactg     840
   acgatggaag tggatggagt gaccacgtcc atcatggaga ggacggcgct ggtggccaac     900
   acttcgaata tgcccgtggc ggcgcgtgaa gcgtccattt acacgggaat tactctggcc     960
   gaatacttcc gcgacatggg tctgaacgtg gccatgatgg ccgactcgac ctcccgatgg    1020
   gccgaggcgc tccgtgagat ttcgggccgt ctcggggaaa tgccggctga ctcgggttac    1080
   cccgcctatt tggccgcacg gctcgcttcc ttctacgaaa gggcgggaaa agtgaaatgt    1140
   tttgggtcgc ctgaacgcga agggtccgtc actattgtcg gcgcagtgtc gccgcccggc    1200
   ggagacttcg ccgaccccgt cacatcggca accctcggca ttgtccaagt gttttgggga    1260
   ctcgacaaaa aattggccca acgaaagcac ttcccttcaa tcaattggct catttcttac    1320
   ag                                                                    1322
<210> 807
<400> 807
   000
<210> 808
   <211> 999
   <212> DNA
   <213> Heterodera glycines
<220>
   <223> genomic DNA sequence=SeqID_538; cDNA sequence=SeqID_583
<220>
   <223> peptide sequence=SeqID_1050
<400> 808
   atgatcaaaa gattgacgaa tggaactgag ctcaaactta gtaaaaacgg tacggaactc 60
   agttttcggc ctggtctaat tcatggagga gaattggagt tcgactgtgg ggttcaacgt 120
   tgtatcagtt actttttgga gcctctcatt ctcatggcgc ctttttgcaa aaaacctttg 180
   aatgtgaatc tgaccggtgt tacaaattcc gtcgatgaac tcagcatcga cgccattcgg 240
   gctacttggc tcccggtctt ttcccgcttt gttcttgccg accaatgcac agaaataaaa 300
   atcaaagcca gaggtttcaa gccgagtggc ggtggttcag tgactttctc cagtcccatc 360
   aaaaggaatt tgcgtgctgt gcaatgcatt aagccaggaa aagtttgcaa gatcagaggt 420
   ctcgcttacg tatgcaaagt gacgccatca atcgcatctc gaatgattga cggagctaaa 480
   aagatgcttc acggaaacat ttctgacatt tacattacga ttgaccagag gaagggcccc 540
   cagggcggac tatctcccgg ctttggaatc ttcttgacgg ctgaaaccac cgaaggcgta 600
   tttttccatg gagaagcgat gtcgcgccct aagggccaaa acgaagacca aacagtgccg 660
   gaagaaattg gggaacaggc ggcaagacgt ttgctggatg aaattcatat gggaggctgt 720
   gcagattcgt cctgtcaatc tttggccgcc actttcatga ctctctgcga cagagatgtt 780
   tcgaaatttc tccttggtcc actcacaatt aacagcgtac acacccttcg gaatttgcaa 840
   atgtttttcg agcaaatgtt taaattggag gaatggtgga aggttatgaa agaaatcgtc 900
   ggaaaaggcg gaaatgcgat gaaaaacgga agtgaggaca aatgtttgat gacctgtgtt 960
   ggggttggtt tcttcaacat caacaaagac caactttaa 999
<210> 809
<400> 809
   000
<210> 810
<400> 810
   000
<210> 811
   <211> 2013
   <212> DNA
   <213> Heterodera glycines
<220>
   <223> genomic DNA sequence=SeqID_508; cDNA sequence=SeqID_547
<220>
   <223> peptide sequence=SeqID_1053
<400> 811 ttggcacaac agcaacaaca tgagcagcaa acg 2013
<210> 812
<400> 812
   000
<210> 813
   <211> 1483
   <212> DNA
   <213> Heterodera glycines
<220>
   <223> genomic DNA sequence=SeqID_509; cDNA sequence=SeqID_548
<220>
   <223> peptide sequence=SeqID_1055
<400> 813
   atgaccattt ctgcgaccgc tgacgttgac aagctccagg atgcggccaa ccgaatgcgc      60
   atttcgtcca ttgagcagac gtgcgccgcc aactcgggcc acccgaccag cagttgcagc     120
   gcagcggaaa ttgtggccac tctgttcttt gccgaaatgc gttatgacgt gaaggacccg     180
   cgcagcgcat ccgccgaccg tttcattctg tccaagggtc acgcttgccc aattctgtac     240
   gccgcttggg aagaggcggg cctgctcagc cgtgaccaag tgctctccct gcgcaagatt     300
   gactccgaca ttgaaggaca tccgaccccg cggctcaact tcatcgacgt ggccaccggc     360
   tcgctgggcc agggtctgtc atgcgctgcc ggcatggcgt acactggcaa atatgtggac     420
   cattcgtcct accgcgtgta ctgtttgatg ggcgacggag agagtgcgga aggctccgtg     480
   tgggaggcag cagcatttgc aagtttctac aagttggaca atctggtggc cattgtggac     540
   gttaaccgac ttggacaaag tcaggagacc caactgcagc acgacattga agcgtatgtg     600
   caacggtttg ccgcgttcgg ttggaatgcc atcggattgg aggacggaca cgacatctcg     660
   gcacttttga atgcttacgc caatgccaag aacgtgactg gcaaacccac ggcaatcatc     720
   gccaaaacgc tcaagggcaa gggcattgac ggcgttgccg acctgaacaa ccagcacggg     780
   aagccggtgt cgaaggacaa ggcggagccc atccgggcat tgctcaaaag tgacaagccg     840
   gcaaattgga ccatcccggc accgattcgg gacgcaccgg cggtcgactt aggcatcggt     900
   gccatcgcaa tgagtgcacc gccgcagtac aaaatgggcg aaaaggtggc cactcgacag     960
   gcgtacggaa acgccttgct gaagctggcg gaggccaaca aacgcgtcat tgccttagac    1020
   ggtgacatct ccaactccac cttctccaac ctggtgcaaa agaagctacc cgagcagttt    1080
   gtccagtgtt acattgctga gcagaacatg gtcggtgttg gagttggcat ggcgtgccgt    1140
   gggcgtgtgc ttccgcatgt cagcactttt gccgccttct tcacccgtgc cggtgaccaa    1200
   attcgcatgg gcgccgtgtc cttcacaaat gtcaaattca ccggctctca ctgcggcgtt    1260
   tccatcggcg aggacggtcc cagtcaaatg gccctcgagg acttggcgct gttcagcgct    1320
   ttgcccgcca gtgtcgtcct ctacccatcc gacgcggtgt cggccgaacg tgcgactgag    1380
   ttggccgcca attacacggg catcacttac acgcgcgtct ctcgacccgc caccccagtg    1440
   ctctacacaa gtgacgagaa gttcgagctg ggcaagtgca agg                      1483
<210> 814
   <211> 776
   <212> DNA
   <213> Heterodera glycines
<220>
   <223> genomic DNA sequence=SeqID_510; cDNA sequence=SeqID_549
<220>
   <223> peptide sequence=SeqID_1056
<400> 814
   aatgatggga gggaagcgaa agaaaaaggg gaaaaaagcg gaactgagcg agaaaaggaa      60
   gcggttattg cgaattggga gccgagtccg ttggtgccgc tttactcgga aagggaacaa     120
   aacgacccga attatcggcc ccgttcaatt gaagggcgaa tgggcaaatt tgttgtcatc     180
   gatgacaaac gatgcgtcaa tttggcgtcg actgattttt tcggctttat cggaaaccag     240
   aaaattgaag aagtagccaa aaatgccatt ttcaaatacg gcgtgggctc atgcgggccc     300
   aggaatttct acggaacggt ggacgtacat ttggacttgg aaagggaatt ggctgagttc     360
   atgggctgtg aagaggccat tctgtacagc tacggattcc ccaccatcgc aagtgccatc     420
   ccagcttatt caaggagggg cgatgtcatt tttgcggaca aaggtgttaa tttcgccata     480
   caatccggac tgatggcgtc gagaagtgac attttatggt ttgaacacaa tgacatggcg     540
   gacttggagc gcaaaatggt cgagttggag gagaaagaca aaatgaatcc gaaaaggaaa     600
   gcgtccattc gccgtttgat cgttgtcgaa ggaatttaca caaattcggg cgacatttgc     660
   ccactgccca aactgatcga gctgaaatgg cgacacaaag tgcgcatttt catcgacgag     720
   tccttctctt tcggagtgtt gggaaagcac ggaaaaggtg gggaattcca tttcaa         776
<210> 815
<400> 815
   000
<210> 816
   <211> 1387
   <212> DNA
   <213> Heterodera glycines
<220>
   <223> genomic DNA sequence=SeqID_524; cDNA sequence=SeqID_565
<220>
   <223> peptide sequence=SeqID_1058
<400> 816
   accaagcaga aggagtacga tttgcagcag aagcaattgg aggctcgaat tgcggccatg      60
   caatcggaga atgtgcgcgt tgcggaggaa gaaaggcgca aaacgttaat tgaggagaca     120
   aaacatgcgc gcgcaagagc cgactaccag gaccaattgg cacggaagcg acaagaggag     180
   gagatgagca tgaaggcacg aatgcaggaa gaaacattgc gcaggcagga ggagtcggtg     240
   aagaagcagg aggcactcag aaaggcgacc attgagcatg agctgtcgct acggcacaaa     300
   tacgacttgg aacgcattga cgcggacgtg cgcgccaagg cgcgtgccgc acgcgacaac     360
   cgtgacgtga acctggagat gatgcgtgcg agtgaggagg agcgacgcaa gacggtcacg     420
   gaacagattc ggacaaccgc cggcgttgtg ggcgctggac tgtccgagtt tctctccgat     480
   cgctccaaaa ttatggcggc agtcggtggt ctgactgtgc tggcggtcgg ttggtacagt     540
   gccaagcgtg gcactggcgt tgtggccaaa caggtggaag cacggcttgg taagccgtcc     600
   ctggtgcgtg aaacgtcccg cctcacactg ttcgagctgt tcaaacatcc aattgcgagt     660
   gtgcggacac tgttccgtgc ccgcgaggac ccgctgaagg gcattgtgct caatccaagc     720
   ctcgagtcgc gtctgcgtga cattgcgatc accacgagga acacaaagcg taactttgga     780
   ctgttccgca atgtgttgtt ctacggaccg ccaggcacgg ggaagacaat gttcgccaag     840
   agtttggctc gccattcggg tctcgactac gccataatga ccggcggtga cgtggcaccg     900
   atgggccgtg acggagtgtc cgcaattcat aaaatttttg actgggccca aacgtcgcgc     960
   aaaggtttgg tgctctttgt ggacgaagcg gacgcttttc tgcgcaagcg aagcacggaa    1020
   cagatcagcg aggacatgcg cgccatgctc aacgcgttcc tcttcagaac cggggaacag    1080
   tcgaggaagt tcatgttggt agtggccagc aaccagccgg agcagttcga ctgggctgtc    1140
   aacgatcgtc tggacgaact tgtcgaattt ggactgcccg gtgagctgga gcgtgaacgt    1200
   ctactgctcc agtacttcaa gcagtacatt gccgacccgg ccacctccgg ggcacggacc    1260
   caacgcctga agctgggcga ctttgactgg ctgtccaaaa tgaagcaaat cgcctcgctg    1320
   actgccggca tgtcgggccg tgagctgtcc aaattggtgt ttggctggca agtgagtggg    1380
   tgggcat                                                              1387
<210> 817
<400> 817
   000
<210> 818
<400> 818
   000
<210> 819
<400> 819
   000
<210> 820
<400> 820
   000
<210> 821
<400> 821
   000
<210> 822
<400> 822
   000
<210> 823
<400> 823
   000
<210> 824
<400> 824
   000
<210> 825
<400> 825
   000
<210> 826
<400> 826
   000
<210> 827
<400> 827
   000
<210> 828
<400> 828
   000

<210> 829
<400> 829
   000
<210> 830
<400> 830
   000
<210> 831
<400> 831
   000
<210> 832
<400> 832
   000
<210> 833
<400> 833
   000
<210> 834
<400> 834
   000
<210> 835
<400> 835
   000
<210> 836
<400> 836
   000
<210> 837
<400> 837
   000
<210> 838
<400> 838
   000
<210> 839
<400> 839
   000
<210> 840
<400> 840
   000
<210> 841
<400> 841
   000
<210> 842
<400> 842
   000
<210> 843
<400> 843
   000
<210> 844
<400> 844
   000
<210> 845
<400> 845
   000
<210> 846
<400> 846
   000
<210> 847
<400> 847
   000
<210> 848
<400> 848
   000
<210> 849
<400> 849
   000
<210> 850
<400> 850
   000
<210> 851
<400> 851
   000
<210> 852
<400> 852
   000
<210> 853
<400> 853
   000
<210> 854
<400> 854
   000
<210> 855
<400> 855
   000
<210> 856
<400> 856
   000
<210> 857
<400> 857
   000
<210> 858
<400> 858
   000
<210> 859
<400> 859
   000
<210> 860
<400> 860
   000
<210> 861
<400> 861
   000
<210> 862
<400> 862
   000
<210> 863
<400> 863
   000
<210> 864
<400> 864
   000
<210> 865
<400> 865
   000
<210> 866
<400> 866
   000
<210> 867
<400> 867
   000
<210> 868
<400> 868
   000
<210> 869
<400> 869
   000
<210> 870
<400> 870
   000
<210> 871
<400> 871
   000
<210> 872
<400> 872
   000
<210> 873
<400> 873
   000
<210> 874
<400> 874
   000
<210> 875
<400> 875
   000
<210> 876
<400> 876
   000
<210> 877
<400> 877
   000
<210> 878
<400> 878
   000
<210> 879
<400> 879
   000
<210> 880
<400> 880
   000
<210> 881
<400> 881
   000
<210> 882
<400> 882
   000
<210> 883
<400> 883
   000
<210> 884
<400> 884
   000
<210> 885
<400> 885
   000
<210> 886
<400> 886
   000
<210> 887
<400> 887
   000
<210> 888
<400> 888
   000
<210> 889
<400> 889
   000
<210> 890
<400> 890
   000
<210> 891
<400> 891
   000
<210> 892
<400> 892
   000
<210> 893
<400> 893
   000
<210> 894
<400> 894
   000
<210> 895
<400> 895
   000
<210> 896
<400> 896
   000
<210> 897
<400> 897
   000
<210> 898
<400> 898
   000
<210> 899
<400> 899
   000
<210> 900
<400> 900
   000
<210> 901
<400> 901
   000
<210> 902
<400> 902
   000
<210> 903
<400> 903
   000
<210> 904
<400> 904
   000
<210> 905
<400> 905
   000
<210> 906
<400> 906
   000
<210> 907
<400> 907
   000
<210> 908
<400> 908
   000
<210> 909
<400> 909
   000
<210> 910
<400> 910
   000
<210> 911
<400> 911
   000
<210> 912
<400> 912
   000
<210> 913
<400> 913
   000
<210> 914
<400> 914
   000
<210> 915
<400> 915
   000
<210> 916
<400> 916
   000
<210> 917
<400> 917
   000
<210> 918
<400> 918
   000
<210> 919
<400> 919
   000
<210> 920
<400> 920
   000
<210> 921
<400> 921
   000
<210> 922
<400> 922
   000
<210> 923
<400> 923
   000
<210> 924
<400> 924
   000
<210> 925
<400> 925
   000
<210> 926
<400> 926
   000
<210> 927
<400> 927
   000
<210> 928
<400> 928
   000
<210> 929
<400> 929
   000
<210> 930
<400> 930
   000
<210> 931
<400> 931
   000
<210> 932
<400> 932
   000
<210> 933
<400> 933
   000
<210> 934
<400> 934
   000
<210> 935
<400> 935
   000
<210> 936
<400> 936
   000
<210> 937
<400> 937
   000
<210> 938
<400> 938
   000
<210> 939
<400> 939
   000
<210> 940
<400> 940
   000
<210> 941
<400> 941
   000
<210> 942
<400> 942
   000
<210> 943
<400> 943
   000
<210> 944
<400> 944
   000
<210> 945
<400> 945
   000
<210> 946
<400> 946
   000
<210> 947
<400> 947
   000
<210> 948
<400> 948
   000
<210> 949
<400> 949
   000
<210> 950
<400> 950
   000
<210> 951
<400> 951
   000
<210> 952
<400> 952
   000
<210> 953
<400> 953
   000
<210> 954
<400> 954
   000
<210> 955
<400> 955
   000
<210> 956
   <211> 743
   <212> DNA
   <213> Heterodera glycines
<220>
   <223> genomic DNA sequence=SeqID_434; cDNA sequence=SeqID_716
<220>
   <223> peptide sequence=SeqID_1175
<400> 956
   gggagccaaa aggcaacaaa ttttgcgttt tggtggctgt agggcaaaag gttacgcctc      60
   taatctatcg actcgaccac ggaaaaccaa cgccacaact cattggcaag atcgaaccgg     120
   ccaacggact gaacacggcg atgtgggccc cgcagggcgg ttgggtggtg gtgatggggg     180
   tgaacacgtc cagcggaatt gtgagcttca tcgacgcgac gggggcagag gcgaacaaag     240
   ctaaaacgat tgagcatact tcactcagtc agggttcttg ggacccgacc gggcgatact     300
   ttgccacgtg cactttcgcg ggaaagagtc gctatgagac gggctatcgc attcactcgt     360
   tccaaggacg tgagctgcga cgtaaggcgg tagacgccct gctgcgattc aaatggcgtc     420
   cgcggccacc cgtttcattg tccgagcaaa aagtgcggga gactcgcaaa tcgttgaagc     480
   aattgtcagc gaagtttgag gaggaggaca aaagggaaat gctcaaagtg tcgaaggaaa     540
   taattgaaca gcgcaaaaag gcattggaac tcttctcgaa cattcgcgtc gctgctgcta     600
   aaacatacga agaagaacgg gaaaaacgga ctgagctcag aggtggcatt gaccaggaaa     660
   ttggcgagaa ggacctcgtt gaagaaacaa taacagtggc actaaacacg gagaaggaga     720
   aaatcagaga cgagagcaaa tag                                             743
<210> 957
<400> 957
   000
<210> 958
<400> 958
   000
<210> 959
<400> 959
   000
<210> 960
<400> 960
   000
<210> 961
<400> 961
   000
<210> 962
<400> 962
   000
<210> 963
<400> 963
   000
<210> 964
<400> 964
   000
<210> 965
<400> 965
   000
<210> 966
<400> 966
   000
<210> 967
<400> 967
   000
<210> 968
<400> 968
   000
<210> 969
<400> 969
   000
<210> 970
<400> 970
   000
<210> 971
<400> 971
   000
<210> 972
<400> 972
   000
<210> 973
<400> 973
   000
<210> 974
<400> 974
   000
<210> 975
<400> 975
   000
<210> 976
<400> 976
   000
<210> 977
<400> 977
   000
<210> 978
<400> 978
   000
<210> 979
<400> 979
   000
<210> 980
<400> 980
   000
<210> 981
<400> 981
   000
<210> 982
<400> 982
   000
<210> 983
<400> 983
   000
<210> 984
<400> 984
   000
<210> 985
<400> 985
   000
<210> 986
<400> 986
   000
<210> 987
<400> 987
   000
<210> 988
<400> 988
   000
<210> 989
<400> 989
   000
<210> 990
<400> 990
   000
<210> 991
<400> 991
   000
<210> 992
<400> 992
   000
<210> 993
<400> 993
   000
<210> 994
<400> 994
   000
<210> 995
<400> 995
   000
<210> 996
<400> 996
   000
<210> 997
<400> 997
   000
<210> 998
<400> 998
   000
<210> 999
<400> 999
   000
<210> 1000
<400> 1000
   000
<210> 1001
<400> 1001
   000
<210> 1002
<400> 1002
   000
<210> 1003
<400> 1003
   000
<210> 1004
<400> 1004
   000
<210> 1005
<400> 1005
   000
<210> 1006
<400> 1006
   000
<210> 1007
<400> 1007
   000
<210> 1008
<400> 1008
   000
<210> 1009
<400> 1009
   000

<210> 1010
<400> 1010
   000
<210> 1011
<400> 1011
   000
<210> 1012
<400> 1012
   000
<210> 1013
<400> 1013
   000
<210> 1014
<400> 1014
   000
<210> 1015
<400> 1015
   000
<210> 1016
<400> 1016
   000
<210> 1017
<400> 1017
   000
<210> 1018
<400> 1018
   000
<210> 1019
<400> 1019
   000
<210> 1020
<400> 1020
   000
<210> 1021
<400> 1021
   000
<210> 1022
<400> 1022
   000
<210> 1023
<400> 1023
   000
<210> 1024
<400> 1024
   000
<210> 1025
<400> 1025
   000
<210> 1026
<400> 1026
   000
<210> 1027
<400> 1027
   000
<210> 1028
<400> 1028
   000
<210> 1029
<400> 1029
   000
<210> 1030
<400> 1030
   000
<210> 1031
<400> 1031
   000
<210> 1032
<400> 1032
   000
<210> 1033
<400> 1033
   000
<210> 1034
<400> 1034
   000
<210> 1035
<400> 1035
   000
<210> 1036
<400> 1036
   000
<210> 1037
<400> 1037
   000
<210> 1038
<400> 1038
   000
<210> 1039
<400> 1039
   000
<210> 1040
<400> 1040
   000
<210> 1041
<400> 1041
   000
<210> 1042
<400> 1042
   000
<210> 1043
<400> 1043
   000
<210> 1044
<400> 1044
   000
<210> 1045
<400> 1045
   000
<210> 1046
<400> 1046
   000
<210> 1047
<400> 1047
   000
<210> 1048
<400> 1048
   000
<210> 1049
<400> 1049
   000
<210> 1050
<400> 1050
   000
<210> 1051
<400> 1051
   000
<210> 1052
<400> 1052
   000
<210> 1053
<400> 1053
   000
<210> 1054
<400> 1054
   000
<210> 1055
<400> 1055
   000
<210> 1056
<400> 1056
   000
<210> 1057
<400> 1057
   000
<210> 1058
<400> 1058
   000
<210> 1059
<400> 1059
   000
<210> 1060
<400> 1060
   000
<210> 1061
<400> 1061
   000
<210> 1062
<400> 1062
   000
<210> 1063
<400> 1063
   000
<210> 1064
<400> 1064
   000
<210> 1065
<400> 1065
   000
<210> 1066
<400> 1066
   000
<210> 1067
<400> 1067
   000
<210> 1068
<400> 1068
   000
<210> 1069
<400> 1069
   000
<210> 1070
<400> 1070
   000
<210> 1071
<400> 1071
   000
<210> 1072
<400> 1072
   000
<210> 1073
<400> 1073
   000
<210> 1074
<400> 1074
   000
<210> 1075
<400> 1075
   000
<210> 1076
<400> 1076
   000
<210> 1077
<400> 1077
   000
<210> 1078
<400> 1078
   000
<210> 1079
<400> 1079
   000
<210> 1080
<400> 1080
   000
<210> 1081
<400> 1081
   000
<210> 1082
<400> 1082
   000
<210> 1083
<400> 1083
   000
<210> 1084
<400> 1084
   000
<210> 1085
<400> 1085
   000
<210> 1086
<400> 1086
   000
<210> 1087
<400> 1087
   000
<210> 1088
<400> 1088
   000
<210> 1089
<400> 1089
   000
<210> 1090
<400> 1090
   000
<210> 1091
<400> 1091
   000
<210> 1092
<400> 1092
   000
<210> 1093
<400> 1093
   000
<210> 1094
<400> 1094
   000
<210> 1095
<400> 1095
   000
<210> 1096
<400> 1096
   000
<210> 1097
<400> 1097
   000
<210> 1098
<400> 1098
   000
<210> 1099
<400> 1099
   000
<210> 1100
<400> 1100
   000
<210> 1101
<400> 1101
   000
<210> 1102
<400> 1102
   000
<210> 1103
<400> 1103
   000
<210> 1104
<400> 1104
   000
<210> 1105
<400> 1105
   000
<210> 1106
<400> 1106
   000
<210> 1107
<400> 1107
   000
<210> 1108
<400> 1108
   000
<210> 1109
<400> 1109
   000
<210> 1110
<400> 1110
   000
<210> 1111
<400> 1111
   000
<210> 1112
<400> 1112
   000
<210> 1113
<400> 1113
   000
<210> 1114
<400> 1114
   000
<210> 1115
<400> 1115
   000
<210> 1116
<400> 1116
   000
<210> 1117
<400> 1117
   000
<210> 1118
<400> 1118
   000
<210> 1119
<400> 1119
   000
<210> 1120
<400> 1120
   000
<210> 1121
<400> 1121
   000
<210> 1122
<400> 1122
   000
<210> 1123
<400> 1123
   000
<210> 1124
<400> 1124
   000
<210> 1125
<400> 1125
   000
<210> 1126
<400> 1126
   000
<210> 1127
<400> 1127
   000
<210> 1128
<400> 1128
   000
<210> 1129
<400> 1129
   000
<210> 1130
<400> 1130
   000
<210> 1131
<400> 1131
   000
<210> 1132
<400> 1132
   000
<210> 1133
<400> 1133
   000
<210> 1134
<400> 1134
   000
<210> 1135
<400> 1135
   000
<210> 1136
<400> 1136
   000
<210> 1137
<400> 1137
   000
<210> 1138
<400> 1138
   000
<210> 1139
<400> 1139
   000
<210> 1140
<400> 1140
   000
<210> 1141
<400> 1141
   000
<210> 1142
<400> 1142
   000
<210> 1143
<400> 1143
   000
<210> 1144
<400> 1144
   000
<210> 1145
<400> 1145
   000
<210> 1146
<400> 1146
   000
<210> 1147
<400> 1147
   000
<210> 1148
<400> 1148
   000
<210> 1149
<400> 1149
   000
<210> 1150
<400> 1150
   000
<210> 1151
<400> 1151
   000
<210> 1152
<400> 1152
   000
<210> 1153
<400> 1153
   000
<210> 1154
<400> 1154
   000
<210> 1155
<400> 1155
   000
<210> 1156
<400> 1156
   000
<210> 1157
<400> 1157
   000
<210> 1158
<400> 1158
   000
<210> 1159
<400> 1159
   000
<210> 1160
<400> 1160
   000
<210> 1161
<400> 1161
   000
<210> 1162
<400> 1162
   000
<210> 1163
<400> 1163
   000
<210> 1164
<400> 1164
   000
<210> 1165
<400> 1165
   000
<210> 1166
<400> 1166
   000
<210> 1167
<400> 1167
   000
<210> 1168
<400> 1168
   000
<210> 1169
<400> 1169
   000
<210> 1170
<400> 1170
   000
<210> 1171
<400> 1171
   000
<210> 1172
<400> 1172
   000
<210> 1173
<400> 1173
   000
<210> 1174
<400> 1174
   000
<210> 1175
<400> 1175
   000
<210> 1176
<400> 1176
   000
<210> 1177
<400> 1177
   000
<210> 1178
<400> 1178
   000
<210> 1179
<400> 1179
   000
<210> 1180
<400> 1180
   000
<210> 1181
<400> 1181
   000
<210> 1182
<400> 1182
   000
<210> 1183
<400> 1183
   000
<210> 1184
<400> 1184
   000
<210> 1185
<400> 1185
   000
<210> 1186
<400> 1186
   000
<210> 1187
<400> 1187
   000
<210> 1188
<400> 1188
   000
<210> 1189
<400> 1189
   000
<210> 1190
<400> 1190
   000
<210> 1191
<400> 1191
   000
<210> 1192
<400> 1192
   000
<210> 1193
<400> 1193
   000
<210> 1194
<400> 1194
   000
<210> 1195

<400> 1195
   000
<210> 1196
<400> 1196
   000
<210> 1197
<400> 1197
   000
<210> 1198
<400> 1198
   000
<210> 1199
<400> 1199
   000
<210> 1200
<400> 1200
   000
<210> 1201
<400> 1201
   000
<210> 1202
<400> 1202
   000
<210> 1203
<400> 1203
   000
<210> 1204
<400> 1204
   000
<210> 1205
<400> 1205
   000
<210> 1206
<400> 1206
   000
<210> 1207
<400> 1207
   000
<210> 1208
<400> 1208
   000
<210> 1209
<400> 1209
   000
<210> 1210
<400> 1210
   000
<210> 1211
<400> 1211
   000
<210> 1212
<400> 1212
   000
<210> 1213
<400> 1213
   000
<210> 1214
<400> 1214
   000
<210> 1215
<400> 1215
   000
<210> 1216
<400> 1216
   000
<210> 1217
<400> 1217
   000
<210> 1218
<400> 1218
   000
<210> 1219
<400> 1219
   000
<210> 1220
<400> 1220
   000
<210> 1221
<400> 1221
   000
<210> 1222
<400> 1222
   000
<210> 1223
<400> 1223
   000
<210> 1224
<400> 1224
   000
<210> 1225
<400> 1225
   000
<210> 1226
<400> 1226
   000
<210> 1227
<400> 1227
   000
<210> 1228
<400> 1228
   000
<210> 1229
<400> 1229
   000
<210> 1230
<400> 1230
   000
<210> 1231
<400> 1231
   000
<210> 1232
<400> 1232
   000
<210> 1233
   <211> 899
   <212> PRT
   <213> Heterodera glycines
<220>
   <221> misc_feature
   <222> (600)..(600)
   <223> Xaa can be any naturally occurring amino acid
<220>
   <221> misc_feature
   <222> (618)..(618)
   <223> Xaa can be any naturally occurring amino acid
<220>
   <221> misc_feature
   <222> (834)..(834)
   <223> Xaa can be any naturally occurring amino acid
<220>
   <223> genomic DNA sequence=SeqID_511; coding sequence=SeqID_790
<400> 1233
   Leu Leu Ser Glu Cys Pro Ala Gly Asp Ser Pro Val Phe Leu Leu His
   1               5                   10                  15
   His Asn Thr Ser Val Ala Ser Gly Thr Val Pro Leu Arg Ser Ala Pro
               20                  25                  30
   Val Pro Gly Gly Phe Leu Glu Cys Thr Glu Leu Cys Ser Val Ala Arg
           35                  40                  45
   Gln Cys Gln Leu Val Gly Pro Lys Ser Ala Ser Glu Gly Ala Val Ala
       50                  55                  60
   Glu Phe Gly Glu Glu Met Ala Thr Arg Thr Val Thr Lys Asn Cys Val
   65                  70                  75                  80
   Lys Ser Glu Arg Ile Cys Ser Ser Pro Phe His Phe Asp Val His Glu
                   85                  90                  95
   Gln Lys Ile Leu Val Gly Phe Ala Arg Glu Val Val Ser Ala Asp Ser
               100                 105                 110
   Val His Arg Cys Leu Ser Ala Cys Leu Asn Ala Phe Asp Thr Phe Gly
           115                 120                 125
   Phe Glu Cys Glu Ser Val Met Tyr Tyr Pro Val Asp Ala Glu Cys Ile
       130                 135                 140
   Leu Asn Thr Glu Asp Arg Leu Asp Arg Pro Asp Leu Phe Val Asp Glu
   145                 150                 155                 160
   His Glu Asp Thr Val Ile Tyr Leu Asp Asn Asn Cys Ala Gly Cys Glu
                   165                 170                 175
   Cys His Trp His Phe Asp Asn Phe Lys Thr Ser Gly Ile Leu Asn Asp
   180 185 190
   Gln Gln Phe Ala Ile Ala Ala Gln Cys Tyr Ala Pro Tyr Val Thr Gln
   195 200 205
   Tyr Val Ala Val Glu Gly Arg Gln Leu Ser Asp Glu Leu Asp His Ser
   210 215 220
   Phe Glu Gly Leu Glu Leu Ser Glu Cys Glu Glu Leu Cys Thr Gln Arg
   225 230 235 240
   Leu Ser Val Thr Ala Asn Asp Phe Asn Cys Lys Ser Phe Met Tyr Ser
   245 250 255
   Asn Leu Thr Arg Ser Cys Val Leu Ser Asp Glu Arg Ser Arg Pro Leu
   260 265 270
   Gly Arg Ala Asn Leu Ala Glu Val Pro Gly Trp Thr Tyr Phe Glu Lys
   275 280 285
   Lys Cys Phe Ala Ser Pro Arg Thr Cys Arg Gly Val Pro Ser Phe Thr
   290 295 300
   Arg Val Pro Gln Met Leu Leu Val Gly Phe Ala Ser Phe Val Met Glu
   305 310 315 320
   Asn Val Pro Ser Val Thr Met Cys Leu Asp Gln Cys Thr Asn Pro Pro
   325 330 335
   Pro Glu Thr Gly Gln Asn Phe Val Cys Lys Ser Val Met Tyr Tyr Tyr
   340 345 350
   Asn Glu Gln Glu Cys Ile Leu Asn Ala Glu Ser Arg His Ser Lys Pro
   355 360 365
   Asp Leu Phe Ile Pro Glu Glu Asp Asp Phe Val Val Asp Tyr Phe Asp
   370 375 380
   Ile Asn Cys Arg Leu Glu Ser Glu Gln Cys Ser Ala Gly Glu Gly Ala
   385 390 395 400
   Arg Ser Pro Lys Ile Val Arg Thr Leu Asn Ala Ala Leu Pro Glu Gly
   405 410 415
   Asp Gly Ala Ile His Val Val Glu Thr Val Gln Ala Asn Val Arg Glu
   420 425 430
   Cys Ala Gln Lys Cys Ala Ala His Ser Pro Asp Lys Cys Arg Ser Phe
   435 440 445
   Asn Phe Asp Lys Ser Ser Gly Ile Cys Asn Leu Leu Tyr Leu Asp Gly
   450 455 460
   Arg Gly Ser Leu Arg Pro Glu Pro Lys Gly Gln Val Asp Leu Tyr Asp
   465 470 475 480
   Leu His Cys Leu Glu Thr Ser Pro Glu Gly Gln Ser Met Gly Cys Val
   485 490 495
   Asp Pro Glu Gly Ala Ala Phe Ser Arg His Leu Tyr Ser Gln Trp Val
   500 505 510
   Gly Gly Glu Leu Ser Lys Glu Leu His Ser Val Pro Leu Ser Lys Cys
   515 520 525
   Leu Asn Leu Cys Ala Gly Ala Gly His Cys Glu Gly Leu Asn Tyr Asn
   530 535 540
   Arg Arg Asn Gly Thr Cys Ala Val Ile Gly Gln Leu Gly Thr Val Ser
   545 550 555 560
   Gly Ala Met Gln Lys Ser Glu Gln Val Asp Phe Tyr Arg Asn Val Cys
   565 570 575
   Ala Leu Lys Glu Val Gln Ser Glu Glu Ser Ser Ala Ala Asn Val Pro
   580 585 590
   Lys Ser Lys Arg Asp Met Gly Xaa Arg Arg Asn Glu Arg Asp Gln Ile
   595 600 605
   Lys Asn Trp Glu Ile Asn Val Lys Lys Xaa Lys Lys Ala Leu Val Ser
   610 615 620
   Gly Thr Ser Glu Gly Lys Gly Ala Lys Glu Asp Lys Lys Val Gly Pro
   625 630 635 640
   Pro Ile Gln Ile Pro Gly Ser Glu Val His Thr Ile Cys Asn Tyr Glu
   645 650 655
   Gly Ile Ser Val Gln Ile Lys His Ser Glu Pro Phe Ser Gly Val Met
   660 665 670
   Phe Val Lys Asn Lys Tyr Asp Thr Cys Arg Val Glu Val Gly Thr Glu
   675 680 685
   Asn Ser Val Thr Leu Val Leu Gly Leu Pro Ala Asn Phe Gly Met Lys
   690 695 700
   Pro Ile Thr Leu Thr Ser Pro Glu Gly Lys Gly Ser Phe Gly Asn Arg
   705 710 715 720
   Glu Lys Gln Cys Ser Lys Lys Ala Phe Ala Ser Phe Ala Ile Met Phe
   725 730 735
   Arg Gln Arg Arg Gln Ser Gln Arg Asp Cys Gly Leu Val Asp Met Asp
   740 745 750
   Asn Gly Thr Tyr Lys Thr Val Val Val Val Gln Thr Asn Asn Leu Gly
   755 760 765
   Ile Pro Gly Leu Val Thr Ser Met Asp Gln Leu Tyr Glu Ile Ser Cys
   770 775 780
   Asn Tyr Ser Ser Met Leu Gly Gly Lys Val Thr Ala Ala Ala Asn Leu
   785 790 795 800
   Thr Leu Gln Gly Pro Gln Pro Ser Leu Ile Gln Pro Arg Gly Lys Ile
   805 810 815
   Glu Leu Gly Asn Pro Val Leu Met Gln Met Met Pro Met Arg Glu Ala
   820 825 830
   Asn Xaa Asn Val Pro Gln Ala Lys Leu Gly Asp Ile Leu Glu Leu Arg
   835 840 845
   Trp Glu Leu Met Ala Met Asp Asp Glu Leu Asp Phe Leu Val Lys Asp
   850 855 860
   Cys Tyr Ala Glu Pro Gly Ile Val Pro Pro Lys Gly Ala Ser Ala Pro
   865 870 875 880
   Lys Leu Ala Met Glu Lys Leu Gln Leu Val Gln Gly Gly Ser Val Gly
   885 890 895
   Thr Ala Phe
<210> 1234
<400> 1234
   000
<210> 1235
<400> 1235
   000
<210> 1236
<400> 1236
   000
<210> 1237
<400> 1237
   000
<210> 1238
<400> 1238
   000
<210> 1239
<400> 1239
   000
<210> 1240
<400> 1240
   000
<210> 1241
<400> 1241
   000
<210> 1242
<400> 1242
   000
<210> 1243
<400> 1243
   000
<210> 1244
<400> 1244
   000
<210> 1245
<400> 1245
   000
<210> 1246
<400> 1246
   000
<210> 1247
<400> 1247
   000
<210> 1248
<400> 1248
   000
<210> 1249
<400> 1249
   000
<210> 1250
<400> 1250
   000
<210> 1251
<400> 1251
   000
<210> 1252
<400> 1252
   000
<210> 1253
<400> 1253
   000
<210> 1254
<400> 1254
   000
<210> 1255
<400> 1255
   000
<210> 1256
<400> 1256
   000
<210> 1257
<400> 1257
   000
<210> 1259
<400> 1259
   000
<210> 1260
<400> 1260
   000
<210> 1261
<400> 1261
   000
<210> 1262
<400> 1262
   000
<210> 1263
<400> 1263
   000
<210> 1264
<400> 1264
   000
<210> 1265
<400> 1265
   000
<210> 1266
<400> 1266
   000
<210> 1267
<400> 1267
   000
<210> 1268
<400> 1268
   000
<210> 1269
<400> 1269
   000
<210> 1270
<400> 1270
   000
<210> 1271
<400> 1271
   000
<210> 1272
<400> 1272
   000
<210> 1273
<400> 1273
   000
<210> 1274
<400> 1274
   000
<210> 1275
<400> 1275
   000
<210> 1276
<400> 1276
   000
<210> 1277
<400> 1277
   000
<210> 1278
<400> 1278
   000
<210> 1279
<400> 1279
   000
<210> 1280
<400> 1280
   000
<210> 1281
<400> 1281
   000
<210> 1282
<400> 1282
   000
<210> 1283
<400> 1283
   000
<210> 1284
<400> 1284
   000
<210> 1285
<400> 1285
   000
<210> 1286
<400> 1286
   000
<210> 1287
<400> 1287
   000
<210> 1288
<400> 1288
   000
<210> 1289
   <211> 1808
   <212> DNA
   <213> Heterodera glycines
<220>
   <223> coding sequence=SeqID_1513; coordinates=40-1380
<220>
   <223> peptide sequence=SeqID_1722
<400> 1289
   gcacgaggtc tatttgtttt ttattcaatt tcgacctgaa tgtcagctgc ggtttcttct     60
   tcgcgtccga tggaagattg gaaatctgag ttagctatgc cagacaagga ccttcgatac    120
   aaaacatctg atgtgactgc gactaagggc gttgaatttg aggaatttgg gctcactcgc    180
   gaccttctta aaggcatttt tgagaagggt tgggagcatc caagtccggt tcaggaagcg    240
   tcgattggaa ttgcacttac aggccaagac attctcgctc gcgccaaaaa tggaactggc    300
   aaaactggcg catattgcat tccctgcatt gagaaaattg acccatcaaa agaatacatc    360
   caagcaatga taatcgttcc aactcgggag cttgccttac aaacatctca gatttgcgta    420
   gaactcagca aacacttgaa gattaagatt atggtgacga ctggcggaac agatcttcgt    480
   gacgatattt tgcgtctcaa tggtattgta catcttgttg tggcaactcc tggccgaatt    540
   ttggatctta tggagaaggg agttgcgaat gttgcgtatt gcaagacgat cgttctcgat    600
   gaagcggaca aactcctttc ccaggacttc caaggtgtgc ttgaccgtct tgttcagttt    660
   ctacccaaag accgtcaaat tatgctgtac tccgcaacat ttccgcgcac tgtcgcgact    720
   tttatggaaa agcacatgag caagccgtat gaaatcaatc tgatggagga actgacgctg    780
   cttggcatta cacagtttta tgcttatgtc caagaaaaac agaaagtgca ctgtctgaac    840
   acgcttttcc gcaagctcca aataaaccaa tccatcatct tttgtaattc gacacaacgg    900
   gtggagttac ttgccaagaa aatcacagaa ttgggctact cctgttacta cattcattcc    960
   aaaatggctc aacaccacag gaacagagtg ttccatgaat ttcgtcaggg tcattgtagg   1020
   aacttggtct gttccgatct gcttactcgt ggtattgaca tccaagccgt caacgtggtc   1080
   attaactttg acttcccgcg caattccgag acatatttgc acagaattgg acgttctggc   1140
   cgttttggcc accttggtat tgctatcaat ctgatcacct ttgaagaccg ttacaacctg   1200
   cgacgcatcg aaaaggagct caagacacac attgacccaa ttccaaagga agtcgatccg   1260
   aaactctatg ttgctgaatt tcaaatggat cacaccaaat tcgactgtgg gaatgtcgtc   1320
   gatggtgcgg tgctgaacaa cggagtcgac caacaacagc agcctcgggc ttcgtcgtag   1380
   ttgcggtggg agcacggagc gaaaactccc acagaattta tcagctcgtc catccacagt   1440
   aaattgaccc taataaaaat gctgcgctgc tctccgatct ctcatttgtt gttaattttc   1500
   ggattttgtt atttaaattc tctcatccgc acacgtccgt cacaccattc aaatttatcg   1560
   tccattcatt gtgcgaattt ggtatttctc tgcgaacaca ttgtgtggat ttctatcttt   1620
   gtgacctttt aataaatgtg agagggtttt gtctttgttc gttctcatca ttcacctctt   1680
   tctctccctc acctaaccaa taatcctcca atttactgct tgtctattaa ctttgttacg   1740
   ggtaaattcc aactatgcct caaaaaaaaa aaaacaaaaa aaaaaaaaaa aaaaaaaaaa   1800
   aaaaaaaa                                                            1808
<210> 1290
   <211> 953
   <212> DNA
   <213> Heterodera glycines
<220>
   <223> coding sequence=SeqID_1514; coordinates=52-795
<220>
   <223> peptide sequence=SeqID_1723
<400> 1290
   cccttgggtt taattaccca agtttgagtt gttgaatgca ataatcctaa aatgttccgc      60
   aaccaatacg atagcgacgt tactgtgttc agcccgcagg gccgtcttca ccaaattgat     120
   tatgcagtcg aagcgatgag acagggcagt gcagcagtag caattcgtag caaagattat     180
   gctgttgtgg tcgcacttat gcgtgcacaa aacgagcttt cttcatatca gcagaaagtt     240
   ttcaaattgg acgatcatct cggcttgtct atgtctgggc ttatttcaga tggacgcatt     300
   ttggcacgct ttatgcaaag tgaatgcgcc gagtttcgct ggaactatca taatccaatt     360
   tcggttgaac tgctcaaccg taaattaggc ttgaaacttc agtccaatac ccaatattac     420
   ggacgccgcc catttggtgt gggactgctc atcattggat atgatgacaa agggcctcac     480
   gttgtgaaag ccgaaccctc gggggacgtg tccgaaatgt ttgctgcttc gattggcgct     540
   cggtcacaaa gtgcgcgaac ttatctcgaa aagaacctac cagaatttga gacggctgac     600
   agagacgcat tgattcgcca tgcgattcgc tccctcaaag agacgctgca ggcggacgct     660
   ggcagtttgg atgagaataa cacgtttatt gcgatcatcg gattgggaca accttttacc     720
   tttttgggga aagacgccac gcgggcgcat attcgttcag ttgaagaaga agcacacagg     780
   gaaatggacg aatagagggg cacttttgtc gttttgtccc cgtgttattc ttttaattat     840
   tcttggatta caaaacgatg ctatttcatt gttgtaattt cataaaaggt gacccaaaaa     900
   aaaaaaaaaa aaaaaaaaaa aaaaaaaact cgagactagt tctctctcaa ggg             953
<210> 1291
<400> 1291
   000
<210> 1292
   <211> 947
   <212> DNA
   <213> Heterodera glycines
<220>
   <223> coding sequence=SeqID_1515; coordinates=26-790
<220>
   <223> peptide sequence=SeqID_1724
<400> 1292
   gcacgagggt ttgagcaaat aaactatgga gcattatgat cgtgcaatta cgattttttc 60
   tccggatggc agactttttc aagttgacta tgcgcaagaa gcagtgaaga agggctcaac 120
   agcggttgcc gtgaaaggaa aaaattgtgt tgtaattgga gtcgaaaaaa aatctatccc 180
   gactctgcaa gacgaacgta ccattcgtaa aatacatgca ttggattcgc atgtgatggt 240
   cgcattcgcc ggactcagcg ccgatgcacg cgtccttatc gaccgtgctc gagttgaatg 300
   cgaaaattac aaattgacgc ttgaggaccg cgtatctgtc atgcatatct ctcgcatcat 360
   agctgatata aaacagagat acactcaaac cactggccgt cggccttttg gaatttcaat 420
   gcttgtcgga ggcttcgatt ctgatgggac ccctcatctt ttcaagacgg aaccgtccgg 480
   agtttactac gaattgttgg ccggttcaat agggcgaaat gaaaagattg tgaaggagta 540
   tctggagaaa gaatacggca acgacaacac agctgatgag gagtctacgc ttaaactgat 600
   gctcaagtct ttagccccag tggtccaatc gggggcgcag aatatcgaaa ttgcggtgct 660
   caaatgggtg gatggcacca atggggagcc tgggtattgt gagcagcaaa ttttaaatgt 720
   cgaagcgatt ggagcgctgt tgagtcaaat tgggcctatc gaacaacaga aaccgatgga 780
   aacttcttga tgaattcaat tatttatcag ctgttgttta gaaaatgatt gaatttggat 840
   ctaaatgttt gatataaatt tgttttctta tttggattac agcaacatat tttgaaaaaa 900
   aaaaaaaaaa aaaaaaaaaa aaaaaaaaaa aactcgaggg ggggccc 947
<210> 1293
   <211> 946
   <212> DNA
   <213> Heterodera glycines
<220>
   <223> coding sequence=SeqID_1516; coordinates=30-785
<220>
   <223> peptide sequence=SeqID_1725
<400> 1293
   gcacgaggga attttcgact tacctataaa tgtttctgac ccgcagcgaa tacgacaggg 60
   gagtgaacac tttttccccg gagggccgtc tgttccaagt ggaatatgcc atcgaaactg 120
   tcaagcttgg ttccacaagc atcggaattc acaccaaaga aggcgttctt ttggctgcgg 180
   aaaggcgttc aatgagcaaa ttggtggtgg acgactcaat gagcaaaatt tcggaagttg 240
   agaagcacat tgccgtcgcc tcggccggtc tcatcgcaga ttcacgcact tgggtcgaac 300
   acgcgcgggt ggaggctcaa cacttttggt ttacttacgg tggcaaaatt cgggtggaag 360
   acattactca aaaggtctca agattggcac tgcattttgg agacgacgac tcaactatca 420
   gtctcggccg tccgtttgga gtttctatgc tttttgccgg cattgatcac acgggtgcgc 480
   atctcttcca tttggacccg tccgggacgt acattaaatg tttggccaag gccatcggtg 540
   ccggctccga tgcagcggaa caaacgctgc aagagcactg taaaaactgc gacaaaaaaa 600
   tggaaatggc cgaggcaaaa caagtcgcac tgaacacact caaacagctg atggaagaga 660
   aaatcaattc caaaaatgtg gaaattgtta tgattaagcc gcagacggac aaggaaggca 720
   aaacgttggg caaaattgtg tggttagagg aatcggagtt gcaagaaatc atttcgcgat 780
   tgtagtcgaa ggggacggat tagagaagga aaatgggctt tgcactgccc cttttatgat 840
   tggatgacct tttgttattc tctgcctttt tgtgactttt cagtgtataa ggcaaatgaa 900
   agcaattaat tgagaaaaaa aaaaaaaaaa aaaaaaaaaa aaaaaa 946
<210> 1294
<400> 1294
   000
<210> 1295
<400> 1295
   000
<210> 1296
<400> 1296
   000
<210> 1297
<400> 1297
   000
<210> 1298
   <211> 1028
   <212> DNA
   <213> Heterodera glycines
<220>
   <223> coding sequence=SeqID_1521; coordinates=1-767
<220>
   <223> peptide sequence=SeqID_1729
<400> 1298
   ccgacacggt cggttgttgc tcgttgcgtt gtgagcacat caaattgcac gatgatatgg     60
   acgacctgaa gtatgttgtg gaattcgact tcctcggcaa agactccatc cgctattaca    120
   accgtgtgcc ggtggaaaag caagtgttca aaaatctgaa gctttttatg gaggagaaag    180
   acccggggga cgatttgttc gaccgattgg atacgagttc gttgaaccaa tacctgaagg    240
   aactgatgga cggactgacc gcgaaagtgt ttcgtacgta taacgcgtcg atcactctcc    300
   aagaacagct ggacaaactg acaaatcctt ccgattccgt ccacgagaaa atgctttcct    360
   acaatcgtgc aaatcgtcag gtggccatcc tttgtaacca tcaaagggca gtgccaaaaa    420
   cgcacgaaaa gtcgatggaa aatttggaga ataaaataaa agagaagaag caggagtaca    480
   aggagttgaa ggatcagctg aagaagagca aatcggtgga cgagaaaatg gcaaaaaaat    540
   acgaaagatt gaaggaacag atcgccaaac tgaacacaca gcgcacggac aaggatgaaa    600
   acaaacaaat tgcactggga acctcaaaac tgaactactt ggatccgagg atttcggttg    660
   catggtgcaa aaagaatgac gtgcccgttg agaaaatatt cagcaaaacg caacgcgaca    720
   aattccgctg ggctctcgac atggcgaagg aggacttcat tttctgacac ttcctcgcaa    780
   ccctttccga tttcccttca ccccctttta ctcgctgttc cttacttttg gagagtgccc    840
   ctttcctctc aatcactctc ttctgctgga cctaaactag tagttgttta tatcttgtca    900
   tgcctagttt gtgtgagcgt ttgttttcat atgtctgatg attggttcgt attttattaa    960
   taaaggaagt gttattgaaa aaaaaaaaaa aaaaaaaaaa aaaaaaaact cgaggggggg   1020
   cccggtac                                                            1028
<210> 1299
<400> 1299
   000
<210> 1300
<400> 1300
   000
<210> 1301
<400> 1301
   000
<210> 1302
   <211> 3516
   <212> DNA
   <213> Heterodera glycines
<220>
   <223> coding sequence=SeqID_1525; coordinates=59-3334
<220>
   <223> peptide sequence=SeqID_1732
<400> 1302
   gcacgagggt ttgagtgata gtatccttat tttataaact caaatttgct gaagtatcat 60
   gggagttcca gcctttttcc gttggctttc acgaaagtat gtgtcgatca ttgttgacgc 120
   ggtggaacag cgcagaagag aaattgacgg agtaaaaatc ccaatagatg caactcaacc 180
   aaatgagaat taccaagagt ttgacaatct ttacttggac atgaacggca ttatccatcc 240
   gtgcacacat ccggaagacc gacccgcacc gaagaccgag gaagaaatgt tcattttgat 300
   cttcgagtat attgaccgcc tcttttccat cgttcggcca agaaaacttc tctacatggc 360
   agttgatggg gtcgccccca gagcaaagat gaaccaacag cgaagtcgtc gatttcgagc 420
   agcacaagaa gcggcggaga agcgcgagct catcgaaaat gtgcgtaagc ggttggagtc 480
   ggaaggaatt ccgttgccgc caccgcgaaa ggaagaggaa cactttgact ccaattgcat 540
   aacacccggc acgccgttta tggcccgtct ttctgttgcc cttcgctatt acgtgcacaa 600
   acggatcacc tacgacccgg catggcaaaa gatccaggtt attctttccg atgcgaatgt 660
   gccgggtgag ggcgaacata aaatcatgga ctacgtccga cgtcagcgcg cttcgccggc 720
   gcatgacccg aacaccgttc actgtctttg tggcgccgat gccgacctta tcatgcttgg 780
   attggccacc cacgagtcca atttcaacat aattcgagaa gagtttttgc cgaatcaacc 840
   gaggccgtgc gaactttgcg gtcaatacgg gcacgaggtc gaacattgtg aagggttggc 900
   acgtgccgag accgggccgg accaagcgga cccgctgaag aaggagaaga acttcatctt 960
   catacgtctg ccagtgcttc gggaatattt ggagcgtgaa ctttccatgc caaatttgcc 1020
   atttccgttc gatttggaga gggccatcga tgattgggtc atgatgtgtt tttttgtggg 1080
   caacgatttt ctcccccatt tgccatcgtt agaaatccgt gaaaatgcca tcgaccgtct 1140
   cgtgaagttg tacaaggaca tggtttatca ggctggcggc tggctcactg aaaacggcat 1200
   agtgaatatc gaaagagtga agaagatcat gggcggactt gggaaggttg aggacgaaat 1260
   attcaaacaa agacagctca aagaattgcg cttcaaagaa tcaaagaaac agcgaaagca 1320
   gcaacaacgg caggcaatgg caaaaattat gccgtacaat gccaatttga tagcaccaat 1380
   gccacttgga cagcaaaagc gaatgtcggg cgaggaaatg cgcagattgg cggcgggaga 1440
   ccgacaggag ttcgtcagac ttgcggatgt cgccagttcg cacaaattgc agtcgctgtt 1500
   gacgcctgtc gaccgacgaa aggccacgga tggcggtgat cggaagcgca agttggcccc 1560
   aagttcagtc gacagtccga acggagcacc accagctaaa tcgtttaagg agggagtggc 1620
   tgctgcttcc gtttcctccg aaacggcctt gaaaccggag ataaaactcg taactccctc 1680
   tgggaagacg gcgcctttgg ccaagggggc ggttggggca ctggcatacg actctgacga 1740
   ggaggaggaa gtgctcgacg aaatccgtct atgggaaagt ggatggaagg accgctacta 1800
   cagagccaag ttccaagtgt ccgagactga ccttgaattt cgcaagaaag tggcatgtgc 1860
   ttatgttgag ggactctgct gggtgttgcg ttattattat cagggatgtg ccagctggaa 1920
   atggtactac ccttaccact acgcgccgtt tgcctccgat tttgaactga tcgccgattt 1980
   ccaacccgac ttcaacatgc ccacgaaacc gttcaagcca ttggagcagt taatgggtgt 2040
   gtttcctgct gctagcagag ctcatattcc tgacgggtgg cgttggctga tgaccgactc 2100
   aagttcgccg ataattgatt tttacccgga aacttttgaa attgatctga acgggaagaa 2160
   atatgcttgg caaggcgtcg ctctgcttcc atttgtcgag gaaaaacgtc ttttggatgc 2220
   acttgactcg gtgtacgcca ctctcacaac cgatgaaatt gcgcggaatg ccaccggacc 2280
   agaccgtctt tttgtcggca ataaacacaa cttgttttcg ctcgtttatg aggtgcactc 2340
   ccaaaagacg acaattccga taaaagatga actaacaaat gatgacacaa aagaaacaag 2400
   cgaagggacg cgagaaactg aaggagaagc cgaggaaact caacaggcgg aaaatggtga 2460
   aagcgaacaa atagcgaaag ccgaagaaac gacggcacaa gaggaacaag cgaacgaaga 2520
   agctatctca aaacctgaac aggcaccgaa acaggaattc gagactgaaa ttgaatttgt 2580
   cgatgacgaa gaaaagaaat gggtgcacat cgacgcagtc aaagcgtatg ggatgtctgg 2640
   cgaagtgtct tatgacgtcg aagcactcgc ttttggtgag gcgcatcggt cgctgttcag 2700
   aaattcgttg gagtttggcg acatcaaaga gaatacctgc attatggttg agttccgaga 2760
   tccccaattc ccaatgcact tcatcttcaa agccaacagg ctggacggca tcaaggaatt 2820
   gcctcgtgtg ctcaaaccga acgattggga ccaacgacgg cacggctcct tccaacccca 2880
   aatcggattc agtcgtgaca tcccacgtgc aaatctctcc atggcgggcc atcgtacgct 2940
   caaccaccac ataaacgaca accgtcgcag ttgtccggcc tctcttttct cagtgcaaac 3000
   atcgccacca gcgcacacac ttccatggca catgagcaca aactcaccac aacaacaaca 3060
   tcaaatgcag taccaacatc agcaacaatg gcagcagggt caattttaca acgggcaaca 3120
   gcagcagcag cccgtgccgt tgttcagttt tgggcagatg gcaatgcccc cgcctccgcc 3180
   gctcccaccg caaaattgga ttactagtca cccgccacca caaatccagt ctcacgtgcc 3240
   gtacgggcag cccccacctc taatatcaca gccacggaat actcagcatc accatcgcag 3300
   aaacgacaac aacagaactt acaataggaa ataattttca tcaaccgctg aatagcggtc 3360
   acgatgtgat taattttcta cgaaaatggt gttgccagac cctttctgaa taaaagcatt 3420
   taatccattt ttgtttcaaa tattttgtgg tgtggttaaa cacaactaag ttcaaaaaaa 3480
   aaaaaaaaaa aaaaaaaaaa aaaaaaaaaa aaaaaa 3516
<210> 1303
   <211> 1751
   <212> DNA
   <213> Heterodera glycines
<220>
   <223> coding sequence=SeqID_1526; coordinates=44-1549
<220>
   <223> peptide sequence=SeqID_1733
<400> 1303
   gcacgaggga ctgttctttt tcaccttcct tcaattcaac aaaatggcga aatctgcaat 60
   tgatgttaat cgcggcatca cagaagacca aacagctacc ataagaaatt atttcacaca 120
   accgcactta atttacaaaa ctgtcactgg agtgcgcggg cctttggtca tattgggcga 180
   tgttaaattc ccacaatacg ctgaaattgt gaaacttaca ctgcctaatg gagaaaaacg 240
   catgggacaa gtactcgaag taacgaagga caaagctgtc gttcaggtgt ttgaaggtac 300
   gtcaggtatc gatgcaaaaa acacaatttg cgagttcact ggcgatatta tgagaacacc 360
   cgtttccgag gatatgcttg gaagaatatt caatggatcc ggcacaccca tcgacaaagg 420
   gcccaatgtt ttggcggagg attacttgga catcgatgga cagcctatca acccatggtc 480
   cagaatttat cccgaagaaa tgattcagac cggcatttcc gcaattgatg ttatgaatag 540
   tatcgccagg ggacagaaaa ttccaatttt ttctgctgca ggtcttccgc ataatgagat 600
   cgctgctcaa attgtacgcc aagggggcct cgtcaaacag ccgaacagaa agaaggaaaa 660
   tgaggtggag ggtgacgacg actttgccat tgtgtttgct gcaatgggtg tcaacatgga 720
   gacggcgcgc tttttcaaac aagactttga agaaaacggt tccatggaaa atgtgtgcct 780
   tttccttaac ttggctaatg acccaaccat cgaacggatc atcacaccac gactcgcgct 840
   gacttgtgca gaatttctgg cttaccaatg ccaaaaacac gtcttggttg tgctcactga 900
   catgtcttat tacgctgaag cacttcgcga agtgtccgcc gcccgagagg aagtgcccgg 960
   tcgtcgtggt tttcccggtt acatgtacac tgacttggcc agaatatacg aacgtgccgg 1020
   gcgtgttgaa ggccgaaagg ggtcaatcac acaaattcca attttgtcta tgcctaatga 1080
   cgacatcacc cacccaattc ccgatttgac cggttacata acggaaggcc aaatttatgt 1140
   ggatcgccaa cttcacaatc gtcagattta ccctccaata aatgtacttc catcgctgtc 1200
   gcgtctgatg aaatcggcca ttggcgaggg aatgacccgt gccgatcatt cggatgtgtc 1260
   caaccaactt tacgcatgtt atgccattgg taaagacgtg caagcaatga aagcggtggt 1320
   tggagaggag gcactgtcgt cggatgattt gctctacttg gagtttctca cgaaatttga 1380
   aaagaatttc atttcacaag ggaattatga aaaccggacg gtgttcgact cactcgacat 1440
   tggttggcag ctgttgcgca tttttccaag agaaatgttg aagcgaattc cagcgaaaac 1500
   attggataaa tattacccaa ggggaggcac tggggcgagg aacgaatgag cgaagagttg 1560
   ttaattagtc tgacagatat ccacttaaag attccataat tattcacttg tgtgatttac 1620
   gttatgtcat tgactggatt tttgcatatt tgcggatagt cattattgct tgtattgtat 1680
   tagtatcagt gatgataaca atatacttta cctactttaa aaaaaaaaaa aaaaaaaaaa 1740
   aaaaaaaaaa a 1751

<210> 1304
   <211> 1040
   <212> DNA
   <213> Heterodera glycines
<220>
   <223> coding sequence=SeqID_1487; coordinates=1-824
<220>
   <223> peptide sequence=SeqID_1889
<400> 1304
   gcacgaggcg cgaatatctt cctaaagtgt tgggctcgtc cttcgccgcc acaattggcc      60
   cttaccgcgg ctatgacccg aacatcgatc cgacggtggc caatgaattc acttcgcagg     120
   cgtatcgttt tggacatgga atgattcagg agttttaccc gcgcttggac agtgccgggc     180
   gcaccattcc ctcgggcgga tttgccttcg tcgacggcac tctccactcg gaccacttca     240
   ttttccaggg cggtccggac ccgatcattc gtggaatgat gtctcaacag ttgaaaaggc     300
   cacaacgatt gacgcggacg atcaccgaac aaatgtttgg caccaccgac ttgggcacaa     360
   ttaacatcca acgagggcgc gaccacgggg tgccgtcgta catgcgcttt cggcaattgt     420
   gcgggctttc caccgcgacg acatttgacc aactgagtcg tgagataatg agtgtcgagg     480
   cacgacagaa tttgcagagg atatacggca cgccagaccg aattgatctg ttcgtcggcg     540
   gaatgctcga agacccggtg gtgcgcggtt tggtcggtcc gacagttgcc tgcattattg     600
   gtcctcaatt tgcacggact cgcgacgggg acaggtttta ttttgaaaat ccgggcattt     660
   tcacccgcgc ccaattggcc gaaattcgcc gttcgtcgct cgcccgagtg atttgcgaca     720
   acggcgacga ctttcggaca gtgccgcgcg aagccttccg cacggggcca attgtccagt     780
   gcacagagat accgcaaatg gacctgagca aatggaagga gtgaacgagg agaaaggaat     840
   gttcagctga ttgatcagaa agggacaccg tacgaatcat tggacatacc gtactctctt     900
   accgtattgt attttggaaa tctccgcctt ttctatggtc cctttgccct cctcattttt     960
   ctgtatttgc ttgcatcaaa tttttgcatt tcaaaaaaaa aaaaaaaaaa aaaaaaaaaa    1020
   aaaaaaaaaa aaaaaaaaaa                                                1040
<210> 1305
<400> 1305
   000
<210> 1306
   <211> 1094
   <212> DNA
   <213> Heterodera glycines
<220>
   <223> coding sequence=SeqID_1528; coordinates=34-987
<220>
   <223> peptide sequence=SeqID_1735
<400> 1306
   gcacgaggca tttgttcact ttaatatttt gccatgtctt ttcttgtcac attagccggc      60
   tatttgacaa ttgccatttt tctccagaaa ctctttcgct ctctgtacaa tgtggttttc     120
   ccctatttgt tcgctgttcc tcagaatatc aaggtgctcg cgggtgccga gtgggcagtg     180
   gtcaccggca gcactgacgg cattggcaag gcgtacgcgc tcgagttggc caagaaaaac     240
   ttcaacgtgg tgctcatctc acgctccatg gacaaactga acgcagtggc ggaggaaatc     300
   aaacagaaat atcccaatgt ggaagtgaag tgcatctcat ttgacttcac caacgcaaat     360
   ctgaaggact acgagcagac aattttctcc caactttcca ccatcgaagt tggaatgctc     420
   gtgaacaatg tcgggatgag ctacgagtac cccgagcgat tggaccgtat tgagggcgga     480
   ctgcaacgcg tttccgacat taccgtgatc aacaccctgc cgactacggt actctccgct     540
   tttgtgctca aacagatgag ggaacgggga cgcggcgtcg tcgtcaattt ggcctcctcg     600
   gcagcgtatt tcaattggtt ttacctggcc gcttattctg cgtcgaagaa atacgtgtgt     660
   tggttgtcct ccattctgcg catggaatac gccgacaccg acattgtcat ccaaacggtg     720
   tgcccgatga tggtggccac caaaatggcc aagattcgca aggcttcctt cttcacgccc     780
   agccccgagg aatttgccgc ccaagcgctc cgcagcattg gcttggtcga cgagaccacc     840
   ggctgtttgt cccaccaaat tcaggccgag ctgatgttcg gctacgtccc cgccccgttg     900
   ctgaacaaat tcgtgcgcga caactcactg cagacgcgca agcgcgccct gaagaagaaa     960
   gaagcgacgg tggcgaagag tgaatgatga gaaaaaagaa agaaaagtga tgtgaaatat    1020
   tgacattcat tgaaacaacc aaagtaaatt aaagttattt aataaaaaaa aaaaaaaaaa    1080
   aaaaaaaaaa aaaa                                                       1094
<210> 1307
   <211> 872
   <212> DNA
   <213> Heterodera glycines
<220>
   <223> coding sequence=SeqID_1529; coordinates=63-713
<220>
   <223> peptide sequence=SeqID_1736
<400> 1307
   gcacgagggt ttgagcactt ttgaaaattt tataattgaa ttttgctcat atttttgcat      60
   aaatgaacca agaacaagac aatatcccaa ctttcaagct tgtgttggtt ggtgacggtg     120
   gtaccggaaa gaccactttt gtgaaacgcc atttgactgg agaattcgag aagaaatata     180
   tcgctacgct tggtgttgag gtccatcccc tcacttttca cacttcgcga ggtcaaatcc     240
   gattcaacgt ttgggacaca gccggccagg agaagtttgg tggacttcgt gatgggtatt     300
   acatccaagg ccaatgtgct attgtcatgt tcgacgtgac cgccagagtc acatacaaaa     360
   atgtgccgaa ttggcaccgc gatttggtcc gagtttgtga gaacatccca attgtgctgg     420
   tggggaacaa agtggatgtc aaagatagga aagtcaaggc gaaaagcatc acttttcacc     480
   gcaagaaaaa tctccagtat tacgacattt ccgcaaagag caactacaat ttcgagaagc     540
   ccttccttta tctggcacga aagctgatcg gcgaccccaa cctcgaattc gtggcgatgc     600
   cggcactcgc tccacccgaa gtacaaatgg atcccgcttt ggctgccaaa tacgagcaag     660
   aagtggccga ggcagcaagc gctgaactcc cggacgatga tgaggacatt tgaagagatg     720
   gaaggacggc tgggacagat tcctgggaca gcaatttccg agcagacaaa gttatgtcca     780
   ttgttttaat tgattgtttg atgagtaaag gaaagcgaat gaaaaaaaaa aaaaaaaaaa     840
   aaaaaaaaaa aaactcgagg gggggcccgg ta                                   872
<210> 1308
<400> 1308
   000
<210> 1309
<400> 1309
   000
<210> 1310
   <211> 1045
   <212> DNA
   <213> Heterodera glycines
<220>
   <223> coding sequence=SeqID_1531; coordinates=1-935
<220>
   <223> peptide sequence=SeqID_1737
<400> 1310
   gcacgaggca cgaaaatgtg gcgcccactg acattgagga gggaatgcga gtgggtgtgg 60
   accgcaacaa ataccagatt catttgcctt tgccggcaaa gattgacgcg tccgttacga 120
   tgatgcaagt ggaggacaag ccggacgtta cctacgcgga cattggcggg tgcgaagaac 180
   agatcaaaaa gttgcgtgaa gtggtcgagt ttccgttgct tcagcctgag cgtttcacga 240
   gtttgggcat tgagcctccg aagggcgttt tgttttttgg tccgccgggc accggcaaaa 300
   ctttgtgtgc ccgcgcggtc gccaatcgga cggacgcgtg tttcatccgc gtcatcggtt 360
   ccgaattagt caaaaaatac gttggcgaag gcgcgcgcat ggtgcgcgag ctgttttcgc 420
   tggctaaaac gaaaaaggcg tgcattctct tcttcgacga agtcgacgcc atcggcggag 480
   cgcgatttga cgacggaaaa gggggcgaca acgaagtgca acggacgatg ctcgagttgg 540
   tcaaccaact ggacggattc gactcacgcg gggccatcaa ggttttgatg gccaccaaca 600
   gaccggacac actcgacccg gcgctcattc gtcccggtcg cattgaccga cgcattgaat 660
   tttccttgcc tgacctcaag gcacgaggaa acattctcca aattcacacc aaacggatga 720
   gcgtcgaccg gaacattcgg tacgaattga ttgctcgact ctgtccaaac acgacgggtg 780
   ccgacttgcg cagcgtttgc actgaggcgg gaatgttcgc tttgcgtgca cgtcgaaagg 840
   tcataacgga gcaagacttt ctcaaggctg ttcagaaagt ggtgaaaagc tacgccaagt 900
   tcagttcaac gccggcgtat atgacgcaca actgacaaca cagttcttac aaaacggact 960
   tttttatatt tgtgcacttt tgtttcatta caatataaat gaggaaacca aaaaaaaaaa 1020
   aaaaaaaaaa aaaaaaaaaa aaaaa 1045
<210> 1311
<400> 1311
   000
<210> 1312
   <211> 1188
   <212> DNA
   <213> Heterodera glycines
<220>
   <223> coding sequence=SeqID_1533; coordinates=1-1074
<220>
   <223> peptide sequence=SeqID_1906
<400> 1312
   gcacgagggt ttgaggtgcc tttggctgtg gtaaaactgt catttcgcag tcgttgtcca      60
   aatattccaa ctccgatgcc attatttacg tgggctgtgg cgagcgtggc aacgaaatgt     120
   cggaagtgtt gcgcgacttt cccgaactga cgatggaagt ggatggagtg accacgtcca     180
   tcatggagag gacggcgctg gtggccaaca cttcgaatat gcccgtggcg gcgcgtgaag     240
   cgtccattta cacgggaatt actctggccg aatacttccg cgacatgggt ctgaacgtgg     300
   ccatgatggc cgactcgacc tcccgatggg ccgaggcgct ccgtgagatt tcgggccgtc     360
   tcggggaaat gccggctgac tcgggttacc ccgcctattt ggccgcacgg ctcgcttcct     420
   tctacgaaag ggcgggaaaa gtgaaatgtt ttgggtcgcc tgaacgcgaa gggtccgtca     480
   ctattgtcgg cgcagtgtcg ccgcccggcg gagacttcgc cgaccccgtc acatcggcaa     540
   ccctcggcat tgtccaagtg ttttggggac tcgacaaaaa attggcccaa cgaaagcact     600
   tcccttcaat caattggctc atttcttaca gcaaatatat gcgtgcgttg gacgattatt     660
   acgagaagaa ttttgccgaa tttgttccac tgagaacgaa atgcaaagaa attttgcagg     720
   aagaggaaga tttgtcagaa attgtccaat tggtcggcaa agcatcgctt gccgaggccg     780
   acaaaatcac tttggagcta tgatcgcttc tgtcccttct acaaaactgt tgggatgctc     840
   aaaaatatga ttggctttta cgactttgcc cggcactcgg tggaaacgac tgcacaatcg     900
   gacaacaaaa tcacgtggaa tcagatcaga gatcacctcg gggacatcat tcacgaactt      960
   tcttcgatga aatttgagga cccatccgaa aaaacggaag atcagatcaa aaaggattac    1020
   gaagaactgt atgaacggat gcagaacaaa ttccgagcgt tagaggatga atgaacaaac    1080
   tgtgatcaat gataacaatt tctcaaaatt ttccctcatt tctcttagtg tagtgatgga    1140
   aaaaacgtct cagtgaaaaa aaaaaaaaaa aaaaaaaaaa aaaaaaaa                 1188
<210> 1313
<400> 1313
   000
<210> 1314
<400> 1314
   000
<210> 1315
<400> 1315
   000
<210> 1316
<400> 1316
   000
<210> 1317
<400> 1317
   000
<210> 1318
   <211> 1745
   <212> DNA
   <213> Heterodera glycines
<220>
   <223> coding sequence=SeqID_1537; coordinates=1-1629
<220>
   <223> peptide sequence=SeqID_1741
<400> 1318
   gcacgagggt ccccttctct ttccgtcgcc aactcaaatg tctctcgaca gcttggccaa     60
   ccaaaagtag cgggcttcaa agataaggat aaaccggaaa gtgttcgtac cagcaacatc    120
   gtggcagcaa aggctgttgc ggatgcggtg cggacgagtc ttgggcctcg gggaatggac    180
   aaaatgatcc aatcgggcaa cggagaagtg acaattacca atgacggggc gactattctc    240
   aaccaaatgt cggttgtgca tcccaccgca aaaatgttgg tcgagctgtc caaagcgcag    300
   gacattgagg cgggcgatgg gaccacaact gttgtggtgt tggccgggtc cttgttggac    360
   gctgcagaaa aacttcttgc caaaggaatt catccgacca ccatttccga ctcattccaa    420
   cgggcggcca cattttctga aaaagttctc gaggaaatgg ccacacccat tgacattaac    480
   gatgacgacg agttggtgaa gctcgccatg acgagtttga actcgaaggt cgtttcgcaa    540
   cattcccatc ttttggcccc aatggcggtg caggcagtga agaaactgat gagttcaccg    600
   gaggactcaa gtgtcaatct gaagctgatc aaagtcgtga aaaagttggg cgaaacggtg    660
   gaggagtcgc agctcattga gggagcgctg atcgatcaga agtcgatggg ccacggaggc    720
   ccggcgcggg tggagaaggc gaagattggc ctcatccaat tccaaatctc accaccaaag    780
   cccaacatgg aaaatcaagt ggtcatctct gactatgctc aaatggaccg tgcgctgaag    840
   gaagaacgaa attacatttt ggagctgtgc aagcaaatca aaaaggccgg gtgcaatgtg    900
   ttgctcatcc aaaagtccat tttacgcgat gccatcaacg aaatggcgct gcactttttc    960
   gcaaaaatga aaattatggt gatcaaagac atcgagcggg atgacatcga attctatgcc   1020
   aaaatcctcg ggtgtcgccc ggtggcatcc gttgaccatt ttgtgccgga atcgctcgga   1080
   acggccgatt tggtggagga aattgagacg gctgatggga aagtcctcaa agtgaccggc   1140
   cttaaacaga gtgggtgcgc tgtgtccatt ttggttcgcg gctccagcaa attggtgttg   1200
   gacgaagccg agcgctctct gcacgacgca ttgtgcgtca ttcgctgttt ggtgaagaaa   1260
   cgcgcccttc tttccggcgg cggtgccccc gaaatggaag tgtccgtccg cgtccgcgca   1320
   gctgctcaga caaaacacgg agccgaacag tattgctgga aggcatttgc ggacgcactc   1380
   gaagtggtgc catacacttt ggcagaaaac gctggactgt cgcccattga aacagtgacg   1440
   gagatgcgca gtcagcacgc aatgggaaac aaagagcacg gagtgaatgt gcgaatgggt   1500
   gccattacgg acattcgtga ggaaaatgtg gtgcagcctc ttttggtcag ttcgttcgcc   1560
   gtcaaacagg cggctgaatg tgtgcgcagc attctcaaaa tcgacgacat tgttctgtcc   1620
   gtgcgttaaa gtgacggaaa tgcaattaga aatttgtgga cagccatttt tgaatgttat   1680
   gacgttctct cgcataaatc gctttttact aaaaaaaaaa aaaaaaaaaa aaaaaaaaaa   1740
   aaaaa                                                               1745
<210> 1319
<400> 1319
   000
<210> 1320
<400> 1320
   000
<210> 1321
<400> 1321
   000
<210> 1322
<400> 1322
   000
<210> 1323
<400> 1323
   000
<210> 1324
<400> 1324
   000
<210> 1325
<400> 1325
   000
<210> 1326
<400> 1326
   000
<210> 1327
   <211> 1083
   <212> DNA
   <213> Heterodera glycines
<220>
   <223> coding sequence=SeqID_1545; coordinates=1-960
<220>
   <223> peptide sequence=SeqID_1748
<400> 1327
   gcacgaggat taaaattgaa agttccaatt ctgctgtatt gttttgttca atgtttaagt 60
   gctgtcaaag tggacaatgc cgagcaatcc gaccaaccaa ctggcgattc ttcctccgga 120
   caattcccaa tttcgtatcc tccaataaaa tatgaaaacg atttggccta tgtggagaaa 180
   ccgagtggca acgagcgttg tctattctac gcggaaggac tttctgtcgt cgtttttgcc 240
   actaaaaaaa gtcaatcggc gtgggcggac tatcgctctg acaccaaaga aagtccgaag 300
   tataccgcgt caattgaatg ccaaggcaat atgaatgtgg gcagcagctt tgatgtctat     360
   ttgaaaattg acggcaaagc ggaaggagtt gtgagagctg acattggaaa aaaggacgca     420
   aaaagtgtct tcactgttga aggagccgaa tttatactca acttcaacgt cacacaagtc     480
   ggcttttggc aattggttgg tgccaccgtg gaaagaattg aagtgaaagg ctcttcggat     540
   ttcctgaaca aaaatttgaa tgtcgacaaa aaatggactt tggagccttc agttgttcca     600
   tccacaaaca ttaacggctt caaagactac agctttgctt gtggtcagac caacccgata     660
   gtgtgggccc taaaggactc aaatgattac gcagtcggaa ttacgctgaa caatgtgcag     720
   ttccaaccat tcaatgcaac ttacactgcc ggtggaaaag aagctgaccg tttaaaattt     780
   gggtggaaag tcaacgattg ccaggggctg ttcagcattg gcacttggat gggcttggta     840
   gttgccactc tgctcatcgg cgtgctcttt ttcggctttc tgatgcttaa ttctgtgcag     900
   acaatggaca gattcgatga tccgaagcag aaacagttga taattagttc gaaagagtga     960
   atttcatcat tttcttttat tacatttccc tcaaaatttt tcattctttg tagctttata    1020
   aatataaatt ttttccttga aaaaaaaaaa aaaaaaaaaa aaaaaaaaaa aaaaaaaaaa    1080
   aaa                                                                  1083
<210> 1328
<400> 1328
   000
<210> 1329
<400> 1329
   000
<210> 1330
   <211> 1452
   <212> DNA
   <213> Heterodera glycines
<220>
   <223> coding sequence=SeqID_1548; coordinates=1-1283
<220>
   <223> peptide sequence=SeqID_1751
<400> 1330
   ttcctctctt tgggataatg tccacggaca gtgctgaccc cctctattgg ctccgtgtga 60
   tcatggcttc caacagaggc acactgatgg agttgggcat ttcgcccatt gtcacttccg 120
   gtctcataat gcaacttctc gctggtgcga agattatcga agtgggcgac acgccaaagg 180
   aacgggcatt gttcaacggg gcgcaaaaac tgttcggaat ggtcatcact attggacagg 240
   caattgtcta cgtggcttcc ggaatgtacg gaaacccaca cgaaatcggc gccggaattt 300
   gcttgcttat cgtaattcaa ctcgtcgtgg ctggtctcat tgtccttctg ctggacgaat 360
   tgcttcagaa agggtatgga cttggctcgg gaatttcgct gtttatcgcc acaaacattt 420
   gcgagaccat cgtttggaag gccttttcac cggcgacgat gaacacgggc cgtggcacgg 480
   aatttgaggg ggcgatcatc gcacttttcc atctgttggc cactcggaat gacaaagtcc 540
   gtgcacttcg tgaagctttc taccgacaga atttgccaaa tttgatgaat ctgctggcca 600
   ctgttctagt ttttgccgtc gtcatttatt tccaaggttt tcgtgtcgat ttgcccataa 660
   aaagtgctcg ctaccgcggc cagcacagtt cctatccgat caagctgttc tacaccagca 720
   acatccccat catcctccaa tctgcgctcg tctccaacct ttacgtgatc tcacaaatgc 780
   tggcggccaa attcggcggc aacattttgg ttaatttgct cgggacgtgg tccgactctt 840
   ccggcacata tcggagcttc ccaaccggtg gtatctgcta ttatctgtcc ccgcccgagt 900
   cgctctccca cattttggag gaccccatcc actgcatcat ttacattgtg ttcatgcttg 960
   gctcgtgcgc cttcttttct aagacttgga tcgacgtcag cggctcaagt gccaaagatg 1020
   tagccaaaca actgaaggag cagcagatga tcatgcgagg ccatcgcgag aaatcgatga 1080
   tccacgagct caaccggtac attccgacag cggctgcttt cggtgggctg tgcatcggcg 1140
   cattgtcagt cacggcagat tttttgggcg caattggaag tggcacgggt attctgctgg 1200
   ctgtcacaat catttaccag tactttgaaa ttttcgtcaa agaacagcaa gaaatgggcg 1260
   gagttgccgg actctttttc tgagccaatt tttcttgcgc agaatttata catatataaa 1320
   cgacacattc actcatttcc atctaaagtt gaactttgtt gttttccctc acttttggca 1380
   tcacataatt ctcttttgtt caaaaaaaaa aaaaaaaaaa aaaaaaaaaa aactcgaggg 1440
   gggggcccgg ta 1452
<210> 1331
<400> 1331
   000
<210> 1332
<400> 1332
   000
<210> 1333
<400> 1333
   000
<210> 1334
<400> 1334
   000
<210> 1335
<400> 1335
   000
<210> 1336
<400> 1336
   000
<210> 1337
<400> 1337
   000
<210> 1338
<400> 1338
   000
<210> 1339
<400> 1339
   000
<210> 1340
<400> 1340
   000
<210> 1341
<400> 1341
   000
<210> 1342
<400> 1342
   000
<210> 1343
<400> 1343
   000
<210> 1344
<400> 1344
   000
<210> 1345
<400> 1345
   000
<210> 1346
<400> 1346
   000
<210> 1347
<400> 1347
   000
<210> 1348
<400> 1348
   000
<210> 1349
<400> 1349
   000
<210> 1350
<400> 1350
   000
<210> 1351
<400> 1351
   000
<210> 1352
<400> 1352
   000
<210> 1353
<400> 1353
   000
<210> 1354
<400> 1354
   000
<210> 1355
<400> 1355
   000
<210> 1356
<400> 1356
   000
<210> 1357
<400> 1357
   000
<210> 1358
<400> 1358
   000
<210> 1359
<400> 1359
   000
<210> 1360
<400> 1360
   000
<210> 1361
<400> 1361
   000
<210> 1362
<400> 1362
   000
<210> 1363
<400> 1363
   000
<210> 1364
<400> 1364
   000
<210> 1365
<400> 1365
   000
<210> 1366
<400> 1366
   000
<210> 1367
<400> 1367
   000
<210> 1368
<400> 1368
   000
<210> 1369
<400> 1369
   000
<210> 1370
<400> 1370
   000
<210> 1371
<400> 1371
   000
<210> 1372
<400> 1372
   000
<210> 1373
<400> 1373
   000
<210> 1374
<400> 1374
   000
<210> 1375
<400> 1375
   000
<210> 1376
<400> 1376
   000
<210> 1377
<400> 1377
   000
<210> 1378
<400> 1378
   000
<210> 1379
<400> 1379
   000
<210> 1380
<400> 1380
   000
<210> 1381
<400> 1381
   000
<210> 1382
<400> 1382
   000
<210> 1383
<400> 1383
   000
<210> 1384
<400> 1384
   000
<210> 1385
<400> 1385
   000
<210> 1386
<400> 1386
   000
<210> 1387
<400> 1387
   000
<210> 1388
<400> 1388
   000
<210> 1389
<400> 1389
   000
<210> 1390
<400> 1390
   000
<210> 1391
<400> 1391
   000
<210> 1392
<400> 1392
   000
<210> 1393
<400> 1393
   000
<210> 1394
<400> 1394
   000
<210> 1395
<400> 1395
   000
<210> 1396
<400> 1396
   000
<210> 1397
<400> 1397
   000
<210> 1398
<400> 1398
   000
<210> 1399
<400> 1399
   000
<210> 1400
<400> 1400
   000
<210> 1401
<400> 1401
   000
<210> 1402
<400> 1402
   000
<210> 1403
<400> 1403
   000
<210> 1404
<400> 1404
   000
<210> 1405
<400> 1405
   000
<210> 1406
<400> 1406
   000
<210> 1407
<400> 1407
   000
<210> 1408
<400> 1408
   000
<210> 1409
<400> 1409
   000
<210> 1410
<400> 1410
   000
<210> 1411
<400> 1411
   000
<210> 1412
<400> 1412
   000
<210> 1413
<400> 1413
   000
<210> 1414
<400> 1414
   000
<210> 1415
<400> 1415
   000
<210> 1416
<400> 1416
   000
<210> 1417
<400> 1417
   000
<210> 1418
<400> 1418
   000
<210> 1419
<400> 1419
   000
<210> 1420
<400> 1420
   000
<210> 1421
<400> 1421
   000
<210> 1422
<400> 1422
   000
<210> 1423
<400> 1423
   000
<210> 1424
<400> 1424
   000
<210> 1425
<400> 1425
   000
<210> 1426
<400> 1426
   000
<210> 1427
<400> 1427
   000

<210> 1428
<400> 1428
   000
<210> 1429
<400> 1429
   000
<210> 1430
<400> 1430
   000
<210> 1431
<400> 1431
   000
<210> 1432
<400> 1432
   000
<210> 1433
<400> 1433
   000
<210> 1434
<400> 1434
   000
<210> 1435
<400> 1435
   000
<210> 1436
<400> 1436
   000
<210> 1437
<400> 1437
   000
<210> 1438
<400> 1438
   000
<210> 1439
<400> 1439
   000
<210> 1440
<400> 1440
   000
<210> 1441
<400> 1441
   000
<210> 1442
<400> 1442
   000
<210> 1443
<400> 1443
   000
<210> 1444
<400> 1444
   000
<210> 1445
<400> 1445
   000
<210> 1446
<400> 1446
   000
<210> 1447
<400> 1447
   000
<210> 1448
<400> 1448
   000
<210> 1449
<400> 1449
   000
<210> 1450
<400> 1450
   000
<210> 1451
<400> 1451
   000
<210> 1452
<400> 1452
   000
<210> 1453
<400> 1453
   000
<210> 1454
<400> 1454
   000
<210> 1455
<400> 1455
   000
<210> 1456
<400> 1456
   000
<210> 1457
<400> 1457
   000
<210> 1458
<400> 1458
   000
<210> 1459
<400> 1459
   000
<210> 1460
<400> 1460
   000
<210> 1461
<400> 1461
   000
<210> 1462
<400> 1462
   000
<210> 1463
<400> 1463
   000
<210> 1464
<400> 1464
   000
<210> 1465
<400> 1465
   000
<210> 1466
<400> 1466
   000
<210> 1467
<400> 1467
   000
<210> 1468
<400> 1468
   000
<210> 1469
<400> 1469
   000
<210> 1470
<400> 1470
   000
<210> 1471
<400> 1471
   000
<210> 1472
<400> 1472
   000
<210> 1473
<400> 1473
   000
<210> 1474
<400> 1474
   000
<210> 1475
<400> 1475
   000
<210> 1476
<400> 1476
   000
<210> 1477
<400> 1477
   000
<210> 1478
<400> 1478
   000
<210> 1479
<400> 1479
   000
<210> 1480
<400> 1480
   000
<210> 1481
<400> 1481
   000
<210> 1482
<400> 1482
   000
<210> 1483
<400> 1483
   000
<210> 1484
<400> 1484
   000
<210> 1485
<400> 1485
   000
<210> 1486
<400> 1486
   000
<210> 1487
   <211> 824
   <212> DNA
   <213> Heterodera glycines
<220>
   <223> cDNA sequence=SeqID_1304; peptide sequence=SeqID_1889
<400> 1487
   gcacgaggcg cgaatatctt cctaaagtgt tgggctcgtc cttcgccgcc acaattggcc      60
   cttaccgcgg ctatgacccg aacatcgatc cgacggtggc caatgaattc acttcgcagg     120
   cgtatcgttt tggacatgga atgattcagg agttttaccc gcgcttggac agtgccgggc     180
   gcaccattcc ctcgggcgga tttgccttcg tcgacggcac tctccactcg gaccacttca     240
   ttttccaggg cggtccggac ccgatcattc gtggaatgat gtctcaacag ttgaaaaggc     300
   cacaacgatt gacgcggacg atcaccgaac aaatgtttgg caccaccgac ttgggcacaa     360
   ttaacatcca acgagggcgc gaccacgggg tgccgtcgta catgcgcttt cggcaattgt     420
   gcgggctttc caccgcgacg acatttgacc aactgagtcg tgagataatg agtgtcgagg     480
   cacgacagaa tttgcagagg atatacggca cgccagaccg aattgatctg ttcgtcggcg     540
   gaatgctcga agacccggtg gtgcgcggtt tggtcggtcc gacagttgcc tgcattattg     600
   gtcctcaatt tgcacggact cgcgacgggg acaggtttta ttttgaaaat ccgggcattt     660
   tcacccgcgc ccaattggcc gaaattcgcc gttcgtcgct cgcccgagtg atttgcgaca     720
   acggcgacga ctttcggaca gtgccgcgcg aagccttccg cacggggcca attgtccagt     780
   gcacagagat accgcaaatg gacctgagca aatggaagga gtga                      824
<210> 1488
<400> 1488
   000
<210> 1489
<400> 1489
   000
<210> 1490
<400> 1490
   000
<210> 1491
<400> 1491
   000
<210> 1492
<400> 1492
   000
<210> 1493
<400> 1493
   000
<210> 1494
<400> 1494
   000
<210> 1495
<400> 1495
   000
<210> 1496
<400> 1496
   000
<210> 1497
<400> 1497
   000
<210> 1498
<400> 1498
   000
<210> 1499
<400> 1499
   000
<210> 1500
<400> 1500
   000
<210> 1501
<400> 1501
   000
<210> 1502
<400> 1502
   000
<210> 1503
<400> 1503
   000
<210> 1504
<400> 1504
   000
<210> 1505
<400> 1505
   000
<210> 1506
<400> 1506
   000
<210> 1507
<400> 1507
   000
<210> 1508
<400> 1508
   000
<210> 1509
<400> 1509
   000
<210> 1510
<400> 1510
   000
<210> 1511
<400> 1511
   000
<210> 1512
<400> 1512
   000
<210> 1513
   <211> 1341
   <212> DNA
   <213> Heterodera glycines
<220>
   <223> cDNA sequence=SeqID_1289; peptide sequence=SeqID_1722
<400> 1513
   atgtcagctg cggtttcttc ttcgcgtccg atggaagatt ggaaatctga gttagctatg      60
   ccagacaagg accttcgata caaaacatct gatgtgactg cgactaaggg cgttgaattt     120
   gaggaatttg ggctcactcg cgaccttctt aaaggcattt ttgagaaggg ttgggagcat     180
   ccaagtccgg ttcaggaagc gtcgattgga attgcactta caggccaaga cattctcgct     240
   cgcgccaaaa atggaactgg caaaactggc gcatattgca ttccctgcat tgagaaaatt     300
   gacccatcaa aagaatacat ccaagcaatg ataatcgttc caactcggga gcttgcctta     360
   caaacatctc agatttgcgt agaactcagc aaacacttga agattaagat tatggtgacg     420
   actggcggaa cagatcttcg tgacgatatt ttgcgtctca atggtattgt acatcttgtt     480
   gtggcaactc ctggccgaat tttggatctt atggagaagg gagttgcgaa tgttgcgtat     540
   tgcaagacga tcgttctcga tgaagcggac aaactccttt cccaggactt ccaaggtgtg     600
   cttgaccgtc ttgttcagtt tctacccaaa gaccgtcaaa ttatgctgta ctccgcaaca     660
   tttccgcgca ctgtcgcgac ttttatggaa aagcacatga gcaagccgta tgaaatcaat     720
   ctgatggagg aactgacgct gcttggcatt acacagtttt atgcttatgt ccaagaaaaa     780
   cagaaagtgc actgtctgaa cacgcttttc cgcaagctcc aaataaacca atccatcatc     840
   ttttgtaatt cgacacaacg ggtggagtta cttgccaaga aaatcacaga attgggctac     900
   tcctgttact acattcattc caaaatggct caacaccaca ggaacagagt gttccatgaa     960
   tttcgtcagg gtcattgtag gaacttggtc tgttccgatc tgcttactcg tggtattgac    1020
   atccaagccg tcaacgtggt cattaacttt gacttcccgc gcaattccga gacatatttg    1080
   cacagaattg gacgttctgg ccgttttggc caccttggta ttgctatcaa tctgatcacc    1140
   tttgaagacc gttacaacct gcgacgcatc gaaaaggagc tcaagacaca cattgaccca    1200
   attccaaagg aagtcgatcc gaaactctat gttgctgaat ttcaaatgga tcacaccaaa    1260
   ttcgactgtg ggaatgtcgt cgatggtgcg gtgctgaaca acggagtcga ccaacaacag    1320
   cagcctcggg cttcgtcgta g                                              1341
<210> 1514
<400> 1514
   000
<210> 1515
<400> 1515
   000
<210> 1516
   <211> 756
   <212> DNA
   <213> Heterodera glycines
<220>
   <223> cDNA sequence=SeqID_1293; peptide sequence=SeqID_1725
<400> 1516
   atgtttctga cccgcagcga atacgacagg ggagtgaaca ctttttcccc ggagggccgt 60
   ctgttccaag tggaatatgc catcgaaact gtcaagcttg gttccacaag catcggaatt 120
   cacaccaaag aaggcgttct tttggctgcg gaaaggcgtt caatgagcaa attggtggtg 180
   gacgactcaa tgagcaaaat ttcggaagtt gagaagcaca ttgccgtcgc ctcggccggt 240
   ctcatcgcag attcacgcac ttgggtcgaa cacgcgcggg tggaggctca acacttttgg 300
   tttacttacg gtggcaaaat tcgggtggaa gacattactc aaaaggtctc aagattggca 360
   ctgcattttg gagacgacga ctcaactatc agtctcggcc gtccgtttgg agtttctatg 420
   ctttttgccg gcattgatca cacgggtgcg catctcttcc atttggaccc gtccgggacg 480
   tacattaaat gtttggccaa ggccatcggt gccggctccg atgcagcgga acaaacgctg 540
   caagagcact gtaaaaactg cgacaaaaaa atggaaatgg ccgaggcaaa acaagtcgca 600
   ctgaacacac tcaaacagct gatggaagag aaaatcaatt ccaaaaatgt ggaaattgtt 660
   atgattaagc cgcagacgga caaggaaggc aaaacgttgg gcaaaattgt gtggttagag 720
   gaatcggagt tgcaagaaat catttcgcga ttgtag 756
<210> 1517
<400> 1517
   000
<210> 1518
<400> 1518
   000
<210> 1519
<400> 1519
   000
<210> 1520
<400> 1520
   000
<210> 1521
   <211> 767
   <212> DNA
   <213> Heterodera glycines
<220>
   <223> cDNA sequence=SeqID_1298; peptide sequence=SeqID_1729
<400> 1521
   ccgacacggt cggttgttgc tcgttgcgtt gtgagcacat caaattgcac gatgatatgg     60
   acgacctgaa gtatgttgtg gaattcgact tcctcggcaa agactccatc cgctattaca    120
   accgtgtgcc ggtggaaaag caagtgttca aaaatctgaa gctttttatg gaggagaaag    180
   acccggggga cgatttgttc gaccgattgg atacgagttc gttgaaccaa tacctgaagg    240
   aactgatgga cggactgacc gcgaaagtgt ttcgtacgta taacgcgtcg atcactctcc    300
   aagaacagct ggacaaactg acaaatcctt ccgattccgt ccacgagaaa atgctttcct    360
   acaatcgtgc aaatcgtcag gtggccatcc tttgtaacca tcaaagggca gtgccaaaaa    420
   cgcacgaaaa gtcgatggaa aatttggaga ataaaataaa agagaagaag caggagtaca    480
   aggagttgaa ggatcagctg aagaagagca aatcggtgga cgagaaaatg gcaaaaaaat    540
   acgaaagatt gaaggaacag atcgccaaac tgaacacaca gcgcacggac aaggatgaaa    600
   acaaacaaat tgcactggga acctcaaaac tgaactactt ggatccgagg atttcggttg    660
   catggtgcaa aaagaatgac gtgcccgttg agaaaatatt cagcaaaacg caacgcgaca    720
   aattccgctg ggctctcgac atggcgaagg aggacttcat tttctga                  767
<210> 1522
<400> 1522
   000
<210> 1523
<400> 1523
   000
<210> 1524
<400> 1524
   000
<210> 1525
   <211> 3276
   <212> DNA
   <213> Heterodera glycines
<220>
   <223> cDNA sequence=SeqID_1302; peptide sequence=SeqID_1732
<400> 1525
   atgggagttc cagccttttt ccgttggctt tcacgaaagt atgtgtcgat cattgttgac      60
   gcggtggaac agcgcagaag agaaattgac ggagtaaaaa tcccaataga tgcaactcaa     120
   ccaaatgaga attaccaaga gtttgacaat ctttacttgg acatgaacgg cattatccat     180
   ccgtgcacac atccggaaga ccgacccgca ccgaagaccg aggaagaaat gttcattttg     240
   atcttcgagt atattgaccg cctcttttcc atcgttcggc caagaaaact tctctacatg     300
   gcagttgatg gggtcgcccc cagagcaaag atgaaccaac agcgaagtcg tcgatttcga      360
   gcagcacaag aagcggcgga gaagcgcgag ctcatcgaaa atgtgcgtaa gcggttggag     420
   tcggaaggaa ttccgttgcc gccaccgcga aaggaagagg aacactttga ctccaattgc 480
   ataacacccg gcacgccgtt tatggcccgt ctttctgttg cccttcgcta ttacgtgcac 540
   aaacggatca cctacgaccc ggcatggcaa aagatccagg ttattctttc cgatgcgaat 600
   gtgccgggtg agggcgaaca taaaatcatg gactacgtcc gacgtcagcg cgcttcgccg 660
   gcgcatgacc cgaacaccgt tcactgtctt tgtggcgccg atgccgacct tatcatgctt 720
   ggattggcca cccacgagtc caatttcaac ataattcgag aagagttttt gccgaatcaa 780
   ccgaggccgt gcgaactttg cggtcaatac gggcacgagg tcgaacattg tgaagggttg 840
   gcacgtgccg agaccgggcc ggaccaagcg gacccgctga agaaggagaa gaacttcatc 900
   ttcatacgtc tgccagtgct tcgggaatat ttggagcgtg aactttccat gccaaatttg 960
   ccatttccgt tcgatttgga gagggccatc gatgattggg tcatgatgtg tttttttgtg 1020
   ggcaacgatt ttctccccca tttgccatcg ttagaaatcc gtgaaaatgc catcgaccgt 1080
   ctcgtgaagt tgtacaagga catggtttat caggctggcg gctggctcac tgaaaacggc 1140
   atagtgaata tcgaaagagt gaagaagatc atgggcggac ttgggaaggt tgaggacgaa 1200
   atattcaaac aaagacagct caaagaattg cgcttcaaag aatcaaagaa acagcgaaag 1260
   cagcaacaac ggcaggcaat ggcaaaaatt atgccgtaca atgccaattt gatagcacca 1320
   atgccacttg gacagcaaaa gcgaatgtcg ggcgaggaaa tgcgcagatt ggcggcggga 1380
   gaccgacagg agttcgtcag acttgcggat gtcgccagtt cgcacaaatt gcagtcgctg 1440
   ttgacgcctg tcgaccgacg aaaggccacg gatggcggtg atcggaagcg caagttggcc 1500
   ccaagttcag tcgacagtcc gaacggagca ccaccagcta aatcgtttaa ggagggagtg 1560
   gctgctgctt ccgtttcctc cgaaacggcc ttgaaaccgg agataaaact cgtaactccc 1620
   tctgggaaga cggcgccttt ggccaagggg gcggttgggg cactggcata cgactctgac 1680
   gaggaggagg aagtgctcga cgaaatccgt ctatgggaaa gtggatggaa ggaccgctac 1740
   tacagagcca agttccaagt gtccgagact gaccttgaat ttcgcaagaa agtggcatgt 1800
   gcttatgttg agggactctg ctgggtgttg cgttattatt atcagggatg tgccagctgg 1860
   aaatggtact acccttacca ctacgcgccg tttgcctccg attttgaact gatcgccgat 1920
   ttccaacccg acttcaacat gcccacgaaa ccgttcaagc cattggagca gttaatgggt 1980
   gtgtttcctg ctgctagcag agctcatatt cctgacgggt ggcgttggct gatgaccgac 2040
   tcaagttcgc cgataattga tttttacccg gaaacttttg aaattgatct gaacgggaag 2100
   aaatatgctt ggcaaggcgt cgctctgctt ccatttgtcg aggaaaaacg tcttttggat 2160
   gcacttgact cggtgtacgc cactctcaca accgatgaaa ttgcgcggaa tgccaccgga 2220
   ccagaccgtc tttttgtcgg caataaacac aacttgtttt cgctcgttta tgaggtgcac 2280
   tcccaaaaga cgacaattcc gataaaagat gaactaacaa atgatgacac aaaagaaaca 2340
   agcgaaggga cgcgagaaac tgaaggagaa gccgaggaaa ctcaacaggc ggaaaatggt 2400
   gaaagcgaac aaatagcgaa agccgaagaa acgacggcac aagaggaaca agcgaacgaa 2460
   gaagctatct caaaacctga acaggcaccg aaacaggaat tcgagactga aattgaattt 2520
   gtcgatgacg aagaaaagaa atgggtgcac atcgacgcag tcaaagcgta tgggatgtct 2580
   ggcgaagtgt cttatgacgt cgaagcactc gcttttggtg aggcgcatcg gtcgctgttc 2640
   agaaattcgt tggagtttgg cgacatcaaa gagaatacct gcattatggt tgagttccga 2700
   gatccccaat tcccaatgca cttcatcttc aaagccaaca ggctggacgg catcaaggaa 2760
   ttgcctcgtg tgctcaaacc gaacgattgg gaccaacgac ggcacggctc cttccaaccc 2820
   caaatcggat tcagtcgtga catcccacgt gcaaatctct ccatggcggg ccatcgtacg 2880
   ctcaaccacc acataaacga caaccgtcgc agttgtccgg cctctctttt ctcagtgcaa 2940
   acatcgccac cagcgcacac acttccatgg cacatgagca caaactcacc acaacaacaa 3000
   catcaaatgc agtaccaaca tcagcaacaa tggcagcagg gtcaatttta caacgggcaa 3060
   cagcagcagc agcccgtgcc gttgttcagt tttgggcaga tggcaatgcc cccgcctccg 3120
   ccgctcccac cgcaaaattg gattactagt cacccgccac cacaaatcca gtctcacgtg 3180
   ccgtacgggc agcccccacc tctaatatca cagccacgga atactcagca tcaccatcgc 3240
   agaaacgaca acaacagaac ttacaatagg aaataa 3276
<210> 1526
   <211> 1506
   <212> DNA
   <213> Heterodera glycines
<220>
   <223> cDNA sequence=SeqID_1303; peptide sequence=SeqID_1733
<400> 1526
   atggcgaaat ctgcaattga tgttaatcgc ggcatcacag aagaccaaac agctaccata      60
   agaaattatt tcacacaacc gcacttaatt tacaaaactg tcactggagt gcgcgggcct     120
   ttggtcatat tgggcgatgt taaattccca caatacgctg aaattgtgaa acttacactg     180
   cctaatggag aaaaacgcat gggacaagta ctcgaagtaa cgaaggacaa agctgtcgtt     240
   caggtgtttg aaggtacgtc aggtatcgat gcaaaaaaca caatttgcga gttcactggc     300
   gatattatga gaacacccgt ttccgaggat atgcttggaa gaatattcaa tggatccggc     360
   acacccatcg acaaagggcc caatgttttg gcggaggatt acttggacat cgatggacag     420
   cctatcaacc catggtccag aatttatccc gaagaaatga ttcagaccgg catttccgca     480
   attgatgtta tgaatagtat cgccagggga cagaaaattc caattttttc tgctgcaggt      540
   cttccgcata atgagatcgc tgctcaaatt gtacgccaag ggggcctcgt caaacagccg      600
   aacagaaaga aggaaaatga ggtggagggt gacgacgact ttgccattgt gtttgctgca     660
   atgggtgtca acatggagac ggcgcgcttt ttcaaacaag actttgaaga aaacggttcc     720
   atggaaaatg tgtgcctttt ccttaacttg gctaatgacc caaccatcga acggatcatc     780
   acaccacgac tcgcgctgac ttgtgcagaa tttctggctt accaatgcca aaaacacgtc     840
   ttggttgtgc tcactgacat gtcttattac gctgaagcac ttcgcgaagt gtccgccgcc     900
   cgagaggaag tgcccggtcg tcgtggtttt cccggttaca tgtacactga cttggccaga     960
   atatacgaac gtgccgggcg tgttgaaggc cgaaaggggt caatcacaca aattccaatt    1020
   ttgtctatgc ctaatgacga catcacccac ccaattcccg atttgaccgg ttacataacg    1080
   gaaggccaaa tttatgtgga tcgccaactt cacaatcgtc agatttaccc tccaataaat    1140
   gtacttccat cgctgtcgcg tctgatgaaa tcggccattg gcgagggaat gacccgtgcc    1200
   gatcattcgg atgtgtccaa ccaactttac gcatgttatg ccattggtaa agacgtgcaa    1260
   gcaatgaaag cggtggttgg agaggaggca ctgtcgtcgg atgatttgct ctacttggag    1320
   tttctcacga aatttgaaaa gaatttcatt tcacaaggga attatgaaaa ccggacggtg    1380
   ttcgactcac tcgacattgg ttggcagctg ttgcgcattt ttccaagaga aatgttgaag    1440
   cgaattccag cgaaaacatt ggataaatat tacccaaggg gaggcactgg ggcgaggaac    1500
   gaatga                                                               1506
<210> 1527
<400> 1527
   000
<210> 1528
   <211> 954
   <212> DNA
   <213> Heterodera glycines
<220>
   <223> cDNA sequence=SeqID_1306; peptide sequence=SeqID_1735
<400> 1528
   atgtcttttc ttgtcacatt agccggctat ttgacaattg ccatttttct ccagaaactc      60
   tttcgctctc tgtacaatgt ggttttcccc tatttgttcg ctgttcctca gaatatcaag     120
   gtgctcgcgg gtgccgagtg ggcagtggtc accggcagca ctgacggcat tggcaaggcg     180
   tacgcgctcg agttggccaa gaaaaacttc aacgtggtgc tcatctcacg ctccatggac     240
   aaactgaacg cagtggcgga ggaaatcaaa cagaaatatc ccaatgtgga agtgaagtgc     300
   atctcatttg acttcaccaa cgcaaatctg aaggactacg agcagacaat tttctcccaa     360
   ctttccacca tcgaagttgg aatgctcgtg aacaatgtcg ggatgagcta cgagtacccc     420
   gagcgattgg accgtattga gggcggactg caacgcgttt ccgacattac cgtgatcaac     480
   accctgccga ctacggtact ctccgctttt gtgctcaaac agatgaggga acggggacgc     540
   ggcgtcgtcg tcaatttggc ctcctcggca gcgtatttca attggtttta cctggccgct     600
   tattctgcgt cgaagaaata cgtgtgttgg ttgtcctcca ttctgcgcat ggaatacgcc     660
   gacaccgaca ttgtcatcca aacggtgtgc ccgatgatgg tggccaccaa aatggccaag     720
   attcgcaagg cttccttctt cacgcccagc cccgaggaat ttgccgccca agcgctccgc     780
   agcattggct tggtcgacga gaccaccggc tgtttgtccc accaaattca ggccgagctg     840
   atgttcggct acgtccccgc cccgttgctg aacaaattcg tgcgcgacaa ctcactgcag     900
   acgcgcaagc gcgccctgaa gaagaaagaa gcgacggtgg cgaagagtga atga           954
<210> 1529
<400> 1529
   000
<210> 1530
<400> 1530
   000
<210> 1531
   <211> 935
   <212> DNA
   <213> Heterodera glycines
<220>
   <223> cDNA sequence=SeqID_1310; peptide sequence=SeqID_1737
<400> 1531
   gcacgaggca cgaaaatgtg gcgcccactg acattgagga gggaatgcga gtgggtgtgg 60
   accgcaacaa ataccagatt catttgcctt tgccggcaaa gattgacgcg tccgttacga 120
   tgatgcaagt ggaggacaag ccggacgtta cctacgcgga cattggcggg tgcgaagaac 180
   agatcaaaaa gttgcgtgaa gtggtcgagt ttccgttgct tcagcctgag cgtttcacga 240
   gtttgggcat tgagcctccg aagggcgttt tgtttttggg tccgccgggc accggcaaaa 300
   ctttgtgtgc ccgcgcggtc gccaatcgga cggacgcgtg tttcatccgc gtcatcggtt 360
   ccgaattagt caaaaaatac gttggcgaag gcgcgcgcat ggtgcgcgag ctgttttcgc 420
   tggctaaaac gaaaaaggcg tgcattctct tcttcgacga agtcgacgcc atcggcggag 480
   cgcgatttga cgacggaaaa gggggcgaca acgaagtgca acggacgatg ctcgagttgg 540
   tcaaccaact ggacggattc gactcacgcg gggccatcaa ggttttgatg gccaccaaca 600
   gaccggacac actcgacccg gcgctcattc gtcccggtcg cattgaccga cgcattgaat 660
   tttccttgcc tgacctcaag gcacgaggaa acattctcca aattcacacc aaacggatga 720
   gcgtcgaccg gaacattcgg tacgaattga ttgctcgact ctgtccaaac acgacgggtg 780
   ccgacttgcg cagcgtttgc actgaggcgg gaatgttcgc tttgcgtgca cgtcgaaagg 840
   tcataacgga gcaagacttt ctcaaggctg ttcagaaagt ggtgaaaagc tacgccaagt 900
   tcagttcaac gccggcgtat atgacgcaca actga 935
<210> 1532
<400> 1532
   000
<210> 1533
   <211> 1074
   <212> DNA
   <213> Heterodera glycines
<220>
   <223> cDNA sequence=SeqID_1312; peptide sequence=SeqID_1906
<400> 1533
   gcacgagggt ttgaggtgcc tttggctgtg gtaaaactgt catttcgcag tcgttgtcca      60
   aatattccaa ctccgatgcc attatttacg tgggctgtgg cgagcgtggc aacgaaatgt     120
   cggaagtgtt gcgcgacttt cccgaactga cgatggaagt ggatggagtg accacgtcca     180
   tcatggagag gacggcgctg gtggccaaca cttcgaatat gcccgtggcg gcgcgtgaag     240
   cgtccattta cacgggaatt actctggccg aatacttccg cgacatgggt ctgaacgtgg     300
   ccatgatggc cgactcgacc tcccgatggg ccgaggcgct ccgtgagatt tcgggccgtc     360
   tcggggaaat gccggctgac tcgggttacc ccgcctattt ggccgcacgg ctcgcttcct     420
   tctacgaaag ggcgggaaaa gtgaaatgtt ttgggtcgcc tgaacgcgaa gggtccgtca     480
   ctattgtcgg cgcagtgtcg ccgcccggcg gagacttcgc cgaccccgtc acatcggcaa     540
   ccctcggcat tgtccaagtg ttttggggac tcgacaaaaa attggcccaa cgaaagcact     600
   tcccttcaat caattggctc atttcttaca gcaaatatat gcgtgcgttg gacgattatt     660
   acgagaagaa ttttgccgaa tttgttccac tgagaacgaa atgcaaagaa attttgcagg     720
   aagaggaaga tttgtcagaa attgtccaat tggtcggcaa agcatcgctt gccgaggccg     780
   acaaaatcac tttggagcta tgatcgcttc tgtcccttct acaaaactgt tgggatgctc     840
   aaaaatatga ttggctttta cgactttgcc cggcactcgg tggaaacgac tgcacaatcg     900
   gacaacaaaa tcacgtggaa tcagatcaga gatcacctcg gggacatcat tcacgaactt     960
   tcttcgatga aatttgagga cccatccgaa aaaacggaag atcagatcaa aaaggattac    1020
   gaagaactgt atgaacggat gcagaacaaa ttccgagcgt tagaggatga atga          1074
<210> 1534
<400> 1534
   000
<210> 1535
<400> 1535
   000
<210> 1536
<400> 1536
   000
<210> 1537
   <211> 1629
   <212> DNA
   <213> Heterodera glycines
<220>
   <223> cDNA sequence=SeqID_1318; peptide sequence=SeqID_1741
<400> 1537
   gcacgagggt ccccttctct ttccgtcgcc aactcaaatg tctctcgaca gcttggccaa      60
   ccaaaagtag cgggcttcaa agataaggat aaaccggaaa gtgttcgtac cagcaacatc     120
   gtggcagcaa aggctgttgc ggatgcggtg cggacgagtc ttgggcctcg gggaatggac     180
   aaaatgatcc aatcgggcaa cggagaagtg acaattacca atgacggggc gactattctc     240
   aaccaaatgt cggttgtgca tcccaccgca aaaatgttgg tcgagctgtc caaagcgcag     300
   gacattgagg cgggcgatgg gaccacaact gttgtggtgt tggccgggtc cttgttggac     360
   gctgcagaaa aacttcttgc caaaggaatt catccgacca ccatttccga ctcattccaa     420
   cgggcggcca cattttctga aaaagttctc gaggaaatgg ccacacccat tgacattaac     480
   gatgacgacg agttggtgaa gctcgccatg acgagtttga actcgaaggt cgtttcgcaa     540
   cattcccatc ttttggcccc aatggcggtg caggcagtga agaaactgat gagttcaccg     600
   gaggactcaa gtgtcaatct gaagctgatc aaagtcgtga aaaagttggg cgaaacggtg     660
   gaggagtcgc agctcattga gggagcgctg atcgatcaga agtcgatggg ccacggaggc     720
   ccggcgcggg tggagaaggc gaagattggc ctcatccaat tccaaatctc accaccaaag     780
   cccaacatgg aaaatcaagt ggtcatctct gactatgctc aaatggaccg tgcgctgaag     840
   gaagaacgaa attacatttt ggagctgtgc aagcaaatca aaaaggccgg gtgcaatgtg     900
   ttgctcatcc aaaagtccat tttacgcgat gccatcaacg aaatggcgct gcactttttc     960
   gcaaaaatga aaattatggt gatcaaagac atcgagcggg atgacatcga attctatgcc    1020
   aaaatcctcg ggtgtcgccc ggtggcatcc gttgaccatt ttgtgccgga atcgctcgga    1080
   acggccgatt tggtggagga aattgagacg gctgatggga aagtcctcaa agtgaccggc    1140
   cttaaacaga gtgggtgcgc tgtgtccatt ttggttcgcg gctccagcaa attggtgttg    1200
   gacgaagccg agcgctctct gcacgacgca ttgtgcgtca ttcgctgttt ggtgaagaaa    1260
   cgcgcccttc tttccggcgg cggtgccccc gaaatggaag tgtccgtccg cgtccgcgca    1320
   gctgctcaga caaaacacgg agccgaacag tattgctgga aggcatttgc ggacgcactc    1380
   gaagtggtgc catacacttt ggcagaaaac gctggactgt cgcccattga aacagtgacg    1440
   gagatgcgca gtcagcacgc aatgggaaac aaagagcacg gagtgaatgt gcgaatgggt    1500
   gccattacgg acattcgtga ggaaaatgtg gtgcagcctc ttttggtcag ttcgttcgcc    1560
   gtcaaacagg cggctgaatg tgtgcgcagc attctcaaaa tcgacgacat tgttctgtcc    1620
   gtgcgttaa                                                            1629
<210> 1538
<400> 1538
   000
<210> 1539
<400> 1539
   000
<210> 1540
<400> 1540
   000
<210> 1541
<400> 1541
   000
<210> 1542
<400> 1542
   000
<210> 1543
<400> 1543
   000
<210> 1544
<400> 1544
   000
<210> 1545
   <211> 960
   <212> DNA
   <213> Heterodera glycines

<220>
   <223> cDNA sequence=SeqID_1327; peptide sequence=SeqID_1748
<400> 1545
   gcacgaggat taaaattgaa agttccaatt ctgctgtatt gttttgttca atgtttaagt 60
   gctgtcaaag tggacaatgc cgagcaatcc gaccaaccaa ctggcgattc ttcctccgga 120
   caattcccaa tttcgtatcc tccaataaaa tatgaaaacg atttggccta tgtggagaaa 180
   ccgagtggca acgagcgttg tctattctac gcggaaggac tttctgtcgt cgtttttgcc 240
   actaaaaaaa gtcaatcggc gtgggcggac tatcgctctg acaccaaaga aagtccgaag 300
   tataccgcgt caattgaatg ccaaggcaat atgaatgtgg gcagcagctt tgatgtctat 360
   ttgaaaattg acggcaaagc ggaaggagtt gtgagagctg acattggaaa aaaggacgca 420
   aaaagtgtct tcactgttga aggagccgaa tttatactca acttcaacgt cacacaagtc 480
   ggcttttggc aattggttgg tgccaccgtg gaaagaattg aagtgaaagg ctcttcggat 540
   ttcctgaaca aaaatttgaa tgtcgacaaa aaatggactt tggagccttc agttgttcca 600
   tccacaaaca ttaacggctt caaagactac agctttgctt gtggtcagac caacccgata 660
   gtgtgggccc taaaggactc aaatgattac gcagtcggaa ttacgctgaa caatgtgcag 720
   ttccaaccat tcaatgcaac ttacactgcc ggtggaaaag aagctgaccg tttaaaattt 780
   gggtggaaag tcaacgattg ccaggggctg ttcagcattg gcacttggat gggcttggta 840
   gttgccactc tgctcatcgg cgtgctcttt ttcggctttc tgatgcttaa ttctgtgcag 900
   acaatggaca gattcgatga tccgaagcag aaacagttga taattagttc gaaagagtga 960
<210> 1546
<400> 1546
   000
<210> 1547
<400> 1547
   000
<210> 1548
   <211> 1283
   <212> DNA
   <213> Heterodera glycines
<220>
   <223> cDNA sequence=SeqID_1330; peptide sequence=SeqID_1751
<400> 1548
   ttcctctctt tgggataatg tccacggaca gtgctgaccc cctctattgg ctccgtgtga 60
   tcatggcttc caacagaggc acactgatgg agttgggcat ttcgcccatt gtcacttccg 120
   gtctcataat gcaacttctc gctggtgcga agattatcga agtgggcgac acgccaaagg 180
   aacgggcatt gttcaacggg gcgcaaaaac tgttcggaat ggtcatcact attggacagg 240
   caattgtcta cgtggcttcc ggaatgtacg gaaacccaca cgaaatcggc gccggaattt 300
   gcttgcttat cgtaattcaa ctcgtcgtgg ctggtctcat tgtccttctg ctggacgaat 360
   tgcttcagaa agggtatgga cttggctcgg gaatttcgct gtttatcgcc acaaacattt 420
   gcgagaccat cgtttggaag gccttttcac cggcgacgat gaacacgggc cgtggcacgg 480
   aatttgaggg ggcgatcatc gcacttttcc atctgttggc cactcggaat gacaaagtcc 540
   gtgcacttcg tgaagctttc taccgacaga atttgccaaa tttgatgaat ctgctggcca 600
   ctgttctagt ttttgccgtc gtcatttatt tccaaggttt tcgtgtcgat ttgcccataa 660
   aaagtgctcg ctaccgcggc cagcacagtt cctatccgat caagctgttc tacaccagca 720
   acatccccat catcctccaa tctgcgctcg tctccaacct ttacgtgatc tcacaaatgc 780
   tggcggccaa attcggcggc aacattttgg ttaatttgct cgggacgtgg tccgactctt 840
   ccggcacata tcggagcttc ccaaccggtg gtatctgcta ttatctgtcc ccgcccgagt 900
   cgctctccca cattttggag gaccccatcc actgcatcat ttacattgtg ttcatgcttg 960
   gctcgtgcgc cttcttttct aagacttgga tcgacgtcag cggctcaagt gccaaagatg 1020
   tagccaaaca actgaaggag cagcagatga tcatgcgagg ccatcgcgag aaatcgatga 1080
   tccacgagct caaccggtac attccgacag cggctgcttt cggtgggctg tgcatcggcg 1140
   cattgtcagt cacggcagat tttttgggcg caattggaag tggcacgggt attctgctgg 1200
   ctgtcacaat catttaccag tactttgaaa ttttcgtcaa agaacagcaa gaaatgggcg 1260
   gagttgccgg actctttttc tga 1283
<210> 1549
<400> 1549
   000
<210> 1550
<400> 1550
   000
<210> 1551
<400> 1551
   000
<210> 1552
<400> 1552
   000
<210> 1553
<400> 1553
   000
<210> 1554
<400> 1554
   000
<210> 1555
<400> 1555
   000
<210> 1556
<400> 1556
   000
<210> 1557
<400> 1557
   000
<210> 1558
<400> 1558
   000
<210> 1559
<400> 1559
   000
<210> 1560
<400> 1560
   000
<210> 1561
<400> 1561
   000
<210> 1562
<400> 1562
   000
<210> 1563
<400> 1563
   000
<210> 1564
<400> 1564
   000
<210> 1565
<400> 1565
   000
<210> 1566
<400> 1566
   000
<210> 1567
<400> 1567
   000
<210> 1568
<400> 1568
   000
<210> 1569
<400> 1569
   000
<210> 1570
<400> 1570
   000
<210> 1571
<400> 1571
   000
<210> 1572
<400> 1572
   000
<210> 1573
<400> 1573
   000
<210> 1574
<400> 1574
   000
<210> 1575
<400> 1575
   000
<210> 1576
<400> 1576
   000
<210> 1577
<400> 1577
   000
<210> 1578
<400> 1578
   000
<210> 1579
<400> 1579
   000
<210> 1580
<400> 1580
   000
<210> 1581
<400> 1581
   000
<210> 1582
<400> 1582
   000
<210> 1583
<400> 1583
   000
<210> 1584
<400> 1584
   000
<210> 1585
<400> 1585
   000
<210> 1586
<400> 1586
   000
<210> 1587
<400> 1587
   000
<210> 1588
<400> 1588
   000
<210> 1589
<400> 1589
   000
<210> 1590
<400> 1590
   000
<210> 1591
<400> 1591
   000
<210> 1592
<400> 1592
   000
<210> 1593
<400> 1593
   000
<210> 1594
<400> 1594
   000
<210> 1595
<400> 1595
   000
<210> 1596
<400> 1596
   000
<210> 1597
<400> 1597
   000
<210> 1598
<400> 1598
   000
<210> 1599
<400> 1599
   000
<210> 1600
<400> 1600
   000
<210> 1601
<400> 1601
   000
<210> 1602
<400> 1602
   000
<210> 1603
<400> 1603
   000
<210> 1604
<400> 1604
   000
<210> 1605
<400> 1605
   000
<210> 1606
<400> 1606
   000
<210> 1607
<400> 1607
   000
<210> 1608
<400> 1608
   000
<210> 1609
<400> 1609
   000
<210> 1610
<400> 1610
   000
<210> 1611
<400> 1611
   000
<210> 1612
<400> 1612
   000
<210> 1613
<400> 1613
   000
<210> 1614
<400> 1614
   000
<210> 1615
<400> 1615
   000
<210> 1616
<400> 1616
   000
<210> 1617
<400> 1617
   000
<210> 1618
<400> 1618
   000
<210> 1619
<400> 1619
   000
<210> 1620
<400> 1620
   000
<210> 1621
<400> 1621
   000
<210> 1622
<400> 1622
   000
<210> 1623
<400> 1623
   000
<210> 1624
<400> 1624
   000
<210> 1625
<400> 1625
   000
<210> 1626
<400> 1626
   000
<210> 1627
<400> 1627
   000
<210> 1628
<400> 1628
   000
<210> 1629
<400> 1629
   000
<210> 1630
<400> 1630
   000
<210> 1631
<400> 1631
   000
<210> 1632
<400> 1632
   000
<210> 1633
<400> 1633
   000
<210> 1634
<400> 1634
   000
<210> 1635
<400> 1635
   000
<210> 1636
<400> 1636
   000
<210> 1637
<400> 1637
   000
<210> 1638
<400> 1638
   000
<210> 1639
<400> 1639
   000
<210> 1640
<400> 1640
   000
<210> 1641
<400> 1641
   000
<210> 1642
<400> 1642
   000
<210> 1643
<400> 1643
   000
<210> 1644
<400> 1644
   000
<210> 1645
<400> 1645
   000
<210> 1646
<400> 1646
   000
<210> 1647
<400> 1647
   000
<210> 1648
<400> 1648
   000
<210> 1649
<400> 1649
   000
<210> 1650
<400> 1650
   000
<210> 1651
<400> 1651
   000
<210> 1652
<400> 1652
   000
<210> 1653
<400> 1653
   000
<210> 1654
<400> 1654
   000
<210> 1655
<400> 1655
   000
<210> 1656
<400> 1656
   000
<210> 1657
<400> 1657
   000
<210> 1658
<400> 1658
   000
<210> 1659
<400> 1659
   000
<210> 1660
<400> 1660
   000
<210> 1661
<400> 1661
   000
<210> 1662
<400> 1662
   000
<210> 1663
<400> 1663
   000
<210> 1664
<400> 1664
   000
<210> 1665
<400> 1665
   000
<210> 1666
<400> 1666
   000
<210> 1667
<400> 1667
   000
<210> 1668
<400> 1668
   000
<210> 1669
<400> 1669
   000
<210> 1670
<400> 1670
   000
<210> 1671
<400> 1671
   000
<210> 1672
<400> 1672
   000
<210> 1673
<400> 1673
   000
<210> 1674
<400> 1674
   000
<210> 1675
<400> 1675
   000
<210> 1676
<400> 1676
   000
<210> 1677
<400> 1677
   000
<210> 1678
<400> 1678
   000
<210> 1679
<400> 1679
   000
<210> 1680
<400> 1680
   000
<210> 1681
<400> 1681
   000
<210> 1682
<400> 1682
   000
<210> 1683
<400> 1683
   000
<210> 1684
<400> 1684
   000
<210> 1685
<400> 1685
   000
<210> 1686
<400> 1686
   000
<210> 1687
<400> 1687
   000
<210> 1688
<400> 1688
   000
<210> 1689
<400> 1689
   000
<210> 1690
<400> 1690
   000
<210> 1691
<400> 1691
   000
<210> 1692
<400> 1692
   000
<210> 1693
<400> 1693
   000
<210> 1694
<400> 1694
   000
<210> 1695
<400> 1695
   000
<210> 1696
<400> 1696
   000
<210> 1697
<400> 1697
   000
<210> 1698
<400> 1698
   000
<210> 1699
<400> 1699
   000
<210> 1700
<400> 1700
   000
<210> 1701
<400> 1701
   000
<210> 1702
<400> 1702
   000
<210> 1703
<400> 1703
   000
<210> 1704
<400> 1704
   000
<210> 1705
<400> 1705
   000
<210> 1706
<400> 1706
   000
<210> 1707
<400> 1707
   000
<210> 1708
<400> 1708
   000
<210> 1709
<400> 1709
   000
<210> 1710
<400> 1710
   000
<210> 1711
<400> 1711
   000
<210> 1712
<400> 1712
   000
<210> 1713
<400> 1713
   000
<210> 1714
<400> 1714
   000
<210> 1715
<400> 1715
   000
<210> 1716
<400> 1716
   000
<210> 1717
<400> 1717
   000
<210> 1718
<400> 1718
   000
<210> 1719
<400> 1719
   000

<210> 1720
<400> 1720
   000
<210> 1721
<400> 1721
   000
<210> 1722
   <211> 446
   <212> PRT
   <213> Heterodera glycines
<220>
   <223> cDNA sequence=SeqID_1289; coding sequence=SeqID_1513
<400> 1722
   Met Ser Ala Ala Val Ser Ser Ser Arg Pro Met Glu Asp Trp Lys Ser
   1               5                   10                  15
   Glu Leu Ala Met Pro Asp Lys Asp Leu Arg Tyr Lys Thr Ser Asp Val
               20                  25                  30
   Thr Ala Thr Lys Gly Val Glu Phe Glu Glu Phe Gly Leu Thr Arg Asp
           35                  40                  45
   Leu Leu Lys Gly Ile Phe Glu Lys Gly Trp Glu His Pro Ser Pro Val
       50                  55                  60
   Gln Glu Ala Ser Ile Gly Ile Ala Leu Thr Gly Gln Asp Ile Leu Ala
   65                  70                  75                  80
   Arg Ala Lys Asn Gly Thr Gly Lys Thr Gly Ala Tyr Cys Ile Pro Cys
                   85                  90                  95
   Ile Glu Lys Ile Asp Pro Ser Lys Glu Tyr Ile Gln Ala Met Ile Ile
               100                 105                 110
   Val Pro Thr Arg Glu Leu Ala Leu Gln Thr Ser Gln Ile Cys Val Glu
           115                 120                 125
   Leu Ser Lys His Leu Lys Ile Lys Ile Met Val Thr Thr Gly Gly Thr
       130                 135                 140
   Asp Leu Arg Asp Asp Ile Leu Arg Leu Asn Gly Ile Val His Leu Val
   145                 150                 155                 160
   Val Ala Thr Pro Gly Arg Ile Leu Asp Leu Met Glu Lys Gly Val Ala
                   165                 170                 175
   Asn Val Ala Tyr Cys Lys Thr Ile Val Leu Asp Glu Ala Asp Lys Leu
               180                 185                 190
   Leu Ser Gln Asp Phe Gln Gly Val Leu Asp Arg Leu Val Gln Phe Leu
   195 200 205
   Pro Lys Asp Arg Gln Ile Met Leu Tyr Ser Ala Thr Phe Pro Arg Thr
   210 215 220
   Val Ala Thr Phe Met Glu Lys His Met Ser Lys Pro Tyr Glu Ile Asn
   225 230 235 240
   Leu Met Glu Glu Leu Thr Leu Leu Gly Ile Thr Gln Phe Tyr Ala Tyr
   245 250 255
   Val Gln Glu Lys Gln Lys Val His Cys Leu Asn Thr Leu Phe Arg Lys
   260 265 270
   Leu Gln Ile Asn Gln Ser Ile Ile Phe Cys Asn Ser Thr Gln Arg Val
   275 280 285
   Glu Leu Leu Ala Lys Lys Ile Thr Glu Leu Gly Tyr Ser Cys Tyr Tyr
   290 295 300
   Ile His Ser Lys Met Ala Gln His His Arg Asn Arg Val Phe His Glu
   305 310 315 320
   Phe Arg Gln Gly His Cys Arg Asn Leu Val Cys Ser Asp Leu Leu Thr
   325 330 335
   Arg Gly Ile Asp Ile Gln Ala Val Asn Val Val Ile Asn Phe Asp Phe
   340 345 350
   Pro Arg Asn Ser Glu Thr Tyr Leu His Arg Ile Gly Arg Ser Gly Arg
   355 360 365
   Phe Gly His Leu Gly Ile Ala Ile Asn Leu Ile Thr Phe Glu Asp Arg
   370 375 380
   Tyr Asn Leu Arg Arg Ile Glu Lys Glu Leu Lys Thr His Ile Asp Pro
   385 390 395 400
   Ile Pro Lys Glu Val Asp Pro Lys Leu Tyr Val Ala Glu Phe Gln Met
   405 410 415
   Asp His Thr Lys Phe Asp Cys Gly Asn Val Val Asp Gly Ala Val Leu
   420 425 430
   Asn Asn Gly Val Asp Gln Gln Gln Gln Pro Arg Ala Ser Ser
   435 440 445
<210> 1723
   <211> 247
   <212> PRT
   <213> Heterodera glycines
<220>
   <223> cDNA sequence=SeqID_1290; coding sequence=SeqID_1514
<400> 1723
   Met Phe Arg Asn Gln Tyr Asp Ser Asp Val Thr Val Phe Ser Pro Gln
   1               5                   10                  15
   Gly Arg Leu His Gln Ile Asp Tyr Ala Val Glu Ala Met Arg Gln Gly
               20                  25                  30
   Ser Ala Ala Val Ala Ile Arg Ser Lys Asp Tyr Ala Val Val Val Ala
           35                  40                  45
   Leu Met Arg Ala Gln Asn Glu Leu Ser Ser Tyr Gln Gln Lys Val Phe
       50                  55                  60
   Lys Leu Asp Asp His Leu Gly Leu Ser Met Ser Gly Leu Ile Ser Asp
   65                  70                  75                  80
   Gly Arg Ile Leu Ala Arg Phe Met Gln Ser Glu Cys Ala Glu Phe Arg
                   85                  90                  95
   Trp Asn Tyr His Asn Pro Ile Ser Val Glu Leu Leu Asn Arg Lys Leu
               100                 105                 110
   Gly Leu Lys Leu Gln Ser Asn Thr Gln Tyr Tyr Gly Arg Arg Pro Phe
           115                 120                 125
   Gly Val Gly Leu Leu Ile Ile Gly Tyr Asp Asp Lys Gly Pro His Val
       130                 135                 140
   Val Lys Ala Glu Pro Ser Gly Asp Val Ser Glu Met Phe Ala Ala Ser
   145                 150                 155                 160
   Ile Gly Ala Arg Ser Gln Ser Ala Arg Thr Tyr Leu Glu Lys Asn Leu
                   165                 170                 175
   Pro Glu Phe Glu Thr Ala Asp Arg Asp Ala Leu Ile Arg His Ala Ile
               180                 185                 190
   Arg Ser Leu Lys Glu Thr Leu Gln Ala Asp Ala Gly Ser Leu Asp Glu
           195                 200                 205
   Asn Asn Thr Phe Ile Ala Ile Ile Gly Leu Gly Gln Pro Phe Thr Phe
       210                 215                 220
   Leu Gly Lys Asp Ala Thr Arg Ala His Ile Arg Ser Val Glu Glu Glu
   225                 230                 235                 240
   Ala His Arg Glu Met Asp Glu
                   245
<210> 1724
   <211> 254
   <212> PRT
   <213> Heterodera glycines
<220>
   <223> cDNA sequence=SeqID_1292; coding sequence=SeqID_1515
<400> 1724
   Met Glu His Tyr Asp Arg Ala Ile Thr Ile Phe Ser Pro Asp Gly Arg
   1               5                   10                  15
   Leu Phe Gln Val Asp Tyr Ala Gln Glu Ala Val Lys Lys Gly Ser Thr
               20                  25                  30
   Ala Val Ala Val Lys Gly Lys Asn Cys Val Val Ile Gly Val Glu Lys
           35                  40                  45
   Lys Ser Ile Pro Thr Leu Gln Asp Glu Arg Thr Ile Arg Lys Ile His
       50                  55                  60
   Ala Leu Asp Ser His Val Met Val Ala Phe Ala Gly Leu Ser Ala Asp
   65                  70                  75                  80
   Ala Arg Val Leu Ile Asp Arg Ala Arg Val Glu Cys Glu Asn Tyr Lys
                   85                  90                  95
   Leu Thr Leu Glu Asp Arg Val Ser Val Met His Ile Ser Arg Ile Ile
               100                 105                 110
   Ala Asp Ile Lys Gln Arg Tyr Thr Gln Thr Thr Gly Arg Arg Pro Phe
           115                 120                 125
   Gly Ile Ser Met Leu Val Gly Gly Phe Asp Ser Asp Gly Thr Pro His
       130                 135                 140
   Leu Phe Lys Thr Glu Pro Ser Gly Val Tyr Tyr Glu Leu Leu Ala Gly
   145                 150                 155                 160
   Ser Ile Gly Arg Asn Glu Lys Ile Val Lys Glu Tyr Leu Glu Lys Glu
                   165                 170                 175
   Tyr Gly Asn Asp Asn Thr Ala Asp Glu Glu Ser Thr Leu Lys Leu Met
               180                 185                 190
   Leu Lys Ser Leu Ala Pro Val Val Gln Ser Gly Ala Gln Asn Ile Glu
           195                 200                 205
   Ile Ala Val Leu Lys Trp Val Asp Gly Thr Asn Gly Glu Pro Gly Tyr
       210                 215                 220
   Cys Glu Gln Gln Ile Leu Asn Val Glu Ala Ile Gly Ala Leu Leu Ser
   225                 230                 235                 240
   Gln Ile Gly Pro Ile Glu Gln Gln Lys Pro Met Glu Thr Ser
                   245                 250
<210> 1725
<400> 1725
   000
<210> 1726
<400> 1726
   000
<210> 1727
<400> 1727
   000
<210> 1728
<400> 1728
   000
<210> 1729
   <211> 254
   <212> PRT
   <213> Heterodera glycines
<220>
   <223> cDNA sequence=SeqID_1298; coding sequence=SeqID_1521
<400> 1729
   Asp Thr Val Gly Cys Cys Ser Leu Arg Cys Glu His Ile Lys Leu His
   1 5 10 15
   Asp Asp Met Asp Asp Leu Lys Tyr Val Val Glu Phe Asp Phe Leu Gly
   20 25 30
   Lys Asp Ser Ile Arg Tyr Tyr Asn Arg Val Pro Val Glu Lys Gln Val
   35 40 45
   Phe Lys Asn Leu Lys Leu Phe Met Glu Glu Lys Asp Pro Gly Asp Asp
   50 55 60
   Leu Phe Asp Arg Leu Asp Thr Ser Ser Leu Asn Gln Tyr Leu Lys Glu
   65 70 75 80
   Leu Met Asp Gly Leu Thr Ala Lys Val Phe Arg Thr Tyr Asn Ala Ser
   85 90 95
   Ile Thr Leu Gln Glu Gln Leu Asp Lys Leu Thr Asn Pro Ser Asp Ser
   100 105 110
   Val His Glu Lys Met Leu Ser Tyr Asn Arg Ala Asn Arg Gln Val Ala
   115 120 125
   Ile Leu Cys Asn His Gln Arg Ala Val Pro Lys Thr His Glu Lys Ser
   130 135 140
   Met Glu Asn Leu Glu Asn Lys Ile Lys Glu Lys Lys Gln Glu Tyr Lys
   145 150 155 160
   Glu Leu Lys Asp Gln Leu Lys Lys Ser Lys Ser Val Asp Glu Lys Met
   165 170 175
   Ala Lys Lys Tyr Glu Arg Leu Lys Glu Gln Ile Ala Lys Leu Asn Thr
   180 185 190
   Gln Arg Thr Asp Lys Asp Glu Asn Lys Gln Ile Ala Leu Gly Thr Ser
   195 200 205
   Lys Leu Asn Tyr Leu Asp Pro Arg Ile Ser Val Ala Trp Cys Lys Lys
   210 215 220
   Asn Asp Val Pro Val Glu Lys Ile Phe Ser Lys Thr Gln Arg Asp Lys
   225 230 235 240
   Phe Arg Trp Ala Leu Asp Met Ala Lys Glu Asp Phe Ile Phe
   245 250
<210> 1730
<400> 1730
   000
<210> 1731
<400> 1731
   000
<210> 1732
   <211> 1091
   <212> PRT
   <213> Heterodera glycines
<220>
   <223> cDNA sequence=SeqID_1302; coding sequence=SeqID_1525
<400> 1732
   Met Gly Val Pro Ala Phe Phe Arg Trp Leu Ser Arg Lys Tyr Val Ser
   1               5                   10                  15
   Ile Ile Val Asp Ala Val Glu Gln Arg Arg Arg Glu Ile Asp Gly Val
               20                  25                  30
   Lys Ile Pro Ile Asp Ala Thr Gln Pro Asn Glu Asn Tyr Gln Glu Phe
           35                  40                  45
   Asp Asn Leu Tyr Leu Asp Met Asn Gly Ile Ile His Pro Cys Thr His
       50                  55                  60
   Pro Glu Asp Arg Pro Ala Pro Lys Thr Glu Glu Glu Met Phe Ile Leu
   65                  70                  75                  80
   Ile Phe Glu Tyr Ile Asp Arg Leu Phe Ser Ile Val Arg Pro Arg Lys
                   85                  90                  95
   Leu Leu Tyr Met Ala Val Asp Gly Val Ala Pro Arg Ala Lys Met Asn
               100                 105                 110
   Gln Gln Arg Ser Arg Arg Phe Arg Ala Ala Gln Glu Ala Ala Glu Lys
           115                 120                 125
   Arg Glu Leu Ile Glu Asn Val Arg Lys Arg Leu Glu Ser Glu Gly Ile
       130                 135                 140
   Pro Leu Pro Pro Pro Arg Lys Glu Glu Glu His Phe Asp Ser Asn Cys
   145                 150                 155                 160
   Ile Thr Pro Gly Thr Pro Phe Met Ala Arg Leu Ser Val Ala Leu Arg
                   165                 170                 175
   Tyr Tyr Val His Lys Arg Ile Thr Tyr Asp Pro Ala Trp Gln Lys Ile
               180                 185                 190
   Gln Val Ile Leu Ser Asp Ala Asn Val Pro Gly Glu Gly Glu His Lys
           195                 200                 205
   Ile Met Asp Tyr Val Arg Arg Gln Arg Ala Ser Pro Ala His Asp Pro
       210                 215                 220
   Asn Thr Val His Cys Leu Cys Gly Ala Asp Ala Asp Leu Ile Met Leu
   225                 230                 235                 240
   Gly Leu Ala Thr His Glu Ser Asn Phe Asn Ile Ile Arg Glu Glu Phe
                   245                 250                 255
   Leu Pro Asn Gln Pro Arg Pro Cys Glu Leu Cys Gly Gln Tyr Gly His
   260 265 270
   Glu Val Glu His Cys Glu Gly Leu Ala Arg Ala Glu Thr Gly Pro Asp
   275 280 285
   Gln Ala Asp Pro Leu Lys Lys Glu Lys Asn Phe Ile Phe Ile Arg Leu
   290 295 300
   Pro Val Leu Arg Glu Tyr Leu Glu Arg Glu Leu Ser Met Pro Asn Leu
   305 310 315 320
   Pro Phe Pro Phe Asp Leu Glu Arg Ala Ile Asp Asp Trp Val Met Met
   325 330 335
   Cys Phe Phe Val Gly Asn Asp Phe Leu Pro His Leu Pro Ser Leu Glu
   340 345 350
   Ile Arg Glu Asn Ala Ile Asp Arg Leu Val Lys Leu Tyr Lys Asp Met
   355 360 365
   Val Tyr Gln Ala Gly Gly Trp Leu Thr Glu Asn Gly Ile Val Asn Ile
   370 375 380
   Glu Arg Val Lys Lys Ile Met Gly Gly Leu Gly Lys Val Glu Asp Glu
   385 390 395 400
   Ile Phe Lys Gln Arg Gln Leu Lys Glu Leu Arg Phe Lys Glu Ser Lys
   405 410 415
   Lys Gln Arg Lys Gln Gln Gln Arg Gln Ala Met Ala Lys Ile Met Pro
   420 425 430
   Tyr Asn Ala Asn Leu Ile Ala Pro Met Pro Leu Gly Gln Gln Lys Arg
   435 440 445
   Met Ser Gly Glu Glu Met Arg Arg Leu Ala Ala Gly Asp Arg Gln Glu
   450 455 460
   Phe Val Arg Leu Ala Asp Val Ala Ser Ser His Lys Leu Gln Ser Leu
   465 470 475 480
   Leu Thr Pro Val Asp Arg Arg Lys Ala Thr Asp Gly Gly Asp Arg Lys
   485 490 495
   Arg Lys Leu Ala Pro Ser Ser Val Asp Ser Pro Asn Gly Ala Pro Pro
   500 505 510
   Ala Lys Ser Phe Lys Glu Gly Val Ala Ala Ala Ser Val Ser Ser Glu
   515 520 525
   Thr Ala Leu Lys Pro Glu Ile Lys Leu Val Thr Pro Ser Gly Lys Thr
   530 535 540
   Ala Pro Leu Ala Lys Gly Ala Val Gly Ala Leu Ala Tyr Asp Ser Asp
   545 550 555 560
   Glu Glu Glu Glu Val Leu Asp Glu Ile Arg Leu Trp Glu Ser Gly Trp
   565 570 575
   Lys Asp Arg Tyr Tyr Arg Ala Lys Phe Gln Val Ser Glu Thr Asp Leu
   580 585 590
   Glu Phe Arg Lys Lys Val Ala Cys Ala Tyr Val Glu Gly Leu Cys Trp
   595 600 605
   Val Leu Arg Tyr Tyr Tyr Gln Gly Cys Ala Ser Trp Lys Trp Tyr Tyr
   610 615 620
   Pro Tyr His Tyr Ala Pro Phe Ala Ser Asp Phe Glu Leu Ile Ala Asp
   625 630 635 640
   Phe Gln Pro Asp Phe Asn Met Pro Thr Lys Pro Phe Lys Pro Leu Glu
   645 650 655
   Gln Leu Met Gly Val Phe Pro Ala Ala Ser Arg Ala His Ile Pro Asp
   660 665 670
   Gly Trp Arg Trp Leu Met Thr Asp Ser Ser Ser Pro Ile Ile Asp Phe
   675 680 685
   Tyr Pro Glu Thr Phe Glu Ile Asp Leu Asn Gly Lys Lys Tyr Ala Trp
   690 695 700
   Gln Gly Val Ala Leu Leu Pro Phe Val Glu Glu Lys Arg Leu Leu Asp
   705 710 715 720
   Ala Leu Asp Ser Val Tyr Ala Thr Leu Thr Thr Asp Glu Ile Ala Arg
   725 730 735
   Asn Ala Thr Gly Pro Asp Arg Leu Phe Val Gly Asn Lys His Asn Leu
   740 745 750
   Phe Ser Leu Val Tyr Glu Val His Ser Gln Lys Thr Thr Ile Pro Ile
   755 760 765
   Lys Asp Glu Leu Thr Asn Asp Asp Thr Lys Glu Thr Ser Glu Gly Thr
   770 775 780
   Arg Glu Thr Glu Gly Glu Ala Glu Glu Thr Gln Gln Ala Glu Asn Gly
   785 790 795 800
   Glu Ser Glu Gln Ile Ala Lys Ala Glu Glu Thr Thr Ala Gln Glu Glu
   805 810 815
   Gln Ala Asn Glu Glu Ala Ile Ser Lys Pro Glu Gln Ala Pro Lys Gln
   820 825 830
   Glu Phe Glu Thr Glu Ile Glu Phe Val Asp Asp Glu Glu Lys Lys Trp
   835 840 845
   Val His Ile Asp Ala Val Lys Ala Tyr Gly Met Ser Gly Glu Val Ser
   850 855 860
   Tyr Asp Val Glu Ala Leu Ala Phe Gly Glu Ala His Arg Ser Leu Phe
   865 870 875 880
   Arg Asn Ser Leu Glu Phe Gly Asp Ile Lys Glu Asn Thr Cys Ile Met
   885 890 895
   Val Glu Phe Arg Asp Pro Gln Phe Pro Met His Phe Ile Phe Lys Ala
   900 905 910
   Asn Arg Leu Asp Gly Ile Lys Glu Leu Pro Arg Val Leu Lys Pro Asn
   915 920 925
   Asp Trp Asp Gln Arg Arg His Gly Ser Phe Gln Pro Gln Ile Gly Phe
   930 935 940
   Ser Arg Asp Ile Pro Arg Ala Asn Leu Ser Met Ala Gly His Arg Thr
   945 950 955 960
   Leu Asn His His Ile Asn Asp Asn Arg Arg Ser Cys Pro Ala Ser Leu
   965 970 975
   Phe Ser Val Gln Thr Ser Pro Pro Ala His Thr Leu Pro Trp His Met
   980 985 990
   Ser Thr Asn Ser Pro Gln Gln Gln His Gln Met Gln Tyr Gln His Gln
   995 1000 1005
   Gln Gln Trp Gln Gln Gly Gln Phe Tyr Asn Gly Gln Gln Gln Gln
   1010 1015 1020
   Gln Pro Val Pro Leu Phe Ser Phe Gly Gln Met Ala Met Pro Pro
   1025 1030 1035
   Pro Pro Pro Leu Pro Pro Gln Asn Trp Ile Thr Ser His Pro Pro
   1040 1045 1050
   Pro Gln Ile Gln Ser His Val Pro Tyr Gly Gln Pro Pro Pro Leu
   1055 1060 1065
   Ile Ser Gln Pro Arg Asn Thr Gln His His His Arg Arg Asn Asp
   1070 1075 1080
   Asn Asn Arg Thr Tyr Asn Arg Lys
   1085 1090
<210> 1733
   <211> 501
   <212> PRT
   <213> Heterodera glycines
<220>
   <223> cDNA sequence=SeqID_1303; coding sequence=SeqID_1526
<400> 1733
   Met Ala Lys Ser Ala Ile Asp Val Asn Arg Gly Ile Thr Glu Asp Gln
   1               5                   10                  15
   Thr Ala Thr Ile Arg Asn Tyr Phe Thr Gln Pro His Leu Ile Tyr Lys
               20                  25                  30
   Thr Val Thr Gly Val Arg Gly Pro Leu Val Ile Leu Gly Asp Val Lys
           35                  40                  45
   Phe Pro Gln Tyr Ala Glu Ile Val Lys Leu Thr Leu Pro Asn Gly Glu
       50                  55                  60
   Lys Arg Met Gly Gln Val Leu Glu Val Thr Lys Asp Lys Ala Val Val
   65                  70                  75                  80
   Gln Val Phe Glu Gly Thr Ser Gly Ile Asp Ala Lys Asn Thr Ile Cys
                   85                  90                  95
   Glu Phe Thr Gly Asp Ile Met Arg Thr Pro Val Ser Glu Asp Met Leu
               100                 105                 110
   Gly Arg Ile Phe Asn Gly Ser Gly Thr Pro Ile Asp Lys Gly Pro Asn
           115                 120                 125
   Val Leu Ala Glu Asp Tyr Leu Asp Ile Asp Gly Gln Pro Ile Asn Pro
       130                 135                 140
   Trp Ser Arg Ile Tyr Pro Glu Glu Met Ile Gln Thr Gly Ile Ser Ala
   145                 150                 155                 160
   Ile Asp Val Met Asn Ser Ile Ala Arg Gly Gln Lys Ile Pro Ile Phe
                   165                 170                 175
   Ser Ala Ala Gly Leu Pro His Asn Glu Ile Ala Ala Gln Ile Val Arg
               180                 185                 190
   Gln Gly Gly Leu Val Lys Gln Pro Asn Arg Lys Lys Glu Asn Glu Val
           195                 200                 205
   Glu Gly Asp Asp Asp Phe Ala Ile Val Phe Ala Ala Met Gly Val Asn
       210                 215                 220
   Met Glu Thr Ala Arg Phe Phe Lys Gln Asp Phe Glu Glu Asn Gly Ser
   225                 230                 235                 240
   Met Glu Asn Val Cys Leu Phe Leu Asn Leu Ala Asn Asp Pro Thr Ile
                   245                 250                 255
   Glu Arg Ile Ile Thr Pro Arg Leu Ala Leu Thr Cys Ala Glu Phe Leu
               260                 265                 270
   Ala Tyr Gln Cys Gln Lys His Val Leu Val Val Leu Thr Asp Met Ser
           275                 280                 285
   Tyr Tyr Ala Glu Ala Leu Arg Glu Val Ser Ala Ala Arg Glu Glu Val
       290                 295                 300
   Pro Gly Arg Arg Gly Phe Pro Gly Tyr Met Tyr Thr Asp Leu Ala Arg
   305                 310                 315                 320
   Ile Tyr Glu Arg Ala Gly Arg Val Glu Gly Arg Lys Gly Ser Ile Thr
                   325                 330                 335
   Gln Ile Pro Ile Leu Ser Met Pro Asn Asp Asp Ile Thr His Pro Ile
               340                 345                 350
   Pro Asp Leu Thr Gly Tyr Ile Thr Glu Gly Gln Ile Tyr Val Asp Arg
           355                 360                 365
   Gln Leu His Asn Arg Gln Ile Tyr Pro Pro Ile Asn Val Leu Pro Ser
       370                 375                 380
   Leu Ser Arg Leu Met Lys Ser Ala Ile Gly Glu Gly Met Thr Arg Ala
   385                 390                 395                 400
   Asp His Ser Asp Val Ser Asn Gln Leu Tyr Ala Cys Tyr Ala Ile Gly
                   405                 410                 415
   Lys Asp Val Gln Ala Met Lys Ala Val Val Gly Glu Glu Ala Leu Ser
               420                 425                 430
   Ser Asp Asp Leu Leu Tyr Leu Glu Phe Leu Thr Lys Phe Glu Lys Asn
           435                 440                 445
   Phe Ile Ser Gln Gly Asn Tyr Glu Asn Arg Thr Val Phe Asp Ser Leu
       450                 455                 460
   Asp Ile Gly Trp Gln Leu Leu Arg Ile Phe Pro Arg Glu Met Leu Lys
   465                 470                 475                 480
   Arg Ile Pro Ala Lys Thr Leu Asp Lys Tyr Tyr Pro Arg Gly Gly Thr
                   485                 490                 495
   Gly Ala Arg Asn Glu
               500
<210> 1734
<400> 1734
   000
<210> 1735
   <211> 317
   <212> PRT
   <213> Heterodera glycines
<220>
   <223> cDNA sequence=SeqID_1306; coding sequence=SeqID_1528
<400> 1735
   Met Ser Phe Leu Val Thr Leu Ala Gly Tyr Leu Thr Ile Ala Ile Phe
   1               5                   10                  15
   Leu Gln Lys Leu Phe Arg Ser Leu Tyr Asn Val Val Phe Pro Tyr Leu
               20                  25                  30
   Phe Ala Val Pro Gln Asn Ile Lys Val Leu Ala Gly Ala Glu Trp Ala
           35                  40                  45
   Val Val Thr Gly Ser Thr Asp Gly Ile Gly Lys Ala Tyr Ala Leu Glu
       50                  55                  60
   Leu Ala Lys Lys Asn Phe Asn Val Val Leu Ile Ser Arg Ser Met Asp
   65                  70                  75                  80
   Lys Leu Asn Ala Val Ala Glu Glu Ile Lys Gln Lys Tyr Pro Asn Val
                   85                  90                  95
   Glu Val Lys Cys Ile Ser Phe Asp Phe Thr Asn Ala Asn Leu Lys Asp
               100                 105                 110
   Tyr Glu Gln Thr Ile Phe Ser Gln Leu Ser Thr Ile Glu Val Gly Met
           115                 120                 125
   Leu Val Asn Asn Val Gly Met Ser Tyr Glu Tyr Pro Glu Arg Leu Asp
       130                 135                 140
   Arg Ile Glu Gly Gly Leu Gln Arg Val Ser Asp Ile Thr Val Ile Asn
   145                 150                 155                 160
   Thr Leu Pro Thr Thr Val Leu Ser Ala Phe Val Leu Lys Gln Met Arg
                   165                 170                 175
   Glu Arg Gly Arg Gly Val Val Val Asn Leu Ala Ser Ser Ala Ala Tyr
               180                 185                 190
   Phe Asn Trp Phe Tyr Leu Ala Ala Tyr Ser Ala Ser Lys Lys Tyr Val
           195                 200                 205
   Cys Trp Leu Ser Ser Ile Leu Arg Met Glu Tyr Ala Asp Thr Asp Ile
       210                 215                 220
   Val Ile Gln Thr Val Cys Pro Met Met Val Ala Thr Lys Met Ala Lys
   225                 230                 235                 240
   Ile Arg Lys Ala Ser Phe Phe Thr Pro Ser Pro Glu Glu Phe Ala Ala
                   245                 250                 255
   Gln Ala Leu Arg Ser Ile Gly Leu Val Asp Glu Thr Thr Gly Cys Leu
               260                 265                 270
   Ser His Gln Ile Gln Ala Glu Leu Met Phe Gly Tyr Val Pro Ala Pro
           275                 280                 285
   Leu Leu Asn Lys Phe Val Arg Asp Asn Ser Leu Gln Thr Arg Lys Arg
       290                 295                 300
   Ala Leu Lys Lys Lys Glu Ala Thr Val Ala Lys Ser Glu
   305                 310                 315
<210> 1736
   <211> 216
   <212> PRT
   <213> Heterodera glycines
<220>
   <223> cDNA sequence=SeqID_1307; coding sequence=SeqID_1529
<400> 1736
   Met Asn Gln Glu Gln Asp Asn Ile Pro Thr Phe Lys Leu Val Leu Val
   1               5                   10                  15
   Gly Asp Gly Gly Thr Gly Lys Thr Thr Phe Val Lys Arg His Leu Thr
               20                  25                  30
   Gly Glu Phe Glu Lys Lys Tyr Ile Ala Thr Leu Gly Val Glu Val His
           35                  40                  45
   Pro Leu Thr Phe His Thr Ser Arg Gly Gln Ile Arg Phe Asn Val Trp
       50                  55                  60
   Asp Thr Ala Gly Gln Glu Lys Phe Gly Gly Leu Arg Asp Gly Tyr Tyr
   65                  70                  75                  80
   Ile Gln Gly Gln Cys Ala Ile Val Met Phe Asp Val Thr Ala Arg Val
                   85                  90                  95
   Thr Tyr Lys Asn Val Pro Asn Trp His Arg Asp Leu Val Arg Val Cys
               100                 105                 110
   Glu Asn Ile Pro Ile Val Leu Val Gly Asn Lys Val Asp Val Lys Asp
           115                 120                 125
   Arg Lys Val Lys Ala Lys Ser Ile Thr Phe His Arg Lys Lys Asn Leu
       130                 135                 140
   Gln Tyr Tyr Asp Ile Ser Ala Lys Ser Asn Tyr Asn Phe Glu Lys Pro
   145                 150                 155                 160
   Phe Leu Tyr Leu Ala Arg Lys Leu Ile Gly Asp Pro Asn Leu Glu Phe
                   165                 170                 175
   Val Ala Met Pro Ala Leu Ala Pro Pro Glu Val Gln Met Asp Pro Ala
               180                 185                 190
   Leu Ala Ala Lys Tyr Glu Gln Glu Val Ala Glu Ala Ala Ser Ala Glu
           195                 200                 205
   Leu Pro Asp Asp Asp Glu Asp Ile
       210                 215
<210> 1737
   <211> 310
   <212> PRT
   <213> Heterodera glycines
<220>
   <223> cDNA sequence=SeqID_1310; coding sequence=SeqID_1531
<400> 1737
   Thr Arg His Glu Asn Val Ala Pro Thr Asp Ile Glu Glu Gly Met Arg
   1               5                   10                  15
   Val Gly Val Asp Arg Asn Lys Tyr Gln Ile His Leu Pro Leu Pro Ala
               20                  25                  30
   Lys Ile Asp Ala Ser Val Thr Met Met Gln Val Glu Asp Lys Pro Asp
           35                  40                  45
   Val Thr Tyr Ala Asp Ile Gly Gly Cys Glu Glu Gln Ile Lys Lys Leu
       50                  55                  60
   Arg Glu Val Val Glu Phe Pro Leu Leu Gln Pro Glu Arg Phe Thr Ser
   65                  70                  75                  80
   Leu Gly Ile Glu Pro Pro Lys Gly Val Leu Phe Phe Gly Pro Pro Gly
                   85                  90                  95
   Thr Gly Lys Thr Leu Cys Ala Arg Ala Val Ala Asn Arg Thr Asp Ala
               100                 105                 110
   Cys Phe Ile Arg Val Ile Gly Ser Glu Leu Val Lys Lys Tyr Val Gly
           115                 120                 125
   Glu Gly Ala Arg Met Val Arg Glu Leu Phe Ser Leu Ala Lys Thr Lys
       130                 135                 140
   Lys Ala Cys Ile Leu Phe Phe Asp Glu Val Asp Ala Ile Gly Gly Ala
   145                 150                 155                 160
   Arg Phe Asp Asp Gly Lys Gly Gly Asp Asn Glu Val Gln Arg Thr Met
                   165                 170                 175
   Leu Glu Leu Val Asn Gln Leu Asp Gly Phe Asp Ser Arg Gly Ala Ile
               180                 185                 190
   Lys Val Leu Met Ala Thr Asn Arg Pro Asp Thr Leu Asp Pro Ala Leu
           195                 200                 205
   Ile Arg Pro Gly Arg Ile Asp Arg Arg Ile Glu Phe Ser Leu Pro Asp
       210                 215                 220
   Leu Lys Ala Arg Gly Asn Ile Leu Gln Ile His Thr Lys Arg Met Ser
   225                 230                 235                 240
   Val Asp Arg Asn Ile Arg Tyr Glu Leu Ile Ala Arg Leu Cys Pro Asn
   245 250 255
   Thr Thr Gly Ala Asp Leu Arg Ser Val Cys Thr Glu Ala Gly Met Phe
   260 265 270
   Ala Leu Arg Ala Arg Arg Lys Val Ile Thr Glu Gln Asp Phe Leu Lys
   275 280 285
   Ala Val Gln Lys Val Val Lys Ser Tyr Ala Lys Phe Ser Ser Thr Pro
   290 295 300
   Ala Tyr Met Thr His Asn
   305 310
<210> 1738
<400> 1738
   000
<210> 1739
<400> 1739
   000
<210> 1740
<400> 1740
   000
<210> 1741
   <211> 542
   <212> PRT
   <213> Heterodera glycines
<220>
   <223> cDNA sequence=SeqID_1318; coding sequence=SeqID_1537
<400> 1741
   Ala Arg Gly Ser Pro Ser Leu Ser Val Ala Asn Ser Asn Val Ser Arg
   1 5 10 15
   Gln Leu Gly Gln Pro Lys Val Ala Gly Phe Lys Asp Lys Asp Lys Pro
   20 25 30
   Glu Ser Val Arg Thr Ser Asn Ile Val Ala Ala Lys Ala Val Ala Asp
   35 40 45
   Ala Val Arg Thr Ser Leu Gly Pro Arg Gly Met Asp Lys Met Ile Gln
   50 55 60
   Ser Gly Asn Gly Glu Val Thr Ile Thr Asn Asp Gly Ala Thr Ile Leu
   65 70 75 80
   Asn Gln Met Ser Val Val His Pro Thr Ala Lys Met Leu Val Glu Leu
   85 90 95
   Ser Lys Ala Gln Asp Ile Glu Ala Gly Asp Gly Thr Thr Thr Val Val
   100 105 110
   Val Leu Ala Gly Ser Leu Leu Asp Ala Ala Glu Lys Leu Leu Ala Lys
   115 120 125
   Gly Ile His Pro Thr Thr Ile Ser Asp Ser Phe Gln Arg Ala Ala Thr
   130 135 140
   Phe Ser Glu Lys Val Leu Glu Glu Met Ala Thr Pro Ile Asp Ile Asn
   145 150 155 160
   Asp Asp Asp Glu Leu Val Lys Leu Ala Met Thr Ser Leu Asn Ser Lys
   165 170 175
   Val Val Ser Gln His Ser His Leu Leu Ala Pro Met Ala Val Gln Ala
   180 185 190
   Val Lys Lys Leu Met Ser Ser Pro Glu Asp Ser Ser Val Asn Leu Lys
   195 200 205
   Leu Ile Lys Val Val Lys Lys Leu Gly Glu Thr Val Glu Glu Ser Gln
   210 215 220
   Leu Ile Glu Gly Ala Leu Ile Asp Gln Lys Ser Met Gly His Gly Gly
   225 230 235 240
   Pro Ala Arg Val Glu Lys Ala Lys Ile Gly Leu Ile Gln Phe Gln Ile
   245 250 255
   Ser Pro Pro Lys Pro Asn Met Glu Asn Gln Val Val Ile Ser Asp Tyr
   260 265 270
   Ala Gln Met Asp Arg Ala Leu Lys Glu Glu Arg Asn Tyr Ile Leu Glu
   275 280 285
   Leu Cys Lys Gln Ile Lys Lys Ala Gly Cys Asn Val Leu Leu Ile Gln
   290 295 300
   Lys Ser Ile Leu Arg Asp Ala Ile Asn Glu Met Ala Leu His Phe Phe
   305 310 315 320
   Ala Lys Met Lys Ile Met Val Ile Lys Asp Ile Glu Arg Asp Asp Ile
   325 330 335
   Glu Phe Tyr Ala Lys Ile Leu Gly Cys Arg Pro Val Ala Ser Val Asp
   340 345 350
   His Phe Val Pro Glu Ser Leu Gly Thr Ala Asp Leu Val Glu Glu Ile
   355 360 365
   Glu Thr Ala Asp Gly Lys Val Leu Lys Val Thr Gly Leu Lys Gln Ser
   370 375 380
   Gly Cys Ala Val Ser Ile Leu Val Arg Gly Ser Ser Lys Leu Val Leu
   385 390 395 400
   Asp Glu Ala Glu Arg Ser Leu His Asp Ala Leu Cys Val Ile Arg Cys
   405 410 415
   Leu Val Lys Lys Arg Ala Leu Leu Ser Gly Gly Gly Ala Pro Glu Met
   420 425 430
   Glu Val Ser Val Arg Val Arg Ala Ala Ala Gln Thr Lys His Gly Ala
   435 440 445
   Glu Gln Tyr Cys Trp Lys Ala Phe Ala Asp Ala Leu Glu Val Val Pro
       450                 455                 460
   Tyr Thr Leu Ala Glu Asn Ala Gly Leu Ser Pro Ile Glu Thr Val Thr
   465                 470                 475                 480
   Glu Met Arg Ser Gln His Ala Met Gly Asn Lys Glu His Gly Val Asn
                   485                 490                 495
   Val Arg Met Gly Ala Ile Thr Asp Ile Arg Glu Glu Asn Val Val Gln
               500                 505                 510
   Pro Leu Leu Val Ser Ser Phe Ala Val Lys Gln Ala Ala Glu Cys Val
           515                 520                 525
   Arg Ser Ile Leu Lys Ile Asp Asp Ile Val Leu Ser Val Arg
       530                 535                 540
<210> 1742
<400> 1742
   000
<210> 1743
<400> 1743
   000
<210> 1744
<400> 1744
   000
<210> 1745
<400> 1745
   000
<210> 1746
<400> 1746
   000
<210> 1747
<400> 1747
   000
<210> 1748
   <211> 319
   <212> PRT
   <213> Heterodera glycines
<220>
   <223> cDNA sequence=SeqID_1327; coding sequence=SeqID_1545
<400> 1748
   Ala Arg Gly Leu Lys Leu Lys Val Pro Ile Leu Leu Tyr Cys Phe Val
   1               5                   10                  15
   Gln Cys Leu Ser Ala Val Lys Val Asp Asn Ala Glu Gln Ser Asp Gln
               20                  25                  30
   Pro Thr Gly Asp Ser Ser Ser Gly Gln Phe Pro Ile Ser Tyr Pro Pro
           35                  40                  45
   Ile Lys Tyr Glu Asn Asp Leu Ala Tyr Val Glu Lys Pro Ser Gly Asn
       50                  55                  60
   Glu Arg Cys Leu Phe Tyr Ala Glu Gly Leu Ser Val Val Val Phe Ala
   65                  70                  75                  80
   Thr Lys Lys Ser Gln Ser Ala Trp Ala Asp Tyr Arg Ser Asp Thr Lys
                   85                  90                  95
   Glu Ser Pro Lys Tyr Thr Ala Ser Ile Glu Cys Gln Gly Asn Met Asn
               100                 105                 110
   Val Gly Ser Ser Phe Asp Val Tyr Leu Lys Ile Asp Gly Lys Ala Glu
           115                 120                 125
   Gly Val Val Arg Ala Asp Ile Gly Lys Lys Asp Ala Lys Ser Val Phe
       130                 135                 140
   Thr Val Glu Gly Ala Glu Phe Ile Leu Asn Phe Asn Val Thr Gln Val
   145                 150                 155                 160
   Gly Phe Trp Gln Leu Val Gly Ala Thr Val Glu Arg Ile Glu Val Lys
                   165                 170                 175
   Gly Ser Ser Asp Phe Leu Asn Lys Asn Leu Asn Val Asp Lys Lys Trp
               180                 185                 190
   Thr Leu Glu Pro Ser Val Val Pro Ser Thr Asn Ile Asn Gly Phe Lys
           195                 200                 205
   Asp Tyr Ser Phe Ala Cys Gly Gln Thr Asn Pro Ile Val Trp Ala Leu
       210                 215                 220
   Lys Asp Ser Asn Asp Tyr Ala Val Gly Ile Thr Leu Asn Asn Val Gln
   225                 230                 235                 240
   Phe Gln Pro Phe Asn Ala Thr Tyr Thr Ala Gly Gly Lys Glu Ala Asp
                   245                 250                 255
   Arg Leu Lys Phe Gly Trp Lys Val Asn Asp Cys Gln Gly Leu Phe Ser
               260                 265                 270
   Ile Gly Thr Trp Met Gly Leu Val Val Ala Thr Leu Leu Ile Gly Val
           275                 280                 285
   Leu Phe Phe Gly Phe Leu Met Leu Asn Ser Val Gln Thr Met Asp Arg
       290                 295                 300
   Phe Asp Asp Pro Lys Gln Lys Gln Leu Ile Ile Ser Ser Lys Glu
   305                 310                 315
<210> 1749
<400> 1749
   000
<210> 1750
<400> 1750
   000
<210> 1751
   <211> 426
   <212> PRT
   <213> Heterodera glycines
<220>
   <223> cDNA sequence=SeqID_1330; coding sequence=SeqID_1548
<400> 1751
   Pro Leu Phe Gly Ile Met Ser Thr Asp Ser Ala Asp Pro Leu Tyr Trp
   1               5                   10                  15
   Leu Arg Val Ile Met Ala Ser Asn Arg Gly Thr Leu Met Glu Leu Gly
               20                  25                  30
   Ile Ser Pro Ile Val Thr Ser Gly Leu Ile Met Gln Leu Leu Ala Gly
           35                  40                  45
   Ala Lys Ile Ile Glu Val Gly Asp Thr Pro Lys Glu Arg Ala Leu Phe
       50                  55                  60
   Asn Gly Ala Gln Lys Leu Phe Gly Met Val Ile Thr Ile Gly Gln Ala
   65                  70                  75                  80
   Ile Val Tyr Val Ala Ser Gly Met Tyr Gly Asn Pro His Glu Ile Gly
                   85                  90                  95
   Ala Gly Ile Cys Leu Leu Ile Val Ile Gln Leu Val Val Ala Gly Leu
               100                 105                 110
   Ile Val Leu Leu Leu Asp Glu Leu Leu Gln Lys Gly Tyr Gly Leu Gly
           115                 120                 125
   Ser Gly Ile Ser Leu Phe Ile Ala Thr Asn Ile Cys Glu Thr Ile Val
       130                 135                 140
   Trp Lys Ala Phe Ser Pro Ala Thr Met Asn Thr Gly Arg Gly Thr Glu
   145                 150                 155                 160
   Phe Glu Gly Ala Ile Ile Ala Leu Phe His Leu Leu Ala Thr Arg Asn
                   165                 170                 175
   Asp Lys Val Arg Ala Leu Arg Glu Ala Phe Tyr Arg Gln Asn Leu Pro
               180                 185                 190
   Asn Leu Met Asn Leu Leu Ala Thr Val Leu Val Phe Ala Val Val Ile
           195                 200                 205
   Tyr Phe Gln Gly Phe Arg Val Asp Leu Pro Ile Lys Ser Ala Arg Tyr
   210 215 220
   Arg Gly Gln His Ser Ser Tyr Pro Ile Lys Leu Phe Tyr Thr Ser Asn
   225 230 235 240
   Ile Pro Ile Ile Leu Gln Ser Ala Leu Val Ser Asn Leu Tyr Val Ile
   245 250 255
   Ser Gln Met Leu Ala Ala Lys Phe Gly Gly Asn Ile Leu Val Asn Leu
   260 265 270
   Leu Gly Thr Trp Ser Asp Ser Ser Gly Thr Tyr Arg Ser Phe Pro Thr
   275 280 285
   Gly Gly Ile Cys Tyr Tyr Leu Ser Pro Pro Glu Ser Leu Ser His Ile
   290 295 300
   Leu Glu Asp Pro Ile His Cys Ile Ile Tyr Ile Val Phe Met Leu Gly
   305 310 315 320
   Ser Cys Ala Phe Phe Ser Lys Thr Trp Ile Asp Val Ser Gly Ser Ser
   325 330 335
   Ala Lys Asp Val Ala Lys Gln Leu Lys Glu Gln Gln Met Ile Met Arg
   340 345 350
   Gly His Arg Glu Lys Ser Met Ile His Glu Leu Asn Arg Tyr Ile Pro
   355 360 365
   Thr Ala Ala Ala Phe Gly Gly Leu Cys Ile Gly Ala Leu Ser Val Thr
   370 375 380
   Ala Asp Phe Leu Gly Ala Ile Gly Ser Gly Thr Gly Ile Leu Leu Ala
   385 390 395 400
   Val Thr Ile Ile Tyr Gln Tyr Phe Glu Ile Phe Val Lys Glu Gln Gln
   405 410 415
   Glu Met Gly Gly Val Ala Gly Leu Phe Phe
   420 425
<210> 1752
<400> 1752
   000
<210> 1753
<400> 1753
   000
<210> 1754
<400> 1754
   000
<210> 1755
<400> 1755
   000
<210> 1756
<400> 1756
   000
<210> 1757
<400> 1757
   000
<210> 1758
<400> 1758
   000
<210> 1759
<400> 1759
   000
<210> 1760
<400> 1760
   000
<210> 1761
<400> 1761
   000
<210> 1762
<400> 1762
   000
<210> 1763
<400> 1763
   000
<210> 1764
<400> 1764
   000

<210> 1765
<400> 1765
   000
<210> 1766
<400> 1766
   000
<210> 1767
<400> 1767
   000
<210> 1768
<400> 1768
   000
<210> 1769
<400> 1769
   000
<210> 1770
<400> 1770
   000
<210> 1771
<400> 1771
   000
<210> 1772
<400> 1772
   000
<210> 1773
<400> 1773
   000
<210> 1774
<400> 1774
   000
<210> 1775
<400> 1775
   000
<210> 1776
<400> 1776
   000
<210> 1777
<400> 1777
   000
<210> 1778
<400> 1778
   000
<210> 1779
<400> 1779
   000
<210> 1780
<400> 1780
   000
<210> 1781
<400> 1781
   000
<210> 1782
<400> 1782
   000
<210> 1783
<400> 1783
   000
<210> 1784
<400> 1784
   000
<210> 1785
<400> 1785
   000
<210> 1786
<400> 1786
   000
<210> 1787
<400> 1787
   000
<210> 1788
<400> 1788
   000
<210> 1789
<400> 1789
   000
<210> 1790
<400> 1790
   000
<210> 1791
<400> 1791
   000
<210> 1792
<400> 1792
   000
<210> 1793
<400> 1793
   000
<210> 1794
<400> 1794
   000
<210> 1795
<400> 1795
   000
<210> 1796
<400> 1796
   000
<210> 1797
<400> 1797
   000
<210> 1798
<400> 1798
   000
<210> 1799
<400> 1799
   000
<210> 1800
<400> 1800
   000
<210> 1801
<400> 1801
   000
<210> 1802
<400> 1802
   000
<210> 1803
<400> 1803
   000
<210> 1804
<400> 1804
   000
<210> 1805
<400> 1805
   000
<210> 1806
<400> 1806
   000
<210> 1807
<400> 1807
   000
<210> 1808
<400> 1808
   000
<210> 1809
<400> 1809
   000
<210> 1810
<400> 1810
   000
<210> 1811
<400> 1811
   000
<210> 1812
<400> 1812
   000
<210> 1813
<400> 1813
   000
<210> 1814
<400> 1814
   000
<210> 1815
<400> 1815
   000
<210> 1816
<400> 1816
   000
<210> 1817
<400> 1817
   000
<210> 1818
<400> 1818
   000
<210> 1819
<400> 1819
   000
<210> 1820
<400> 1820
   000
<210> 1821
<400> 1821
   000
<210> 1822
<400> 1822
   000
<210> 1823
<400> 1823
   000
<210> 1824
<400> 1824
   000
<210> 1825
<400> 1825
   000
<210> 1826
<400> 1826
   000
<210> 1827
<400> 1827
   000
<210> 1828
<400> 1828
   000
<210> 1829
<400> 1829
   000
<210> 1830
<400> 1830
   000
<210> 1831
<400> 1831
   000
<210> 1832
<400> 1832
   000
<210> 1833
<400> 1833
   000
<210> 1834
<400> 1834
   000
<210> 1835
<400> 1835
   000
<210> 1836
<400> 1836
   000
<210> 1837
<400> 1837
   000
<210> 1838
<400> 1838
   000
<210> 1839
<400> 1839
   000
<210> 1840
<400> 1840
   000
<210> 1841
<400> 1841
   000
<210> 1842
<400> 1842
   000
<210> 1843
<400> 1843
   000
<210> 1844
<400> 1844
   000
<210> 1845
<400> 1845
   000
<210> 1846
<400> 1846
   000
<210> 1847
<400> 1847
   000
<210> 1848
<400> 1848
   000
<210> 1849
<400> 1849
   000
<210> 1850
<400> 1850
   000
<210> 1851
<400> 1851
   000
<210> 1852
<400> 1852
   000
<210> 1853
<400> 1853
   000
<210> 1854
<400> 1854
   000
<210> 1855
<400> 1855
   000
<210> 1856
<400> 1856
   000
<210> 1857
<400> 1857
   000
<210> 1858
<400> 1858
   000
<210> 1859
<400> 1859
   000
<210> 1860
<400> 1860
   000
<210> 1861
<400> 1861
   000
<210> 1862
<400> 1862
   000
<210> 1863
<400> 1863
   000
<210> 1864
<400> 1864
   000
<210> 1865
<400> 1865
   000
<210> 1866
<400> 1866
   000
<210> 1867
<400> 1867
   000
<210> 1868
<400> 1868
   000
<210> 1869
<400> 1869
   000
<210> 1870
<400> 1870
   000
<210> 1871
<400> 1871
   000
<210> 1872
<400> 1872
   000
<210> 1873
<400> 1873
   000
<210> 1874
<400> 1874
   000
<210> 1875
<400> 1875
   000
<210> 1876
<400> 1876
   000
<210> 1877
<400> 1877
   000
<210> 1878
<400> 1878
   000
<210> 1879
<400> 1879
   000
<210> 1880
<400> 1880
   000
<210> 1881
<400> 1881
   000
<210> 1882
<400> 1882
   000
<210> 1883
<400> 1883
   000
<210> 1884
<400> 1884
   000
<210> 1885
<400> 1885
   000
<210> 1886
<400> 1886
   000
<210> 1887
<400> 1887
   000
<210> 1888
<400> 1888
   000
<210> 1889
   <211> 273
   <212> PRT
   <213> Heterodera glycines
<220>
   <223> cDNA sequence=SeqID_1304; coding sequence=SeqID_1487
<400> 1889
   Thr Arg Arg Glu Tyr Leu Pro Lys Val Leu Gly Ser Ser Phe Ala Ala
   1               5                   10                  15
   Thr Ile Gly Pro Tyr Arg Gly Tyr Asp Pro Asn Ile Asp Pro Thr Val
               20                  25                  30
   Ala Asn Glu Phe Thr Ser Gln Ala Tyr Arg Phe Gly His Gly Met Ile
           35                  40                  45
   Gln Glu Phe Tyr Pro Arg Leu Asp Ser Ala Gly Arg Thr Ile Pro Ser
       50                  55                  60
   Gly Gly Phe Ala Phe Val Asp Gly Thr Leu His Ser Asp His Phe Ile
   65                  70                  75                  80
   Phe Gln Gly Gly Pro Asp Pro Ile Ile Arg Gly Met Met Ser Gln Gln
                   85                  90                  95
   Leu Lys Arg Pro Gln Arg Leu Thr Arg Thr Ile Thr Glu Gln Met Phe
               100                 105                 110
   Gly Thr Thr Asp Leu Gly Thr Ile Asn Ile Gln Arg Gly Arg Asp His
           115                 120                 125
   Gly Val Pro Ser Tyr Met Arg Phe Arg Gln Leu Cys Gly Leu Ser Thr
       130                 135                 140
   Ala Thr Thr Phe Asp Gln Leu Ser Arg Glu Ile Met Ser Val Glu Ala
   145                 150                 155                 160
   Arg Gln Asn Leu Gln Arg Ile Tyr Gly Thr Pro Asp Arg Ile Asp Leu
                   165                 170                 175
   Phe Val Gly Gly Met Leu Glu Asp Pro Val Val Arg Gly Leu Val Gly
               180                 185                 190
   Pro Thr Val Ala Cys Ile Ile Gly Pro Gln Phe Ala Arg Thr Arg Asp
           195                 200                 205
   Gly Asp Arg Phe Tyr Phe Glu Asn Pro Gly Ile Phe Thr Arg Ala Gln
       210                 215                 220
   Leu Ala Glu Ile Arg Arg Ser Ser Leu Ala Arg Val Ile Cys Asp Asn
   225                 230                 235                 240
   Gly Asp Asp Phe Arg Thr Val Pro Arg Glu Ala Phe Arg Thr Gly Pro
                   245                 250                 255
   Ile Val Gln Cys Thr Glu Ile Pro Gln Met Asp Leu Ser Lys Trp Lys
               260                 265                 270
   Glu
<210> 1890
<400> 1890
   000
<210> 1891
<400> 1891
   000
<210> 1892
<400> 1892
   000
<210> 1893
<400> 1893
   000
<210> 1894
<400> 1894
   000
<210> 1895
<400> 1895
   000
<210> 1896
<400> 1896
   000
<210> 1897
<400> 1897
   000
<210> 1898
<400> 1898
   000
<210> 1899
<400> 1899
   000
<210> 1900
<400> 1900
   000
<210> 1901
<400> 1901
   000
<210> 1902
<400> 1902
   000
<210> 1903
<400> 1903
   000
<210> 1904
<400> 1904
   000
<210> 1905
<400> 1905
   000
<210> 1906
   <211> 353
   <212> PRT
   <213> Heterodera glycines
<220>
   <221> misc_feature
   <222> (262)..(262)
   <223> Xaa can be any naturally occurring amino acid
<220>
   <223> cDNA sequence=SeqID_1312; coding sequence=SeqID_1533
<400> 1906
   Gly Ala Phe Gly Cys Gly Lys Thr Val Ile Ser Gln Ser Leu Ser Lys
   1 5 10 15
   Tyr Ser Asn Ser Asp Ala Ile Ile Tyr Val Gly Cys Gly Glu Arg Gly
   20 25 30
   Asn Glu Met Ser Glu Val Leu Arg Asp Phe Pro Glu Leu Thr Met Glu
   35 40 45
   Val Asp Gly Val Thr Thr Ser Ile Met Glu Arg Thr Ala Leu Val Ala
   50 55 60
   Asn Thr Ser Asn Met Pro Val Ala Ala Arg Glu Ala Ser Ile Tyr Thr
   65 70 75 80
   Gly Ile Thr Leu Ala Glu Tyr Phe Arg Asp Met Gly Leu Asn Val Ala
   85 90 95
   Met Met Ala Asp Ser Thr Ser Arg Trp Ala Glu Ala Leu Arg Glu Ile
   100 105 110
   Ser Gly Arg Leu Gly Glu Met Pro Ala Asp Ser Gly Tyr Pro Ala Tyr
   115 120 125
   Leu Ala Ala Arg Leu Ala Ser Phe Tyr Glu Arg Ala Gly Lys Val Lys
   130 135 140
   Cys Phe Gly Ser Pro Glu Arg Glu Gly Ser Val Thr Ile Val Gly Ala
   145 150 155 160
   Val Ser Pro Pro Gly Gly Asp Phe Ala Asp Pro Val Thr Ser Ala Thr
   165 170 175
   Leu Gly Ile Val Gln Val Phe Trp Gly Leu Asp Lys Lys Leu Ala Gln
   180 185 190
   Arg Lys His Phe Pro Ser Ile Asn Trp Leu Ile Ser Tyr Ser Lys Tyr
   195 200 205
   Met Arg Ala Leu Asp Asp Tyr Tyr Glu Lys Asn Phe Ala Glu Phe Val
       210                 215                 220
   Pro Leu Arg Thr Lys Cys Lys Glu Ile Leu Gln Glu Glu Glu Asp Leu
   225                 230                 235                 240
   Ser Glu Ile Val Gln Leu Val Gly Lys Ala Ser Leu Ala Glu Ala Asp
                   245                 250                 255
   Lys Ile Thr Leu Glu Xaa Tyr Asp Arg Phe Cys Pro Phe Tyr Lys Thr
               260                 265                 270
   Val Gly Met Leu Lys Asn Met Ile Gly Phe Tyr Asp Phe Ala Arg His
           275                 280                 285
   Ser Val Glu Thr Thr Ala Gln Ser Asp Asn Lys Ile Thr Trp Asn Gln
       290                 295                 300
   Ile Arg Asp His Leu Gly Asp Ile Ile His Glu Leu Ser Ser Met Lys
   305                 310                 315                 320
   Phe Glu Asp Pro Ser Glu Lys Thr Glu Asp Gln Ile Lys Lys Asp Tyr
                   325                 330                 335
   Glu Glu Leu Tyr Glu Arg Met Gln Asn Lys Phe Arg Ala Leu Glu Asp
               340                 345                 350
   Glu
<210> 1907
<400> 1907
   000
<210> 1908
<400> 1908
   000
<210> 1909
<400> 1909
   000
<210> 1910
<400> 1910
   000
<210> 1911
<400> 1911
   000
<210> 1912
<400> 1912
   000
<210> 1913
<400> 1913
   000
<210> 1914
<400> 1914
   000
<210> 1915
<400> 1915
   000
<210> 1916
<400> 1916
   000
<210> 1917
<400> 1917
   000
<210> 1918
<400> 1918
   000
<210> 1919
<400> 1919
   000
<210> 1920
   <211> 2335
   <212> DNA
   <213> Heterodera glycines
<220>
   <223> coding sequence=SeqID_1925; coordinates=5-2335
<220>
   <223> peptide sequence=SeqID_1930
<400> 1920
   gaatatggca atgatggctg aggcaaatgg agagacccca ctgttgaatg gtggtgacct     60
   aatggactcg aattcaagtc aagaagggtc ctccattggc acgggcaccg aaaacggatt    120
   gtccaattcc tcatctgaag ctgctgttat taacaatgag caccagcaaa atggtgggaa    180
   caatgttcct gcaccaaaag gaggacaaag tgaggggacc gacttggaca atgttccatg    240
   gggcgaacgg atacgcgaca ccacggggaa acgaaaagaa gaaaccagca atgagaagaa    300
   aaaggggaaa aaagctgagg aagttatgcc gaaaaagtcg aaggaatcga cctcaaaaag    360
   tcgaaaaaag aacgaaagca gcggagagga agaagacttt gacgacgact ccgacgggga    420
   agaggaggaa tatcagccaa agaaaaagaa acaaaagtcg gacccatccc cggcgaagaa    480
   aagcgacagc aaaaagtcaa cgaaaacaac aaaagttacc aaggtggagg tcaaggaaga    540
   gagcggcgct ggggttgagc acaagaaacg gaagacaacg aaagttgaag tgaaggaaga    600
   gccctcctct tcccagggaa gtccgtccaa gaaacgtgtg aagaaacagg aggaagaaga    660
   ggaagtgtgg cgctggtggg aggaggacga gtcgaacgcg ggcgcctccg atgggtccat    720
   caaatggcaa acgcttgtcc acaaagggcc cgtctttgca ccggcctatg tgccattgcc    780
   aaaacacatc aagttcaaat acgacggaaa ggagatggat ttgtctttgc cggccgaaga    840
   agtggcttcc ttttacgcga aaatgctcga ccacgactac acgtcaaaac cggtgttcaa    900
   ccagaacttt ttcaaggatt gggtcaaagt gatgacaaag gcggagcgag ccgaaattaa    960
   agatttggcc aaatgcgatt ttaggcaaat gcacgctttt tacgtgtcgg agtcggagaa   1020
   gcgcaaagcg cgcagcaaag aggagaaaaa ggcggagaaa gaggagaagg aaggcgaggc   1080
   aaaggtgtac gggttcgcca tgttggacgg atacaaacag aaaattggca acttccgaat   1140
   agaaccgccg ggactattca gggggagagg tgaccatcca aagatgggtt ctctgaagaa   1200
   acgggtccag ccggaggatg tggtcatcaa ctgctcaaga ggactgaaga ttatgccgcc   1260
   ggaggggcac aaatggaagg aaatccgtca tgataacact gtcacttggc tgtgttcatg   1320
   gaccgaaaat gtgttgggca gcaacaagta cataatgctg aatccgtcca gcaaactcaa   1380
   aggccaaaag gattgggaga agtttgagaa ggcgagaaag ttaaaaggac atgtcggtca   1440
   aatccgccag cagtacctcg ccgactttaa gtctaaggaa atgcgtatcc gtcagcgtgc   1500
   cgtggcacta tattttatcg acaaattggc acttcgtgcg ggcaatgaaa aagacaccga   1560
   cgaagcggcc gacacggtcg gttgttgctc gttgcgttgt gagcacatca aattgcacga   1620
   tgatatggac gacctgaagt atgttgtgga attcgacttc ctcggcaaag actccatccg   1680
   ctattacaac cgtgtgccgg tggaaaagca agtgttcaaa aatctgaagc tttttatgga   1740
   ggagaaagac ccgggggacg atttgttcga ccgattggat acgagttcgt tgaaccaata   1800
   cctgaaggaa ctgatggacg gactgaccgc gaaagtgttt cgtacgtata acgcgtcgat   1860
   cactctccaa gaacagctgg acaaactgac aaatccttcc gattccgtcc acgagaaaat 1920
   gctttcctac aatcgtgcaa atcgtcaggt ggccatcctt tgtaaccatc aaagggcagt 1980
   gccaaaaacg cacgaaaagt cgatggaaaa tttggagaat aaaataaaag agaagaagca 2040
   ggagtacaag gagttgaagg atcagctgaa gaagagcaaa tcggtggacg agaaaatggc 2100
   aaaaaaatac gaaagattga aggaacagat cgccaaactg aacacacagc gcacggacaa 2160
   ggatgaaaac aaacaaattg cactgggaac ctcaaaactg aactacttgg atccgaggat 2220
   ttcggttgca tggtgcaaaa agaatgacgt gcccgttgag aaaatattca gcaaaacgca 2280
   acgcgacaaa ttccgctggg ctctcgacat ggcgaaggag gacttcattt tttga 2335

<210> 1921
   <211> 1263
   <212> DNA
   <213> Heterodera glycines
<220>
   <223> coding sequence=SeqID_1926; coordinates=255-1214
<220>
   <223> peptide sequence=SeqID_1931
<400> 1921
   atggagtgca tgagaatgtt gattgtccgt ctccgtggtg agtttgagaa acgcgtccgt      60
   ttcctccgtc cgtccgatgt ttgcattttc catcgaattg aatgaaaaat tccgcggatc     120
   tgctgagggg gcgctaaggc taacatttcg gggtcgattt gcgcgaccgc ttctcttcgg     180
   ctgtgcaaac ctcgctttct cccatcccaa ctttgccttt gcccaatccc tcatttgtcc     240
   ctaattattt caaaatgcaa cggttcgctt ctcaaaagga aactatccat tccgtgccgg     300
   atgacgacga cactttggag gagccgcccc gactgcccca gggcaatcgc cgtcggttgg     360
   gcgtgtcggc ggaggtgccg gacgaaaatg aggcggccaa ttaccagcga gtggtcatcc     420
   cgaaagacga cgacgtgcaa aaggctttgc gagcggcgat gtgcaaaaat gtgctgtttg     480
   cgcatttgga cttggacgag gaaaaggcca ttttcgacgc catgtttccg gtggagaaac     540
   acctcgggga gacaatcatt gagcagggtg aagagggcga caatttttat gtgattgaca     600
   gtggcgaggt cgatgtgttc gtcaatggcg agttcgtgtt gagcattaag gagggaggtt     660
   catttggcga gttggcactg atttatggca ctccgcgcgc agccaccgta gtcgcgaaga     720
   cgaacacggt caaactgtgg gssatcgacc gcatcactta ccgcgccatt cttatggggt     780
   cgacgatgcg aaagcggaaa atgtacgacg aattcctgag caaagttcag actcttgccg     840
   acttagacaa atgggaaagg gccaatgtgg cggatgcgtt ggaacagtgc caattcccct     900
   cgggcaccca cgtggtggag caaggacatc ctggggacga attcttcatc attgtggagg     960
   gcgaagctga ggtgctgcag agaccgaacg acgacgcgcc cttcgaagtg gtcggaaaat    1020
   tgggaccctc tgactatttc ggggaaatcg ccctccttct cgatcgccct cgtgccgcca    1080
   ccgtcgtggc caaaactctg ctgaaatgtc tgaaattgga ccgggcacga tttgagcgcg    1140
   tgatgggtcc ggtgcgtgag attctcaagc gcgacgtgtc caattacaat tcgtacgtga    1200
   agctgatgac ttgacgagcc ggcagatctt tggcggccaa agaattgatc gccgatcact    1260
   cta                                                                  1263
<210> 1922
<400> 1922
   000
<210> 1923
   <211> 3557
   <212> DNA
   <213> Heterodera glycines
<220>
   <223> coding sequence=SeqID_1928; coordinates=263-3433
<220>
   <223> peptide sequence=SeqID_1933
<400> 1923
   aaatttttcg ttgactgatt ttgtaataaa aagaacacaa taaagtggtt tttgctcaca     60
   ttccaattct ttcttttagt ytgctccgtt gaatttttat tctcatttat tggcggtggt    120
   caatagccyt acaattagag ccgaatatta gccgtttgat tgaataacat tcggagttat    180
   tttgatgatt tttgacccaa taattgctcg gatgcaattg gccactgaaa attagattcg    240
   ttttttccat ttccagtggt ttatgcagtc atctgacctt attattgagg aaccagcaac    300
   gaaaaaggca aaaatcatct gtgaactgcc gaactccgag gcatcagaaa acgcaccaaa    360
   gagtaacgga gaacaaatgg cggccatcga caccaatttg tactctcggc agatttatgc    420
   gcttggacat tccgccatgg aacacttgcg caaggcgtcg gttctcgtca gtggcctcgg    480
   ctccgtgggc gtggaggtgg cgaagaatct gatccttggc ggtgttcgct cggtgactct    540
   tcacgacaca aaagcgttgg cgttcaaaga tttgtcggcg catttctacg ccaaggaaag    600
   cgacgtggga acgaaccgtg ccaaagtttg cttcgacaaa ttggcggagc tcaacgacac    660
   tgtgaactgt gcattgtctg tggaaaatct gtcggaggat ttcgtcaaac aattcgactt    720
   ggtcgttctc accgacgcgt ccttttccac tcagacggaa gtgaacggtt ggatgcgcaa    780
   ccatcgcaaa atgttcatct cggcagacgc gcgcggactt ttttcttatg catttgtgga    840
   cttgggcgac gaattcgttg tgaacgattc cgacggggag acgtgcaaag aggtgctcgt    900
   tgagtacatc gactgcgaaa gcggcgacat gacgacactc gaaggcgcat ttcaccagtt    960
   ggaggacggg gaccacgttg tgttcaccga ctttgagcac gccgaactga acgaacttcc   1020
   accagtgcca gtcactttga agggaagcaa agtcttcaac attggaaatg ctctcgcggc   1080
   cttttctggc gcagttcaag gccccgtttc gcgtggacgt gttcagcaag tcaaagtgcc 1140
   caagaaaatg tccttcaagc cattgtctct cgcactcgcc gagcccgact ttatgatttg 1200
   ggactttgcc aagttcgacg aacctgcgca acttcacgcc ctttggcagg ctttgtacag 1260
   ttttgaggag atgaaaaact acagcccttt gccccgttcc gatgccgacg caaaattgtt 1320
   aaaggaaatg ctgccaaaag atgcgcccga aatgccggac tcactgctct tcaacttctc 1380
   ttaccaggcg tgtggaaatc tccaacccat cgcttcgctc atcggcggtt tggtggccca 1440
   agaggcgatg aaggcagtga cgcaccacac gactcccctc aagcagtttc tgtacaccga 1500
   ttctcttgag gcgttgccgg gcgcagattc gacctttgac gccgccaagt tggagaagaa 1560
   cgattgtgca ccgcgcaaca atcgctacga cggacaggcg gccgttttcg ggtgggcctt 1620
   tcaggaggcg ctgcagcaac agaaatggtt cattgttggc gccggagcga ttggctgtga 1680
   gctgctgaag aacatggcga tgatgggctt gggctgtggc aacaatggct cgctgaccat 1740
   caccgacccc gacaccatcg aagtgtccaa tttgaaccgc cagtttctgt ttcgtcgtcc 1800
   tgatgttggg aaaaagaagt ccgaagtcgc cgcccgttcc gtcaaagcgt ttaactcgac 1860
   cattcacatc aatccgatgt ctgagatcgt cgccgagtcg accgagcaca tcttcggcga 1920
   cgacttcttc gcccgtctga acggcgtctg taatgcgctg gacaatgtcg aagcacgccg 1980
   ttacgttgac cgccgctgcg tgtactatca gcttccgctg ctcgagtccg gcacaatggg 2040
   cgcaaagggc aacacgcaag tggtcttccc gcacttgacg gagtcttacg gctccacgtc 2100
   cgacccgccc gagaaggaga cgcccatctg cacgctgcgc aatttcccct accaaatcca 2160
   tcacaccatt caatgggcgc gagcgaagtt caccgactat ttcacttcgg ttgcggagac 2220
   ggtcaaccaa tacttggaca atgtgaacga ctttttgcac cgagtgaacc aaatgacttt 2280
   ttctcagcgg atcgaagtgc tcagtacgct tgagcatgcg ttggtcgtcg aatacccaaa 2340
   aaatgcggaa gaatgtgtga gttgggcccg tgaactattc gacacgcttt accacaacga 2400
   cattgcccaa atgctgcaca atttcccttc cagcatgctt actgcccaag gcgacaaatt 2460
   ttggagtgga accaaacgct gtccgcacac tttgaacttc gacgttaaca accctgaaca 2520
   cttcgagttc gtcttctccg cttccatttt gcgtgcccaa tcttacagtt tggctccgat 2580
   cactgaccgc aaaaaagtgg cacaattggc catggcgtac tgctccaagc cgttgagacc 2640
   acaagaggga gtgcgcatcg ctgtgaccga cgccgaggcg acggccaatg atggaaacac 2700
   agcaaatggc gatgcagacg aaactgaact tcggctgaac gatctgaacg tgaaattggc 2760
   caggctgaag ttggagaaca ttcgccgcat gacgcccatt gactttgaga aggacgacga 2820
   ctccaaccac cacgtcgact tcgtgacgag tgcctccaat ttgcgagccg ataattacga 2880
   catcgaaaag gctgatcgga tgaaaacaaa acaaattgcg ggcaaaatca tcccggcgtt 2940
   ggccaccaca actgcgctcg tctcagggct cgtctgcatc gaactctaca aaaccattga 3000
   ggcggacgga aagcgctcga cgactccgat cgaacacttc aaaaatgcat ttatcaactt 3060
   ggctacgcct ttcattgcgt tcagcgagcc gggcaaagcg cagaagaaga agtacctcga   3120
   cattgacttc actctttggg accgtttcga agttgacgga ccgatgacat tggggcaatt   3180
   gattgattcg gttgagagta agagtggcct caccgtttca atgatttctt ccggcgtctc   3240
   ccttttgtat gcgttctttc agcccgcaaa aaaagttgcc gagcgcaagg acatggatct   3300
   tattgcggtt ttcgaggaag tgtctcgtgg caaagtgccg gaccatcgcc gagcgatcgt   3360
   tcttgaggcg ttgacccaga atgaggaaaa cgaagacgtt gatatcccat tcattaaata   3420
   taatttccgt taaatcattg tttgagtacg ccggtatatt ggatattgtt gtgtccatat   3480
   aagtgattgt ctgttccttc gttctcgaaa taataaactc agtcagctaa aaaaaaaaaa   3540
   aaaaaaaaaa aaaaaaa                                                  3557
<210> 1924
<400> 1924
   000
<210> 1925
   <211> 2331
   <212> DNA
   <213> Heterodera glycines
<220>
   <223> cDNA sequence=SeqID_1920; peptide sequence=SeqID_1930
<400> 1925
   atggcaatga tggctgaggc aaatggagag accccactgt tgaatggtgg tgacctaatg    60
   gactcgaatt caagtcaaga agggtcctcc attggcacgg gcaccgaaaa cggattgtcc   120
   aattcctcat ctgaagctgc tgttattaac aatgagcacc agcaaaatgg tgggaacaat   180
   gttcctgcac caaaaggagg acaaagtgag gggaccgact tggacaatgt tccatggggc   240
   gaacggatac gcgacaccac ggggaaacga aaagaagaaa ccagcaatga gaagaaaaag   300
   gggaaaaaag ctgaggaagt tatgccgaaa aagtcgaagg aatcgacctc aaaaagtcga   360
   aaaaagaacg aaagcagcgg agaggaagaa gactttgacg acgactccga cggggaagag   420
   gaggaatatc agccaaagaa aaagaaacaa aagtcggacc catccccggc gaagaaaagc   480
   gacagcaaaa agtcaacgaa aacaacaaaa gttaccaagg tggaggtcaa ggaagagagc   540
   ggcgctgggg ttgagcacaa gaaacggaag acaacgaaag ttgaagtgaa ggaagagccc   600
   tcctcttccc agggaagtcc gtccaagaaa cgtgtgaaga aacaggagga agaagaggaa   660
   gtgtggcgct ggtgggagga ggacgagtcg aacgcgggcg cctccgatgg gtccatcaaa   720
   tggcaaacgc ttgtccacaa agggcccgtc tttgcaccgg cctatgtgcc attgccaaaa   780
   cacatcaagt tcaaatacga cggaaaggag atggatttgt ctttgccggc cgaagaagtg   840
   gcttcctttt acgcgaaaat gctcgaccac gactacacgt caaaaccggt gttcaaccag   900
   aactttttca aggattgggt caaagtgatg acaaaggcgg agcgagccga aattaaagat     960
   ttggccaaat gcgattttag gcaaatgcac gctttttacg tgtcggagtc ggagaagcgc    1020
   aaagcgcgca gcaaagagga gaaaaaggcg gagaaagagg agaaggaagg cgaggcaaag    1080
   gtgtacgggt tcgccatgtt ggacggatac aaacagaaaa ttggcaactt ccgaatagaa    1140
   ccgccgggac tattcagggg gagaggtgac catccaaaga tgggttctct gaagaaacgg    1200
   gtccagccgg aggatgtggt catcaactgc tcaagaggac tgaagattat gccgccggag    1260
   gggcacaaat ggaaggaaat ccgtcatgat aacactgtca cttggctgtg ttcatggacc    1320
   gaaaatgtgt tgggcagcaa caagtacata atgctgaatc cgtccagcaa actcaaaggc    1380
   caaaaggatt gggagaagtt tgagaaggcg agaaagttaa aaggacatgt cggtcaaatc    1440
   cgccagcagt acctcgccga ctttaagtct aaggaaatgc gtatccgtca gcgtgccgtg    1500
   gcactatatt ttatcgacaa attggcactt cgtgcgggca atgaaaaaga caccgacgaa    1560
   gcggccgaca cggtcggttg ttgctcgttg cgttgtgagc acatcaaatt gcacgatgat    1620
   atggacgacc tgaagtatgt tgtggaattc gacttcctcg gcaaagactc catccgctat    1680
   tacaaccgtg tgccggtgga aaagcaagtg ttcaaaaatc tgaagctttt tatggaggag    1740
   aaagacccgg gggacgattt gttcgaccga ttggatacga gttcgttgaa ccaatacctg    1800
   aaggaactga tggacggact gaccgcgaaa gtgtttcgta cgtataacgc gtcgatcact    1860
   ctccaagaac agctggacaa actgacaaat ccttccgatt ccgtccacga gaaaatgctt    1920
   tcctacaatc gtgcaaatcg tcaggtggcc atcctttgta accatcaaag ggcagtgcca    1980
   aaaacgcacg aaaagtcgat ggaaaatttg gagaataaaa taaaagagaa gaagcaggag    2040
   tacaaggagt tgaaggatca gctgaagaag agcaaatcgg tggacgagaa aatggcaaaa    2100
   aaatacgaaa gattgaagga acagatcgcc aaactgaaca cacagcgcac ggacaaggat    2160
   gaaaacaaac aaattgcact gggaacctca aaactgaact acttggatcc gaggatttcg    2220
   gttgcatggt gcaaaaagaa tgacgtgccc gttgagaaaa tattcagcaa aacgcaacgc    2280
   gacaaattcc gctgggctct cgacatggcg aaggaggact tcattttttg a             2331
<210> 1926
   <211> 960
   <212> DNA
   <213> Heterodera glycines
<220>
   <223> cDNA sequence=SeqID_1921; peptide sequence=SeqID_1931
<400> 1926
   atgcaacggt tcgcttctca aaaggaaact atccattccg tgccggatga cgacgacact     60
   ttggaggagc cgccccgact gccccagggc aatcgccgtc ggttgggcgt gtcggcggag    120
   gtgccggacg aaaatgaggc ggccaattac cagcgagtgg tcatcccgaa agacgacgac    180
   gtgcaaaagg ctttgcgagc ggcgatgtgc aaaaatgtgc tgtttgcgca tttggacttg    240
   gacgaggaaa aggccatttt cgacgccatg tttccggtgg agaaacacct cggggagaca    300
   atcattgagc agggtgaaga gggcgacaat ttttatgtga ttgacagtgg cgaggtcgat    360
   gtgttcgtca atggcgagtt cgtgttgagc attaaggagg gaggttcatt tggcgagttg    420
   gcactgattt atggcactcc gcgcgcagcc accgtagtcg cgaagacgaa cacggtcaaa    480
   ctgtgggssa tcgaccgcat cacttaccgc gccattctta tggggtcgac gatgcgaaag    540
   cggaaaatgt acgacgaatt cctgagcaaa gttcagactc ttgccgactt agacaaatgg    600
   gaaagggcca atgtggcgga tgcgttggaa cagtgccaat tcccctcggg cacccacgtg    660
   gtggagcaag gacatcctgg ggacgaattc ttcatcattg tggagggcga agctgaggtg    720
   ctgcagagac cgaacgacga cgcgcccttc gaagtggtcg gaaaattggg accctctgac    780
   tatttcgggg aaatcgccct ccttctcgat cgccctcgtg ccgccaccgt cgtggccaaa    840
   actctgctga aatgtctgaa attggaccgg gcacgatttg agcgcgtgat gggtccggtg    900
   cgtgagattc tcaagcgcga cgtgtccaat tacaattcgt acgtgaagct gatgacttga    960
<210> 1927
<400> 1927
   000
<210> 1928
   <211> 3171
   <212> DNA
   <213> Heterodera glycines
<220>
   <223> cDNA sequence=SeqID_1923; peptide sequence=SeqID_1933
<400> 1928
   atgcagtcat ctgaccttat tattgaggaa ccagcaacga aaaaggcaaa aatcatctgt    60
   gaactgccga actccgaggc atcagaaaac gcaccaaaga gtaacggaga acaaatggcg   120
   gccatcgaca ccaatttgta ctctcggcag atttatgcgc ttggacattc cgccatggaa   180
   cacttgcgca aggcgtcggt tctcgtcagt ggcctcggct ccgtgggcgt ggaggtggcg   240
   aagaatctga tccttggcgg tgttcgctcg gtgactcttc acgacacaaa agcgttggcg   300
   ttcaaagatt tgtcggcgca tttctacgcc aaggaaagcg acgtgggaac gaaccgtgcc   360
   aaagtttgct tcgacaaatt ggcggagctc aacgacactg tgaactgtgc attgtctgtg   420
   gaaaatctgt cggaggattt cgtcaaacaa ttcgacttgg tcgttctcac cgacgcgtcc   480
   ttttccactc agacggaagt gaacggttgg atgcgcaacc atcgcaaaat gttcatctcg   540
   gcagacgcgc gcggactttt ttcttatgca tttgtggact tgggcgacga attcgttgtg   600
   aacgattccg acggggagac gtgcaaagag gtgctcgttg agtacatcga ctgcgaaagc   660
   ggcgacatga cgacactcga aggcgcattt caccagttgg aggacgggga ccacgttgtg 720
   ttcaccgact ttgagcacgc cgaactgaac gaacttccac cagtgccagt cactttgaag 780
   ggaagcaaag tcttcaacat tggaaatgct ctcgcggcct tttctggcgc agttcaaggc 840
   cccgtttcgc gtggacgtgt tcagcaagtc aaagtgccca agaaaatgtc cttcaagcca 900
   ttgtctctcg cactcgccga gcccgacttt atgatttggg actttgccaa gttcgacgaa 960
   cctgcgcaac ttcacgccct ttggcaggct ttgtacagtt ttgaggagat gaaaaactac 1020
   agccctttgc cccgttccga tgccgacgca aaattgttaa aggaaatgct gccaaaagat 1080
   gcgcccgaaa tgccggactc actgctcttc aacttctctt accaggcgtg tggaaatctc 1140
   caacccatcg cttcgctcat cggcggtttg gtggcccaag aggcgatgaa ggcagtgacg 1200
   caccacacga ctcccctcaa gcagtttctg tacaccgatt ctcttgaggc gttgccgggc 1260
   gcagattcga cctttgacgc cgccaagttg gagaagaacg attgtgcacc gcgcaacaat 1320
   cgctacgacg gacaggcggc cgttttcggg tgggcctttc aggaggcgct gcagcaacag 1380
   aaatggttca ttgttggcgc cggagcgatt ggctgtgagc tgctgaagaa catggcgatg 1440
   atgggcttgg gctgtggcaa caatggctcg ctgaccatca ccgaccccga caccatcgaa 1500
   gtgtccaatt tgaaccgcca gtttctgttt cgtcgtcctg atgttgggaa aaagaagtcc 1560
   gaagtcgccg cccgttccgt caaagcgttt aactcgacca ttcacatcaa tccgatgtct 1620
   gagatcgtcg ccgagtcgac cgagcacatc ttcggcgacg acttcttcgc ccgtctgaac 1680
   ggcgtctgta atgcgctgga caatgtcgaa gcacgccgtt acgttgaccg ccgctgcgtg 1740
   tactatcagc ttccgctgct cgagtccggc acaatgggcg caaagggcaa cacgcaagtg 1800
   gtcttcccgc acttgacgga gtcttacggc tccacgtccg acccgcccga gaaggagacg 1860
   cccatctgca cgctgcgcaa tttcccctac caaatccatc acaccattca atgggcgcga 1920
   gcgaagttca ccgactattt cacttcggtt gcggagacgg tcaaccaata cttggacaat 1980
   gtgaacgact ttttgcaccg agtgaaccaa atgacttttt ctcagcggat cgaagtgctc 2040
   agtacgcttg agcatgcgtt ggtcgtcgaa tacccaaaaa atgcggaaga atgtgtgagt 2100
   tgggcccgtg aactattcga cacgctttac cacaacgaca ttgcccaaat gctgcacaat 2160
   ttcccttcca gcatgcttac tgcccaaggc gacaaatttt ggagtggaac caaacgctgt 2220
   ccgcacactt tgaacttcga cgttaacaac cctgaacact tcgagttcgt cttctccgct 2280
   tccattttgc gtgcccaatc ttacagtttg gctccgatca ctgaccgcaa aaaagtggca 2340
   caattggcca tggcgtactg ctccaagccg ttgagaccac aagagggagt gcgcatcgct 2400
   gtgaccgacg ccgaggcgac ggccaatgat ggaaacacag caaatggcga tgcagacgaa 2460
   actgaacttc ggctgaacga tctgaacgtg aaattggcca ggctgaagtt ggagaacatt 2520
   cgccgcatga cgcccattga ctttgagaag gacgacgact ccaaccacca cgtcgacttc 2580
   gtgacgagtg cctccaattt gcgagccgat aattacgaca tcgaaaaggc tgatcggatg 2640
   aaaacaaaac aaattgcggg caaaatcatc ccggcgttgg ccaccacaac tgcgctcgtc   2700
   tcagggctcg tctgcatcga actctacaaa accattgagg cggacggaaa gcgctcgacg   2760
   actccgatcg aacacttcaa aaatgcattt atcaacttgg ctacgccttt cattgcgttc   2820
   agcgagccgg gcaaagcgca gaagaagaag tacctcgaca ttgacttcac tctttgggac   2880
   cgtttcgaag ttgacggacc gatgacattg gggcaattga ttgattcggt tgagagtaag   2940
   agtggcctca ccgtttcaat gatttcttcc ggcgtctccc ttttgtatgc gttctttcag   3000
   cccgcaaaaa aagttgccga gcgcaaggac atggatctta ttgcggtttt cgaggaagtg   3060
   tctcgtggca aagtgccgga ccatcgccga gcgatcgttc ttgaggcgtt gacccagaat   3120
   gaggaaaacg aagacgttga tatcccattc attaaatata atttccgtta a            3171
<210> 1929
<400> 1929
   000
<210> 1930
   <211> 776
   <212> PRT
   <213> Heterodera glycines
<220>
   <223> cDNA sequence=SeqID_1920; coding sequence=SeqID_1925
<400> 1930
   Met Ala Met Met Ala Glu Ala Asn Gly Glu Thr Pro Leu Leu Asn Gly
   1               5                   10                  15
   Gly Asp Leu Met Asp Ser Asn Ser Ser Gln Glu Gly Ser Ser Ile Gly
               20                  25                  30
   Thr Gly Thr Glu Asn Gly Leu Ser Asn Ser Ser Ser Glu Ala Ala Val
           35                  40                  45
   Ile Asn Asn Glu His Gln Gln Asn Gly Gly Asn Asn Val Pro Ala Pro
       50                  55                  60
   Lys Gly Gly Gln Ser Glu Gly Thr Asp Leu Asp Asn Val Pro Trp Gly
   65                  70                  75                  80
   Glu Arg Ile Arg Asp Thr Thr Gly Lys Arg Lys Glu Glu Thr Ser Asn
                   85                  90                  95
   Glu Lys Lys Lys Gly Lys Lys Ala Glu Glu Val Met Pro Lys Lys Ser
               100                 105                 110
   Lys Glu Ser Thr Ser Lys Ser Arg Lys Lys Asn Glu Ser Ser Gly Glu
           115                 120                 125
   Glu Glu Asp Phe Asp Asp Asp Ser Asp Gly Glu Glu Glu Glu Tyr Gln
       130                 135                 140
   Pro Lys Lys Lys Lys Gln Lys Ser Asp Pro Ser Pro Ala Lys Lys Ser
   145 150 155 160
   Asp Ser Lys Lys Ser Thr Lys Thr Thr Lys Val Thr Lys Val Glu Val
   165 170 175
   Lys Glu Glu Ser Gly Ala Gly Val Glu His Lys Lys Arg Lys Thr Thr
   180 185 190
   Lys Val Glu Val Lys Glu Glu Pro Ser Ser Ser Gln Gly Ser Pro Ser
   195 200 205
   Lys Lys Arg Val Lys Lys Gln Glu Glu Glu Glu Glu Val Trp Arg Trp
   210 215 220
   Trp Glu Glu Asp Glu Ser Asn Ala Gly Ala Ser Asp Gly Ser Ile Lys
   225 230 235 240
   Trp Gln Thr Leu Val His Lys Gly Pro Val Phe Ala Pro Ala Tyr Val
   245 250 255
   Pro Leu Pro Lys His Ile Lys Phe Lys Tyr Asp Gly Lys Glu Met Asp
   260 265 270
   Leu Ser Leu Pro Ala Glu Glu Val Ala Ser Phe Tyr Ala Lys Met Leu
   275 280 285
   Asp His Asp Tyr Thr Ser Lys Pro Val Phe Asn Gln Asn Phe Phe Lys
   290 295 300
   Asp Trp Val Lys Val Met Thr Lys Ala Glu Arg Ala Glu Ile Lys Asp
   305 310 315 320
   Leu Ala Lys Cys Asp Phe Arg Gln Met His Ala Phe Tyr Val Ser Glu
   325 330 335
   Ser Glu Lys Arg Lys Ala Arg Ser Lys Glu Glu Lys Lys Ala Glu Lys
   340 345 350
   Glu Glu Lys Glu Gly Glu Ala Lys Val Tyr Gly Phe Ala Met Leu Asp
   355 360 365
   Gly Tyr Lys Gln Lys Ile Gly Asn Phe Arg Ile Glu Pro Pro Gly Leu
   370 375 380
   Phe Arg Gly Arg Gly Asp His Pro Lys Met Gly Ser Leu Lys Lys Arg
   385 390 395 400
   Val Gln Pro Glu Asp Val Val Ile Asn Cys Ser Arg Gly Leu Lys Ile
   405 410 415
   Met Pro Pro Glu Gly His Lys Trp Lys Glu Ile Arg His Asp Asn Thr
   420 425 430
   Val Thr Trp Leu Cys Ser Trp Thr Glu Asn Val Leu Gly Ser Asn Lys
   435 440 445
   Tyr Ile Met Leu Asn Pro Ser Ser Lys Leu Lys Gly Gln Lys Asp Trp
   450 455 460
   Glu Lys Phe Glu Lys Ala Arg Lys Leu Lys Gly His Val Gly Gln Ile
   465 470 475 480
   Arg Gln Gln Tyr Leu Ala Asp Phe Lys Ser Lys Glu Met Arg Ile Arg
   485 490 495
   Gln Arg Ala Val Ala Leu Tyr Phe Ile Asp Lys Leu Ala Leu Arg Ala
                   500                 505                 510
   Gly Asn Glu Lys Asp Thr Asp Glu Ala Ala Asp Thr Val Gly Cys Cys
           515                 520                 525
   Ser Leu Arg Cys Glu His Ile Lys Leu His Asp Asp Met Asp Asp Leu
       530                 535                 540
   Lys Tyr Val Val Glu Phe Asp Phe Leu Gly Lys Asp Ser Ile Arg Tyr
   545                 550                 555                 560
   Tyr Asn Arg Val Pro Val Glu Lys Gln Val Phe Lys Asn Leu Lys Leu
                   565                 570                 575
   Phe Met Glu Glu Lys Asp Pro Gly Asp Asp Leu Phe Asp Arg Leu Asp
               580                 585                 590
   Thr Ser Ser Leu Asn Gln Tyr Leu Lys Glu Leu Met Asp Gly Leu Thr
           595                 600                 605
   Ala Lys Val Phe Arg Thr Tyr Asn Ala Ser Ile Thr Leu Gln Glu Gln
       610                 615                 620
   Leu Asp Lys Leu Thr Asn Pro Ser Asp Ser Val His Glu Lys Met Leu
   625                 630                 635                 640
   Ser Tyr Asn Arg Ala Asn Arg Gln Val Ala Ile Leu Cys Asn His Gln
                   645                 650                 655
   Arg Ala Val Pro Lys Thr His Glu Lys Ser Met Glu Asn Leu Glu Asn
               660                 665                 670
   Lys Ile Lys Glu Lys Lys Gln Glu Tyr Lys Glu Leu Lys Asp Gln Leu
           675                 680                 685
   Lys Lys Ser Lys Ser Val Asp Glu Lys Met Ala Lys Lys Tyr Glu Arg
       690                 695                 700
   Leu Lys Glu Gln Ile Ala Lys Leu Asn Thr Gln Arg Thr Asp Lys Asp
   705                 710                 715                 720
   Glu Asn Lys Gln Ile Ala Leu Gly Thr Ser Lys Leu Asn Tyr Leu Asp
                   725                 730                 735
   Pro Arg Ile Ser Val Ala Trp Cys Lys Lys Asn Asp Val Pro Val Glu
               740                 745                 750
   Lys Ile Phe Ser Lys Thr Gln Arg Asp Lys Phe Arg Trp Ala Leu Asp
           755                 760                 765
   Met Ala Lys Glu Asp Phe Ile Phe
       770                 775
<210> 1931
   <211> 319
   <212> PRT
   <213> Heterodera glycines
<220>
   <223> cDNA sequence=SeqID_1921; coding sequence=SeqID_1926
<220>
   <221> misc_feature
<210> 1932
<400> 1932
   000
<210> 1933
   <211> 1056
   <212> PRT
   <213> Heterodera glycines
<220>
   <223> cDNA sequence=SeqID_1923; coding sequence=SeqID_1928
<400> 1933
   Met Gln Ser Ser Asp Leu Ile Ile Glu Glu Pro Ala Thr Lys Lys Ala
   1               5                   10                  15
   Lys Ile Ile Cys Glu Leu Pro Asn Ser Glu Ala Ser Glu Asn Ala Pro
               20                  25                  30
   Lys Ser Asn Gly Glu Gln Met Ala Ala Ile Asp Thr Asn Leu Tyr Ser
           35                  40                  45
   Arg Gln Ile Tyr Ala Leu Gly His Ser Ala Met Glu His Leu Arg Lys
       50                  55                  60
   Ala Ser Val Leu Val Ser Gly Leu Gly Ser Val Gly Val Glu Val Ala
   65                  70                  75                  80
   Lys Asn Leu Ile Leu Gly Gly Val Arg Ser Val Thr Leu His Asp Thr
                   85                  90                  95
   Lys Ala Leu Ala Phe Lys Asp Leu Ser Ala His Phe Tyr Ala Lys Glu
               100                 105                 110
   Ser Asp Val Gly Thr Asn Arg Ala Lys Val Cys Phe Asp Lys Leu Ala
           115                 120                 125
   Glu Leu Asn Asp Thr Val Asn Cys Ala Leu Ser Val Glu Asn Leu Ser
       130                 135                 140
   Glu Asp Phe Val Lys Gln Phe Asp Leu Val Val Leu Thr Asp Ala Ser
   145                 150                 155                 160
   Phe Ser Thr Gln Thr Glu Val Asn Gly Trp Met Arg Asn His Arg Lys
                   165                 170                 175
   Met Phe Ile Ser Ala Asp Ala Arg Gly Leu Phe Ser Tyr Ala Phe Val
               180                 185                 190
   Asp Leu Gly Asp Glu Phe Val Val Asn Asp Ser Asp Gly Glu Thr Cys
           195                 200                 205
   Lys Glu Val Leu Val Glu Tyr Ile Asp Cys Glu Ser Gly Asp Met Thr
       210                 215                 220
   Thr Leu Glu Gly Ala Phe His Gln Leu Glu Asp Gly Asp His Val Val
   225                 230                 235                 240
   Phe Thr Asp Phe Glu His Ala Glu Leu Asn Glu Leu Pro Pro Val Pro
                   245                 250                 255
   Val Thr Leu Lys Gly Ser Lys Val Phe Asn Ile Gly Asn Ala Leu Ala
   260 265 270
   Ala Phe Ser Gly Ala Val Gln Gly Pro Val Ser Arg Gly Arg Val Gln
   275 280 285
   Gln Val Lys Val Pro Lys Lys Met Ser Phe Lys Pro Leu Ser Leu Ala
   290 295 300
   Leu Ala Glu Pro Asp Phe Met Ile Trp Asp Phe Ala Lys Phe Asp Glu
   305 310 315 320
   Pro Ala Gln Leu His Ala Leu Trp Gln Ala Leu Tyr Ser Phe Glu Glu
   325 330 335
   Met Lys Asn Tyr Ser Pro Leu Pro Arg Ser Asp Ala Asp Ala Lys Leu
   340 345 350
   Leu Lys Glu Met Leu Pro Lys Asp Ala Pro Glu Met Pro Asp Ser Leu
   355 360 365
   Leu Phe Asn Phe Ser Tyr Gln Ala Cys Gly Asn Leu Gln Pro Ile Ala
   370 375 380
   Ser Leu Ile Gly Gly Leu Val Ala Gln Glu Ala Met Lys Ala Val Thr
   385 390 395 400
   His His Thr Thr Pro Leu Lys Gln Phe Leu Tyr Thr Asp Ser Leu Glu
   405 410 415
   Ala Leu Pro Gly Ala Asp Ser Thr Phe Asp Ala Ala Lys Leu Glu Lys
   420 425 430
   Asn Asp Cys Ala Pro Arg Asn Asn Arg Tyr Asp Gly Gln Ala Ala Val
   435 440 445
   Phe Gly Trp Ala Phe Gln Glu Ala Leu Gln Gln Gln Lys Trp Phe Ile
   450 455 460
   Val Gly Ala Gly Ala Ile Gly Cys Glu Leu Leu Lys Asn Met Ala Met
   465 470 475 480
   Met Gly Leu Gly Cys Gly Asn Asn Gly Ser Leu Thr Ile Thr Asp Pro
   485 490 495
   Asp Thr Ile Glu Val Ser Asn Leu Asn Arg Gln Phe Leu Phe Arg Arg
   500 505 510
   Pro Asp Val Gly Lys Lys Lys Ser Glu Val Ala Ala Arg Ser Val Lys
   515 520 525
   Ala Phe Asn Ser Thr Ile His Ile Asn Pro Met Ser Glu Ile Val Ala
   530 535 540
   Glu Ser Thr Glu His Ile Phe Gly Asp Asp Phe Phe Ala Arg Leu Asn
   545 550 555 560
   Gly Val Cys Asn Ala Leu Asp Asn Val Glu Ala Arg Arg Tyr Val Asp
   565 570 575
   Arg Arg Cys Val Tyr Tyr Gln Leu Pro Leu Leu Glu Ser Gly Thr Met
   580 585 590
   Gly Ala Lys Gly Asn Thr Gln Val Val Phe Pro His Leu Thr Glu Ser
   595 600 605
   Tyr Gly Ser Thr Ser Asp Pro Pro Glu Lys Glu Thr Pro Ile Cys Thr
   610 615 620
   Leu Arg Asn Phe Pro Tyr Gln Ile His His Thr Ile Gln Trp Ala Arg
   625 630 635 640
   Ala Lys Phe Thr Asp Tyr Phe Thr Ser Val Ala Glu Thr Val Asn Gln
   645 650 655
   Tyr Leu Asp Asn Val Asn Asp Phe Leu His Arg Val Asn Gln Met Thr
   660 665 670
   Phe Ser Gln Arg Ile Glu Val Leu Ser Thr Leu Glu His Ala Leu Val
   675 680 685
   Val Glu Tyr Pro Lys Asn Ala Glu Glu Cys Val Ser Trp Ala Arg Glu
   690 695 700
   Leu Phe Asp Thr Leu Tyr His Asn Asp Ile Ala Gln Met Leu His Asn
   705 710 715 720
   Phe Pro Ser Ser Met Leu Thr Ala Gln Gly Asp Lys Phe Trp Ser Gly
   725 730 735
   Thr Lys Arg Cys Pro His Thr Leu Asn Phe Asp Val Asn Asn Pro Glu
   740 745 750
   His Phe Glu Phe Val Phe Ser Ala Ser Ile Leu Arg Ala Gln Ser Tyr
   755 760 765
   Ser Leu Ala Pro Ile Thr Asp Arg Lys Lys Val Ala Gln Leu Ala Met
   770 775 780
   Ala Tyr Cys Ser Lys Pro Leu Arg Pro Gln Glu Gly Val Arg Ile Ala
   785 790 795 800
   Val Thr Asp Ala Glu Ala Thr Ala Asn Asp Gly Asn Thr Ala Asn Gly
   805 810 815
   Asp Ala Asp Glu Thr Glu Leu Arg Leu Asn Asp Leu Asn Val Lys Leu
   820 825 830
   Ala Arg Leu Lys Leu Glu Asn Ile Arg Arg Met Thr Pro Ile Asp Phe
   835 840 845
   Glu Lys Asp Asp Asp Ser Asn His His Val Asp Phe Val Thr Ser Ala
   850 855 860
   Ser Asn Leu Arg Ala Asp Asn Tyr Asp Ile Glu Lys Ala Asp Arg Met
   865 870 875 880
   Lys Thr Lys Gln Ile Ala Gly Lys Ile Ile Pro Ala Leu Ala Thr Thr
   885 890 895
   Thr Ala Leu Val Ser Gly Leu Val Cys Ile Glu Leu Tyr Lys Thr Ile
   900 905 910
   Glu Ala Asp Gly Lys Arg Ser Thr Thr Pro Ile Glu His Phe Lys Asn
   915 920 925
   Ala Phe Ile Asn Leu Ala Thr Pro Phe Ile Ala Phe Ser Glu Pro Gly
   930 935 940
   Lys Ala Gln Lys Lys Lys Tyr Leu Asp Ile Asp Phe Thr Leu Trp Asp
   945 950 955 960
   Arg Phe Glu Val Asp Gly Pro Met Thr Leu Gly Gln Leu Ile Asp Ser
   965 970 975
   Val Glu Ser Lys Ser Gly Leu Thr Val Ser Met Ile Ser Ser Gly Val
   980 985 990
   Ser Leu Leu Tyr Ala Phe Phe Gln Pro Ala Lys Lys Val Ala Glu Arg
   995 1000 1005
   Lys Asp Met Asp Leu Ile Ala Val Phe Glu Glu Val Ser Arg Gly
   1010 1015 1020
   Lys Val Pro Asp His Arg Arg Ala Ile Val Leu Glu Ala Leu Thr
   1025 1030 1035
   Gln Asn Glu Glu Asn Glu Asp Val Asp Ile Pro Phe Ile Lys Tyr
   1040 1045 1050
   Asn Phe Arg
   1055
<210> 1934
<400> 1934
   000

## Description

### BACKGROUND OF THE INVENTION

### 1. Field of the Invention

The present invention relates generally to genetic control of plant disease caused by plant-parasitic nematodes. More specifically, the present invention relates to identification of target coding sequences, and to use of recombinant DNA technologies for post-transcriptionally repressing or inhibiting expression of target coding sequences in the cells of a plant-parasitic nematode to provide a plant protective effect.

### 2. Description of Related Art

Plants are subject to multiple potential disease causing agents, including plant-parasitic nematodes, which are active, flexible, elongate, organisms that live on moist surfaces or in liquid environments, including films of water within soil and moist tissues within other organisms. There are numerous plant-parasitic nematode species, including various cyst nematodes (*e.g*. *Heterodera sp.),* root knot nematodes *(e.g. Meloidogyne sp.),* lesion nematodes *(e.g. Pratylenchus sp.),* dagger nematodes *(e.g. Xiphinema sp.)* and stem and bulb nematodes *(e.g. Ditylenchus sp.),* among others. Tylenchid nematodes (members of the order Tylenchida), including the families Heteroderidae, Meloidogynidae, and Pratylenchidae, are the largest and most economically important group of plant-parasitic nematodes. Other important plant-parasitic nematodes include Dorylaimid nematodes (*e.g. Xiphinema sp.),* among others. Nematode species grow through a series of lifecycle stages and molts. Typically, there are five stages and four molts: egg stage; J1 (*i.e.* first juvenile stage); M1 *(i.e.* first molt); J2 (second juvenile stage; sometimes hatch from egg); M2; J3; M3; J4; M4; A (adult). Juvenile ("J") stages are also sometimes referred to as larval ("L") stages. Gene expression may be specific to one or more lifecycle stages.

Some species of nematodes have evolved as very successful parasites of both plants and animals and are responsible for significant economic losses in agriculture and livestock and for morbidity and mortality in humans. Nematode parasites of plants can inhabit all parts of plants, including roots, developing flower buds, leaves, and stems. Plant parasites are classified on the basis of their feeding habits into the broad categories migratory ectoparasites, migratory endoparasites, and sedentary endoparasites. Sedentary endoparasites, which include the root knot nematodes *(Meloidogyne)* and cyst nematodes *(Globodera* and *Heterodera)* induce feeding sites ("syncytia") and establish long-term infections within roots that are often very damaging to crops. It is estimated that parasitic nematodes cost the horticulture and agriculture industries in excess of $78 billion worldwide a year, based on an estimated average 12% annual loss spread across all major crops. For example, it is estimated that nematodes cause soybean losses of approximately $3.2 billion annually worldwide (Barker *et al.,* 1994).

Compositions, methods, and agents for controlling infestations by nematodes have been provided in several forms. Biological and cultural control methods, including plant quarantines, have been attempted in numerous instances. In some crops, plant resistance genes have been identified that allow nematode resistance or tolerance. Chemical compositions such as nematocides have typically been applied to soil in which plant parasitic nematodes are present. However, there is an urgent need for safe and effective nematode controls. Factors relating to the disadvantages of current control strategies include heightened concern for the sustainability of agriculture, and new government regulations that may prevent or severely restrict the use of many available agricultural chemical antihelminthic agents.

Chemical agents are often not selective, and exert their effects on non-target organisms, effectively disrupting populations of beneficial microorganisms, for a period of time following application of the agent. Chemical agents may persist in the environment and only be slowly metabolized. Nematocidal soil fumigants such as chloropicrin and methyl bromide and related compounds are highly toxic, and methyl bromide has been identified as an ozone-depleting compound. Thus its registration for use in the United States is not being renewed. These agents may also accumulate in the water table or the food chain, and in higher trophic level species. These agents may also act as mutagens and/or carcinogens to cause irreversible and deleterious genetic modifications. Thus, alternative methods for nematode control, such as genetic methods, are increasingly being studied.

The organism *Caenorhabditis elegans,* a bacteriovorous nematode, is the most widely studied nematode genetic model. Public and private databases hold a wealth of information on its genetics and development, but practically applying this information for control of plant-parasitic nematodes remains a challenge (McCarter *et al.* 2003; McCarter 2004). It has previously been impractical to routinely identify a large number of target genes in nematodes other than *C. elegans,* such as plant-parasitic nematodes, for subsequent functional analysis *e.g.* by RNAi analysis. Therefore, there has existed a need for improved methods of identifying target genes, suppression of expression of which leads to control of nematode infestation.

Many genes in *C. elegans* have orthologs in metazoan animals including insects and vertebrates as well as other nematodes. In recent years, a greatly expanded expressed sequence tag (EST) collection has been generated from over 30 parasitic nematode species of plants and animals (Parkinson *et al.,* 2004). As of 2005 there were approximately 560,874 nucleotide sequences in Genbank from nematodes other than *Caenorhabditis* species and public projects are underway to generate draft sequences of *Meloidogyne hapla* (root knot nematode), *Haemonchus contortus* (parasite of sheep), *Trichinella spiralis* (parasite of humans and other mammals) (430,000 sequence traces submitted), and *Prislionchus pacificus* (free living nematode) (149,000 sequence traces submitted). 20,109 ESTs are available from *Heterodera glycines* representing portions of approximately 9,000 genes (see, *e.g.,* U.S. Patent Appl. Ser. No. 11/360,355, filed Feb 23, 2006). Conserved genes are expected to often retain the same or very similar functions in different nematodes. This functional equivalence has been demonstrated in some cases by transforming *C. elegans* with homologous genes from other nematodes (Kwa *et al.,* 1995; Redmond *et al.* 2001). Such equivalence has been shown in cross phyla comparisons for conserved genes and is expected to be more robust among species within a phylum.

RNA interference (RNAi) is a process utilizing endogenous cellular pathways whereby a double stranded RNA (dsRNA) specific target gene results in the degradation of the mRNA of interest. In recent years, RNAi has been used to perform gene "knockdown" in a number of species and experimental systems, from the nematode *C*. *elegans,* to plants, to insect embryos and cells in tissue culture (Fire *et al.,* 1998; Martinez *et al.,* 2002; McManus and Sharp, 2002). RNAi works through an endogenous pathway including the Dicer protein complex that generates ∼21-nucleotide small interfering RNAs (siRNAs) from the original dsRNA and the RNA-induced silencing complex (RISC) that uses siRNA guides to recognize and degrade the corresponding mRNAs. Only transcripts complementary to the siRNA are cleaved and degraded, and thus the knock-down of mRNA expression is usually sequence specific. The gene silencing effect of RNAi persists for days and, under experimental conditions, can lead to a decline in abundance of the targeted transcript of 90% or more, with consequent decline in levels of the corresponding protein.

dsRNA-mediated gene suppression by RNAi can be achieved in *C*. *elegans* by feeding, by soaking the nematodes in solutions containing double stranded or small interfering RNA molecules, and by injection of the dsRNA molecules (Kamath *et al.,* 2001; Maeda *et al.,* 2001. Several large-scale surveys of *C*. *elegans* genes by RNAi have been performed so that RNAi knockdown information is available for >90% of *C*. *elegans* genes *(Gonczy et al.,* 2000; Fraser *et al.,* 2000; Piano *et al.,* 2000; Maeda *et al.,* 2001; Kamath *et al.,* 2003; Simmer *et al.,* 2003; Ashrafi *et al.,* 2003; Sonnichsen *et al.,* 2005).

To date, only limited published technical or patent information exists on RNAi-mediated gene suppression in plant parasitic nematodes, wherein the double-stranded (dsRNA) or small interfering (siRNA) molecules are taken up from artificial growth media *(in vitro*) or from plant tissue *(in planta*). RNAi has been observed to function in several parasitic nematodes including the plant parasites *Heterodera glycines* and *Globodera pallida* (Urwin *et al.,* 2002; US Publications 2004/0098761; 2003/0150017; 2003/0061626 and 2004/0133943; Fairbaim *et al.* 2005), *Meloidogyne javanica* (W02005/019408), and the mammalian parasites *Nippostrongylus brasiliensis* (Hussein *et al.,* 2002), *Brugia malayi* (Aboobaker *et al.,* 2003), and *Onchocerca volvulus* (Lustigman *et al.,* 2004). Production of parasite-specific dsRNA in plant cells has been suggested as a direct strategy for control of plant parasitic nematodes including the soybean cyst nematode, *Heterodera glycines (e.g.* Fire et al., 1998; US Publications 2004/0098761 and 2005/0188438; WO 03/052110). US Publication 2006/0037101 describes use of *H. glycines* sequences, such as from *pas5,* to modulate SCN gene expression. However, no systematic method for identifying target nematode genes for use in such strategies has been reported, and only a limited number of plant-parasitic nematode genes have been proposed as potential targets for RNAi-mediated gene suppression studies.

### BRIEF DESCRIPTION OF THE FIGURES

FIG. 1: *C. elegans* RNAi Phenotype Ranking System.
FIG. 2: Results of *C*. *elegans* P0 RNAi feeding studies.
FIG. 3A-3D: Top 300 List of *H. glycines* Gene Targets Based on *C*. *elegans* orthologs.

### SUMMARY OF THE INVENTION

The present invention is directed toward compositions and methods for controlling diseases caused by plant-parasitic nematodes. The present invention provides exemplary nucleic acid compositions that are homologous to at least a portion of one or more native nucleic acid sequences in a target plant-parasitic nematode. In certain embodiments, the nematode is selected from *Heterodera sp., Meloidogyne sp., Globodera sp., Helicotylenchus sp., Ditylenchus sp., Pratylenchus sp., Paratylenchus sp., Rotylenchus sp., Tylenchulus sp., Tylenchorhynchus sp., Hoplolaimus sp., Belonolaimus sp., Anguina sp., Subanguina sp.* and *Nacobbus sp.* In particular, the nematode may be a *Heterodera sp.,* such as *H. glycines.* Specific examples of such nucleic acids provided by the invention are given in the attached sequence listing as SEQ ID NO:1920. Thus, in one aspect, the invention provides a polynucleotide comprising a first, a second and a third polynucleotide sequence, wherein the first polynucleotide sequence is selected from:
(a) a fragment of at least 21 contiguous nucleotides of a nucleic acid sequence of SEQ ID NO:1920, wherein uptake by a plant-parasitic nematode of a double stranded ribonucleotide sequence comprising at least one strand that is complementary to said fragment inhibits the growth of said nematode and
(b) a complement of the sequence of (a), wherein the third polynucleotide sequence is linked to the first polynucleotide sequence by the second polynucleotide sequence, and wherein the third polynucleotide sequence is substantially the reverse complement of the first polynucleotide sequence such that the first and the third polynucleotide sequences hybridize when transcribed into a ribonucleic acid to form a double stranded ribonucleotide molecule stabilized by the linked second ribonucleotide sequence,
wherein the polynucleotide is operably linked to a heterologous promoter. In yet another aspect, the invention provides this isolated polynucleotide further defined as comprised on a plant transformation vector. As used herein uptake by a plant-parasitic nematode includes ingestion of one or more sequences by the nematode, for example, by feeding. In specific non-limiting embodiments, uptake may be achieved by contacting a plant-parasitic nematode with a composition comprising one or more nucleic acid(s) according to the invention. For instance, uptake may also be achieved by soaking of plant-parasitic nematodes with a solution comprising the nucleic acid(s).

The invention is also directed to a double stranded ribonucleotide sequence produced from the expression of the above polynucleotide, wherein the taking up of said ribonucleotide sequence by a plant-parasitic nematode inhibits the growth of said nematode. The invention further provides a double stranded ribonucleotide sequence produced by preparing a recombinant polynucleotide sequence comprising a first, a second and a third polynucleotide sequence, wherein the first polynucleotide sequence comprises an isolated polynucleotide, uptake of which by a plant-parasitic nematode inhibits the growth, feeding, or development of said nematode, wherein the third polynucleotide sequence is linked to the first polynucleotide sequence by the second polynucleotide sequence, and wherein the third polynucleotide sequence is substantially the reverse complement of the first polynucleotide sequence such that the first and the third polynucleotide sequences hybridize when transcribed into a ribonucleic acid to form the double stranded ribonucleotide molecule stabilized by the linked second ribonucleotide sequence. Inhibition of nematode growth, feeding, or development may be accomplished by inhibiting expression of a nucleotide sequence in the plant-parasitic nematode that is substantially complementary to the sequence of the first polynucleotide.

The invention further provides a plant transformation vector comprising the above mentioned nucleotide sequence, wherein the sequence is operably linked to a heterologous promoter functional in a plant cell, and to cells transformed with the vector. The cells may be prokaryotic or eukaryotic. In particular, they may be plant cells. Plants and seeds derived from such transformed plant cells are also contemplated. The invention further provides a commodity product produced from such a plant, wherein said commodity product comprises a detectable amount of the above polynucleotide or a ribonucleotide expressed therefrom. Methods to produce such a commodity product are also contemplated, by obtaining such transformed plants and preparing food or feed from them. In particular, the food or feed is defined as oil, meal, protein, starch, flour or silage.

The invention also relates to methods for controlling a population of a plant-parasitic nematode, such as *H. glycines,* comprising providing an agent comprising a double stranded ribonucleotide sequence that functions upon being taken up by the nematode to inhibit a biological function within said nematode, wherein
(i) the agent comprises a fragment of at least 21 contiguous nucleotides of a nucleotide sequence of SEQ ID NO:1920 or complements thereof; or
(ii) said polynucleotide sequence exhibits from 95 to 100% nucleotide sequence identity along at least from 19 to 25 contiguous nucleotides to a coding sequence derived from said nematode and is hybridized to a second polynucleotide sequence that is complementary to said first polynucleotide sequence, and wherein said coding sequence derived from said nematode is SEQ ID NO:1920 or complements thereof.

The invention also relates to a method for controlling a plant-parasitic nematode population comprising providing in the host plant of a plant-parasitic nematode a transformed plant cell expressing the above polynucleotide sequence, wherein the polynucleotide is expressed to produce a double stranded ribonucleic acid that functions upon being taken up by the plant-parasitic nematode to inhibit the expression of a target sequence within said nematode and results in decreased growth of the nematode or nematode population, relative to growth on a host lacking the transformed plant cell.

The invention further provides a method for reducing the number of *Heterodera* feeding sites established in the root tissue of a host plant, comprising providing in the host plant of a *Heterodera sp.* a transformed plant cell expressing the above polynucleotide sequence, wherein the polynucleotide is expressed to produce a double stranded ribonucleic acid that functions upon being taken up by the *Heterodera sp.* to inhibit the expression of a target sequence within said nematode and results in a decrease in the number of feeding sites established, relative to growth on a host lacking the transformed plant cell.

The invention further relates to a method of controlling plant nematode pest infestation in a plant comprising providing in the diet of a plant nematode pest a dsRNA comprising:
a) a sense nucleotide sequence; and
b) an antisense nucleotide sequence complementary to said sense nucleotide sequence,
wherein said sense nucleotide sequence comprises the first nucleotide sequence as defined above.

The present invention also relates to a method for improving the yield of a crop produced from a crop plant subjected to plant-parasitic nematode infection or improving drought tolerance, said method comprising the steps of: a) introducing the above polynucleotide into said crop plant; b) cultivating the crop plant to allow the expression of said polynucleotide, wherein expression of said polynucleotide inhibits plant-parasitic nematode infection or growth and loss of yield due to plant-parasitic nematode infection or improves drought tolerance. In particular, the crop plant may be soybean *(Glycine max),* and the plant-parastic nematode is a Tylenchid nematode such as *H. glycines.*

The invention additionally provides a method for modulating the expression of a target gene in a plant-parasitic nematode cell, the method comprising: (a) transforming a plant cell with a vector comprising a nucleic acid sequence encoding a dsRNA of SEQ ID NO:1920, operatively linked to a promoter and a transcription termination sequence, wherein said dsRNA inhibits the function of the polypeptide having the sequence of SEQ ID NO:1920; (b) culturing the transformed plant cell under conditions sufficient to allow for development of a plant cell culture comprising a plurality of transformed plant cells; (b) culturing the transformed plant cell under conditions sufficient to allow for development of a plant cell culture comprising a plurality of transformed plant cells; (c) selecting for transformed plant cells that have integrated the nucleic acid sequence into their genomes; (d) screening the transformed plant cells for expression of the dsRNA encoded by the nucleic acid sequence; and (e) selecting a plant cell that expresses the dsRNA. Plants may also be regenerated from such plant cells. In particular, "modulating expression" may comprise inhibiting expression.

### DETAILED DESCRIPTION OF THE INVENTION

The following is a detailed description of the invention provided to aid those skilled in the art in practicing the present invention.

The present invention provides methods and compositions for genetic control of plant-parasitic nematode infestations. Methods for identifying genes essential to the lifecycle of a plant-parasitic nematode for use as a target for dsRNA-mediated control of a nematode population are also provided. DNA plasmid vectors encoding dsRNA molecules are designed to suppress nematode genes essential for growth and development. For example, the present invention provides methods and recombinant DNA technologies for post-transcriptionally repressing or inhibiting expression of a target coding sequence in a plant-parasitic nematode to provide a protective effect by allowing the plant-parasitic nematode to ingest one or more double stranded or small interfering ribonucleic acid (RNA) molecules transcribed from all or a portion of a target coding sequence, thereby controlling the infection. Therefore, the present invention relates to sequence-specific inhibition of expression of coding sequences using double-stranded RNA (dsRNA), including small interfering RNA (siRNA), to achieve the intended levels of nematode control.

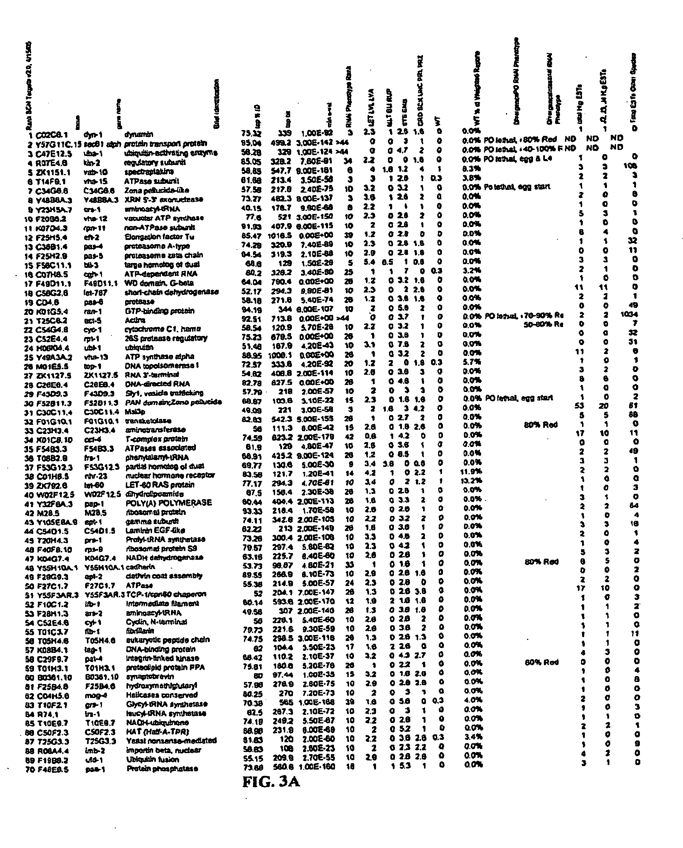

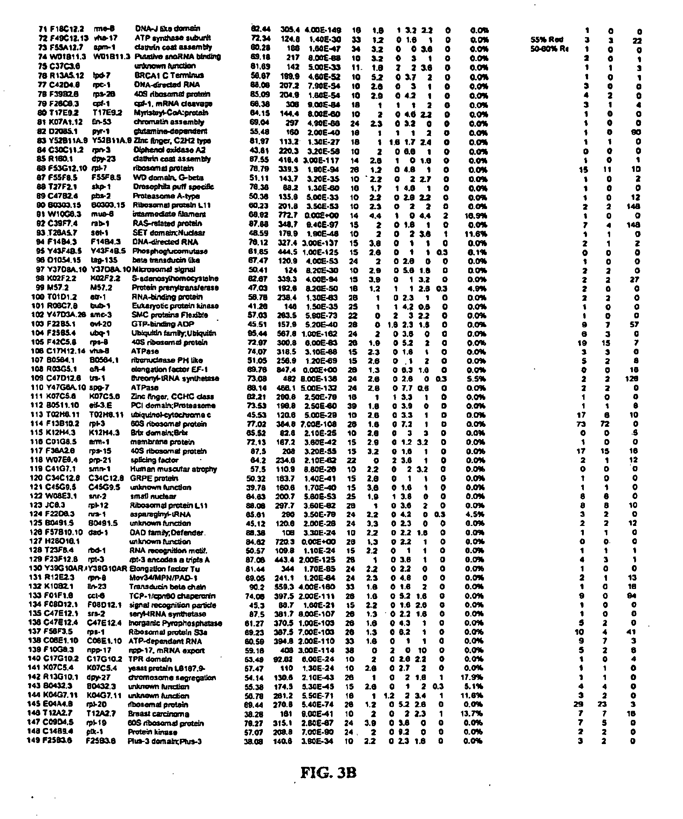

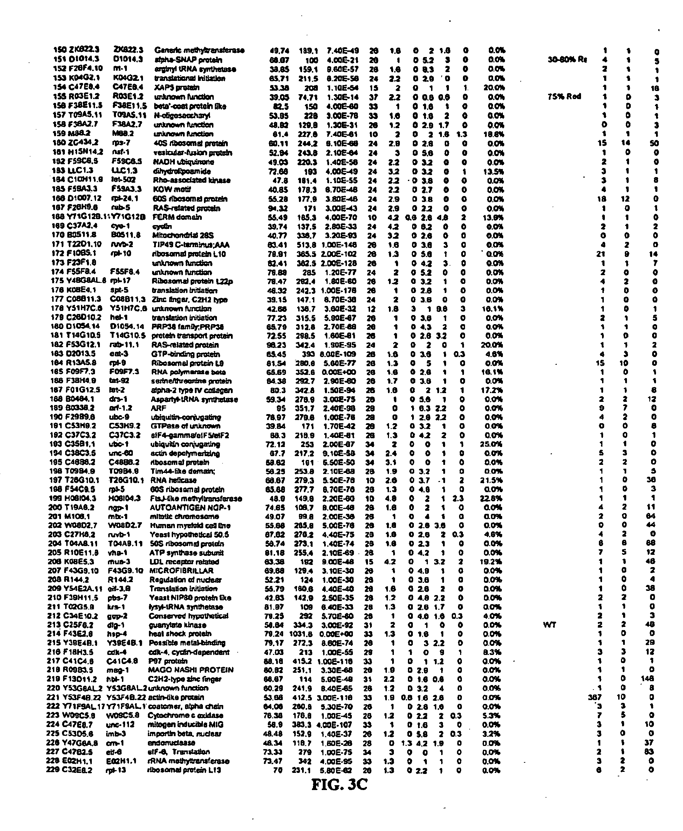

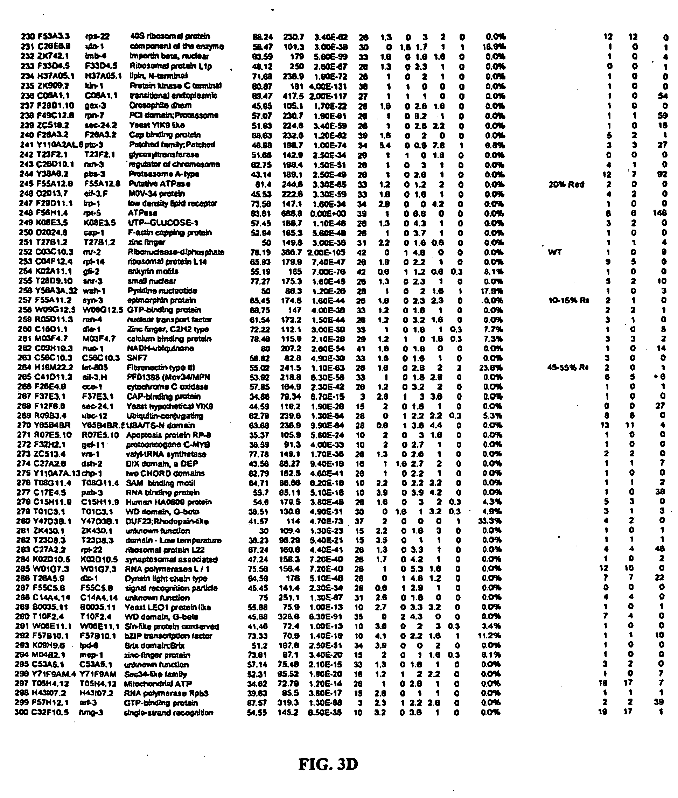

A method for inhibiting target gene function within the plant pathogen soybean cyst nematode, *Heterodera glycines,* is also provided by the present invention, and can be accomplished by RNA interference, resulting in disruption of the pathogen's lifecycle. Optimal target genes for disruption include life-cycle essential genes where disruption results in high penetrance death of the parasite populations or "genetic death" by blocking of reproduction with minimal feeding damage to the plant, reduction in number of established feeding sites, and minimal viable escaping worms reaching the next generation. Another aspect of the present invention provides the nucleic acids of each of the 300 target genes predicted to be essential to *H. glycines* growth and/or development (FIG 3). Features used to predict such targets include orthology to known *C. elegans* genes with strong and reproducible RNA interference phenotypes, and expression pattern in *H. glycines.*

In yet another aspect of the present invention, a set of isolated and purified nucleotide sequences as set forth in SEQ ID NO:1920 is provided. The present invention provides a stabilized dsRNA molecule for the expression of one or more RNAs for inhibition of expression of a target gene in a plant-parasitic nematode, expressed from these sequences and fragments thereof. A stabilized dsRNA, including a dsRNA or siRNA molecule can comprise at least two coding sequences that are arranged in a sense and an antisense orientation relative to at least one promoter, wherein the nucleotide sequence that comprises a sense strand and an antisense strand are linked or connected by a spacer sequence of at least from five to one thousand nucleotides, wherein the sense strand and the antisense strand may be a different length, and wherein each of the two coding sequences shares at least 80% sequence identity, at least 90%, at least 95%, at least 98%, or 100% sequence identity, to any one or more nucleotide sequence(s) set forth in SEQ ID NO:1920.

In yet another aspect, the invention provides recombinant DNA constructs comprising a nucleic acid molecule encoding a dsRNA molecule described herein. The dsRNA may be formed by transcription of one strand of the dsRNA molecule from a nucleotide sequence which is at least from 80% to 100% identical to a nucleotide sequence SEQ ID NO:1920. Such recombinant DNA constructs may be defined as producing dsRNA molecules capable of inhibiting the expression of endogenous target gene(s) in a plant-parasitic nematode cell upon ingestion. The construct may comprise a nucleotide sequence of the invention operably linked to a promoter sequence that functions in the host cell such as a plant cell. Such a promoter may be tissue-specific and may, for example, be specific to a tissue type which is the subject of plant-parasitic nematode attack. In the case of a root or foliar pathogen, respectively for example, it may be desired to use a promoter providing root or leaf-preferred expression, respectively.

Nucleic acid constructs in accordance with the invention may comprise at least one non-naturally occurring nucleotide sequence that can be transcribed into a single stranded RNA capable of forming a dsRNA molecule *in vivo* through intermolecular hybridization. Such dsRNA sequences self assemble and can be provided in the nutrition source of a plant-parasitic nematode to achieve the desired inhibition.

A recombinant DNA construct may comprise two different non-naturally occurring sequences which, when expressed *in vivo* as dsRNA sequences and provided in the tissues of the host plant of a plant-parasitic nematode, inhibit the expression of at least two different target genes in the plant-parasitic nematode. In certain embodiments, at least 2, 3, 4, 5, 6, 8 or 10 or more different dsRNAs are produced in a cell, or plant comprising the cell, that have a nematode-inhibitory effect. The dsRNAs may be expressed from multiple constructs introduced in different transformation events or could be introduced on a single nucleic acid molecule. The dsRNAs may be expressed using a single promoter or multiple promoters. In one embodiment of the invention, single dsRNAs are produced that comprise nucleic acids homologous to multiple loci within a plant-parasitic nematode.

In still yet another aspect, the invention provides a recombinant host cell having in its genome at least one recombinant DNA sequence that is transcribed to produce at least one dsRNA molecule that functions when ingested by a plant-parasitic nematode to inhibit the expression of a target gene in the nematode. The dsRNA molecule may be encoded by any of the nucleic acids described herein and as set forth in the sequence listing. The present invention also provides a transformed plant cell having in its genome at least one recombinant DNA sequence described herein. Transgenic plants comprising such a transformed plant cell are also provided, including progeny plants of any generation, seeds, and plant products, each comprising the recombinant DNA. The dsRNA molecules of the present invention may be found in the transgenic plant cell, for instance in the cytoplasm. They may also be found in an apoplastic space.

The invention also provides one or more stabilization sequences, or "clamps", which may be unrelated to the gene of interest. A clamp preferably comprises a GC rich region that serves to thermodynamically stabilize the dsRNA molecule, and may increase gene silencing.

Further provided by the invention is a fragment of a nucleic acid sequence SEQ ID NO:1920. The fragment may be defined as causing the death, growth inhibition, or cessation of infection or feeding by a plant-parasitic nematode, when expressed as a dsRNA and taken up by the nematode. The fragment may, for example, comprise at least 19, 21, 23, 25, 40, 60, 80, 100, 125 or more contiguous nucleotides of SEQ ID NO:1920, or a complement thereof. One beneficial DNA segment for use in the present invention is at least from 19 to 23, or 23 to 100 nucleotides, but less than 2000 nucleotides, in length. Particularly useful will be dsRNA sequences including 23 to 300 nucleotides homologous to a nematode target sequence. The invention also provides a ribonucleic acid expressed from any of such sequences including a dsRNA. A sequence selected for use in expression of a gene suppression agent can be constructed from a single sequence derived from one or more target plant-parasitic nematode species and intended for use in expression of an RNA that functions in the suppression of a single gene or gene family in the one or more target pathogens, or that the DNA sequence can be constructed as a chimera from a plurality of DNA sequences.

In another embodiment, the invention provides a method for modulating expression of a target gene in a nematode cell, the method comprising: (a) transforming a plant cell with a vector comprising a nucleic acid sequence encoding a dsRNA operatively linked to a promoter and a transcription termination sequence; (b) culturing the transformed plant cell under conditions sufficient to allow for development of a plant cell culture comprising a plurality of transformed plant cells; (c) selecting for transformed plant cells that have integrated the vector into their genomes; (d) screening the transformed plant cells for expression of the dsRNA encoded by the vector; (e) selecting a plant cell that expresses the dsRNA; (f) optionally regenerating a plant from the plant cell that expresses the dsRNA; whereby expression of the gene in the plant is sufficient to modulate the expression of a target gene in a cell of a plant parasitic nematode that contacts the transformed plant or plant cell. Modulation of gene expression may include partial or complete suppression of such expression.

In yet another aspect, the invention provides a method for suppression of gene expression in a plant-parasitic nematode, comprising the provision in the tissue of the host of the nematode a gene-suppressive amount of at least one dsRNA molecule transcribed from a nucleotide sequence as described herein, at least one segment of which is complementary to an mRNA sequence within the cells of the plant-parasitic nematode. The method may further comprise observing the death or growth inhibition of the plant-parasitic nematode, and the degree of host symptomatology. A dsRNA molecule, including its modified form such as an siRNA molecule, ingested by a pathogenic microorganism in accordance with the invention may be at least from 80, 81, 82, 83, 84, 85, 86, 87, 88 89, 90, 91, 92, 93, 94, 95, 96, 97, 98, 99, or 100% identical to a RNA molecule transcribed from a nucleotide sequence SEQ ID NO:1920.

Isolated and substantially purified nucleic acid molecules including, but not limited to, non-naturally occurring nucleotide sequences and recombinant DNA constructs for transcribing dsRNA molecules of the present invention are therefore provided, which suppress or inhibit the expression of an endogenous coding sequence or a target coding sequence in the plant-parasitic nematode when introduced thereto. Transgenic plants that (a) contain nucleotide sequences encoding the isolated and substantially purified nucleic acid molecules and the non-naturally occurring recombinant DNA constructs for transcribing the dsRNA molecules for controlling plant-parasitic nematode infections, and (b) display resistance and/or enhanced tolerance to the infections, are also contemplated. Compositions containing the dsRNA nucleotide sequences of the present invention for use in topical applications onto plants or onto animals or into the environment of an animal to achieve the elimination or reduction of plant-parasitic nematode infection are also included.

cDNA sequences encoding proteins or parts of proteins essential for survival, such as amino acid sequences involved in various metabolic or catabolic biochemical pathways, cell division, reproduction, energy metabolism and digestion may be selected for use in preparing double stranded RNA molecules to be provided in the host plant of a plant-parasitic nematode. As described herein, ingestion of compositions by a target organism containing one or more dsRNAs, at least one segment of which corresponds to at least a substantially identical segment of RNA produced in the cells of the target pathogen, can result in the death or other inhibition of the target. These results indicate that a nucleotide sequence, either DNA or RNA, derived from a plant-parasitic nematode can be used to construct plant cells resistant to infestation by the nematode. The host plant of the nematode, for example, can be transformed to contain one or more of the nucleotide sequences derived from the nematode as provided herein. The nucleotide sequence transformed into the host may encode one or more RNAs that form into a dsRNA sequence in the cells or biological fluids within the transformed host, thus making the dsRNA available if/when the plant-parasitic nematode forms a nutritional relationship with the transgenic host. This may result in the suppression of expression of one or more genes in the cells of the plant-parasitic nematode and ultimately death or inhibition of its growth or development.

The present invention relates generally to genetic control of plant-parasitic nematodes in host organisms. More particularly, the present invention includes methods for delivery of nematode control agents to plant-parasitic nematodes. Such control agents cause, directly or indirectly, an impairment in the ability of the plant-parasitic nematode to feed, grow or otherwise cause disease in a target host. The present invention provides in one embodiment a method comprising delivery of stabilized dsRNA molecules to plant-parasitic nematodes as a means for suppression of targeted genes in the plant-parasitic nematode, thus achieving desired control of plant disease in the nematode host.

In accomplishing the foregoing, the present invention provides a method of inhibiting expression of a target gene in a plant-parasitic nematode, resulting in the cessation of growth, development, reproduction, and/or feeding, and eventually may result in the death of the plant-parasitic nematode. The method comprises in one embodiment introducing partial or fully stabilized double-stranded RNA (dsRNA) nucleotide molecules, including its modified forms such as small interfereing RNA (siRNA) sequences, into a nutritional composition for the plant-parasitic nematode, and making the nutritional composition or food source available to the plant-parasitic nematode. Ingestion of the nutritional composition containing the double stranded or siRNA molecules results in the uptake of the molecules by the cells of the nematode, resulting in the inhibition of expression of at least one target gene in the cells of the nematode. Inhibition of the target gene exerts a deleterious effect upon the nematode. The methods and associated compositions may be used for limiting or eliminating infection or parasitization of a plant or plant cell by a nematode, in or on any host tissue or environment in which a the nematode is present by providing one or more compositions comprising the dsRNA molecules described herein in the host of the nematode.

In certain embodiments, dsRNA molecules provided by the invention comprise nucleotide sequences complementary to a sequence as set forth in SEQ ID NO:1920, the inhibition of which in a plant-parasitic nematode results in the reduction or removal of a protein or nucleotide sequence agent that is essential for the nematode's growth and development or other biological function. The nucleotide sequence selected may exhibit from 80% to 100% sequence identity to the nucleotide sequences of SEQ ID NO:1920, including the complement thereof. Such inhibition can be described as specific in that a nucleotide sequence from a portion of the target gene is chosen from which the inhibitory dsRNA or siRNA is transcribed. The method is effective in inhibiting the expression of at least one target gene and can be used to inhibit many different types of target genes in the plant-parasitic nematode.

The sequences identified as having a nematode-protective effect may be readily expressed as dsRNA molecules through the creation of appropriate expression constructs. For example, such sequences can be expressed as a hairpin and stem and loop structure by taking a first segment corresponding to a sequence of SEQ ID NO:1920, or a fragment thereof, linking this sequence to a second segment spacer region that is not homologous or complementary to the first segment, and linking this to a third segment that transcribes an RNA, wherein at least a portion of the third segment is substantially complementary to the first segment. Such a construct forms a stem and loop structure by hybridization of the first segment with the third segment and a loop structure forms comprising the second segment (WO94/01550, WO98/05770, US 2002/0048814, and US 2003/0018993. dsRNA may be generated for instance in the form of a double stranded structure such as a stem loop structure (*e.g*. hairpin), whereby production of siRNA targeted for a nematode sequence is enhanced by co-expression of a fragment of the targeted gene, for instance on an additional plant expressible cassette, that leads to enhanced siRNA production, or reduces methylation to prevent transcriptional gene silencing of the dsRNA hairpin promoter (*e.g.* WO05/019408).

The methods and compositions of the present invention may be applied to any monocot and dicot plant, depending on the pathogen (*e.g.* nematode) control desired. Examples of such plants include, without limitation, alfalfa, artichoke, asparagus, barley, beans, beet, broccoli, cabbage, canola, carrot, cassava, cauliflower, corn, cotton, cucumber, grape, oat, onion, pea, peanut, potato, rice, rye, sorghum, soybean, spinach, squash, sugarbeet, sugarcane, sunflower, tobacco, tomato, turfgrass, and wheat plants.

Exemplary plant-parasitic nematodes from which plants may be protected by the present invention, and their corresponding plants, include, but are not limited to: alfalfa: *Ditylenchus dipsaci, Meloidogyne hapla, Meloidogyne incognita, Meloidogyne javanica, Pratylenchus spp., Paratylenchus spp., Xiphinema spp.;* banana: *Radopholus similis, Helicotylenchus multicinctus, M. incognita, M. arenaria, M. javanica, Pratylenchus coffeae, Rotylenchulus reniformis;* beans and peas: *Meloidogyne spp., Heterodera spp., Belonolaimus spp., Helicotylenchus spp., Rotylenchulus reniformis, Paratrichodorus anemones, Trichodorus spp.;* cassava: *Rotylenchulus reniformis, Meloidogyne spp.;* cereals: *Anguina tritici* (Emmer, rye, spelt wheat), *Bidera avenae* (oat, wheat), *Ditylenchus dipsaci* (rye, oat), *Subanguina radicicola* (oat, barley, wheat, rye), *Meloidogyne naasi* (barley, wheat, rye), *Pratylenchus spp.* (oat, wheat, barley, rye), *Paratylenchus spp.* (wheat), *Tylenchorhynchus spp.* (wheat, oat); chickpea: *Heterodera cajani, Rotylenchulus reniformis, Hoplolaimus seinhorsti, Meloidogyne spp., Pratylenchus spp.;* citrus: *Tylenchulus semipenetrans, Radopholus similis, Radopholus citrophilus, Hemicycliophora arenaria Pratylenchus spp., Meloidogyne spp., Bolonolaimus longicaudatus, Trichodorus spp., Paratrichodorus spp., Xiphinema spp.;* clover: *Meloidogyne spp., Heterodera trifolii;* corn: *Pratylenchus spp., Paratrichodorus minor, Longidorus spp., Hoplolaimus columbus;* cotton: *Meloidogyne incognita, Belonolaimus longicaudatus, Rotylenchulus reniformis, Hoplolaimus galeatus, Pratylenchus spp., Tylenchorhynchus spp., Paratrichodorus minor;* grapes: *Xiphinema spp., Pratylenchus vulnus, Meloidogyne spp., Tylenchulus semipenetrans, Rotylenchulus reniformis;* grasses: *Pratylenchus spp., Longidorus spp., Paratrichodorus christiei, Xiphinema spp., Ditylenchus spp.;* peanut: *Pratylenchus spp., Meloidogyne hapla., Meloidogyne arenaria, Criconemella spp., Belonolaimus longicaudatus;* pigeon pea: *Heterodera cajani, Rotylenchulus reniformis, Hoplolaimus seinhorsti, Meloidogyne spp., Pratylenchus spp.;* potato: *Globodera rostochiensis, Globodera pallida, Meloidogyne spp., Pratylenchus spp., Trichodorus primitivus, Ditylenchus spp., Paratrichodorus spp., Nacobbus aberrans;* rice: *Aphelenchiodes besseyi, Ditylenchus angustus, Hirchmanniella spp., Heterodera oryzae, Meloidogyne spp.;* small fruits: *Meloidogyne spp.; Pratylenchus spp., Xiphinema spp., Longidorus spp., Paratrichodorus christiei, Aphelenchoides spp.;* soybean: *Heterodera glycines, Meloidagyne incognita, Meloidogyne javanica, Belonolaimus spp., Hoplolaimus columbus;* sugar beet: *Heterodera schachtii, Ditylenchus dipsaci, Meloidogyne spp., Nacobbus aberrans, Trichodorus spp., Longidorus spp., Paratrichodorus spp.;* sugar cane: *Meloidogyne spp., Pratylenchus spp., Radopholus spp., Heterodera spp., Hoplolaimus spp., Helicotylenchus spp., Scutellonema spp., Belonolaimus spp., Tylenchorhynchus spp., Xiphinema spp., Longidorus spp., Paratrichodorus spp.;* tobacco: *Meloidogyne spp., Pratylenchus spp., Tylenchorhynchus claytoni, Globodera tabacum, Trichodorus spp., Xiphinema americanum, Ditylenchus dipsaci, Paratrichodorus spp.;* and tomato: *Pratylenchus spp., Meloidogyne spp.*

The invention also provides combinations of methods and compositions for controlling infection by plant-parasitic nematodes. One means provides a dsRNA method as described herein for protecting plants from plant-parasitic nematodes along with one or more chemical agents that exhibit features different from those exhibited by the dsRNA methods and compositions, and can interfere with nematode growth or development.

### A. Nucleic Acid Compositions and Constructs

The invention provides recombinant DNA constructs for use in achieving stable transformation of particular host targets. Transformed host targets may express effective levels of preferred dsRNA or siRNA molecules from the recombinant DNA constructs. Pairs of isolated and purified nucleotide sequences may be provided from cDNA library and/or genomic library information. The pairs of nucleotide sequences may be derived from any nematode for use as thermal amplification primers to generate the dsRNA and siRNA molecules of the present invention.

As used herein, the term "nucleic acid" refers to a single or double-stranded polymer of deoxyribonucleotide or ribonucleotide bases read from the 5' to the 3' end. The "nucleic acid" may also optionally contain non-naturally occurring or altered nucleotide bases that permit correct read through by a polymerase and do not reduce expression of a polypeptide encoded by that nucleic acid. The term "nucleotide sequence" or "nucleic acid sequence" refers to both the sense and antisense strands of a nucleic acid as either individual single strands or in the duplex. The term "ribonucleic acid" (RNA) is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA) and the term "deoxyribonucleic acid" (DNA) is inclusive of cDNA and genomic DNA and DNA-RNA hybrids. The words "nucleic acid segment", "nucleotide sequence segment", or more generally "segment" will be understood by those in the art as a functional term that includes both genomic sequences, ribosomal RNA sequences, transfer RNA sequences, messenger RNA sequences, operon sequences and smaller engineered nucleotide sequences that express or may be adapted to express, proteins, polypeptides or peptides.

Provided according to the invention are nucleotide sequences, the expression of which results in an RNA sequence which is substantially homologous to all or part of an RNA molecule of a targeted gene in a plant-parasitic nematode that comprises an RNA sequence encoded by a nucleotide sequence within the genome of the nematode. Thus, after ingestion of the stabilized RNA sequence down-regulation of the nucleotide sequence of the target gene in the cells of the plant-parasitic nematode may be obtained resulting in a deleterious effect on the growth, viability, proliferation, or reproduction of the nematode.

As used herein, the term "substantially homologous" or "substantial homology", with reference to a nucleic acid sequence, includes a nucleotide sequence that hybridizes under stringent conditions to the coding sequence of SEQ ID NO:1920, as set forth in the sequence listing, or the complements thereof. Sequences that hybridize under stringent conditions or the complements thereof, are those that allow an antiparallel alignment to take place between the two sequences, and the two sequences are then able, under stringent conditions, to form hydrogen bonds with corresponding bases on the opposite strand to form a duplex molecule that is sufficiently stable under the stringent conditions to be detectable using methods well known in the art. Substantially homologous sequences have preferably from 70% to 80% sequence identity, or more preferably from 80% to 85% sequence identity, or most preferable from 90% to 95% sequence identity, to 99% sequence identity, to the referent nucleotide sequences as set forth in SEQ ID NO:1920, in the sequence listing, or the complements thereof.

As used herein, the term "ortholog" refers to a gene in two or more species that has evolved from a common ancestral nucleotide sequence, and may retain the same function in the two or more species.

As used herein, the term "sequence identity", "sequence similarity" or "homology" is used to describe sequence relationships between two or more nucleotide sequences. The percentage of "sequence identity" between two sequences is determined by comparing two optimally aligned sequences over a comparison window, wherein the portion of the sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity. A sequence that is identical at every position in comparison to a reference sequence is said to be identical to the reference sequence and vice-versa. A first nucleotide sequence when observed in the 5' to 3' direction is said to be a "complement" of, or complementary to, a second or reference nucleotide sequence observed in the 3' to 5' direction if the first nucleotide sequence exhibits complete complementarity with the second or reference sequence. As used herein, nucleic acid sequence molecules are said to exhibit "complete complementarity" when every nucleotide of one of the sequences read 5' to 3' is complementary to every nucleotide of the other sequence when read 3' to 5'. A nucleotide sequence that is complementary to a reference nucleotide sequence will exhibit a sequence identical to the reverse complement sequence of the reference nucleotide sequence. These terms and descriptions are well defined in the art and are easily understood by those of ordinary skill in the art.

As used herein, a "comparison window" refers to a conceptual segment of at least 6 contiguous positions, usually 50 to 100, more usually 100 to 150, in which a sequence is compared to a reference sequence of the same number of contiguous positions after the two sequences are optimally aligned. The comparison window may comprise additions or deletions (*i.e.* gaps) of 20% or less as compared to the reference sequence (which does not comprise additions or deletions) for optimal alignment of the two sequences Those skilled in the art should refer to the detailed methods used for sequence alignment in the Wisconsin Genetics Software Package Release 7.0, Genetics Computer Group, 575 Science Drive Madison, Wis., USA) or refer to Ausubel *et al.* (1998) for a detailed discussion of sequence analysis.

The present invention provides DNA sequences capable of being expressed as an RNA in a cell or microorganism to inhibit target gene expression in a cell, tissue or organ of a plant-parasitic nematode. The sequences comprise a DNA molecule coding for one or more different nucleotide sequences, wherein each of the different nucleotide sequences comprises a sense nucleotide sequence and an antisense nucleotide sequence. The sequences may be connected by a spacer sequence coding for a dsRNA molecule of the present invention. The spacer sequence can constitute part of the sense nucleotide sequence or the antisense nucleotide sequence and forms within the dsRNA molecule between the sense and antisense sequences. The sense nucleotide sequence or the antisense nucleotide sequence is substantially identical to the nucleotide sequence of the target gene or a derivative thereof or a complementary sequence thereto. The dsDNA molecule may be placed operably under the control of a promoter sequence that functions in the cell, tissue or organ of the host expressing the dsDNA to produce dsRNA molecules. In one embodiment, the DNA sequence may be derived from a nucleotide sequence as set forth in SEQ ID NO: 1920, in the sequence listing.

The invention also provides a DNA sequence for expression in a cell of a plant that, upon expression of the DNA to RNA and ingestion by a plant-parasitic nematode achieves suppression of a target gene in a cell, tissue or organ of a plant-parasitic nematode. The dsRNA may comprise one or multiple structural gene sequences, wherein each of the structural gene sequences comprises a sense nucleotide sequence and an antisense nucleotide sequence that may be connected by a spacer sequence that forms a loop within the complementary and antisense sequences. The sense nucleotide sequence or the antisense nucleotide sequence is substantially identical to the nucleotide sequence of the target gene, derivative thereof, or sequence complementary thereto. The one or more structural gene sequences may be placed operably under the control of one or more promoter sequences, at least one of which is operable in the cell, tissue or organ of a prokaryotic or eukaryotic organism, particularly a plant cell. Methods to express a gene suppression molecule in plants are known (*e.g.* WO06073727; US Publication 2006/0200878), and may be used to express a nucleotide sequence of the present invention.

A gene sequence or fragment for plant-parasitic nematode control according to the invention may be cloned between two tissue specific promoters, such as two root specific promoters which are operable in a transgenic plant cell and therein expressed to produce mRNA in the transgenic plant cell that form dsRNA molecules thereto. Examples of root specific promoters are known in the art (*e.g*. the nematode-induced RB7 promoter; U.S. Patent 5,459,252; Opperman et al. 1994). The dsRNA molecules contained in plant tissues are ingested by a plant-parasitic nematode so that the intended suppression of the target gene expression is achieved.

The cauliflower mosaic virus 35S promoter, an archetypal strong promoter common in transgenic plant applications, or a related promoter such as the E35S or the FMV promoter, may be employed for driving nematode resistance genes, particularly for cyst nematodes (see Example 8). Promoters have also been identified that direct gene expression at nematode-induced feeding structures within a plant (*e.g*. Gheysen and Fenoll, 2002). Thus, a promoter identified from among genes that are reproducibly expressed in feeding sites may be utilized. Examples of genes up-regulated in feeding sites (syncytia) formed by nematodes include Hs1pro-1 (Thurau *et al.* 2003), AtSUC2 normally expressed in companion cells (Juergensen *et al.* 2003), At17.1 expressed in vascular tissues and root tips (Mazarei *et al.* 2004), FGAM synthase (phosphoribosylformyl-glycinamidine synthase) (Vaghchhipawala *et al.* 2004), and ABI3 (De Meutter *et al.* 2005), among others. Syncytia formed in response to cyst nematodes have been described as symplastically isolated lacking plasmodesmata to surrounding tissues (Bockenhoff and Grundler 1994; Bockenhoff *et al.* 1996), however, it has been shown that macromolecules up to 30 kD can move from phloem companion cells into the syncytium (Hoth *et al.* 2005). Therefore, gene expression in the phloem may also be suited for delivery of effector molecules into feeding sites.

A nucleotide sequence provided by the present invention may comprise an inverted repeat separated by a "spacer sequence." The spacer sequence may be a region comprising any sequence of nucleotides that facilitates secondary structure formation between each repeat, where this is required. In one embodiment of the present invention, the spacer sequence is part of the sense or antisense coding sequence for mRNA. The spacer sequence may alternatively comprise any combination of nucleotides or homologues thereof that are capable of being linked covalently to a nucleic acid molecule. The spacer sequence may comprise, for example, a sequence of nucleotides of at least 10-100 nucleotides in length, or alternatively at least 100-200 nucleotides in length, at least 200-400 nucleotides in length, or at least 400-500 nucleotides in length.

The nucleic acid molecules or fragments of the nucleic acid molecules or other nucleic acid molecules in the sequence listing are capable of specifically hybridizing to other nucleic acid molecules under certain circumstances. As used herein, two nucleic acid molecules are said to be capable of specifically hybridizing to one another if the two molecules are capable of forming an anti-parallel, double-stranded nucleic acid structure. A nucleic acid molecule is said to be the complement of another nucleic acid molecule if they exhibit complete complementarity. Two molecules are said to be "minimally complementary" if they can hybridize to one another with sufficient stability to permit them to remain annealed to one another under at least conventional "low-stringency" conditions. Similarly, the molecules are said to be complementary if they can hybridize to one another with sufficient stability to permit them to remain annealed to one another under conventional "high-stringency" conditions. Conventional stringency conditions are described by Sambrook, *et al.* (1989), and by Haymes *et al.* (1985).

Departures from complete complementarity are therefore permissible, as long as such departures do not completely preclude the capacity of the molecules to form a double-stranded structure. Thus, in order for a nucleic acid molecule or a fragment of the nucleic acid molecule to serve as a primer or probe it needs only be sufficiently complementary in sequence to be able to form a stable double-stranded structure under the particular solvent and salt concentrations employed.

Appropriate stringency conditions which promote DNA hybridization are, for example, 6.0 x sodium chloride/sodium citrate (SSC) at 45°C, followed by a wash of 2.0 x SSC at 50°C, are known to those skilled in the art or can be found in Current Protocols in Molecular Biology (1989). For example, the salt concentration in the wash step can be selected from a low stringency of 2.0 x SSC at 50°C to a high stringency of 0.2 x SSC at 50°C. In addition, the temperature in the wash step can be increased from low stringency conditions at room temperature, 22°C, to high stringency conditions at 65°C. Both temperature and salt may be varied, or either the temperature or the salt concentration may be held constant while the other variable is changed. A nucleic acid for use in the present invention may specifically hybridize to one or more of nucleic acid molecules from a nematode or complements thereof under such conditions. Preferably, a nucleic acid for use in the present invention will exhibit at least from 80%, or at least from 90%, or at least from 95%, or at least from 98% or even 100% sequence identity with one or more nucleic acid molecules as set forth in SEQ ID NO:1920, in the sequence listing.

Nucleic acids of the present invention may also be synthesized, either completely or in part, especially where it is desirable to provide plant-preferred sequences, by methods known in the art. Thus, all or a portion of the nucleic acids of the present invention may be synthesized using codons preferred by a selected host. Species-preferred codons may be determined, for example, from the codons used most frequently in the proteins expressed in a particular host species. Other modifications of the nucleotide sequences may result in mutants having slightly altered activity. dsRNA or siRNA nucleotide sequences comprise double strands of polymerized ribonucleotide and may include modifications to either the phosphate-sugar backbone or the nucleoside. Modifications in RNA structure may be tailored to allow specific genetic inhibition. In one embodiment, the dsRNA molecules may be modified through an enzymatic process so that siRNA molecules may be generated. The siRNA can effciently mediate the down-regulation effect for some target genes in some pathogens. This enzymatic process may be accomplished by utilizing an RNAse III enzyme or a DICER enzyme, present in the cells of an insect, a vertebrate animal, a fungus or a plant in the eukaryotic RNAi pathway (Elbashir *et al.,* 2001; Hamilton and Baulcombe, 1999). This process may also utilize a recombinant DICER or RNAse III introduced into the cells of a target insect through recombinant DNA techniques that are readily known to the skilled in the art. Both the DICER enzyme and RNAse III, being naturally occurring in a pathogen or being made through recombinant DNA techniques, cleave larger dsRNA strands into smaller oligonucleotides. The DICER enzymes specifically cut the dsRNA molecules into siRNA pieces each of which is 19-25 nucleotides in length while the RNAse III enzymes normally cleave the dsRNA molecules into 12-15 base-pair siRNA. The siRNA molecules produced by either of the enzymes have 2 to 3 nucleotide 3' overhangs, and 5' phosphate and 3' hydroxyl termini. The siRNA molecules generated by RNAse III enzyme are the same as those produced by Dicer enzymes in the eukaryotic RNAi pathway and are hence then targeted and degraded by an inherent cellular RNA-degrading mechanism after they are subsequently unwound, separated into single-stranded RNA and hybridize with the RNA sequences transcribed by the target gene. This process results in the effective degradation or removal of the RNA sequence encoded by the nucleotide sequence of the target gene in the pathogen. The outcome is the silencing of a particularly targeted nucleotide sequence within the pathogen. Detailed descriptions of enzymatic processes can be found in Hannon (2002).

A nucleotide sequence of the present invention can be recorded on computer readable media. As used herein, "computer readable media" refers to any tangible medium of expression that can be read and accessed directly by a computer. Such media include, but are not limited to: magnetic storage media, such as floppy discs, hard disc, storage medium, and magnetic tape: optical storage media such as CD-ROM; electrical storage media such as RAM and ROM; optical character recognition formatted computer files, and hybrids of these categories such as magnetic/optical storage media. A skilled artisan can readily appreciate that any of the presently known computer readable mediums can be used to create a manufacture comprising computer readable medium having recorded thereon a nucleotide sequence of the present invention.

As used herein, "recorded" refers to a process for storing information on computer readable medium. A skilled artisan can readily adopt any of the presently known methods for recording information on computer readable medium to generate media comprising the nucleotide sequence information of the present invention. A variety of data storage structures are available to a skilled artisan for creating a computer readable medium having recorded thereon a nucleotide sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII text file, stored in a database application, such as DB2, Sybase or Oracle. The skilled artisan can readily adapt any number of data processor structuring formats (*e.g*. text file or database) in order to obtain computer readable medium having recorded thereon the nucleotide sequence information of the present invention.

Computer software is publicly available which allows a skilled artisan to access sequence information provided in a computer readable medium. Software that implements the BLAST (Altschul *et al..* 1990) and BLAZE (Brutlag, *et al.,* 1993) search algorithms on a Sybase system can be used to identify open reading frames (ORFs) within sequences such as the Unigenes and EST's that are provided herein and that contain homology to ORFs or proteins from other organisms. Such ORFs are protein-encoding fragments within the sequences of the present invention and are useful in producing commercially important proteins such as enzymes used in amino acid biosynthesis, metabolism, transcription, translation, RNA processing, nucleic acid and a protein degradation, protein modification, and DNA replication, restriction, modification, recombination, and repair.

The present invention further provides systems, particularly computer-based systems, which contain the sequence information described herein. Such systems are designed to identify commercially important fragments of the nucleic acid molecule of the present invention. As used herein, "a computer-based system" refers to the hardware means, software means, and data storage means used to analyze the nucleotide sequence information of the present invention. The minimum hardware means of the computer-based systems of the present invention comprises a central processing unit (CPU), input means, output means, and data storage means. A skilled artisan can readily appreciate that any one of the currently available computer-based system are suitable for use in the present invention.

As used herein, "a target structural motif," or "target motif," refers to any rationally selected sequence or combination of sequences in which the sequences or sequence(s) are chosen based on a three-dimensional configuration that is formed upon the folding of the target motif. There are a variety of target motifs known in the art. Protein target motifs include, but are not limited to, enzymatic active sites and signal sequences. Nucleic acid target motifs include, but are not limited to, promoter sequences, cis elements, hairpin structures and inducible expression elements (protein binding sequences).

### B. Recombinant Vectors and Host Cell Transformation

A recombinant DNA vector may, for example, be a linear or a closed circular plasmid. The vector system may be a single vector or plasmid or two or more vectors or plasmids that together contain the total DNA to be introduced into the genome of the bacterial host. In addition, a bacterial vector may be an expression vector. Nucleic acid molecules as set forth in SEQ ID NO:1920, or fragments thereof, can, for example, be suitably inserted into a vector under the control of a suitable promoter that functions in one or more microbial hosts to drive expression of a linked coding sequence or other DNA sequence. Many vectors are available for this purpose, and selection of the appropriate vector will depend mainly on the size of the nucleic acid to be inserted into the vector and the particular host cell to be transformed with the vector. Each vector contains various components depending on its function (amplification of DNA or expression of DNA) and the particular host cell with which it is compatible. The vector components for bacterial transformation generally include one or more of the following: a signal sequence, an origin of replication, one or more selectable marker genes, and an inducible promoter allowing the expression of exogenous DNA.

Expression and cloning vectors generally contain a selection gene, also referred to as a selectable marker. This gene encodes a protein necessary for the survival or growth of transformed host cells grown in a selective culture medium. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, e.g., the gene encoding D-alanine racemase for Bacilli. Those cells that are successfully transformed with a heterologous protein or fragment thereof produce a protein conferring drug resistance and thus survive the selection regimen.

An expression vector for producing a mRNA can also contain an inducible promoter that is recognized by a host bacterial organism and is operably linked to the nucleic acid. Inducible promoters suitable for use with bacterial hosts include β-lactamase promoter, E *coli* λ phage PL and PR promoters, and *E. coli* galactose promoter, arabinose promoter, alkaline phosphatase promoter, tryptophan (trp) promoter, and the lactose operon promoter and variations thereof and hybrid promoters such as the tac promoter. However, other known bacterial inducible promoters are suitable.

The term "operably linked", as used in reference to a regulatory sequence and a structural nucleotide sequence, means that the regulatory sequence causes regulated expression of the linked structural nucleotide sequence. "Regulatory sequences" or "control elements" refer to nucleotide sequences located upstream (5' noncoding sequences), within, or downstream (3' non-translated sequences) of a structural nucleotide sequence, and which influence the timing and level or amount of transcription, RNA processing or stability, or translation of the associated structural nucleotide sequence. Regulatory sequences may include promoters, translation leader sequences, introns, enhancers, stem-loop structures, repressor binding sequences, and polyadenylation recognition sequences.

Construction of suitable vectors containing one or more of the above-listed components employs standard recombinant DNA techniques. Isolated plasmids or DNA fragments are cleaved, tailored, and re-ligated in the form desired to generate the plasmids required. Examples of available bacterial expression vectors include the multifunctional E. *coli* cloning and expression vectors such as Bluescript™ (Stratagene, La Jolla, CA), in which, for example, a nucleic acid, or fragment thereof, shown in FIG 3 may be ligated into the vector in frame with sequences for the amino-terminal Met and the subsequent 7 residues of β-galactosidase so that a hybrid protein is produced; and pIN vectors (Van Heeke and Schuster, 1989).

The present invention also contemplates transformation of a nucleotide sequence of the present invention into a plant to achieve nematode-inhibitory levels of expression of one or more dsRNA molecules. A transformation vector can be readily prepared using methods available in the art. The transformation vector comprises one or more nucleotide sequences that is/are capable of being transcribed to an RNA molecule and that is/are substantially homologous and/or complementary to one or more nucleotide sequences encoded by the genome of the target nematode, such that upon uptake of the RNA transcribed from the one or more nucleotide sequences by the target plant-parasitic nematode, there is down-regulation of expression of at least one of the respective nucleotide sequences of the genome of the nematode.

The transformation vector may be termed a dsDNA construct and may also be defined as a recombinant molecule, a disease control agent, a genetic molecule or a chimeric genetic construct. A chimeric genetic construct of the present invention may comprise, for example, nucleotide sequences encoding one or more antisense transcripts, one or more sense transcripts, one or more of each of the aforementioned, wherein all or part of a transcript there from is homologous to all or part of an RNA molecule comprising an RNA sequence encoded by a nucleotide sequence within the genome of a pathogen.

In one embodiment a plant transformation vector comprises an isolated and purified DNA molecule comprising a heterologous promoter operatively linked to one or more nucleotide sequences of the present invention. The nucleotide sequence is SEQ ID NO: 1920, as set forth in the sequence listing. The nucleotide sequence includes a segment coding all or part of an RNA present within a targeted nematode RNA transcript and may comprise inverted repeats of all or a part of a targeted nematode RNA. The DNA molecule comprising the expression vector may also contain a functional intron sequence positioned either upstream of the coding sequence or even within the coding sequence, and may also contain a five prime (5') untranslated leader sequence (*i.e.,* a UTR or 5'-UTR) positioned between the promoter and the point of translation initiation.

A plant transformation vector may contain sequences from more than one gene, thus allowing production of more than one dsRNA for inhibiting expression of two or more genes in cells of a target nematode. One skilled in the art will readily appreciate that segments of DNA whose sequence corresponds to that present in different genes can be combined into a single composite DNA segment for expression in a transgenic plant. Alternatively, a plasmid of the present invention already containing at least one DNA segment can be modified by the sequential insertion of additional DNA segments between the enhancer and promoter and terminator sequences. In the disease control agent of the present invention designed for the inhibition of multiple genes, the genes to be inhibited can be obtained from the same plant-parasitic nematode species in order to enhance the effectiveness of the control agent. In certain embodiments, the genes can be derived from different plant-parasitic nematodes in order to broaden the range of nematodes against which the agent(s) is/are effective. When multiple genes are targeted for suppression or a combination of expression and suppression, a polycistronic DNA element can be fabricated as illustrated and disclosed in US Publication 2004-0029283.

Promoters that function in different plant species are also well known in the art. Promoters useful for expression of polypeptides in plants include those that are inducible, viral, synthetic, or constitutive as described in Odell *et al.* (1985), and/or promoters that are temporally regulated, spatially regulated, and spatio-temporally regulated. Preferred promoters include the enhanced CaMV35S promoters, and the FMV35S promoter. A fragment of the CaMV35S promoter exhibiting root-specificity may also be preferred. For the purpose of the present invention, it may be preferable to achieve the highest levels of expression of these genes within the root tissues of plants. A number of root-specific promoters have been identified and are known in the art (*e.g*. US Patents 5,110,732; 5,837,848 and 5,459,252; Hirel et al. 1992).

A recombinant DNA vector or construct of the present invention may comprise a selectable marker that confers a selectable phenotype on plant cells. Selectable markers may also be used to select for plants or plant cells that contain the exogenous nucleic acids encoding polypeptides or proteins of the present invention. The marker may encode biocide resistance, antibiotic resistance *(e.g.,* kanamycin, G418 bleomycin, hygromycin), or herbicide resistance *(e.g.,* glyphosate). Examples of selectable markers include, but are not limited to, a neo gene which codes for kanamycin resistance and can be selected for using kanamycin, G418 a bar gene which codes for bialaphos resistance; a mutant EPSP synthase gene which encodes glyphosate resistance; a nitrilase gene which confers resistance to bromoxynil; a mutant acetolactate synthase gene (ALS) which confers imidazolinone or sulfonylurea resistance; and a methotrexate resistant DHFR gene. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, and tetracycline. Examples of such selectable markers are illustrated in U.S. Patents 5,550,318; 5,633,435; 5,780,708 and 6,118,047.

A recombinant vector or construct of the present invention may also include a screenable marker. Screenable markers may be used to monitor expression. Exemplary screenable markers include a β-glucuronidase or *uidA* gene (GUS) which encodes an enzyme for which various chromogenic substrates are known (Jefferson *et al.,* 1987); an R-locus gene, which encodes a product that regulates the production of anthocyanin pigments (red color) in plant tissues (Dellaporta *et al.,* 1988); a □-lactamase gene (Sutcliffe *et al.,* 1978), a gene which encodes an enzyme for which various chromogenic substrates are known *(e.g.,* PADAC, a chromogenic cephalosporin); a luciferase gene (Ow *et al.,* 1986) a *xylE* gene (Zukowski *et al.,* 1983) which encodes a catechol dioxygenase that can convert chromogenic catechols; an α-amylase gene (Ikatu *et al.,* 1990); a tyrosinase gene (Katz *et al.,* 1983) which encodes an enzyme capable of oxidizing tyrosine to DOPA and dopaquinone which in turn condenses to melanin; an α -galactosidase, which catalyzes a chromogenic α-galactose substrate.

Preferred plant transformation vectors include those derived from a Ti plasmid of *Agrobacterium tumefaciens (e.g.* U.S. Patent Nos. 4,536,475; 4,693,977; 4,886,937 and 5,501,967; and EP 0122791). *Agrobacterium rhizogenes* plasmids (or "Ri") are also useful and known in the art. Other preferred plant transformation vectors include those disclosed, *e.g.,* by Herrera-Estrella (1983); Bevan (1983), Klee (1985) and EP 0120516.

In general it may be preferred to introduce a functional recombinant DNA at a non-specific location in a plant genome. In special cases it may be useful to insert a recombinant DNA construct by site-specific integration. Several site-specific recombination systems exist which are known to function implants include cre-lox as disclosed in U.S. Patent 4,959,317 and FLP-FRT as disclosed in U.S. Patent 5,527,695.

Suitable methods for transformation of host cells for use with the current invention are believed to include virtually any method by which DNA can be introduced into a cell (see, for example, Miki *et al.,* 1993), such as by transformation of protoplasts (U.S. Patent No. 5,508,184; Omirulleh *et al.,* 1993), by desiccation/inhibition-mediated DNA uptake (Potrykus *et al.,* 1985), by electroporation (U.S. Patent No. 5,384,253), by agitation with silicon carbide fibers (Kaeppler et al., 1990; U.S. Patent Nos. 5,302,523 and 5,464,765), by *Agrobacterium*-mediated transformation (U.S. Patent Nos. 5,563,055; 5,591,616; 5,693,512; 5,824,877; 5,981,840 and 6,384,301) and by acceleration of DNA coated particles (U.S. Patent Nos. 5,015,580; 5,550,318; 5,538,880; 6,160,208; 6,399,861 and 6,403,865; Padgette et al. 1995). Through the application of techniques such as these, the cells of virtually any species may be stably transformed. In the case of multicellular species, the transgenic cells may be regenerated into transgenic organisms. The most widely utilized method for introducing an expression vector into plants is based on the natural transformation system of *Agrobacterium* (for example, Horsch *et al.,* 1985). *A. tumefaciens* and *A. rhizogenes* are plant pathogenic soil bacteria which genetically transform plant cells. The Ti and Ri plasmids of *A. tumefaciens* and *A. rhizogenes,* respectively, carry genes responsible for genetic transformation of the plant. Descriptions *of Agrobacterium* vector systems and methods for *Agrobacterium-mediated* gene transfer are provided by numerous references, including Gruber *et al* .1993; *Miki et al.,* 1993, Moloney *et al.,* 1989, and U.S. Patent Nos: 4,940,838 and 5,464,763. Other bacteria such as *Sinorhizobium, Rhizobium,* and *Mesorhizobium* that interact with plants naturally can be modified to mediate gene transfer to a number of diverse plants. These plant-associated symbiotic bacteria can be made competent for gene transfer by acquisition of both a disarmed Ti plasmid and a suitable binary vector (Broothaerts *et al.* 2005).

Methods for the creation of transgenic plants and expression of heterologous nucleic acids in plants in particular are known and may be used with the nucleic acids provided herein to prepare transgenic plants that exhibit reduced susceptibility to feeding by a target nematode. Plant transformation vectors can be prepared, for example, by inserting the dsRNA producing nucleic acids disclosed herein into plant transformation vectors and introducing these into plants. One known vector system has been derived by modifying the natural gene transfer system of *Agrobacterium tumefaciens.* The natural system comprises large Ti (tumor-inducing)-plasmids containing a large segment, known as T-DNA, which is transferred to transformed plants. Another segment of the Ti plasmid, the vir region, is responsible for T-DNA transfer. The T-DNA region is bordered by terminal repeats. In the modified binary vectors the tumor-inducing genes have been deleted and the functions of the vir region are utilized to transfer foreign DNA bordered by the T-DNA border sequences. The T-region may also contain a selectable marker for efficient recovery of transgenic plants and cells, and a multiple cloning site for inserting sequences for transfer such as a dsRNA encoding nucleic acid.

A transgenic plant formed using *Agrobacterium* transformation methods typically contains a single simple recombinant DNA sequence inserted into one chromosome and is referred to as a transgenic event. Such transgenic plants can be referred to as being heterozygous for the inserted exogenous sequence. A transgenic plant homozygous with respect to a transgene can be obtained by sexually mating (selfing) an independent segregant transgenic plant that contains a single exogenous gene sequence to itself, for example an F0 plant, to produce F1 seen. One fourth of the F1 seed produced will be homozygous with respect to the transgene. Germinating F1 seed results in plants that can be tested for heterozygosity, typically using a SNP assay or a thermal amplification assay that allows for the distinction between heterozygotes and homozygotes (*i.e.,* a zygosity assay). Crossing a heterozygous plant with itself or another heterozygous plant results in only heterozygous progeny.

### C. Nucleic Acid Expression and Target Gene Suppression

The present invention provides, as an example, a transformed host plant of a pathogenic target organism, transformed plant cells and transformed plants and their progeny. The transformed plant cells and transformed plants may be engineered to express one or more of the dsRNA or siRNA sequences, under the control of a heterologous promoter, described herein to provide a pathogen-protective effect. These sequences may be used for gene suppression in a pathogen, thereby reducing the level or incidence of disease caused by the pathogen on a protected transformed host organism. As used herein the words "gene suppression" are intended to refer to any of the well-known methods for reducing the levels of protein produced as a result of gene transcription to mRNA and subsequent translation of the mRNA.

Gene suppression is also intended to mean the reduction of protein expression from a gene or a coding sequence including posttranscriptional gene suppression and transcriptional suppression. Posttranscriptional gene suppression is mediated by the homology between of all or a part of a mRNA transcribed from a gene or coding sequence targeted for suppression and the corresponding double stranded RNA used for suppression, and refers to the substantial and measurable reduction of the amount of available mRNA available in the cell for binding by ribosomes. The transcribed RNA can be in the sense orientation to effect what is called co-suppression, in the anti-sense orientation to effect what is called anti-sense suppression, or in both orientations producing a dsRNA to effect what is called RNA interference (RNAi). Transcriptional suppression is mediated by the presence in the cell of a dsRNA gene suppression agent exhibiting substantial sequence identity to a promoter DNA sequence or the complement thereof to effect what is referred to as promoter *trans* suppression. Gene suppression may be effective against a native plant gene associated with a trait, e.g., to provide plants with reduced levels of a protein encoded by the native gene or with enhanced or reduced levels of an affected metabolite. Gene suppression can also be effective against target genes in a plant-parasitic nematode that may ingest or contact plant material containing gene suppression agents, specifically designed to inhibit or suppress the expression of one or more homologous or complementary sequences in the cells of the nematode. Post-transcriptional gene suppression by anti-sense or sense oriented RNA to regulate gene expression in plant cells is disclosed in U.S. Pat. Nos. 5,107,065; 5,759,829; 5,283,184 and 5,231,020. The use of dsRNA to suppress genes in plants is disclosed in WO 99/53050, WO 99/49029, U.S. Publications 2003/0175965 and 2003/0061626, U.S. Patent Application No.10/465,800, and U.S. Patent Nos. 6,506,559 and 6,326,193.

A beneficial method of post transcriptional gene suppression versus a plant-parasitic nematode employs both sense-oriented and anti-sense-oriented, transcribed RNA which is stabilized, *e.g.,* as a hairpin and stem and loop structure. A preferred DNA construct for effecting post transcriptional gene suppression is one in which a first segment encodes an RNA exhibiting an anti-sense orientation exhibiting substantial identity to a segment of a gene targeted for suppression, which is linked to a second segment encoding an RNA exhibiting substantial complementarity to the first segment. Such a construct forms a stem and loop structure by hybridization of the first segment with the second segment and a loop structure from the nucleotide sequences linking the two segments (see WO94/01550, WO98/05770, US 2002/0048814, and US 2003/0018993). Co-expression with an additional target gene segment may also be employed, as noted above (*e.g*. WO05/019408).

According to one embodiment of the present invention, there is provided a nucleotide sequence, for which *in vitro* expression results in transcription of a stabilized RNA sequence that is substantially homologous to an RNA molecule of a targeted gene in a plant-parasitic nematode that comprises an RNA sequence encoded by a nucleotide sequence within the genome of the nematode. Thus, after the plant-parasitic nematode ingests the stabilized RNA sequence, a down-regulation of the nucleotide sequence corresponding to the target gene in the cells of a target nematode is effected.

In certain embodiments of the invention, expression of a fragment of at least 21 contiguous nucleotides of a nucleic acid sequence of SEQ ID NO:1920 may be utilized, including expression of a fragment of up to 21, 36, 60, 100, 550, or 1000 contiguous nucleotides, or sequences displaying 90-100% identity with such seqences, or their complements. In specific embodiments, a nucleotide provided by the invention may comprise a sequence selected from the group described in Table 4, including a location on such sequence spanning nucleotides as described in Table 4. In yet other embodiments, a nucleotide provided by the invention may be described as comprising one or more of nucleotides 1-21,22-50, 51-100,101-150, 151-200, 201-250, 251-300, 301-350, 351-400, 401-450, 451-500, 501-550, 551-600, 601-650, 651-700, 701-750, 751-800, 801-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, 2001-2050, 2051-2100, 23-75, 76-125, 126-175, 176-225, 226-275, 276-325, 326-375, 376-425, 426-475, 476-525, 526-575, 576-625, 626-675, 676-725, 726-775, 776-825, 826-875, 876-925, 926-975, 976-1025, 1026-1075, 1076-1125, 1126-1175, 1176-1225, 1226-1275, 1276-1325, 1326-1375, 1376-1425, 1426-1475, 1476-1525, 1526-1575, 1576-1625, 1626-1675, 1676-1725, 1726-1775, 1776-1825, 1826-1875,1876-1925, 1926-1975, 1976-2025, 2026-2075, 2076-2125, 1-550, 200-750, 300-850, 400-950, 500-1050, 600-1150, 700-1250, 800-1350, 900-1450, 1000-1550, 1100-1650, 1200-1750, 1300-1850, 1400-1950, 1500-2050, up to the full length of the sequence, of SEQ ID NO:1920. Methods for selecting specific sub-sequences as targets for siRNA-mediated gene suppression are known in the art (*e.g*. *Reynolds et al..* 2004).

Inhibition of a target gene using the stabilized dsRNA technology of the present invention is sequence-specific in that nucleotide sequences corresponding to the duplex region of the RNA are targeted for genetic inhibition. RNA containing a nucleotide sequences identical to a portion of the target gene is preferred for inhibition. RNA sequences with insertions, deletions, and single point mutations relative to the target sequence have also been found to be effective for inhibition. In performance of the present invention, it is preferred that the inhibitory dsRNA and the portion of the target gene share at least from 80% sequence identity, or from 90% sequence identity, or from 95% sequence identity, or from 99% sequence identity, or even 100% sequence identity. Alternatively, the duplex region of the RNA may be defined functionally as a nucleotide sequence that is capable of hybridizing with a portion of the target gene transcript. A less than full length sequence exhibiting a greater homology compensates for a longer less homologous sequence. The length of the identical nucleotide sequences may be at least 25, 50, 100, 200, 300, 400, 500 or at least 1000 bases. Normally, a sequence of greater than 20-100 nucleotides should be used, though a sequence of greater than 200-300 nucleotides would be preferred, and a sequence of greater than 500-1000 nucleotides would be especially preferred depending on the size of the target gene. The invention has the advantage of being able to tolerate sequence variations that might be expected due to genetic mutation, strain polymorphism, or evolutionary divergence. The introduced nucleic acid molecule may not need to be absolute homology, may not need to be full length, relative to either the primary transcription product or fully processed mRNA of the target gene. Therefore, those skilled in the art need to realize that, as disclosed herein, 100% sequence identity between the RNA and the target gene is not required to practice the present invention.

Inhibition of target gene expression may be quantified by measuring either the endogenous target RNA or the protein produced by translation of the target RNA and the consequences of inhibition can be confirmed by examination of the outward properties of the cell or organism. Techniques for quantifying RNA and proteins are well known to one of ordinary skill in the art. In certain embodiments gene expression is inhibited by at least 10%, preferably by at least 33%, more preferably by at least 50%, and yet more preferably by at least 80%. In particularly preferred embodiments of the invention gene expression is inhibited by at least 80%, more preferably by at least 90%, more preferably by at least 95%, or by at least 99% within cells in the pathogen so a significant inhibition takes place. Significant inhibition is intended to refer to sufficient inhibition that results in a detectable phenotype (e.g., cessation of growth, feeding, development, mortality) or a detectable decrease in RNA and/or protein corresponding to the target gene being inhibited. Although in certain embodiments of the invention inhibition occurs in substantially all cells of the plant-parasitic nematode, in other preferred embodiments inhibition occurs in only a subset of cells expressing the gene.

dsRNA molecules may be synthesized either *in vivo* or *in vitro.* The dsRNA may be formed by a single self-complementary RNA strand or from two complementary RNA strands. Endogenous RNA polymerase of the cell may mediate transcription *in vivo,* or cloned RNA polymerase can be used for transcription *in vivo* or *in vitro.* Inhibition may be targeted by specific transcription in an organ, tissue, or cell type; stimulation of an environmental condition (*e.g*., infection, stress, temperature, chemical inducers); and/or engineering transcription at a developmental stage or age. The RNA strands may or may not be polyadenylated; the RNA strands may or may not be capable of being translated into a polypeptide by a cell's translational apparatus.

A RNA, dsRNA, siRNA, or miRNA of the present invention may be produced chemically or enzymatically by one skilled in the art through manual or automated reactions or *in vivo* in another organism. RNA may also be produced by partial or total organic synthesis; any modified ribonucleotide can be introduced by *in vilro* enzymatic or organic synthesis. The RNA may be synthesized by a cellular RNA polymerase or a bacteriophage RNA polymerase (e.g., T3, T7, SP6). The use and production of an expression construct are known in the art (see, for example, WO 97/32016; U.S. Pat. Nos. 5,593,874; 5,698,425; 5,712,135; 5,789,214 and 5,804,693). If synthesized chemically or by *in vitro* enzymatic synthesis, the RNA may be purified prior to introduction into the cell. For example, RNA can be purified from a mixture by extraction with a solvent or resin, precipitation, electrophoresis, chromatography, or a combination thereof. Alternatively, the RNA may be used with no or a minimum of purification to avoid losses due to sample processing. The RNA may be dried for storage or dissolved in an aqueous solution. The solution may contain buffers or salts to promote annealing, and/or stabilization of the duplex strands.

For transcription from a transgene *in vivo* or an expression construct, a regulatory region (e.g., promoter, enhancer, silencer, and polyadenylation) may be used to transcribe the RNA strand (or strands). Therefore, in one embodiment, the nucleotide sequences for use in producing RNA molecules may be operably linked to one or more promoter sequences functional in a microorganism, a fungus or a plant host cell. Ideally, the nucleotide sequences are placed under the control of an endogenous promoter, normally resident in the host genome. The nucleotide sequence of the present invention, under the control of an operably linked promoter sequence, may further be flanked by additional sequences that advantageously affect its transcription and/or the stability of a resulting transcript. Such sequences are generally located upstream of the operably linked promoter and/or downstream of the 3' end of the expression construct and may occur both upstream of the promoter and downstream of the 3' end of the expression construct, although such an upstream sequence only is also contemplated.

As used herein, the term "disease control agent", or "gene suppression agent" refers to a particular RNA molecule consisting of a first RNA segment and a second RNA segment linked by a third RNA segment. The first and the second RNA segments lie within the length of the RNA molecule and are substantially inverted repeats of each other and are linked together by the third RNA segment. The complementarity between the first and the second RNA segments results in the ability of the two segments to hybridize *in vivo* and *in vitro* to form a double stranded molecule, *i.e.,* a stem, linked together at one end of each of the first and second segments by the third segment which forms a loop, so that the entire structure forms into a stem and loop structure, or even more tightly hybridizing structures may form into a stem-loop knotted structure. The first and the second segments correspond invariably and not respectively to a sense and an antisense sequence with respect to the target RNA transcribed from the target gene in the target pathogen that is suppressed by the ingestion of the dsRNA molecule. The control agent can also be a substantially purified (or isolated) nucleic acid molecule and more specifically nucleic acid molecules or nucleic acid fragment molecules thereof from a genomic DNA (gDNA) or cDNA library. Alternatively, the fragments may comprise smaller oligonucleotides having from 15 to 250 nucleotide residues, and more preferably, 15 to 30 nucleotide residues.

As used herein, the term "genome" as it applies to cells of a plant-parasitic nematode or a host encompasses not only chromosomal DNA found within the nucleus, but organelle DNA found within subcellular components of the cell. The DNA's of the present invention introduced into plant cells can therefore be either chromosomally integrated or organelle-localized. The term "genome" as it applies to bacteria encompasses both the chromosome and plasmids within a bacterial host cell. The DNA's of the present invention introduced into bacterial host cells can therefore be either chromosomally integrated or plasmid-localized.

As used herein, the term "plant-parasitic nematode" refers to those nematodes that may infect, colonize, parasitize, or cause disease on host plant material transformed to express or coated with a double stranded gene suppression agent. As used herein, a "nematode resistance" trait is a characteristic of a transgenic plant, transgenic animal, or other transgenic host that causes the host to be resistant to attack from a nematode that typically is capable of inflicting damage or loss to the host. Such resistance can arise from a natural mutation or more typically from incorporation of recombinant DNA that confers plant-parasitic nematode resistance. To impart nematode resistance to a transgenic plant a recombinant DNA can, for example, be transcribed into a RNA molecule that forms a dsRNA molecule within the tissues or fluids of the recombinant plant. The dsRNA molecule is comprised in part of a segment of RNA that is identical to a corresponding RNA segment encoded from a DNA sequence within a plant-parasitic nematode that prefers to cause disease on the host plant. Expression of the gene within the target plant-parasitic nematode is suppressed by the dsRNA, and the suppression of expression of the gene in the target plant-parasitic nematode results in the plant being resistant to the nematode. Fire et al. (U.S. Patent No. 6,506,599) generically describes inhibition of pest infestation, providing specifics only several nucleotide sequences that were effective for inhibition of gene function in the nematode species *Caenorhabditis elegans.* Similarly, US 2003/0061626 describes the use of dsRNA for inhibiting gene function in a variety of nematode pests. US 2003/0150017 describes using dsDNA sequences to transform host cells to express corresponding dsRNA sequences that are substantially identical to target sequences in specific pests, and particularly describe constructing recombinant plants expressing such dsRNA sequences for ingestion by various plant-parasitic nematode, facilitating down-regulation of a gene in the genome of the target organism and improving the resistance of the plant to the plant-parasitic nematode.

The modulatory effect of dsRNA is applicable to a variety of genes expressed in the plant-parasitic nematode including, for example, endogenous genes responsible for cellular metabolism or cellular transformation, including house keeping genes, transcription factors, molting-related genes, and other genes which encode polypeptides involved in cellular metabolism or normal growth and development.

As used herein, the phrase "inhibition of gene expression" or "inhibiting expression of a target gene in the cell of a plant-parasitic nematode" refers to the absence (or observable decrease) in the level of protein and/or mRNA product from the target gene. Specificity refers to the ability to inhibit the target gene without manifest effects on other genes of the cell and without any effects on any gene within the cell that is producing the dsRNA molecule. The inhibition of gene expression of the target gene in the plant-parasitic nematodes may result in novel phenotypic traits in the nematode.

The present invention provides in part a delivery system for the delivery of the nematode control agents by ingestion of host cells or the contents of the cells. In accordance with another embodiment, the present invention involves generating a transgenic plant cell or a plant that contains a recombinant DNA construct transcribing the stabilized dsRNA molecules of the present invention. As used herein, "taking up" refers to the process of an agent coming in contact with cells of a target organism, such as a nematode. This may occur, for instance, by nematode feeding, by soaking, or by injection. As used herein, the phrase "generating a transgenic plant cell or a plant" refers to the methods of employing the recombinant DNA technologies readily available in the art (*e.g.,* by Sambrook, *et al.,* 1989) to construct a plant transformation vector transcribing the stabilized dsRNA molecules of the present invention, to transform the plant cell or the plant and to generate the transgenic plant cell or the transgenic plant that contain the transcribed, stabilized dsRNA molecules.

It is envisioned that the compositions of the present invention can be incorporated within the seeds of a plant species either as a product of expression from a recombinant gene incorporated into a genome of the plant cells, or incorporated into a coating or seed treatment that is applied to the seed before planting. The plant cell containing a recombinant gene is considered herein to be a transgenic event.

The present invention provides in part a delivery system for the delivery of disease control agents to plant-parasitic nematodes. The stabilized dsRNA or siRNA molecules of the present invention may be directly introduced into the cells of a plant-parasitic nematode. Methods for introduction may include direct mixing of RNA with host tissue for the plant-parasitic nematode, as well as engineered approaches in which a species that is a host is engineered to express the dsRNA or siRNA. In one embodiment, the RNA may be sprayed onto a plant surface. In still another embodiment, the dsRNA or siRNA may be expressed by microorganisms and the microorganisms may be applied onto a plant surface or introduced into a root, stem by a physical means such as an injection. In still another embodiment, a plant may be genetically engineered to express the dsRNA or siRNA in an amount sufficient to kill the plant-parasitic nematodes known to infest the plant.

It is also anticipated that dsRNAs produced by chemical or enzymatic synthesis may be formulated in a manner consistent with common agricultural practices and used as spray-on products for controlling plant disease. The formulations may include the appropriate stickers and wetters required for efficient foliar coverage as well as UV protectants to protect dsRNAs from UV damage. Such additives are commonly used in the bioinsecticide industry and are well known to those skilled in the art. Such applications could be combined with other spray-on insecticide applications, biologically based or not, to enhance plant protection from plant-parasitic nematodes

The present invention also relates to recombinant DNA constructs for expression in a microorganism. Exogenous nucleic acids from which an RNA of interest is transcribed can be introduced into a microbial host cell, such as a bacterial cell or a fungal cell, using methods known in the art.

The nucleotide sequences of the present invention may be introduced into a wide variety of prokaryotic and eukaryotic microorganism hosts to produce the stabilized dsRNA or siRNA molecules. The term "microorganism" includes prokaryotic and eukaryotic species such as bacteria and fungi, as well as nematodes. Fungi include yeasts and filamentous fungi, among others. Illustrative prokaryotes, both Gram-negative and Gram-positive, include Entero-bacteriaceae, such as *Escherichia, Erwinia,* and *Serratia;* Bacillaceae; Rhizobiaceae, such as *Rhizobium*; Spirillaceae, such as photobacterium, *Zymomonas;* Lactobacillaceae; Pseudomonadaceae, such as *Pseudomonas* and *Acetobacter;* Azotobacteraceae, Actinomycetales, and Nitrobacteraceae. Among eukaryotes are fungi, such as Phycomycetes and Ascomycetes, including *Saccharomyces* and *Schizosaccharomyces;* and Basidiomycetes, such as *Rhodotorula, Aureobasidium.*

### D. Transgenic Plants

The present invention provides seeds and plants having one or more transgenic event. Combinations of events are referred to as "stacked" transgenic events. These stacked transgenic events can be events that are directed at the same target organism, or they can be directed at different target pathogens or pests. In one embodiment, a seed having the ability to express a nucleic acid provided herein also has the ability to express at least one other agent, including, but not limited to, an RNA molecule the sequence of which is derived from the sequence of an RNA expressed in a target pathogen such as a nematode and that forms a double stranded RNA structure upon expressing in the seed or cells of a plant grown from the seed, wherein the ingestion of one or more cells of the plant by the target results in the suppression of expression of the RNA in the cells of the target.

In certain embodiments, a seed having the ability to express a dsRNA the sequence of which is derived from a target plant-parasitic nematode also has a transgenic event that provides herbicide tolerance. One beneficial example of a herbicide tolerance gene provides resistance to glyphosate, N- (phosphonomethyl) glycine, including the isopropylamine salt form of such herbicide.

Benefits provided by the present invention may include, but are not limited to: the ease of introducing dsRNA into the plant-parasitic nematode cells, the low concentration of dsRNA which can be used, the stability of dsRNA, and the effectiveness of the inhibition. The ability to use a low concentration of a stabilized dsRNA avoids several disadvantages of anti-sense interference. The present invention is not limited to *in vitro* use or to specific sequence compositions, to a particular set of target genes, a particular portion of the target gene's nucleotide sequence, or a particular transgene or to a particular delivery method, as opposed to the some of the available techniques known in the art, such as antisense and co-suppression. Furthermore, genetic manipulation becomes possible in organisms that are not classical genetic models.

In order to achieve inhibition of a target gene selectively within a plant-parasitic nematode species that it is desired to control, the target gene should preferably exhibit a low degree of sequence identity with corresponding genes in a plant or a vertebrate animal. Preferably the degree of the sequence identity is less than 80%. More preferably the degree of the sequence identity is less than 70%. Most preferably the degree of the sequence identity is less than 60%.

In addition to direct transformation of a plant with a recombinant DNA construct, transgenic plants can be prepared by crossing a first plant having a recombinant DNA construct with a second plant lacking the construct. For example, recombinant DNA for gene suppression can be introduced into first plant line that is amenable to transformation to produce a transgenic plant that can be crossed with a second plant line to introgress the recombinant DNA for gene suppression into the second plant line.

The present invention can be, in practice, combined with other disease control traits in a plant to achieve desired traits for enhanced control of plant disease. Combining disease control traits that employ distinct modes-of-action can provide protected transgenic plants with superior durability over plants harboring a single control trait because of the reduced probability that resistance will develop in the field.

The invention also relates to commodity products containing one or more of the sequences of the present invention, and produced from a recombinant plant or seed containing one or more of the nucleotide sequences of the present invention are specifically contemplated as embodiments of the present invention. A commodity product containing one or more of the sequences of the present invention is intended to include, but not be limited to, meals, oils, crushed or whole grains or seeds of a plant, or any food product comprising any meal, oil, or crushed or whole grain of a recombinant plant or seed containing one or more of the sequences of the present invention. The detection of one or more of the sequences of the present invention in one or more commodity or commodity products contemplated herein is defacto evidence that the commodity or commodity product is composed of a transgenic plant designed to express one or more of the nucleotides sequences of the present invention for the purpose of controlling plant disease using dsRNA mediated gene suppression methods.

### E. Obtaining Nucleic acids

The present invention provides methods for obtaining a nucleic acid comprising a nucleotide sequence for producing a dsRNA or siRNA. In one embodiment, such a method comprises: (a) analyzing one or more target gene(s) for their expression, function, and phenotype upon dsRNA-mediated gene suppression in a nematode; (b) probing a cDNA or gDNA library with a hybridization probe comprising all or a portion of a nucleotide sequence or a homolog thereof from a targeted nematode that displays an altered, e.g. reduced, nematode growth or development phenotype in a dsRNA-mediated suppression analysis; (c) identifying a DNA clone that hybridizes with the hybridization probe; (d) isolating the DNA clone identified in step (b) ; and (e) sequencing the cDNA or gDNA fragment that comprises the clone isolated in step (d) wherein the sequenced nucleic acid molecule transcribes all or a substantial portion of the RNA nucleotide acid sequence or a homolog thereof.

In another embodiment, a method of the present invention for obtaining a nucleic acid fragment comprising a nucleotide sequence for producing a substantial portion of a dsRNA or siRNA comprises: (a) synthesizing first and a second oligonucleotide primers corresponding to a portion of one of the nucleotide sequences from a targeted pathogen; and (b) amplifying a cDNA or gDNA insert present in a cloning vector using the first and second oligonucleotide primers of step (a) wherein the amplified nucleic acid molecule transcribes a substantial portion of the a substantial portion of a dsRNA or siRNA of the present invention.

In practicing the present invention, a target gene may be derived from *H. glycines* or another nematode. It is contemplated that several criteria may be employed in the selection of preferred target genes. The *H. glycines* gene may be one which has a *C*. *elegans* ortholog with a likelihood for a strong phenotype upon RNAi knockdown of expression, including a **P0** phenotype. Such targets are often those with protein products involved in core cellular processes such as DNA replication, cell cycle, transcription, RNA processing, translation, protein trafficking, secretion, protein modification, protein stability and degradation, energy production, intermediary metabolism, cell structure, signal transduction, channels and transporters, and endocytosis. In certain embodiments it is advantageous to select a gene for which a small drop in expression level results in deleterious effects for the pathogen.

As used herein, the term "derived from" refers to a specified nucleotide sequence that may be obtained from a particular specified source or species, albeit not necessarily directly from that specified source or species.

For the purpose of the present invention, the dsRNA or siRNA molecules may be obtained by polymerase chain (PCR™) amplification of a target gene sequences derived from a gDNA or cDNA library or portions thereof. The DNA library may be prepared using methods known to the ordinary skilled in the art and DNA/RNA may be extracted. Genomic DNA or cDNA libraries generated from a target organism may be used for PCR™ amplification for production of the dsRNA or siRNA.

The target genes may be then be PCR™ amplified and sequenced using the methods readily available in the art. One skilled in the art may be able to modify the PCR™ conditions to ensure optimal PCR™ product formation. The confirmed PCR™ product may be used as a template for *in vitro* transcription to generate sense and antisense RNA with the included minimal promoters. In one embodiment, the present invention comprises isolated and purified nucleotide sequences that may be used as plant-parasitic nematode control agents. The isolated and purified nucleotide sequences may comprise those as set forth in the sequence listing.

As used herein, the phrase "coding sequence", "structural nucleotide sequence" or "structural nucleic acid molecule" refers to a nucleotide sequence that is translated into a polypeptide, usually via mRNA, when placed under the control of appropriate regulatory sequences. The boundaries of the coding sequence are determined by a translation start codon at the 5'-terminus and a translation stop codon at the 3'-terminus. A coding sequence can include, but is not limited to, genomic DNA, cDNA, EST and recombinant nucleotide sequences.

The term " recombinant DNA" or "recombinant nucleotide sequence" refers to DNA that contains a genetically engineered modification through manipulation via mutagenesis or restriction enzymes.

For many of the plant-parasitic nematodes that are potential targets for control by the present invention, there may be limited information regarding the sequences of most genes or the phenotype resulting from mutation of particular genes. Therefore, it is contemplated that selection of appropriate genes for use in the present invention may be accomplished through use of information available from study of the corresponding genes in a model organism such in *C*. *elegans,* in which the genes have been characterized, according to the analysis described in Examples 1-8. In some cases it will be possible to obtain the sequence of a corresponding gene from a target nematode by searching databases such as GenBank using either the name of the gene or the sequence from, for example, a nematode from which the gene has been cloned. Once the sequence is obtained, PCR™ may be used to amplify an appropriately selected segment of the gene in the target nematode for use in the present invention. PCR™ primers may be designed based on the sequence as found in another organism from which the gene has been cloned. The primers are designed to amplify a DNA segment of sufficient length for use in the present invention. DNA (either genomic DNA or cDNA) is prepared from the target plant-parasitic nematode, and the PCR™ primers are used to amplify the DNA segment. Amplification conditions are selected so that amplification will occur even if the primers do not exactly match the target sequence. Alternately, the gene (or a portion thereof) may be cloned from a gDNA or cDNA library prepared from a plant-parasitic nematode species, using the known gene as a probe. Techniques for performing PCR™ and cloning from libraries are known. Further details of the process by which DNA segments from target plant-parasitic nematodes species may be isolated based on the sequence of genes previously cloned from other species are provided in the Examples. One of ordinary skill in the art will recognize that a variety of techniques may be used to isolate gene segments from plant-parasitic nematodes that correspond to genes previously isolated from other species.

### EXAMPLES

The inventors herein have identified a means for controlling plant-parasitic nematodes by providing double stranded ribonucleic acid molecules to plant-parasitic nematodes, and a means to select sequences that encode these double stranded ribonucleic acid molecules. Double stranded ribonucleic acid molecules that function upon ingestion to inhibit a biological function in a nematode may result, for example, in one or more of the following attributes: reduction in growth of a nematode, inhibition of development of a nematode, or reduction of viability. Any one or any combination of these attributes can result in an effective inhibition of plant infection or colonization, and in the case of a plant pathogenic nematode, inhibition of plant disease, and/or reduction in severity of disease symptoms. Examples relating to matter not embraced by the claims are for illustrative purposes.

### EXAMPLE 1

### Criteria for Target Gene Selection

To rank genes by likely essentiality of function in the nematodes lifecycle, information from *C*. *elegans* RNA interference (RNAi) experiments available at wormbase.org was combined with additional experimental information. Using the >40,000 phenotype data points in wormbase, all ~20,000 *C*. *elegans* genes were ranked for their potency of phenotype, level of confidence in phenotype, and likelihood of effects at multiple stages in the lifecycle. 715 *C*. *elegans* genes with BLAST amino acid homology to *H. glycines* of at least e-10 (see orthology determination below) had scores in this phenotype scoring system of 44 or better, where 1 is best. Below, phenotype statistics are provided for just the targets eventually making the top 300 list (using all criteria). As the phenotype ranking is dependent upon currently available information, it is not meant to be all inclusive capturing every gene that could be a good target, but rather to provide a list of high quality targets likely to be successful in *H. glycines* RNAi based on information in hand.

The following analysis of phenotypes evident following RNAi treatment was performed prior to the recent publication of Sonnichsen *et al. C. elegans* genome RNAi survey (Sonnichsen *et al.,* 2005). This group tested 19,075 genes by injection of dsRNA into the gonads of *C*. *elegans* wild type strain N2 hermaphrodites and looked for effects on that animal and its progeny. They found effects for just 1,668 genes (8.7%). Providing additional support to the robustness of the top 300 list (FIG 3), 293 were among the genes tested by Sonnichsen *et al.* and 257 had phenotypes (87.7%) including 176-Emb (embryonic lethal), 34 Lva (larval arrest), 34 Ste (sterile), 8 Lvl (larval lethal), 2 Stp (sterile progeny), 1 Bmd (body morphological defect), 1 Dpy (dumpy), 1 Egl (egg laying abnormal). 36 had wild type (WT) reports. Wild type reports increased toward the bottom of the list: 7 WT reports in 1-100, 13 WT in 101-200, 16 WT in 201-300.

RNAi phenotype reports were categorized into four groups. First, reports of lethality in the larval stages (lethal = let, larval lethal = lvl, larval arrest = lva) were placed together and given greatest weight. Such phenotypes if mimicked in *H. glycines* would be advantageous as dsRNA delivery from the plant to the nematode would begin as the second stage juvenile worm, a stage analogous to the dauer larva, enters the plant. Delivery would continue through development of J2, J3, and J4 juvenile or larval stages at the feeding site. Disrupting nematode physiology during these larval stages could prevent feeding site formation in the plant roots in the **P0** (primary) generation.

Second, reports of molting (mlt), blistering (bli), and rupture (rup) defects were placed together and given second greatest weight. Such phenotypes are rare relative to other more commonly observed effects such as lethality or sterility. Nematodes, including both *C*. *elegans* and *H. glycines* are covered by a cuticle of collagen. Molting defects could block shedding and reformation of the cuticle between larval stages (J2 -> J3, J3 -> J4, J4 ->A) and therefore developmental progression. Blister phenotypes often also involve defects in the cuticle. Rupture phenotypes in *C. elegans* are not well understood but may involve loss of body integrity or osmotic shifts. Rupture phenotypes are particularly attractive for SCN control as they are likely irreversible with no opportunity for the worm to recover as may be the case for temporary blocks to metabolism or development.

Third, sterile (ste) and embryonic (emb) lethality phenotypes were placed together and given third greatest weight. In *C*. *elegans,* the germ line tissue, where many genes involved in fertility and embryonic development are active (maternal factors), is particularly sensitive to RNAi. In the context of an SCN infection, a sterile or embryonic lethal block to the lifecycle alone could prevent formation of a second generation but would allow initial feeding site formation and plant root system damage. Fourth, other major defects in the physiology including growth defective (gro), sick (sck), uncoordinated (unc), paralyzed (prl, prz) phenotypes were placed together and given fourth greatest weight. In the female *H. glycines,* once a feeding site is formed the worm discontinues moving and body wall musculature plays a limited role in survival. However, male worms become mobile as adults so that they can seek, find, and fertilize females. Therefore, blocking of muscle and nervous system structures important for normal movement could interfere with fertilization and formation of a second generation. For the purposes of this ranking, other non-lethal RNAi phenotypes (dumpy, short, long, body morphology defect, etc.) were not included. Phenotypes observed by genetic mutation were also not used in this ranking system.

To rank genes based on wormbase RNAi information, genes with multiple phenotypic reports and multiple different phenotypes were favored over genes with single phenotypic reports and single phenotypes. Multiple reports of phenotypes are helpful as they add confidence to the assignment of phenotype to a gene. Based on experiments from multiple groups, *C*. *elegans* RNAi experiments have a false negative and false positive rate of ~10-15%. Evidence of phenotypes from more than one report decreases the likelihood of inclusion of false positives in the list. Because *C. elegans* RNAi does not result in complete transcript elimination (knockdown is estimated at 90%) and is often non-uniform through the population, single gene RNAi experiments often result in the observation of multiple phenotypes over the course of the life cycle. Therefore, reporting of the phenotype, of a given gene as "ste, emb, lvl" may mean that it is required throughout the lifecycle whereas a phenotype of "emb" alone may mean that the gene is only required for one step in embryonic development. For the purposes of parasite control, genes required for multiple steps and multiple processes in the lifecycle are more attractive targets than those required at just one stage as this provides more opportunities for a dsRNA to interfere with the lifecycle and block infection.

In each of these four categories, *C*. *elegans* RNAi reports were totaled and weighted. The first report of a phenotype in an N2 (wild type) background for a gene from a given laboratory was given a weight of 1. The second, third, etc. report of a phenotype from that same laboratory was given a discounted weight of 0.7. Therefore, independent reports from two laboratories (score = 2) would be favored over two reports from the same laboratory (score = 1.7) that might be vulnerable to a systematic error (*e.g*. wrong clone selected and used twice). Reports of phenotypes in the rrf-3(-/-) genetic background which is more sensitive to RNAi than N2 were given a discounted score of 0.6.

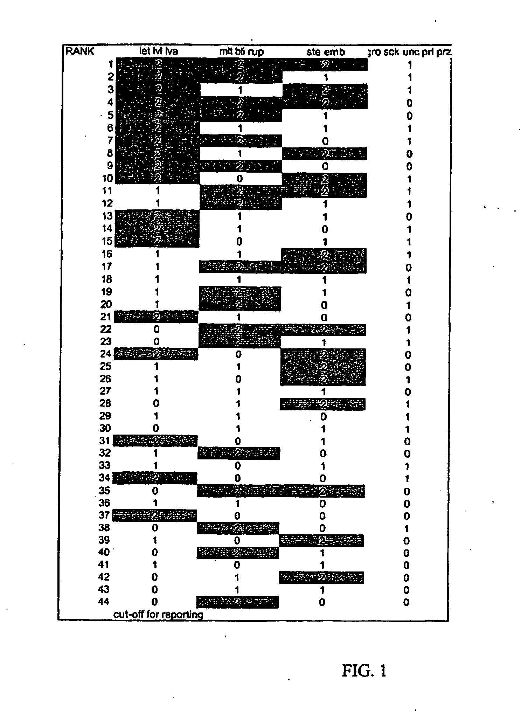

With these categories and weightings, targets were ranked on a scale from a score of 1 to 44 or higher based on their tally in the matrix shown in FIG 1. For instance, to receive a score of 3, targets had to have a let lvl Iva score of ≥ 2, a mlt bli rup score of X ≥ 1, a ste emb score of ≥ 2, and an other phenotype score of ≥ 1. Most genes in *C*. *elegans* have a score worse than 44. 715 genes had a score of 44 or better and a potential homolog in *H. glycines.* For the top 300 targets, following ranking on all criteria, the average phenotype score was 22±9. The top score was a 3 (6 targets) and only 10 targets had a score less than 10. The most common categories were scores of 10 (61 targets with a let lvl Iva score of ≥ 2, a mlt bli rup score of 0, a ste emb score of ≥ 2 and an other phenotype score of ≥ 1), 15 (23 targets), 24 (26 targets), 26 (93 targets), and 33 (19 targets). Averages and standard deviations for tallies in each category were let lvl lva score = 1.9±1, mlt bli rup score = 0.3±0.7, ste emb score = 2.8±1.7, and an other phenotype score = 1.6±1.3. mlt bli rup were the most rare phenotypes with only 56 targets having a score ≥ 0. Totals of targets with scores ≥ 0 in the other categories were 284 for let lvl lva, 281 for ste emb score of ≥ 2, and 258 for other phenotype.

In addition to reports of visible phenotypes from wormbase, reports of wild type (i.e. no phenotype) for each target were also recorded. Wild type findings were viewed as a negative for target ranking and most targets with high numbers of wild type reports were removed from consideration for the top 300 list. All wild type reports were given a score of 1 except those from Vidal *et al.* which were given a score of 0.3 since this group's methodology seems to have resulted in a higher percentage of wild type reports than all other reporting groups. In addition to total wild type tally, % wild type relative to all other reports was also calculated. For the top 300 targets, the averages and standard deviations for wild type tally and wild type percent were 0.14±0.41 and 1.9±5.1%. 250 of the 300 targets had no wild type reports. For the 50 targets with wild type reports, the wild type tally and percentages were 0.85±0.65 and 11.3±7.3%. In only 24 cases were wild type reports >10% of all reports with the highest being 33%.

Next, RNAi data was compared to that available in wormbase. RNAi experiments have been performed on more than 1,500 *C*. *elegans* genes of interest in feeding assays with N2, *rrf-3,* and other strains. Of the 715 *C*. *elegans* genes under consideration with BLAST amino acid homology to the *H. glycines* and wormbase phenotype scores of 44 or better (FIG 1), information was available for about 75. Additionally, 3 genes with P0 (first generation) effects in assays not on the list of 715 were added to the list. 10 target genes of the original 715 showed no phenotype in an assay (multiple replicates with sequence confirmation of constructs) and were therefore excluded from the top 300. Phenotypes were observed for 39 target genes in the top 300 including lvl, lva, rup, ste, emb, unc, sck and additional phenotypes not included in the ranking system such as growth defective (gro), sterile progeny (stp), dumpy (dpy), and body morphology defect (bmd).

### EXAMPLE 2

### C. elegans P-zero (P0) RNAi screens

In addition to the standard RNAi assay, additional *C*. *elegans* RNAi screens were performed. One of these was a **P0** lethal screen.

### P-zero (P0) or Rapid Onset RNAi Effects in C. elegans

To control *Heterodera glycines* by providing dsRNA from a transgenic soy plant, finding genes that are sensitive to rapid onset RNAi effects could be useful. Such genes might block mature feeding site formation by the J2 while slow onset genes might not show an effect until the next generation. *C*. *elegans* genes where effects are seen in the exposed animal, so called P0 (P-zero) generation effects, might predict *H. glycines* orthologs with high and rapid sensitivity to RNAi.

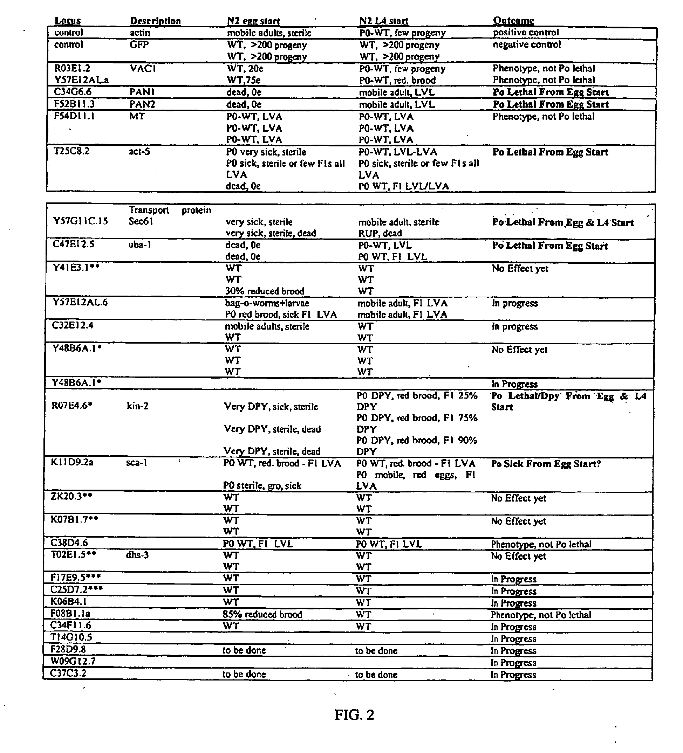

All large scale screens for RNAi effects in *C*. *elegans* have exposed initial worms as L4s or adults (the genetic P0 (P-zero) generation) to dsRNA and looked for phenotypes in their progeny, the F1 and F2 generation. Sterility of the initial P0 animal was also observed in many cases. None of the microinjection screens (Gonczy *et al.,* 2000; Piano *et al.* 2000; Piano *et al.,* 2002) or feeding screens (Fraser *et al.,* 2000; Kamath *et al.,* 2003) note effects on the P0 animals other than sterility. Likewise, in a screen of over 1,200 *C*. *elegans* genes by feeding from L4 start, P0 phenotypes other than sterility were not observed. Wormbase (www.wormbase.org) records of RNAi phenotypes do not even note the generation in which an effect is observed (P0, F1, F2, etc.) so finding P0 candidates from these records is not possible. Maeda *et al.* 2000 performed RNAi on 2,500 *C. elegans* genes by soaking of L4s. Interestingly, in the supplementary online material accompanying the publication, Maeda *et al.* note 26 cases where P0 effects other than sterility were observed ranging from unhealthy to slow growing to lethality. These targets were investigated experimentally to see if any of the genes were actually legitimate P0 effects (FIG 2).

Of the 26 Maeda *et al.* entries 21 corresponded to wormbase.org entries while 5 were clones of unclear origin. 19 genes have been successfully cloned into RNAi E. *coli* feeding constructs and sequence verified while 2 had cloning difficulties. 17 of the 19 completed clones have so far been tested for P0 phenotypes. Experiments are in progress to finish testing of two more clones and repeat test six others that have only been tested once to confirm results. Additionally, 7 targets of interest were tested in the same assay for a total of 24. Data was collected from two life-cycle starting points. First, a P0 L4 start was performed to mimic both the Maeda assay and the standard *E. coli* feeding assay. Second, a P0 egg start was performed to expose the P0 animal to dsRNA throughout development, a similar situation to that which may be encountered by a *Heterodera glycines* nematode upon infection of a plant producing a dsRNA. For the egg start, the assumption is that the first dsRNA exposure occurs as the L1 larva hatches and begins to feed.

From the 17 tested Maeda *et al.* (2001) candidates and 7 additional targets, 6 were found to have P0 phenotypes other than sterility from an egg start in the feeding assay (3 from Maeda and 3 from the additional list). These were Y57G11C.15 (sec61 alpha), C47E12.5 (uba-1), and R07E4.6 (kin-2) from Maeda and C34G6.6 (pan-1), F52B11.3 (pan-2), and T25C8.2 (act-5) from the additional list. For the L4 start, only three genes showed a P0 phenotypes other than sterility. The genes with L4 start phenotypes were a subset of the 6 showing an egg start phenotype: Y57G11C.15 (sec61 alpha) and R07E4.6 (kin-2) from Maeda and T25C8.2 (act-5) from the additional list. Of the remaining genes tested to date from the Maeda candidates, 4 had less severe phenotypes such as sterility and F1 larval arrest while 9 are fully wild type and were likely false positives in the Maeda screen. It remains possible however that genes lacking a phenotype in a bacterial feeding assay would have a phenotype in a soaking assay.

In addition to studies with N2 *C*. *elegans* worms (laboratory standard strain), studies were also performed for 22 of the genes in a *fog-2(q71)* mutant background. *fog-2*(q71) is a mutant that feminizes the germ line of the hermaphrodite and is maintained as a male/female strain. Female worms were picked away from males as larva prior to fertilization. Such virgin female worms are easy to maintain for many days as they age since there is no F1 generation present to overgrow the plate and may therefore aid in seeing late onset effects in the P0 adult worm. P0 results observed in *fog-2(q71)* were identical to those seen in N2 and no additional phenotypes were detected. *fog-2* tests on subsequent genes were therefore discontinued.

**The studies showed that in *C*. *elegans* obtaining a P0 phenotype other than** sterility from an L4 start is a very rare event but that examples do exist such as Y57Gl1C.15 (sec61 alpha), R07E4.6 (kin-2), and T25C8.2 (act-5) (FIG 2). Evidence suggests that most of the P0 phenotypes other than sterility recorded by Maeda *et al.* in their supplementary materials were false positives. This is supported by other evidence: in addition to the present data at least six genes had been tested by other laboratories and found to have only wild type phenotypes in all generations (P0, F1, etc.). P0 phenotypes other than sterility from an L4 start are presumably rare as the adult already has many of the proteins necessary for survival and the slow kinetics of RNAi takes time to degrade all transcripts. Genes showing an effect under such conditions may be ones where the worm is especially sensitive to their disruption and therefore ranked highly among targets for consideration. P0 phenotypes other than sterility from an egg start are less rare and are seen in several of the targets of interest known to have severe phenotypes (*e.g.* pan-1, pan-2). Presumably, a larger survey of targets with strong phenotypes would find more.

Of the six targets in which a P0 lethal phenotype was confirmed, all have reasonably strong orthologs among the *H. glycines* sequences. kin-2 was already confirmed as having a P0 phenotype and was therefore ranked in the top 10. pan-1 and pan-2 are both already among the top 100 using our wormbase RNAi phenotype ranking system (FIG 1) and additional *H. glycines* sequence information and will also be considered. Pan-1, which was missing from the ESTs, is newly identified in *H. glycines* based on Genome Survey sequencing. act-5, uba-1, and sec61 alpha were not yet among the top several hundred targets using the wormbase RNAi phenotype ranking system because they lacked let, lvl., lva, mlt, bli, and rup phenotype reports in wormbase. Final rank positions of these six targets among the top 300 are Y57G11C.15 (sec61 alpha) = #2, C47E12.5 (uba-1) = #3, and R07E4.6 (kin-2) = #4, C34G6.6 (pan-1) = #7, F52B11.3 (pan-2) = #30, and T25C8.2 (act-5) = #21.

### EXAMPLE 3

### C. elegans intestine-specific RNAi screen

A second additional screen was also performed to aid in the selection of target genes (Table 1). A strain of *C*. *elegans* that is sensitive to RNAi only in the intestine was previously generated. This strain can be used to rapidly screen the set of genes previously shown to be essential to viability and development by RNAi to identify those that are essential specifically in the intestine. Such genes may be advantageous targets with plant-delivered *H. glycines* RNAi as the intestine is believed to be the first tissue to come in contact with the entering dsRNA. Further, genes encoding secreted and transmembrane proteins may be vulnerable to disruption by the expression in the plant of nematocidal proteins that disrupt the function of these gene products.

The *C*. *elegans* intestine-specific RNAi strain was generated by introducing a transgene that drives expression of the wild-type *sid-1* gene under the control of an intestine specific-promoter in an otherwise *sid-1* minus background (Strain HC75). *sid-1* is essential for systemic RNAi in *C*. *elegans* and encodes a transmembrane protein that is a putative dsRNA transporter (Winston, *et al.,* 2002). Three lines, each driving expression from a different intestinal promoter, have been tested for sensitivity to RNAi. The promoters used are 5' regions of *elt-2, ges-1,* and *ile-1.* Feeding dsRNA from *unc-54* (body wall muscle), *dpy-7* (hypodermis), and *act-1* (multiple tissues) showed no phenotype in these strains although all three dsRNAs produced the expected phenotype in wild-type (N2) worm controls. On the other hand, feeding dsRNA from *act-5,* an intestine-specific actin, and *ile-1,* a gene with intestinal localization, in these strains resulted in sterility. These observations support the conclusion that these strains are sensitive to RNAi in the intestine but not in a number of other tissues. The ges-1::sid-1 strain was selected for screening. Phenotypes in this strain are weaker than in N2 even for genes that only have roles in the intestine, probably because ges-1 expression is non-uniform or weaker than endogenous SID-1 intestinal expression such that SID-1 is not always present at the levels it would be in N2. However, phenotypes, such as 50% sterility are easily scored relative to controls and are reproducible. 130 genes have been examined to date in the intestinal-RNAi strain, with a focus on secreted and transmembrane targets and 57 have been observed to have phenotypes (Table 1). Of the 715 gene targets under consideration for the top 300 list, 24 had phenotypes in the intestinal RNAi strain whereas 3 were wild type. In ranking otherwise equally weighted targets preference was given to genes showing intestinal RNAi strain phenotypes and 14 such genes made the top 300 list. The 10 that did not either had weak intestinal RNAi phenotypes (*e.g.* 25% sterile) or other drawbacks (weak homology, non-orthology, etc.).

**Table 1. C. elegans Intestinal RNAi Strain Phenotypes**

| Gene | Intestinal RNAi Phenotype | Gene | Intestinal RNAi Phenotype |
|---|---|---|---|
| act-5 | 80% sterile | C01G8.5 | 40% sterile |
| Ile-1 | 80% sterile | R13H4.4 | 30% sterile |
| C16A3.3 | 20-70% sterile | C25A11.4 | 40% sterile |
| C47E12.5 | 40-99% sterile | T26FE3.3 | 15% sterile |
| C48E7.6 | 20-40% sterile | F54G8.3 | 20% sterile |
| D1014.3 | 30-80% sterile | ZK1058.2 | 50-80% sterile |
| D1069.3 | 15-50% sterile | K07D8.1 | 25% sterile |
| T10H9.3 | 20-40% sterile | H19M22.2 | 45% sterile |
| T24H7.1 | 15-35% sterile | F42C5.10 | 65% sterile |
| ZK973.6 | 20-30% sterile | T03E6.7 | EMB |
| C01F1.2 | 15-40% sterile | R03E1.2 | 75% sterile |
| C54G4.8 | 50-80% Sterile | Y55H10A.1 | LVA, 80% sterile |
| F10D7.5 | 15-40% sterile | C23H3.4 | 80% sterile |
| F11G11.5 | 15-50% sterile | F43D9.3 | 55% sterile |
| F32B5.8 | 20-25% sterile | Y57G11C.15 | 80% sterile |
| F33A8.1 | 60-97% sterile | F49C12.13 | 155% sterile |
| F34D10.2 | 20-30% sterile | T01H3.1 | 60% sterile |
| F39B2.11 | 20-25% sterile | T04A8.9 | 20-80% sterile |
| F54C9.2 | 15-25% sterile | H19M22.2 | 45-55% sterile |
| F55A11.2 | 10-15% sterile | K02B12.3 | 20-50% sterile |
| F55A12.7 | 50-60% sterile | W04A4.5 | 25-50% sterile |
| F55A12.8 | 20% sterile | Y47G6A.23 | 25-50% sterile |
| F55F10.1 | 20-25% sterile | C16D9.2 | 30-35% sterile |
| K07A12.3 | 15-25% sterile | F52C6.3 | 35% sterile |
| K07B1.5 | 25-30% sterile | F53B8.1 | 65% Sterile |
| K07D8.1 | 25% sterile | C49C3.11 | 35% sterile |
| K12D12.2 | 40-70% sterile | F41G3.4 | 35% sterile |
| R04F11.2 | 40-70% sterile | Y57G11C.31 | 25% sterile |
| T01B11.3 | 10-30% sterile | | |

### EXAMPLE 4

### Information on the C. elegans Gene Orthologs - Putative Molecular Function

Besides phenotype, additional information extracted from wormbase included gene name, wormpep protein ID, expression pattern, subcellular localization, prominent motifs, brief identification, concise description, and gene ontology terms. Preference for ranking was given to target genes with known molecular function over those with completely unknown function. We observed that genes with strong phenotypes were in general those with protein products involved in core cellular processes such as DNA replication, cell cycle, transcription, RNA processing, translation including ribosome and tRNA function, protein trafficking, secretion, protein modification, protein stability and degradation, energy production including mitochondrial function, intermediary metabolism, cell structure, signal transduction, channels and transporters, and endocytosis. Of the 300 selected targets, 275 belong to one of these categories (Table 2). Of the top 100, 98 belonged to one of these categories. The most frequently observed categories among targets with strong phenotypes were translation / ribosome / tRNAs, transcription / RNA processing, and Protein stability / degradation / proteosome.

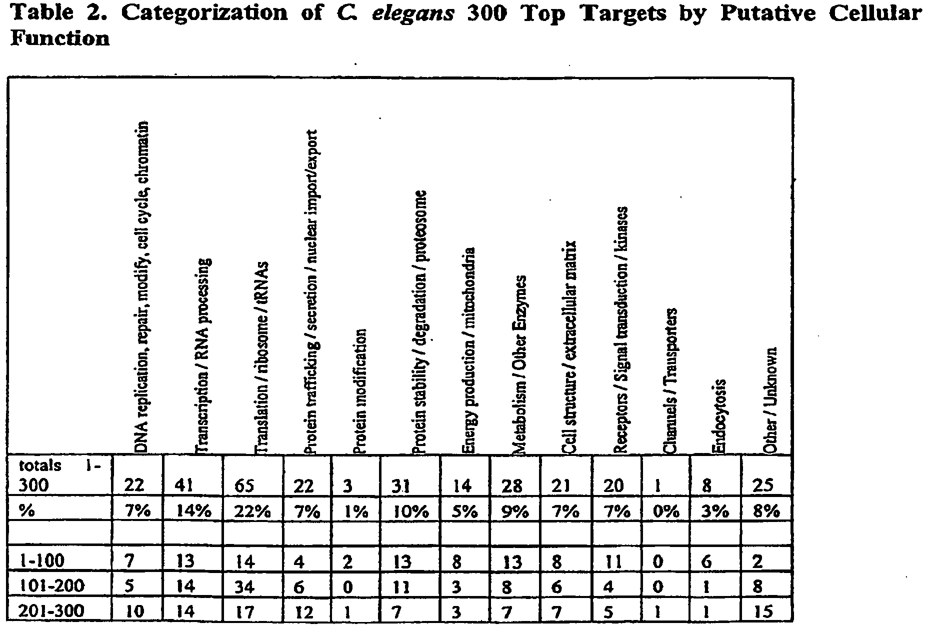

Tracking of putative cellular function insures that suitable diversity is maintained in the top targets list and that the target list does not become too dependent upon one or a few processes which might function differently in *H*. *glycines* than *C*. *elegans.* In conception, low priority is given to developmental processes believed to be rapidly evolving in nematodes such as sex determination since such processes are less likely to be conserved between *H. glycines* and *C*. *elegans.* In practice, few such genes scores well enough in RNAi phenotype to come into consideration for the top 300 list. Interestingly, considering strength of *C*. *elegans* phenotype alone (score from 1-44 and wild type reports) even prior to inclusion of information on sequence conservation, highly ranked targets greatly over represent core cellular processes involving large multi-subunit complexes such as the ribosome and proteosome. This may result simply from the importance of translation and protein degradation to cellular survival. One additional interpretation of this finding is that subunits of large complexes tend to have strong phenotype following RNAi knockdown as they are more dosage sensitive than proteins not acting in complexes. For example, while a monomer enzyme may retain adequate flux through a metabolic pathway with only 10% of its normal protein dose, imbalance between subunit doses in a large complex may result in complex misassembly or other functional failure. Recent findings in *S*. *cerevisiae* support this interpretation (Papp *et al.,* 2003).

### EXAMPLE 5

### Sequence Homology, Identity, and Orthology Assignment

To identify *Heterodera glycines* orthologs of characterized *C*. *elegans* genes, homology searches were performed using the BLAST suite of programs (Altschul *et al.,* 1990). Using predicted protein sequences for *C*. *elegans* genes (Wormpep) TBLASTN was used to search both *H. glycines* clustered ESTs and other sequences available from Genbank as well as recently generated proprietary genome survey sequences. Bitscore, e-value, and % identity were tracked. For consideration as a target, a *C*. *elegans* gene had to have a TBLASTN match to an ortholog or homolog in *H. glycines* with an e-value of at least e-10 or better. 715 *C*. *elegans* genes with RNAi phenotypes ranking from 1-44 (FIG 1) met this minimal criterion. For these 715 genes, *H. glycines* matches were:

| BLAST | Mean±SD | Range |
|---|---|---|
| % ID | 56±18 | 96 - 22 |
| Bitscore | 184±141 | 1031 - 52 |
| E-value | e-48 (median) | 0 - e-11 |

To build the top 300 list (FIG 3), favorable amino acid level sequence similar between *C*, *elegans* and *H. glycines* was the second most important factor in ranking after RNAi phenotype. Targets features of % ID, bitscore, and e-value were divided into quartiles. Top ranking targets in addition to having strong RNAi phenotypes also had favorable homology features indicative of orthology: bitscore (>100), e-value (<e-20), and percentage identity (>40%). To insure orthology assignment, targets were also tested for reciprocal BLAST matching so that when the selected *H. glycines* sequence was checked by BLAST back to the *C*. *elegans* wormpep list (BLASTX), the original starting sequence was identified. 25 genes that would otherwise have made the top 300 failed this reciprocal BLAST test indicating that the genes in *C*. *elegans* and *H. glycines* were unlikely to be orthologs. Of the final 300, 296 appeared to be clear orthologs with reciprocal BLAST top matches in both directions, 1 was tied for top match (2 close homologs in *C*. *elegans*)*,* and 3 were within 5% of the top bitscore (these were kept because of strong phenotypes among all the top *C. elegans* homologs). For the top 300 genes, *H. glycines* matches were:

| BLAST | Mean±SD | Range |
|---|---|---|
| % ID | 65±15 | 96 - 30 |
| Bitscore | 253±158 | 1031 - 71 |
| E-value | e-61 (median) | 0 - e-13 |

Of the top 300 targets, 63 appear to have been identified specifically due to Genome Survey sequencing. Matches in the ESTs or other publicly available sequences are either missing or of substantially weaker scores (mostly paralogs). The rank of these genes among the top 300 is as follows:
23 in top 100: 1, 5, 7, 9, 15, 33, 35, 37, 38, 40, 44, 51, 53, 58, 60, 67, 68, 75, 80, 82, 83, 88, 96
15 in 2nd 100: 102, 115, 134, 142, 148, 151, 152, 156, 163, 164, 191, 193, 199, 200 26 in 3rd 100: 206, 208, 211, 219, 226, 231, 237, 241, 242, 247, 251, 254, 256, 258, 260, 261, 263, 267, 271, 272, 280, 281, 289, 292, 298, 300.

### EXAMPLE 6

### Gene Expression in H. glycines and Other Tylenchids

The expression of target genes in *H. glycines* and other Tylenchid plant parasitic nematodes was also monitored. Even within *H. glycines,* this process was imprecise since the identified transcript from the top 300 list may be the actual gene of interest in the cases where the top hit was an EST (237 out of 300) or a related homolog where the top match was to the genomic sequence (63 out of 300). Likewise, matches in related Tylenchids may be orthologs or homologs. Nevertheless, this information provides a starting point for looking at stage of expression. Expression in J2, J3, and J4 is particularly attractive as double stranded RNA delivery should be possible from the plant at these stages through the feeding site while the cyst is forming. Of genes with EST representation in *H. glycines, 47%* were represented by a single EST. 53% of genes were represented by two or more ESTs. 64% had representation in stages J2, J3, or J4. 165 of the 300 targets had orthologs or homologs among ESTs and other sequencing available from other Tylenchid plant parasitic nematodes including *Heterodera schachtii, Globodera rostochiensis, Globodera pallida, Meloidogyne incognita, Meloidogyne javanica, Meloidogyne arenaria, Meloidogyne hapla, Meloidogyne chitwoodi, Meloidogyne paranaensis, Pratylenchus vulnus, Pratylenchus penetrans, Radopholus similis* (Table 3).

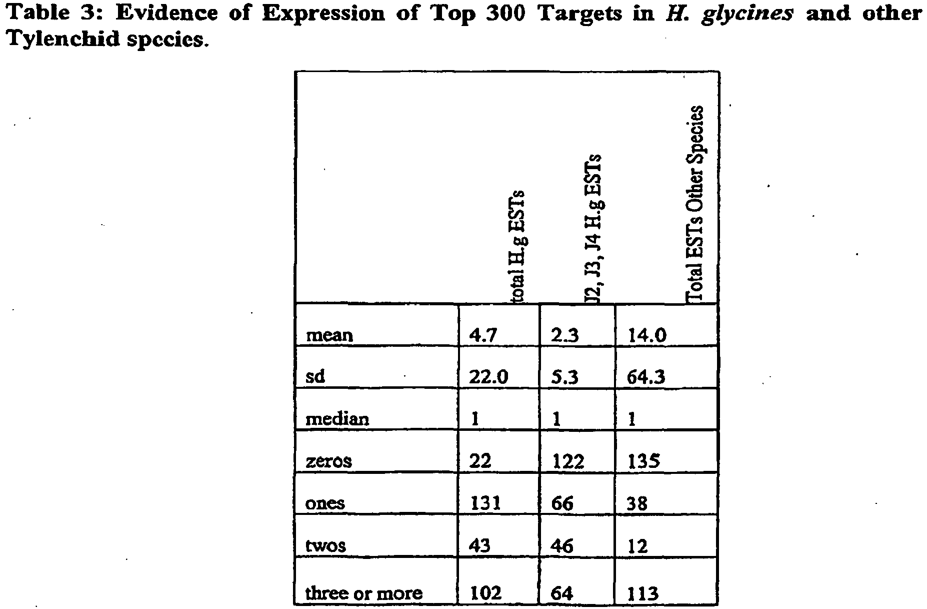

### EXAMPLE 7

### Target list for H. glycines RNAi

Disruption of target gene function within the plant pathogen soybean cyst nematode, *Heterodera glycines,* can be accomplished by RNA interference and can result in disruption of the pathogen's lifecycle. Optimal target genes for disruption include life-cycle essential genes where disruption results in high penetrance death of the parasite populations or "genetic death" by blocking of reproduction with minimal damage to the plant and minimal viable escaping worms reaching the next generation. The inventors have provided a process for selecting RNAi targets and a list of 300 target genes predicted to be essential to *H. glycines* reproduction. Key features used to predict targets include orthology to *C. elegans* known genes with strong and reproducible RNA interference phenotypes. The complete top 300 target list is provided in FIG 3. Table 4 lists selected gene targets for assaying in plant tissues.

**Table 4: Selected Gene Targets for Soybean Hairy Root and in planta Assays**

| **Gene Name** | **Matching Nucleotide Sequence** | **Location on Nucleotide Sequence** | **Peptide Sequence** |
|---|---|---|---|
| Bli-3 | SeqID_1304 | 12-552 | SeqID_1889 |
| Bli-3 | SeqID_1487 | 12-552 | SeqID_1889 |
| C23H3.4 | SeqID_510 | 925-735 | SeqID_1056 |
| C23H3.4 | SeqID_814 | 586-776 | SeqID_1056 |
| C26E6.4 | SeqID_539 | 2836-3029 | SeqID_1051 |
| C34G6.6 | SeqID_511 | 3917-3429 | SeqID_1233 |
| C34G6.6 | SeqID_790 | 244-732 | SeqID_1233 |
| Cct-4 | SeqID_1318 | 193-568 | SeqID_1741 |
| Cct-4 | SeqID_1537 | 193-568 | SeqID_1741 |
| Cgh-1 | SeqID_1289 | 319-795 | SeqID_1722 |
| Cqh-1 | SeqID_1513 | 280-756 | seqID_1722 |
| Cgh-1 | SeqID_1289 | 1587-1761 | SeqID_1722 |
| Crs-1 | SeqID_535 | 1783-1487 | SeqID_1035 |
| Crs-1 | SeqID_792 | 1353-1649 | SeqID_1035 |
| Cyc-1 | SeqID_513 | 1375-1154 | SeqID_1046 |
| Cyc-1 | seqID_804 | 529-750 | SeqID_1046 |
| F01G10.1 | SeqID_509 | 3122-3454 | SeqID_1055 |
| F01G10.1 | SeqID_813 | 690-1022 | SeqID_1055 |
| F52B11.3 | SeqID_508 | 3280-3088 | SeqID_1053 |
| F52B11.3 | SeqID_811 | 1036-1228 | SegID_1053 |
| F54B3.3 | SeqID_524 | 2457-2222 | SeqID_1058 |
| F54B3.3 | SeqID_816 | 266-501 | SeqID_1058 |
| Kin-2 | SeqID_506 | 1310-1023 | SeqID_1032 |
| Kin-2 | SeqID_787 | 79-366 | SeqID_1032 |
| Kin-2 | SeqID_1921 | 15-464 | SeqID_1931 |
| Kin-2 | SeqID_1921 | 465-714 | SeqID_1931 |
| Kin-2 | SegID_1926 | 211-460 | SeqID_1931 |
| Kin-2 | SeqID_787 | 265-514 | SeqID_1032 |
| Kin-2 | SeqID_787 | 514-663 | SeqID_1032 |
| Kin-2 | SeqID_1921 | 340-586 | SeqID_1931 |
| Kin-2 | SeqID_1926 | 86-332 | SeqID_1931 |
| Kin-2 | SeqID_787 | 140-386 | SeqID_1032 |
| Let-767 | SeqID_1306 | 328-687 | SeqID_1735 |
| Let-767 | SeqID_1528 | 295-654 | SeqID_1735 |
| Pas-4 | SeqID_1292 | 564-894 | SeqID_1724 |
| Pas-6 | SeqID_1290 | 314-859 | SeqID_1723 |
| Ran-1 | SeqID_1307 | 550-777 | SeqID_1736 |
| Rpt-1 | SeqID_1310 | 328-581 | SeqID_1737 |
| Rpt-1 | SeqID_1531 | 328-581 | SeqID_1737 |
| Rpt-1 | SeqID_523 | 1356-1609 | SeqID_1047 |
| Rpt-1 | SeqID_805 | 668-921 | SeqID_1047 |
| Sec61 alpha | SeqID_1330 | 93-488 | SeqID_1751 |
| Sec61 alpha | SeqID_1548 | 93-488 | SeqID_1751 |
| Top-1 | SeqID_1298 | 1-449 | SeqID_1729 |
| Top-1 | SeqID_1521 | 1-449 | SeqID_1729 |
| Top-1 | SeqID_1920 | 1569-2017 | SeqID_1930 |
| Top-1 | SeqID_1925 | 1565-2013 | SeqID_1930 |
| Uba-1 | SeqID_1923 | 1977-2184 | SeqID_1933 |
| Uba-1 | SeqID_1928 | 1715-1922 | SeqID_1933 |
| Uba-1 | SeqID_505 | 2500-2708 | SeqID_1031 |
| Uba-1 | SeqID_786 | 1562-1770 | SeqID_1031 |
| Vab-10 | SeqID_542 | 2871-3759 | SeqID_1028 |
| Vab-10 | SeqID_788 | 444-1332 | SeqID_1028 |
| Vha-12 | SeqID_1303 | 44-496 | SeqID_1733 |
| Vha-12 | SeqID_1526 | 1-453 | SeqID_1733 |
| Vha-12 | SeqID_793 | 1-453 | SeqID_1036 |
| Vha-13 | SeqID_1312 | 16-255 | SeqID_1906 |
| Vha-13 | SeqID_1533 | 16-255 | SeqID_1906 |
| Vha-13 | SeqID_514 | 1979-2218 | SeqID_1048 |
| Vha-13 | SeqID_806 | 707-946 | SeqID_1048 |
| Vha-15 | SeqID_519 | 1318-1137 | SeqID_1033 |
| Vha-15 | SeqID_789 | 997-1178 | SeqID_1033 |
| Y48B6A.3 | SeqID_1302 | 1755-2384 | SeqID_1732 |
| Y48B6A.3 | SeqID_1525 | 1697-2326 | SegID_1732 |
| Y55H10A.1 | SeqID_1327 | 524-830 | SeqID_1748 |
| Y55H10A.1 | SeqID_1545 | 524-830 | SeqID_1748 |
| ZK1127.5 | SeqID_538 | 285-535 | SeqID_1050 |
| ZK1127.5 | SeqID_808 | 50-300 | SeqID_1050 |

### EXAMPLE 8

### Selected H. glycines genes showing efficacy in blocking soybean cyst nematode infection of soybean

Results from soaking assays show that orthologs (and fragments of such orthologs) of pas-4 (*e.g.* found at nucleotides 1-544. of SEQ ID NO:1292), pas-5 (*e.g.* found in SEQ ID NOs:525, 569, 797, nucleotides 1-672 of SEQ ID NO:1293, or in SEQ ID NO:1516), pas-6 *(e.g.* found at nucleotides 314-859 of SEQ ID NO:1290), and cgh-1 (*e.g.* found at nucleotides 931-1567 of SEQ ID NO:1289), among others, demonstrate efficacy in blocking soybean cyst nematode (SCN) reproduction. Each of these genes are found among the top 16 entries in FIG 3.

Selected genes or gene fragments were also tested in a soybean hairy root assay (*e.g.* Narayanan *et al.,* 1999), and plant tissues demonstrated resistance to SCN as shown in Table 5:

**Table 5. Hairy root assay results demonstrating SCN resistance activity.**

| Gene Name | Locus | Class I | Class II | Class III | Class IV | Total Exp |
|---|---|---|---|---|---|---|
| Sec61 alpha | Y57G11C.15 | 2 | 1 | 2 | 0 | 7 |
| Uba-1 | C47E12.5 | 8 | 2 | 2 | 0 | 16 |
| Kin-2 | R07E4.6 | 20 | 8 | 3 | 4 | 32 |
| Vab-10 | ZK1151.1 | 1 | 1 | 2 | 0 | 7 |
| Vha-15 | T14F9.1 | 4 | 2 | 1 | 0 | 8 |
| C34G6.6 | C34G6.6 | 1 | 2 | 0 | 1 | 7 |
| Y48B6A.3 | Y48B6A.3 | 0 | 1 | 0 | 0 | 2 |
| Crs-1 | Y23H5A.7 | 1 | 0 | 0 | 0 | 2 |
| Vha-12 | F20B62 | 0 | 1 | 0 | 0 | 4 |
| Pas-4 | C36B1.4 | 2 | 0 | 0 | 0 | 6 |
| Bli-3 | F56C11.1 | 3 | 0 | 0 | 0 | 3 |
| Cgh-1 | C07H6.5 | 1 | 0 | 0 | 1 | 9 |
| Let-767 | C56G2.6 | 3 | 0 | 1 | 0 | 4 |
| Pas-6 | CD4.6 | 2 | 5 | 0 | 1 | 7 |
| Ran-1 | K01G5.4 | 2 | 2 | 0 | 0 | 5 |
| Cyc-1 | C54G4.8 | 1 | 0 | 0 | 0 | 2 |
| Rpt-1 | C52E4.4 | 2 | 0 | 0 | 0 | 6 |
| Vha-13 | Y49A3A.2 | 3 | 2 | 1 | 0 | 8 |
| Top-1 | M01E5.5 | 18 | 8 | 2 | 4 | 26 |
| ZK1127.5 | ZK1127.5 | 0 | 2 | 0 | 0 | 2 |
| C26E6.4 | C26E6.4 | 1 | 1 | 1 | 0 | 3 |
| F52B11.3 | F52B11.3 | 2 | 1 | 1 | 0 | 3 |
| F01G10.1 | F01G10.1 | 6 | 1 | 3 | 1 | 10 |
| C23H3.4 | C23H3.4 | 2 | 1 | 0 | 0 | 5 |
| Cct-4 | K01C8.10 | 3 | 1 | 0 | 0 | 4 |
| F54B3.3 | F54B3.3 | 0 | 1 | 0 | 0 | 2 |
| Y55H10A.1 | Y55H10A.1 | 3 | 1 | 1 | 0 | 6 |

| | | | | | | |
|---|---|---|---|---|---|---|
| Classification of Efficacy for Table 5 Class IV: greater than or equal to 50% reduction in mean cyst number relative to mean cyst number for transgenic empty vector wild type control; Class III: 35 - 49% reduction in mean cyst number relative to mean cyst number for transgenic empty vector wild type control; Class II: 20 - 34% reduction in mean cyst number relative to mean cyst number for transgenic empty vector wild type control Class I: having individual transformation events with greater efficacy than highest event efficacy seen with transgenic empty vector wild type control | | | | | | |

Transgenic soybean plants were prepared expressing fragments of genes encoding, for instance, Pas-4, Pas-5, Cgh-1, or Pas-6, and these plants were tested for SCN resistance. Results are shown in Table 6, demonstrating efficacy in reducing SCN infection and/or reproduction.

**Table 6. Transgenic plant analysis for SCN resistance**

| **Gene Name** | **Locus** | **Promoter** | **Soy line** | **Class I** | **Class II** | **Class III** | **Class IV** | **Total Events tested** |
|---|---|---|---|---|---|---|---|---|
| Pas-4 | C36B1.4 | FMV | Lee 74 | 6 | 3 | 1 | 0 | 12 |
| | | | A3244 | 6 | 2 | 0 | 0 | |
| Pas-5 | F25H2.9 | FMV | Lee 74 | 4 | 5 | 5 | 1 | 18 |
| | | | A3244 | 0 | 1 | 0 | 0 | |
| Pas-5 | F25H2.9 | E35S | Lee 74 | 2 | 4 | 4 | 2 | 16 |
| | | | A3244 | 1 | 1 | 1 | 0 | |
| Cgh-1 | C07H6.5 | FMV | Lee 74 | 4 | 2 | 2 | 2 | 11 |
| | | | A3244 | 0 | 2 | 1 | 1 | |
| Cgh-1 | C07H6.5 | E35S | Lee 74 | 5 | 13 | 2 | 2 | 27 |
| | | | A3244 | 6 | 2 | 0 | 2 | |
| Pas-6 | CD4.6 | FMV | Lee 74 | 5 | 1 | 2 | 1 | 14 |
| | | | A3244 | 1 | 1 | 2 | 0 | |
| Pas-6 | CD4.6 | E35S | Lee 74 | 7 | 5 | 0 | 0 | 20 |
| | | | A3244 | 3 | 1 | 0 | 0 | |

| | | | | | | | | |
|---|---|---|---|---|---|---|---|---|
| Classification of Efficacy for Table 6: Class I: 10-20% reduction in mean cyst number relative to mean cyst number for Lee 74 or A3244; Class II: 20 - 35% reduction in mean cyst number relative to mean cyst number for Lee 74 or A3244; Class III: 36 - 50% reduction in mean cyst number relative to mean cyst number for Lee 74 or A3244; Class IV: >50% reduction in mean cyst number relative to mean cyst number for Lee 74 or A3244 | | | | | | | | |

### References

U.S. Patents 4,536,475; 4,693,977; 4,886,937; 4,940,838; 4,959,317; 5,015,580; 5,107,065; 5,110,732; 5,231,020; 5,283,184; 5,302,523; 5,384,253; 5,464,763; 5,464,765; 5,501,967; 5,508,184; 5,527,695; 5,538,880; 5,459,252; 5,550,318; 5,563,055; 5,591,616; 5,593, 874; 5,633,435; 5,693,512; 5,698,425; 5,712,135; 5,759,829; 5,780,708; 5,789,214; 5,804,693; 5,824,877; 5,837,848; 5,981,840; 6,118,047; 6,160,208; 6,326,193; 6,384,301; 6,399,861; 6,403,865; and 6,506,559.
U.S. Patent Appl. Ser. Nos. 10/465,800; and 11/360,355
U.S. Pubs. 2002/0048814; 2003/0018993; 2003/0061626; 2003/0150017; 2003/0175965; 2004-0029283; 2004/0098761; 2004/0133943; 2005/0188438; 2006/0037101; 2006/0200878. Aboobaker and Blaxter, Mol. Biochem. Parasitol., 129:41-51, 2003.
Altschul et al., J. Mol. Biol., 215:403-410, 1990.
Ashrafi et al., Nature, 421:268-72, 2003.
Ausubel et al., In: Current Protocols in Molecular Biology, John, Wiley & Sons, Inc, New York, 1998.
Barker et al., In: Plant and Soil Nematodes: Societal Impact and Focus for the Future, Comm. Natl. Needs Priorities Nematol., Cooperative State Research Service, US Dept. Arig. Soc. Nematologists, 1994.
Bevan et al., Nature, 304:184-187, 1983.
Böckenhoff and Grundler Parasitology 109: 249-254,1994.
Böckenhoff et al. Plant Physiol 112:1421-7, 1996.
Broothaerts et al., Nature, 433:629-633, 2005.
Brutlag et al., Computers and Chemistry, 17:203-207, 1993.
De Meutter et al. Mol Plant Path 6:321-325, 2005.
Dellaporta et al., In: Chromosome Structure and Function: Impact of New Concepts, 18th Stadler Genetics Symposium, 11:263-282, 1988.
Elbashir et al., Genes Dev., 5(2):188-200, 2001.
EP 0 120 516 and EP 0122 791.
Fairbairn et al., "Plant Delivered RNAi (PD- RNAi): A novel strategy to control plant parasitic nematodes by inactivating nematode genes in planta", (Annual Meeting Abstract #372) American Society for Plant Biology, 2005.
Fire et al., Nature, 391(6669):806-811, 1998.
Fraser et al., Nature, 408:325-330, 2000.
Gheysen and Fenoll, Annu. Rev. Phytopathol. 40:191-219, 2002. Gonczy et al., Nature, 408:331-336, 2000.
Gruber et al., In: Vectors for Plant Transformation, Methods in Plant Molecular Biology and Biotechnology, Glick and Thompson (Eds.), CRC Press, Inc., Boca Raton, 89-119, 1993. Hamilton and Baulcombe, Science, 286:950-952, 1999.
Hannon, Nature, 418:244-251, 2002
Haymes et al., In: Nucleic acid hybridization, a practical approach, IRL Press, Washington, DC, 1985.
Herrera-Estrella et al., Nature, 303:209-213, 1983.
Hirel et al., Plant Molecular Biology, 20:207-218, 1992.
Horsch et al., Science, 227:1229, 1985.
Hoth et al. Plant Physiol 138:383-392, 2005.
Hussein et al., Mol. Biochem. Parasitol., 122:91-94, 2002.
Ikatu et al., Bio/Technol., 8:241-242, 1990.
Jefferson et al., EMBO J., 6:3901-3907, 1987.
Juergensen et al. Plant Physiol 131:61-69, 2003.
Kaeppler et al., Plant Cell Rep., 8:415-418, 1990.
Karnath et al., Genome Biol., 2:R02, 2001.
Kamath et al., Nature, 421:231-237, 2003.
Katz et al., J. Gen Microbiol., 129:2703-2714, 1983.
Klee et al., Bio/Technol., 3:637-642, 1985.
Kwa et al., J. Mol. Biol., 3:246:500-510, 1995.
Lustigman et al., Mol. Biochem. Parasitol., 138:165-70, 2004.
Maeda et al., Curr. Biol., 11: 171-176, 2001.
Mazarei et al. Mol Plant Path 5:409-423, 2004.
Martinez et al., Cell, 110:563-574, 2002.
McCarter et al., Genome Biology, 4:R26,1-19, 2003.
McCarter, Trends in Parasitology, 20:462-468, 2004.
McManus and Sharp, Nat. Rev. Genet., 3:737-47, 2002.
Miki et al., In: Procedures for Introducing Foreign DNA into Plants, Methods in Plant Molecular Biology and Biotechnology, Glick and Thompson (Eds.), CRC Press, Inc., Boca Raton, 67-88, 1993.
Moloney et al., Plant Cell Reports, 8:238, 1989.
Narayanan et al., Crop Sci. 39:1680-1686, 1999.
Odell et al., Nature, 313:810-812, 1985.
Omirulleh et al., Plant Mol. Biol., 21:415-428, 1993.
Opperman et al., Science 263:221-223, 1994.
Ow et al., Science, 234:856-859, 1986.
Padgette et al., Crop Sci., 35:1451-1461, 1995.
Papp et al., Nature, 424:194-197, 2003.
Parkinson et al., Nature Genetics, 36:1259-1267, 2004.
WO 06/073727; WO 05/019408; WO 03/052110; WO 97/32016; WO 99/49029; WO 99/53050; WO94/01550; and WO98/05770.
Piano et al., Curr Biol., 10:1619-1622, 2000.
Piano et al., Curr Biol., 12:1959-64, 2002.
Potrykus et al., Mol. Gen. Genet., 199:183-188, 1985.
Redmond et al., Mol. Biochem. Parasitol., 112:125-31, 2001.
Reynolds et al.. Nat Biotechnol. 22:326-330, 2004.
Sambrook et al., (ed.), Molecular Cloning Cold Spring Harbor Laboratory Press, Cold Spring Harbor, NY, 1989.
Simmer et al., PLoS Biol., 1:E12, 2003.
Sonnichsen et al., Nature, 434:462-469, 2005.
Sutcliffe, Proc. Natl. Acad Sci. USA, 75:3737-3741, 1978.
Thurau et al. Plant Mol Biol 52:643-660, 2003.
Urwin et al., Mol. Plant Microbe. Interact., 15:747-752, 2002.
Vaghchhipawala et al. Genome 47:404-413, 2004.
Van Heeke and Schuster, J. Biol. Chem., 264:5503-5509, 1989.
Winston et al., Science, 295:2456-2459, 2002.
Zukowski et al., Proc. Natl. Acad Sci. USA, 80:1101-1105, 1983.

## Claims

1. A polynucleotide comprising a first, a second and a third polynucleotide sequence, wherein the first polynucleotide sequence is selected from:
(a) a fragment of at least 21 contiguous nucleotides of a nucleic acid sequence of SEQ ID NO:1920, wherein uptake by a plant-parasitic nematode of a double stranded ribonucleotide sequence comprising at least one strand that is complementary to said fragment inhibits the growth of said nematode and
(b) a complement of the sequence of (a), wherein the third polynucleotide sequence is linked to the first polynucleotide sequence by the second polynucleotide sequence, and wherein the third polynucleotide sequence is substantially the reverse complement of the first polynucleotide sequence such that the first and the third polynucleotide sequences hybridize when transcribed into a ribonucleic acid to form a double stranded ribonucleotide molecule stabilized by the linked second ribonucleotide sequence,
wherein the polynucleotide is operably linked to a heterologous promoter.

2. The polynucleotide of claim 1, which is defined as comprised on a plant transformation vector.

3. A double stranded ribonucleotide sequence obtainable by the expression of a polynucleotide according to claim 1, wherein the taking up of said ribonucleotide sequence by a plant-parasitic nematode inhibits the growth of said nematode.

4. The double stranded ribonucleotide sequence of claim 3,
(i) which is obtainable by transcribing a recombinant polynucleotide sequence comprising a first, a second and a third polynucleotide sequence of claim 1, into a ribonucleic acid to form the double stranded ribonucleotide molecule; or
(ii) wherein the taking up of the polynucleotide sequence by the plant-parasitic nematode inhibits the expression of a nucleotide sequence substantially complementary to said polynucleotide sequence.

5. A plant transformation vector comprising the polynucleotide sequence of claim 1.

6. A cell or a plant comprising the polynucleotide of claim 1.

7. The cell of claim 6, defined as
(i) prokaryotic cell; or
(ii) a eukaryotic cell provided that said eukaryotic cell is not a human embryonic stem cell and/or germinal cell; or
(iii) a plant cell.

8. A seed of the plant of claim 6, wherein the seed comprises the polynucleotide of claim 1.

9. The plant of claim 6, wherein said polynucleotide is expressed in the plant cell as a double stranded ribonucleotide sequence, preferably
(i) the plant-parasitic nematode is *Heterodera glycines*; or
(ii) the taking up of the plant-parasitic nematode inhibitory amount of the double stranded ribonucleotide sequence inhibits growth of the nematode.

10. A method for controlling a plant-parasitic nematode population comprising providing an agent comprising a double stranded ribonucleotide sequence that functions upon being taken up by the nematode to inhibit a biological function within said nematode, wherein
(i) the agent comprises a fragment of at least 21 contiguous nucleotides of a nucleotide sequence of SEQ ID NO:1920 or complements thereof; or
(ii) said polynucleotide sequence exhibits from about 95 to about 100% nucleotide sequence identity along at least from about 19 to about 25 contiguous nucleotides to a coding sequence derived from said nematode and is hybridized to a second polynucleotide sequence that is complementary to said first polynucleotide sequence, and wherein said coding sequence derived from said nematode is SEQ ID NO:1920 or complements thereof.

11. The method of claim 10, wherein said nematode is *Heterodera glycines.*

12. A method for controlling a plant-parasitic nematode population comprising providing in the host plant of a plant-parasitic nematode a transformed plant cell expressing a polynucleotide sequence according to claim 1, wherein the polynucleotide is expressed to produce a double stranded ribonucleic acid that functions upon being taken up by the plant-parasitic nematode to inhibit the expression of a target sequence within said nematode and results in decreased growth of the nematode or nematode population, relative to growth on a host lacking the transformed plant cell.

13. The method of claim 12, wherein
(i) the nematode exhibits decreased growth following infection of the host plant; or
(ii) said nematode is selected from the group consisting of *Heterodera sp., Meloidogyne sp., Globodera sp., Helicotylenchus sp., Ditylenchus sp., Pratylenchus sp., Paratylenchus sp., Rotylenchus sp., Tylenchulus sp., Tylenchorhynchus sp., Hoplolaimus sp., Belonolaimus sp., Anguina sp., Subanguina sp.* and *Nacobhus sp.,* preferably the nematode is *Heterodera glycines.*

14. The method of claim 12, wherein the polynucleotide functions upon being taken up by the plant-parasitic nematode to suppress a gene that performs a function essential for nematode survival or growth, said function being DNA replication.

15. A method for reducing the number of Heterodera feeding sites established in the root tissue of a host plant, comprising providing in the host plant of a *Heterodera sp.* a transformed plant cell expressing a polynucleotide sequence according to claim 1, wherein the polynucleotide is expressed to produce a double stranded ribonucleic acid that functions upon being taken up by the *Heterodera sp.* to inhibit the expression of a target sequence within said nematode and results in a decrease in the number of feeding sites established, relative to growth on a host lacking the transformed plant cell.

16. A method of controlling plant nematode pest infestation in a plant comprising providing in the diet of a plant nematode pest a dsRNA comprising:
a) a sense nucleotide sequence; and
b) an antisense nucleotide sequence complementary to said sense nucleotide sequence,
wherein said sense nucleotide sequence comprises the first nucleotide sequence according to claim 1.

17. The method of claim 16, wherein said diet comprises a plant cell transformed to express said sense and said antisense nucleotide sequence.

18. A method for improving the yield of a crop produced from a crop plant subjected to plant-parasitic nematode infection, or for improving the drought tolerance of a crop plant subjected to plant-parasitic nematode infection, said method comprising the steps of,
a) introducing a polynucleotide according to claim 1 into said crop plant;
b) cultivating the crop plant to allow the expression of said polynucleotide; wherein expression of the polynucleotide inhibits plant-parasitic nematode infection or growth and loss of yield due to plant-parasitic nematode infection, or improves the drought tolerance of the crop plant.

19. The method of claim 18, wherein the crop plant is soybean *(Glycine max*).

20. The method of claim 18, wherein the expression of the polynucleotide produces an RNA molecule that suppresses at least a first target gene in a plant-parasitic nematode that has contacted a portion of said crop plant, wherein the target gene performs an essential function in DNA replication.

21. The method of claim 20, wherein the plant-parasitic nematode is a Tylenchid nematode, preferably the plant-parasitic nematode is a *Heterodera sp.,* most preferably the plant-parasitic nematode is *Heterodera glycines.*

22. A method of producing food or feed, comprising obtaining a plant according to claim 6 or a part thereof, and preparing food or feed from the plant or part thereof.

23. The method of claim 22, wherein the food or feed is defined as oil, meal, protein, starch, flour or silage.

24. A method for modulating the expression of a target gene in a plant-parasitic nematode cell, the method comprising:
(a) transforming a plant cell with a vector comprising a nucleic acid sequence encoding a dsRNA of SEQ ID NO:1920, operatively linked to a promoter and a transcription termination sequence, wherein said dsRNA inhibits the function of the polypeptide having the sequence of SEQ ID NO:1920;
(b) culturing the transformed plant cell under conditions sufficient to allow for development of a plant cell culture comprising a plurality of transformed plant cells;
(c) selecting for transformed plant cells that have integrated the nucleic acid sequence into their genomes;
(d) screening the transformed plant cells for expression of the dsRNA encoded by the nucleic acid sequence; and
(e) selecting a plant cell that expresses the dsRNA.

25. The method of claim 24, further comprising regenerating a plant from the plant cell that expresses the dsRNA; whereby expression of the gene in the plant is sufficient to modulate the expression of a target gene in a plant-parasitic nematode cell that contacts the transformed plant or plant cell.

## Patentansprüche

1. Polynucleotid, umfassend eine erste, eine zweite und eine dritte Polynucleotidsequenz, wobei die erste Polynucleotidsequenz ausgewählt ist aus:
(a) einem Fragment von wenigstens 21 aufeinanderfolgenden Nucleotiden einer Nucleinsäuresequenz von SEQ ID Nr. 1920, wobei die Aufnahme einer doppelsträngigen Ribonucleotidsequenz, die wenigstens einen Strang umfasst, der zu dem Fragment komplementär ist, durch einen pflanzenparasitischen Nematoden das Wachstum des Nematoden hemmt; und
(b) einem Komplement der Sequenz von (a), wobei die dritte Polynucleotidsequenz durch die zweite Polynucleotidsequenz mit der ersten Polynucleotidsequenz verknüpft ist und wobei die dritte Polynucleotidsequenz im Wesentlichen das reverse Komplement der ersten Polynucleotidsequenz ist, so dass die erste und die dritte Polynucleotidsequenz, wenn sie zu einer Ribonucleinsäure transcribiert sind, unter Bildung eines doppelsträngigen Ribonucleotidmoleküls, das durch die verknüpfte zweite Ribonucleotidsequenz stabilisiert ist, hybridisieren,
wobei das Polynucleotid funktionell mit einem heterologen Promotor verknüpft ist.

2. Polynucleotid gemäß Anspruch 1, das dadurch definiert ist, dass es auf einem Pflanzentransformationsvektor vorhanden ist.

3. Doppelsträngige Ribonucleotidsequenz, die durch die Expression eines Polynucleotids gemäß Anspruch 1 erhältlich ist, wobei die Aufnahme der Ribonucleotidsequenz durch einen pflanzenparasitischen Nematoden das Wachstum des Nematoden hemmt.

4. Doppelsträngige Ribonucleotidsequenz gemäß Anspruch 3,
(i) die durch Transcribieren einer rekombinanten Polynucleotidsequenz, welche eine erste, eine zweite und eine dritte Polynucleotidsequenz gemäß Anspruch 1 umfasst, zu einer Ribonucleinsäure unter Bildung des doppelsträngigen Ribonucleotidmoleküls erhältlich ist; oder
(ii) wobei die Aufnahme der Polynucleotidsequenz durch den pflanzenparasitischen Nematoden die Expression einer Nucleotidsequenz, die zu der Polynucleotidsequenz im Wesentlichen komplementär ist, hemmt.

5. Pflanzentransformationsvektor, der die Polynucleotidsequenz gemäß Anspruch 1 umfasst.

6. Zelle oder Pflanze, die das Polynucleotid gemäß Anspruch 1 umfasst.

7. Zelle gemäß Anspruch 6, die definiert ist als
(i) prokaryontische Zelle; oder
(ii) eukaryontische Zelle, mit der Maßgabe, dass die eukaryontische Zelle keine humane embryonale Stammzelle und/oder Keimzelle ist; oder
(iii) eine Pflanzenzelle.

8. Samen der Pflanze gemäß Anspruch 6, wobei der Samen das Polynucleotid gemäß Anspruch 1 umfasst.

9. Pflanze gemäß Anspruch 6, wobei das Polynucleotid in der Pflanzenzelle als doppelsträngige Ribonucleotidsequenz exprimiert wird, wobei vorzugsweise
(i) es sich bei dem pflanzenparasitischen Nematoden um *Heterodera glycines* handelt; oder
(ii) die Aufnahme der den pflanzenparasitischen Nematoden hemmenden Menge der doppelsträngigen Ribonucleotidsequenz das Wachstum des Nematoden hemmt.

10. Verfahren zur Bekämpfung einer pflanzenparasitischen Nematodenpopulation, umfassend das Bereitstellen eines Mittels, das eine doppelsträngige Ribonucleotidsequenz umfasst, die bei Aufnahme durch den Nematoden die Funktion hat, eine biologische Funktion innerhalb des Nematoden zu hemmen, wobei
(i) das Mittel ein Fragment von wenigstens 21 aufeinanderfolgenden Nucleotiden einer Nucleotidsequenz von SEQ ID Nr. 1920 oder Komplementen davon umfasst; oder
(ii) die Polynucleotidsequenz etwa 95 bis etwa 100% Nucleotidsequenzidentität entlang wenigstens etwa 19 bis etwa 25 aufeinanderfolgenden Nucleotiden mit einer codierenden Sequenz aufweist, die von dem Nematoden abgeleitet ist und mit einer zweiten Polynucleotidsequenz, die zu der ersten Polynucleotidsequenz komplementär ist, hybridisiert wird, und wobei es sich bei der von dem Nematoden abgeleiteten codierenden Sequenz um SEQ ID Nr. 1920 oder Komplemente davon handelt.

11. Verfahren gemäß Anspruch 10, wobei es sich bei dem Nematoden um *Heterodera glycines* handelt.

12. Verfahren zur Bekämpfung einer pflanzenparasitischen Nematodenpopulation, umfassend das Bereitstellen einer transformierten Pflanzenzelle, die eine Polynucleotidsequenz gemäß Anspruch 1 exprimiert, in der Wirtspflanze eines pflanzenparasitischen Nematoden, wobei das Polynucleotid so exprimiert wird, dass eine doppelsträngige Ribonucleinsäure entsteht, die bei Aufnahme durch den pflanzenparasitischen Nematoden die Funktion hat, die Expression einer Zielsequenz innerhalb des Nematoden zu hemmen, und zu einem verminderten Wachstum des Nematoden oder der Nematodenpopulation relativ zum Wachstum auf einem Wirt, dem die transformierte Pflanzenzelle fehlt, führt.

13. Verfahren gemäß Anspruch 12, wobei
(i) der Nematode nach Infektion der Wirtspflanze ein vermindertes Wachstum aufweist; oder
(ii) der Nematode aus der Gruppe ausgewählt ist, die aus *Heterodera* sp., *Meloidogyne* sp., *Globodera* sp., *Helicotylenchus* sp., *Ditylenchus* sp., *Pratylenchus* sp., *Paratylenchus* sp., *Rotylenchus* sp., *Tylenchulus* sp., *Tylenchorhynchus* sp., *Hoplolaimus* sp., *Belonolaimus* sp., *Anguina* sp., *Subanguina* sp. und *Nacobhus* sp. besteht, wobei es sich bei dem Nematoden vorzugsweise um *Heterodera glycines* handelt.

14. Verfahren gemäß Anspruch 12, wobei das Polynucleotid bei Aufnahme durch den pflanzenparasitischen Nematoden die Funktion hat, ein Gen zu unterdrücken, das eine Funktion erfüllt, die für das Überleben oder Wachstum des Nematoden essentiell ist, wobei es sich bei der Funktion um die DNA-Replikation handelt.

15. Verfahren zum Reduzieren der Anzahl von Heterodera-Fressstellen, die sich im Wurzelgewebe einer Wirtspflanze etabliert haben, umfassend das Bereitstellen einer transformierten Pflanzenzelle, die eine Polynucleotidsequenz gemäß Anspruch 1 exprimiert, in der Wirtspflanze einer *Heterodera* sp., wobei das Polynucleotid so exprimiert wird, dass eine doppelsträngige Ribonucleinsäure entsteht, die bei Aufnahme durch die *Heterodera* sp. die Funktion hat, die Expression einer Zielsequenz innerhalb des Nematoden zu hemmen, und zu einer Abnahme der Anzahl der etablierten Fressstellen relativ zum Wachstum auf einem Wirt, dem die transformierte Pflanzenzelle fehlt, führt.

16. Verfahren zur Bekämpfung eines Schädlingsbefalls durch einen Pflanzennematoden bei einer Pflanze, umfassend das Bereitstellen einer dsRNA, die Folgendes umfasst, in der Nahrung eines Nematoden-Pflanzenschädlings:
a) eine Sense-Nucleotidsequenz; und
b) eine Antisense-Nucleotidsequenz, die zu der Sense-Nucleotidsequenz komplementär ist;
wobei die Sense-Nucleotidsequenz die erste Nucleotidsequenz gemäß Anspruch 1 umfasst.

17. Verfahren gemäß Anspruch 16, wobei die Nahrung eine Pflanzenzelle umfasst, die so transformiert ist, dass sie die Sense- und die Antisense-Nucleotidsequenz exprimiert.

18. Verfahren zur Verbesserung des Ertrags einer aus einer Kulturpflanze, die einer Infektion mit einem pflanzenparasitischen Nematoden unterliegt, erzeugten Feldfrucht oder zur Verbesserung der Dürretoleranz einer Kulturpflanze, die einer Infektion mit einem pflanzenparasitischen Nematoden unterliegt, wobei das Verfahren die Schritte umfasst:
a) Einführen eines Polynucleotids gemäß Anspruch 1 in die Kulturpflanze;
b) Anbauen der Kulturpflanze, um die Expression des Polynucleotids zu ermöglichen; wobei die Expression des Polynucleotids die Infektion mit oder das Wachstum von dem pflanzenparasitischen Nematoden hemmt und den Ertragsverlust aufgrund der Infektion mit dem pflanzenparasitischen Nematoden reduziert oder die Dürretoleranz der Kulturpflanze verbessert.

19. Verfahren gemäß Anspruch 18, wobei es sich bei der Kulturpflanze um die Sojabohne (*Glycine max*) handelt.

20. Verfahren gemäß Anspruch 18, wobei die Expression des Polynucleotids ein RNA-Molekül erzeugt, das wenigstens ein erstes Target-Gen in einem pflanzenparasitischen Nematoden, der mit einem Teil der Kulturpflanze in Kontakt gekommen ist, unterdrückt, wobei das Target-Gen eine essentielle Funktion bei der DNA-Replikation erfüllt.

21. Verfahren gemäß Anspruch 20, wobei es sich bei dem pflanzenparasitischen Nematoden um einen Nematoden der Ordnung Tylenchida handelt, wobei es sich bei dem pflanzenparasitischen Nematoden vorzugsweise um eine *Heterodera* sp. handelt, wobei es sich bei dem pflanzenparasitischen Nematoden am meisten bevorzugt um *Heterodera glycines* handelt.

22. Verfahren zur Produktion eines Nahrungs- oder Futtermittels, umfassend das Beschaffen einer Pflanze gemäß Anspruch 6 oder eines Teils davon und das Herstellen eines Nahrungs- oder Futtermittels aus der Pflanze oder ihrem Teil.

23. Verfahren gemäß Anspruch 22, wobei das Nahrungs- oder Futtermittel als Öl, Schrot, Protein, Stärke, Mehl oder Silage definiert ist.

24. Verfahren zum Modulieren der Expression eines Target-Gens in einer Zelle eines pflanzenparasitischen Nematoden, wobei das Verfahren umfasst:
a) Transformieren einer Pflanzenzelle mit einem Vektor, der eine Nucleinsäuresequenz umfasst, die eine dsRNA der SEQ ID Nr. 1920 codiert und funktionell mit einem Promotor und einer Transcriptionsterminationssequenz verknüpft ist, wobei die dsRNA die Funktion des Polypeptids mit der Sequenz von SEQ ID Nr. 1920 hemmt;
b) Kultivieren der transformierten Pflanzenzelle unter ausreichenden Bedingungen, um die Entwicklung einer Pflanzenzellenkultur, die eine Vielzahl von transformierten Pflanzenzellen umfasst, zu ermöglichen;
c) Selektieren von transformierten Pflanzenzellen, die die Nucleinsäuresequenz in ihr Genom integriert haben;
d) Screening der transformierten Pflanzenzellen nach einer Expression der dsRNA, die von der Nucleinsäuresequenz codiert wird; und
e) Auswählen einer Pflanzenzelle, die die dsRNA exprimiert.

25. Verfahren gemäß Anspruch 24, weiterhin umfassend das Regenerieren einer Pflanze aus der Pflanzenzelle, die die dsRNA exprimiert; wobei die Expression des Gens in der Pflanze ausreichend ist, um die Expression eines Target-Gens in einer Zelle eines pflanzenparasitischen Nematoden, der mit der Pflanze oder Pflanzenzelle in Kontakt gekommen ist, zu modulieren.

## Revendications

1. Polynucléotide comprenant une première, une deuxième et une troisième séquence polynucléotidique, dans lequel la première séquence polynucléotidique est choisie parmi :
(a) un fragment d'au moins 21 nucléotides contigus d'une séquence d'acide nucléique de SEQ ID NO:1920, où l'absorption par un nématode parasite des plantes d'une séquence ribonucléotidique double brin comprenant au moins un brin qui est complémentaire audit fragment inhibe la croissance dudit nématode et
(b) un complément de la séquence de (a), où la troisième séquence polynucléotidique est liée à la première séquence polynucléotidique par la deuxième séquence polynucléotidique, et où la troisième séquence polynucléotidique est essentiellement le complément inverse de la première séquence polynucléotidique de sorte que les première et troisième séquences polynucléotidiques s'hybrident lorsqu'elles sont transcrites dans un acide ribonucléique pour former une molécule ribonucléotidique double brin stabilisée par la deuxième séquence ribonucléotidique liée,
où le polynucléotide est lié de manière fonctionnelle à un promoteur hétérologue.

2. Polynucléotide de la revendication 1, qui est défini comme étant composé d'un vecteur de transformation de plantes.

3. Séquence ribonucléotidique double brin pouvant être obtenue par l'expression d'un polynucléotide selon la revendication 1, dans laquelle l'absorption de ladite séquence ribonucléotidique par un nématode parasite des plantes inhibe la croissance dudit nématode.

4. Séquence ribonucléotidique double brin de la revendication 3,
(i) qui peut être obtenue par transcription d'une séquence polynucléotidique recombinante comprenant une première, une deuxième et une troisième séquence polynucléotidique de la revendication 1, dans un acide ribonucléique pour former la molécule ribonucléotidique double brin ; ou
(ii) dans laquelle l'absorption de la séquence polynucléotidique par le nématode parasite des plantes inhibe l'expression d'une séquence nucléotidique essentiellement complémentaire à ladite séquence polynucléotidique.

5. Vecteur de transformation de plantes comprenant la séquence polynucléotidique de la revendication 1.

6. Cellule ou plante comprenant le polynucléotide de la revendication 1.

7. Cellule de la revendication 6, définie comme étant
(i) une cellule procaryote ; ou
(ii) une cellule eucaryote à condition que ladite cellule eucaryote ne soit pas une cellule souche embryonnaire humaine et/ou une cellule germinale ; ou
(iii) une cellule végétale.

8. Graine de la plante de la revendication 6, dans laquelle la graine comprend le polynucléotide de la revendication 1.

9. Plante de la revendication 6, dans laquelle ledit polynucléotide est exprimé dans la cellule végétale sous forme d'une séquence ribonucléotidique double brin, de préférence
(i) le nématode parasite des plantes est Heterodera glycines ; ou
(ii) l'absorption de la quantité d'inhibition du nématode parasite des plantes de la séquence ribonucléotidique double brin inhibe la croissance du nématode.

10. Procédé permettant de lutter contre une population de nématodes parasites de plantes comprenant le fait de fournir un agent comprenant une séquence ribonucléotidique double brin qui fonctionne, lorsqu'il est absorbé par le nématode pour inhiber une fonction biologique dans ledit nématode, où
(i) l'agent comprend un fragment d'au moins 21 nucléotides contigus d'une séquence nucléotidique de SEQ ID NO:1920 ou de compléments de celle-ci ; ou
(ii) ladite séquence polynucléotidique présente d'environ 95 à environ 100% d'identité de séquence nucléotidique le long d'au moins environ 19 à environ 25 nucléotides contigus à une séquence codante dérivée dudit nématode et est hybridée à une deuxième séquence polynucléotidique qui est complémentaire à ladite première séquence polynucléotidique, et où ladite séquence codante dérivée dudit nématode est SEQ ID NO:1920 ou des compléments de celle-ci.

11. Procédé de la revendication 10, dans lequel ledit nématode est Heterodera glycines.

12. Procédé permettant de lutter contre une population de nématodes parasites des plantes comprenant le fait de fournir dans la plante hôte d'un nématode parasite des plantes une cellule végétale transformée exprimant une séquence polynucléotidique selon la revendication 1, où le polynucléotide est exprimé pour produire un acide ribonucléique double brin qui fonctionne lorsqu'il est absorbé par le nématode parasite des plantes pour inhiber l'expression d'une séquence cible dans ledit nématode et aboutit à une croissance réduite du nématode ou de la population de nématodes, par rapport à la croissance sur un hôte dépourvu de la cellule végétale transformée.

13. Procédé de la revendication 12, dans lequel
(i) le nématode présente une croissance réduite suite à une infection de la plante hôte ; ou
(ii) ledit nématode est choisi dans le groupe constitué de Heterodera sp., Meloidogyne sp., Globodera sp., Helicotylenchus sp., Ditylenchus sp., Pratylenchus sp., Paratylenchus sp., Rotylenchus sp., Tylenchulus sp., Tylenchorhynchus sp., Hoplolaimus sp., Belonolaimus sp., Anguina sp., Subanguina sp. et de Nacobhus sp., de préférence le nématode est Heterodera glycines.

14. Procédé de la revendication 12, dans lequel le polynucléotide fonctionne lorsqu'il est absorbé par le nématode parasite des plantes pour supprimer un gène qui remplit une fonction essentielle pour la survie ou la croissance du nématode, ladite fonction étant la réplication de l'ADN.

15. Procédé permettant de réduire le nombre de sites d'alimentation d'Heterodera établis dans le tissu des racines d'une plante hôte, comprenant le fait de fournir dans la plante hôte d'Heterodera sp. une cellule végétale transformée exprimant une séquence polynucléotidique selon la revendication 1, où le polynucléotide est exprimé pour produire un acide ribonucléique double brin qui fonctionne lorsqu'il est absorbé par l'Heterodera sp. pour inhiber l'expression d'une séquence cible dans ledit nématode et entraîne une diminution du nombre de sites d'alimentation établis, par rapport à la croissance sur un hôte dépourvu de la cellule végétale transformée.

16. Procédé permettant de lutter contre l'infestation par les parasites nématodes de plantes dans une plante, comprenant le fait de fournir dans la nourriture d'un parasite nématode de plantes un ARN à double brin comprenant :
a) une séquence nucléotidique sens ; et
b) une séquence nucléotidique antisens complémentaire à ladite séquence nucléotidique sens,
où ladite séquence nucléotidique sens comprend la première séquence nucléotidique selon la revendication 1.

17. Procédé de la revendication 16, dans lequel ladite nourriture comprend une cellule végétale transformée pour exprimer ladite séquence nucléotidique sens et ladite séquence nucléotidique antisens.

18. Procédé permettant d'améliorer le rendement d'une culture produite à partir d'une plante cultivée sujette à une infection par des nématodes parasites des plantes, ou d'améliorer la tolérance à la sécheresse d'une plante cultivée sujette à une infection par des nématodes parasites des plantes, ledit procédé comprenant les étapes qui consistent,
a) à introduire un polynucléotide selon la revendication 1 dans ladite plante cultivée ;
b) à cultiver la plante cultivée pour permettre l'expression dudit polynucléotide ; où l'expression du polynucléotide inhibe l'infection par les nématodes parasites des plantes ou la croissance de ces derniers et les pertes de rendement dues à l'infection par les nématodes parasites des plantes, ou améliore la tolérance à la sécheresse de la plante cultivée.

19. Procédé de la revendication 18, dans lequel la plante cultivée est le soja (Glycine max).

20. Procédé de la revendication 18, dans lequel l'expression du polynucléotide produit une molécule d'ARN qui supprime au moins un premier gène cible dans un nématode parasite des plantes qui a été en contact avec une partie de ladite plante cultivée, où le gène cible remplit une fonction essentielle dans la réplication de l'ADN.

21. Procédé de la revendication 20, dans lequel le nématode parasite des plantes est un nématode de l'ordre de Tylenchida, de préférence le nématode parasite des plantes est Heterodera sp., plus préférablement, le nématode parasite des plantes est Heterodera glycines.

22. Procédé de production d'un aliment humain ou animal, comprenant l'obtention d'une plante selon la revendication 6 ou d'une partie de celle-ci, et la préparation de l'aliment humain ou animal à partir de la plante ou de la partie de celle-ci.

23. Procédé de la revendication 22, dans lequel l'aliment humain ou animal est défini comme étant une huile, une farine de graine, une protéine, un amidon, une farine ou un ensilage.

24. Procédé permettant de moduler l'expression d'un gène cible dans une cellule de nématode parasite des plantes, le procédé comprenant le fait :
(a) de transformer une cellule végétale avec un vecteur comprenant une séquence d'acide nucléique codant pour un ARN à double brin de SEQ ID NO:1920, liée de manière fonctionnelle à un promoteur et à une séquence de terminaison de transcription, où ledit ARN à double brin inhibe la fonction du polypeptide ayant la séquence de SEQ ID NO:1920 ;
(b) de cultiver la cellule végétale transformée dans des conditions suffisantes pour permettre le développement d'une culture de cellules végétales comprenant une pluralité de cellules végétales transformées ;
(c) de sélectionner des cellules végétales transformées qui ont intégré la séquence d'acide nucléique dans leurs génomes ;
(d) de cribler les cellules végétales transformées pour l'expression de l'ARN à double brin codé par la séquence d'acide nucléique ; et
(e) de sélectionner une cellule végétale qui exprime l'ARN à double brin.

25. Procédé de la revendication 24, comprenant en outre le fait de régénérer une plante à partir de la cellule végétale qui exprime l'ARN à double brin ; moyennant quoi l'expression du gène dans la plante est suffisante pour moduler l'expression d'un gène cible dans une cellule de nématode parasite des plantes qui entre en contact avec la plante ou la cellule végétale transformée.
